(19) Europäisches Patentamt
European Patent Office
Office européen des brevets

(11) EP 1 541 571 A1

(12) EUROPEAN PATENT APPLICATION
published in accordance with Art. 158(3) EPC

(43) Date of publication:
**15.06.2005 Bulletin 2005/24**

(21) Application number: **03765317.7**

(22) Date of filing: **17.07.2003**

(51) Int Cl.[7]: **C07D 471/04**, A61K 31/444, A61P 1/00, A61P 1/16, A61P 3/10, A61P 9/00, A61P 9/10, A61P 11/06, A61P 13/12, A61P 17/06, A61P 19/02, A61P 19/10, A61P 25/04, A61P 25/28, A61P 29/00, A61P 31/12, A61P 35/00, A61P 37/02, A61P 37/08, A61P 43/00

(86) International application number:
**PCT/JP2003/009110**

(87) International publication number:
**WO 2004/009592 (29.01.2004 Gazette 2004/05)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **19.07.2002 JP 2002211836**

(71) Applicant: **Sankyo Company, Limited**
**Tokyo 103-8426 (JP)**

(72) Inventors:
- **KIMURA, Tomio**
  **Shinagawa-ku, Tokyo 140-8710 (JP)**
- **OHKAWA, Nobuyuki**
  **Shinagawa-ku, Tokyo 140-8710 (JP)**
- **AOKI, Kazumasa**
  **Shinagawa-ku, Tokyo 140-8710 (JP)**
- **NAKAO, Akira**
  **Shinagawa-ku, Tokyo 140-8710 (JP)**
- **NAGASAKI, Takayoshi**
  **Shinagawa-ku, Tokyo 140-8710 (JP)**

(74) Representative:
**Gibson, Christian John Robert et al**
**Marks & Clerk**
**90 Long Acre**
**London WC2E 9RA (GB)**

# (54) BICYCLIC UNSATURATED TERTIARY AMINE COMPOUND

(57) The present invention provides a bicyclic unsaturated tertiary amine compound capable of inhibiting the production of inflammatory cytokines.

A compound of the following formula (1):

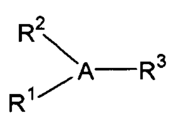

(I)

(wherein,
A represents pyrrole or pyrazole, $R^1$ represents an aryl group or a heteroaryl group which may be substituted, $R^2$ represents a heteroaryl group which may be substituted, and $R^3$ represents an indolizine group), or a pharmacologically acceptable salt thereof, a pharmacologically acceptable ester thereof or other pharmacologically acceptable derivative thereof.

EP 1 541 571 A1

## Description

[Technical Field]

The present invention relates to bicyclic unsaturated tertiary amine compounds that are useful as pharmaceuticals. More particularly, the present invention relates to bicyclic unsaturated tertiary amine compounds that have an action that inhibits the production of inflammatory cytokines such as interleukin (IL)-1, IL-6, IL-8 and tumor necrotizing factor (TNF), and are useful as antipyretics, analgesics, anti-inflammatory drugs as well as therapeutic drugs for autoimmune diseases such as articular rheumatism, osteoarthritis, septicaemia, diabetes (particularly type I diabetes), bone diseases such as osteoporosis, and other diseases in which the aforementioned cytokines are involved.

[Background Art]

In the past, non-steroid anti-inflammatory drugs (NSAIDs) were frequently used for various types of inflammatory and pain-associated diseases as a result of having a pharmacological action consisting primarily of antipyretic, analgesic and anti-inflammatory action based on a mechanism of inhibiting the biosynthesis of prostaglandins (PGs) by inhibiting cyclooxygenase. NSAIDs have been used for the purpose of nosotropic treatment while disease-modifying anti-rheumatic drugs (DMARDs) have been used for the purpose of etiotropic therapy against diseases such as articular rheumatism.

Conventional NSAIDs can induce undesirable side effects including gastrointestinal disorders such as gastric ulcers, resulting in difficulties for any patient who has to take such a drug for an extended period of time. On the other hand, DMARDs have not yet shown stable and clear pharmacological effects. In recent years, active substances, generally referred to as cytokines, have been discovered, that are produced by immunocompetent cells. Among these, interleukin (IL)-1, IL-6, IL-8 and tumor necrotizing factor (TNF) are referred to as inflammatory cytokines, and have been determined to have a diverse range of functions as inflammation mediators, examples of which include activation of the arachidonic acid metabolism system, the system by which PGs are produced, migration of leukocytes, induction of acute phase proteins, and activation of osteoclasts. Inhibitors of the production of these inflammatory cytokines are expected to serve as next-generation antipyretics, analgesics, anti-inflammatory drugs as well as therapeutic agents against autoimmune diseases such as articular rheumatism, bone diseases such as osteoporosis, and other diseases in which the aforementioned cytokines are involved, by a mechanism that differs from the mechanism employed by conventional drugs.

Although known examples of these compounds that have the action of inhibiting the production of inflammatory cytokines include the heteroaryl compounds described in the publications of WO96/21452, WO97/5877, WO97/23479, WO98/52937, WO00/31063, EP1070711 and J. Med. Chem., Vol. 39, 3929-3937 (1996), there is still a need to develop compounds that are even more superior in terms of pharmacological effect, pharmacokinetics and safety.

[Disclosure of the Invention]

The present inventors earnestly investigated the synthesis and the pharmacological action of compounds capable of inhibiting production of inflammatory cytokines over a long period of time. As a result, they have found that the bicyclic unsaturated tertiary amine compounds have excellent action in inhibiting inflammatory cytokine production, and have thereby accomplished the present invention.

The present invention relates to (1) a compound of the following formula (1):

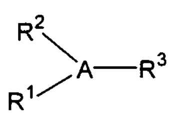

(I)

{wherein
A represents a trivalent group selected from pyrrole and pyrazole,
$R^1$ represents an aryl group; an aryl group substituted by group(s) selected from substituent group α and substituent group β; a heteroaryl group; or a heteroaryl group substituted by group(s) selected from substituent group α and substituent group β,
$R^2$ represents a heteroaryl group having at least one nitrogen atom; or a heteroaryl group having at least one nitrogen atom substituted by group(s) selected from substituent group α and substituent group β,
$R^3$ represents a group of the following formula (II):

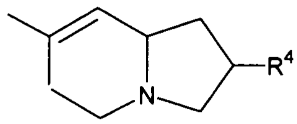
(II)

[wherein
$R^4$ represents a hydrogen atom, a hydroxyl group, a lower alkyl group, a lower alkoxy group, a group of formula: -$NR^{\alpha}R^{\beta}$ (wherein $R^{\alpha}$ and $R^{\beta}$ are the same or different and each represents a hydrogen atom, a lower alkylsulfonyl group or a lower aliphatic acyl group), an aryl group, an aryl group substituted by group(s) selected from substituent group α and substituent group β, an aryl group substituted by an aryl group or an aryl group substituted by an aryloxy group,
substituent group α represents the group consisting of a hydroxyl group, a nitro group, a cyano group, a halogen atom, a lower alkoxy group, a halogeno lower alkoxy group, a lower acyloxy group, a lower alkylthio group, a halogeno lower alkylthio group and a group of formula: -$NR^aR^b$ (wherein $R^a$ and $R^b$ are the same or different and each represents a hydrogen atom, a lower alkyl group, a lower alkenyl group, a lower alkynyl group, an aralkyl group, a lower alkylsulfonyl group or a lower aliphatic acyl group, or $R^a$ and $R^b$, together with the nitrogen atom to which $R^a$ and $R^b$ are bonded, form a heterocyclyl group),
substituent group β represents the group consisting of a lower alkyl group, a lower alkenyl group, a lower alkynyl group, an aralkyl group, a cycloalkyl group, a lower alkyl group substituted by group(s) selected from substituent group α, a lower alkenyl group substituted by group(s) selected from substituent group α and an alkynyl group substituted by group(s) selected from substituent group α],
provided that the atoms on ring A to which $R^1$ and $R^3$ are bonded are each adjacent to the atom on ring A to which $R^2$ is bonded}
or a pharmacologically acceptable salt thereof, a pharmacologically acceptable ester thereof or other pharmacologically acceptable derivative thereof.

In the above compound (I), the compound is preferably:

(2) the compound of (1), wherein $R^1$ is an aryl group; or an aryl group substituted by group(s) selected from substituent group α and substituent group β, or a pharmacologically acceptable salt thereof, a pharmacologically acceptable ester thereof or other pharmacologically acceptable derivative thereof,
(3) the compound of (1), wherein $R^1$ is phenyl, naphthyl, or phenyl or naphthyl substituted by group(s) selected from substituent group α and substituent group β, or a pharmacologically acceptable salt thereof, a pharmacologically acceptable ester thereof or other pharmacologically acceptable derivative thereof,
(4) the compound of (1), wherein $R^1$ is phenyl or phenyl substituted by group(s) selected from substituent group $\alpha^1$ and substituent group $\beta^1$, substituent group $\alpha^1$ represents the group consisting of a cyano group, a halogen atom, a lower alkoxy group, a halogeno lower alkoxy group and a group of formula: -$NR^aR^b$ (wherein one of $R^a$ and $R^b$ represents a hydrogen atom or a lower alkyl group, and the other represents a hydrogen atom, a lower alkyl group or an aralkyl group),
substituent group $\beta^1$ represents the group consisting of a lower alkyl group, a halogeno lower alkyl group, a hydroxy - lower alkyl group, a nitro - lower alkyl group, an amino - lower alkyl group, a lower alkylamino - lower alkyl group, a di(lower alkyl)amino - lower alkyl group and an aralkylamino - lower alkyl group, or a pharmacologically acceptable salt thereof, a pharmacologically acceptable ester thereof or other pharmacologically acceptable derivative thereof,
(5) the compound of (1), wherein $R^1$ is phenyl or phenyl substituted by group(s) selected from the substituent group consisting of a cyano group, a halogen atom, a lower alkoxy group, a halogeno lower alkyl group and a halogeno lower alkoxy group, or a pharmacologically acceptable salt thereof, a pharmacologically acceptable ester thereof or other pharmacologically acceptable derivative thereof,
(6) the compound of (1), wherein $R^1$ is phenyl, 4-fluorophenyl, 3-fluorophenyl, 4-chlorophenyl, 3-chlorophenyl, 2,4-difluorophenyl, 3,4-difluorophenyl, 3,4-dichlorophenyl, 3,4,5-trifluorophenyl, 3-chloro-4-fluorophenyl, 3-difluoromethoxyphenyl, 3-trifluoromethoxyphenyl, 3-trifluoromethylphenyl, 3-methoxyphenyl, 3-cyanophenyl or 4-fluoro-3-methoxyphenyl, or a pharmacologically acceptable salt thereof, a pharmacologically acceptable ester thereof or other pharmacologically acceptable derivative thereof,
(7) the compound of (1), wherein $R^1$ is phenyl, 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl, 3-trifluoromethylphenyl, 3-methoxyphenyl, 3-cyanophenyl or 4-fluoro-3-methoxyphenyl, or a pharmacologically acceptable salt thereof, a pharmacologically acceptable ester thereof or other pharmacologically acceptable derivative thereof,
(8) the compound of (1), wherein $R^1$ is phenyl, 4-fluorophenyl, 3-fluorophenyl, 3-chlorophenyl, 3,4-difluorophenyl, 3-chloro-4-fluorophenyl or 3-trifluoromethylphenyl, or a pharmacologically acceptable salt thereof, a pharmacologically acceptable ester thereof or other pharmacologically acceptable derivative thereof,

(9) the compound of any one selected from (1) to (8), wherein $R^2$ is a 5- or 6-membered heteroaryl group containing 1 or 2 nitrogen atoms; or a 5- or 6-membered heteroaryl group containing 1 or 2 nitrogen atoms substituted by group(s) selected from substituent group α and substituent group β, or a pharmacologically acceptable salt thereof, a pharmacologically acceptable ester thereof or other pharmacologically acceptable derivative thereof,
(10) the compound of any one selected from (1) to (8), wherein $R^2$ is pyridyl, pyrimidinyl, or pyridyl or pyrimidinyl substituted by group(s) selected from substituent group α and substituent group β, or a pharmacologically acceptable salt thereof, a pharmacologically acceptable ester thereof or other pharmacologically acceptable derivative thereof,
(11) the compound of any one selected from (1) to (8), wherein $R^2$ is 4-pyridyl, 4-pyrimidinyl, or 4-pyridyl or 4-pyrimidinyl substituted by group(s) selected from substituent group α and substituent group β, or a pharmacologically acceptable salt thereof, a pharmacologically acceptable ester thereof or other pharmacologically acceptable derivative thereof,
(12) the compound of any one selected from (1) to (8), wherein $R^2$ is 4-pyridyl, 4-pyrimidinyl, or 4-pyridyl or 4-pyrimidinyl wherein the 2-position is substituted by one group selected from substituent group α and substituent group β, or a pharmacologically acceptable salt thereof, a pharmacologically acceptable ester thereof or other pharmacologically acceptable derivative thereof,
(13) the compound of any one selected from (1) to (8), wherein $R^2$ is 4-pyridyl, 4-pyrimidinyl, or 4-pyridyl or 4-pyrimidinyl wherein the 2-position is substituted by one group selected from the substituent group consisting of methoxy, amino, methylamino, benzylamino and α-methylbenzylamino, or a pharmacologically acceptable salt thereof, a pharmacologically acceptable ester thereof or other pharmacologically acceptable derivative thereof,
(14) the compound of any one selected from (1) to (13), wherein $R^4$ is a group independently selected from a hydrogen atom, a lower alkyl group, a group of formula: $-NR^{\alpha}R^{\beta}$ (wherein $R^{\alpha}$ and $R^{\beta}$ are the same or different and each represents a hydrogen atom, a lower alkylsulfonyl group or a lower aliphatic acyl group), an aryl group and an aryl group substituted by group(s) selected from substituent group α and substituent group β, or a pharmacologically acceptable salt thereof, a pharmacologically acceptable ester thereof or other pharmacologically acceptable derivative thereof,
(15) the compound of any one selected from (1) to (13), wherein $R^4$ is a group independently selected from a hydrogen atom, a methyl group, an ethyl group, a propyl group, a butyl group, an amino group, a phenyl group and a phenyl group substituted by group(s) selected from substituent group α and substituent group β, or a pharmacologically acceptable salt thereof, a pharmacologically acceptable ester thereof or other pharmacologically acceptable derivative thereof,
(16) the compound of any one selected from (1) to (13), wherein $R^4$ is a methyl group, an ethyl group, a propyl group, a butyl group, a phenyl group; or a phenyl group substituted by 1 or 2 substituent(s) selected from the group consisting of a hydroxyl group, a halogen atom, a lower acyloxy group, a lower alkoxy group, a halogeno lower alkoxy group, a lower alkyl group and a halogeno lower alkyl group, or a pharmacologically acceptable salt thereof, a pharmacologically acceptable ester thereof or other pharmacologically acceptable derivative thereof,
(17) the compound of any one selected from (1) to (13), wherein $R^4$ is a methyl group, an ethyl group, a propyl group, a butyl group, a phenyl group, or a phenyl group substituted by 1 or 2 substituent(s) selected from the group consisting of a hydroxyl group, a fluorine atom, a chlorine atom, a bromine atom, a formyloxy group, an acetoxy group, a propionyloxy group, a butyryloxy group, an isobutyryloxy group, a valeryloxy group, an isovaleryloxy group, a pivaloyloxy group, a methyl group, an ethyl group, a propyl group, a butyl group, a tert-butyl group, a difluoromethyl group, a trifluoromethyl group, a methoxy group, an ethoxy group, a propoxy group, a butoxy group, a tert-butoxy group, a difluoromethoxy group and a trifluoromethoxy group, or a pharmacologically acceptable salt thereof, a pharmacologically acceptable ester thereof or other pharmacologically acceptable derivative thereof,
(18) the compound of any one selected from (1) to (13), wherein $R^4$ is a methyl group, an ethyl group, a propyl group, a butyl group, a phenyl group; or a phenyl group substituted by 1 or 2 substituent(s) selected from the group consisting of a hydroxyl group, a fluorine atom, a chlorine atom, an acetyloxy group, a methyl group, an ethyl group, a methoxy group and an ethoxy group, or a pharmacologically acceptable salt thereof, a pharmacologically acceptable ester thereof or other pharmacologically acceptable derivative thereof,
(19) the compound of any one selected from (1) to (18), wherein A is pyrrole, or a pharmacologically acceptable salt thereof, a pharmacologically acceptable ester thereof or other pharmacologically acceptable derivative thereof,
(20) the compound of any one selected from (1) to (19), wherein the compound of formula (I) is a compound represented by any one of the following formulae:

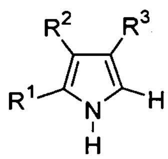 or 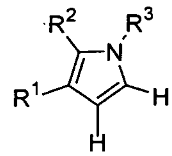

or a pharmacologically acceptable salt thereof, a pharmacologically acceptable ester thereof or other pharmacologically acceptable derivative thereof,
(21) the compound of any one selected from (1) to (19), wherein the compound of formula (I) is a compound represented by the following formula:

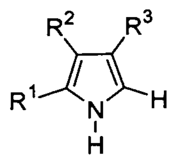

or a pharmacologically acceptable salt thereof, a pharmacologically acceptable ester thereof or other pharmacologically acceptable derivative thereof,
(22) the compound of any one selected from (1) to (18), wherein A is pyrazole, or a pharmacologically acceptable salt thereof, a pharmacologically acceptable ester thereof or other pharmacologically acceptable derivative thereof,
(23) the compound of any one selected from (1) to (18) and (22), wherein the compound of formula (I) is a compound represented by any one of the following formulae:

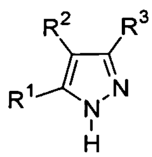 or 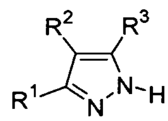

or a pharmacologically acceptable salt thereof, a pharmacologically acceptable ester thereof or other pharmacologically acceptable derivative thereof
and
(24) the compound of any one selected from (1) to (18) and (22), wherein the compound of formula (I) is a compound represented by the following formula:

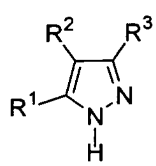

or a pharmacologically acceptable salt thereof, a pharmacologically acceptable ester thereof or other pharmacologically acceptable derivative thereof.

In the compound of formula (I) described in the above (1), compounds satisfying a combination of the requirements arbitrarily selected from the four groups consisting of (2) to (8); (9) to (13); (14) to (18); and (19) to (21) and compounds satisfying a combination of the requirements arbitrarily selected from the four groups consisting of (2) to (8); (9) to (13); (14) to (18); and (22) to (24) are also preferred.

Further, in the compound of formula (I) described in the above (1), the following compounds are also preferred.
(25) the compound of (1), wherein the compound of formula (I) is a compound represented by the following formula:

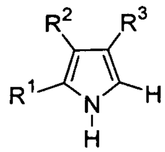

wherein $R^1$ is a phenyl group, a halogeno phenyl group or a halogeno lower alkylphenyl group,
$R^2$ is a pyridyl group,
$R^3$ is a group of the formula:

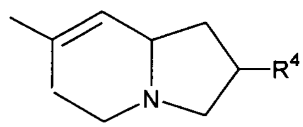

(wherein $R^4$ is a phenyl group or an alkyl group having from 1 to 4 carbon atoms), or a pharmacologically acceptable salt thereof, a pharmacologically acceptable ester thereof or other pharmacologically acceptable derivative thereof,
(26) the compound of (25),
wherein $R^1$ is a phenyl group, a fluorophenyl group, a chlorophenyl group, a difluorophenyl group, a chlorofluorophenyl group or a trifluoromethylphenyl group, or a pharmacologically acceptable salt thereof, a pharmacologically acceptable ester thereof or other pharmacologically acceptable derivative thereof,
(27) the compound of (25),
wherein $R^1$ is a fluorophenyl group and $R^3$ is a group of the following formula:

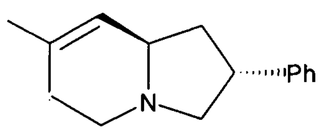

or a pharmacologically acceptable salt thereof, a pharmacologically acceptable ester thereof or other pharmacologically acceptable derivative thereof,
(28) the compound of (25),
wherein $R^1$ is a chlorofluorophenyl group and $R^3$ is a group of the following formula:

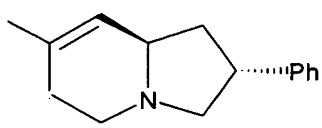

or a pharmacologically acceptable salt thereof, a pharmacologically acceptable ester thereof or other pharmacologically acceptable derivative thereof,
(29) the compound of (1),
wherein the compound of formula (I) is a compound represented by the following formula:

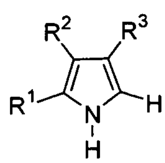

wherein
$R^1$ is a 4-halogeno phenyl group or a halogeno lower alkylphenyl group,
$R^2$ is a pyridyl group,

$R^3$ is a group of the following formula:

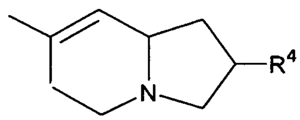

(wherein $R^4$ is a halogeno phenyl group, a 4-methylphenyl group or a 4-methoxyphenyl group), or a pharmacologically acceptable salt thereof, a pharmacologically acceptable ester thereof or other pharmacologically acceptable derivative thereof,
(30) the compound of (29),
wherein $R^1$ is a 4-fluorophenyl group or a trifluoromethylphenyl group and
$R^4$ is a fluorophenyl group, a 4-methylphenyl group or a 4-methoxyphenyl group, or a pharmacologically acceptable salt thereof, a pharmacologically acceptable ester thereof or other pharmacologically acceptable derivative thereof,
(31) the compound of (1),
wherein the compound is selected from the following compounds:

4-[(2S,8aS)-2-butyl-1,2,3,5,6,8a-hexahydroindolizin-7-yl]-2-(4-fluorophenyl)-3-(pyridin-4-yl)-1H-pyrrole,

2-(4-fluorophenyl)-4-[(2S,8aS)-2-phenyl-1,2,3,5,6,8a-hexahydroindolizin-7-yl]-3-(pyridin-4-yl)-1H-pyrrole,

2-(4-fluorophenyl)-4-[(2R,8aS)-2-(4-fluorophenyl)-1,2,3,5,6,8a-hexahydroindolizin-7-yl]-3-(pyridin-4-yl)-1H-pyrrole,

2-(3,4-difluorophenyl)-4-[(2S,8aS)-2-phenyl-1,2,3,5,6,8a-hexahydroindolizin-7-yl]-3-(pyridin-4-yl)-1H-pyrrole,

2-(4-fluorophenyl)-4-[(2S,8aS)-2-(4-methylphenyl)-1,2,3,5,6,8a-hexahydroindolizin-7-yl]-3-(pyridin-4-yl)-1H-pyrrole,

2-(3-chlorophenyl)-4-[(2S,8aS)-2-phenyl-1,2,3,5,6,8a-hexahydroindolizin-7-yl]-3-(pyridin-4-yl)-1H-pyrrole,

4-[(2S,8aS)-2-(4-methylphenyl)-1,2,3,5,6,8a-hexahydroindolizin-7-yl]-3-(pyridin-4-yl)-2-(3-trifluoromethylphenyl)-1H-pyrrole,

2-(4-fluorophenyl)-4-[(2S,8aS)-2-(4-methoxyphenyl)-1,2,3,5,6,8a-hexahydroindolizin-7-yl]-3-(pyridin-4-yl)-1H-pyrrole,

4-[(2S,8aS)-2-phenyl-1,2,3,5,6,8a-hexahydroindolizin-7-yl]-3-(pyridin-4-yl)-2-(3-trifluoromethylphenyl)-1H-pyrrole,

2-phenyl-4-[(2S,8aS)-2-phenyl-1,2,3,5,6,8a-hexahydroindolizin-7-yl]-3-(pyridin-4-yl)-1H-pyrrole,

4-[(2S,8aS)-2-(4-ethylphenyl)-1,2,3,5,6,8a-hexahydroindolizin-7-yl]-2-(4-fluorophenyl)-3-(pyridin-4-yl)-1H-pyrrole,

4- [(2S,8aS)-2-(4-ethylphenyl)-1,2,3,5,6,8a-hexahydroindolizin-7-yl]-2-(3,4-difluorophenyl)-3-(pyridin-4-yl)-1H-pyrrole and

2-(3-chloro-4-fluorophenyl)-4-[(2S,8aS)-2-phenyl-1,2,3,5,6,8a-hexahydroindolizin-7-yl]-3-(pyridin-4-yl)-1H-pyrrole,
or a pharmacologically acceptable salt thereof, a pharmacologically acceptable ester thereof or other pharmacologically acceptable derivative thereof,

(32) the compound of (1),
wherein the compound of formula (I) is a compound represented by any one of the following formulae:

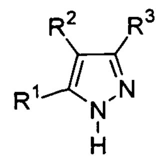

or

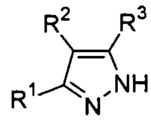

wherein $R^1$ is a phenyl group or a halogeno phenyl group,
$R^2$ is a pyridyl group and
$R^3$ is a group of the following formula:

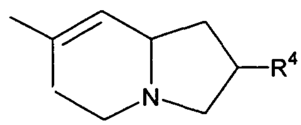

(wherein $R^4$ is a hydrogen atom, a lower alkyl group, a phenyl group, a lower alkylphenyl group or a lower alkoxyphenyl group), or a pharmacologically acceptable salt thereof, a pharmacologically acceptable ester thereof or other pharmacologically acceptable derivative thereof,
(33) the compound of (32),
wherein $R^1$ is a phenyl group, a fluorophenyl group or a chlorofluorophenyl group, and
$R^4$ is a hydrogen atom, an alkyl group having from 1 to 4 carbon atoms, a phenyl group, an alkylphenyl group having from 1 to 3 carbon atoms or an alkoxyphenyl group having from 1 to 3 carbon atoms, or a pharmacologically acceptable salt thereof, a pharmacologically acceptable ester thereof or other pharmacologically acceptable derivative thereof,
(34) the compound of (1),
which is selected from the following:

3-[(2S,8aS)-2-butyl-1,2,3,5,6,8a-hexahydroindolizin-7-yl]-5-(4-fluorophenyl)-4-(pyridin-4-yl)pyrazole,

5-(4-fluorophenyl)-3-[(2S,8aS)-2-propyl-1,2,3,5,6,8a-hexahydroindolizin-7-yl]-4-(pyridin-4-yl)pyrazole,

5-(4-fluorophenyl)-3-[(2S,8aS)-2-phenyl-1,2,3,5,6,8a-hexahydroindolizin-7-yl]-4-(pyridin-4-yl)pyrazole,

5-(4-fluorophenyl)-3-[(2S,8aS)-2-(4-methylphenyl)-1,2,3,5,6,8a-hexahydroindolizin-7-yl]-4-(pyridin-4-yl)pyrazole and

5-(3-chloro-4-fluorophenyl)-3-[(2S,8aS)-2-phenyl-1,2,3,5,6,8a-hexahydroindolizin-7-yl]-4-(pyridin-4-yl)pyrazole,
or a pharmacologically acceptable salt thereof, a pharmacologically acceptable ester thereof or other pharmacologically acceptable derivative thereof,

(35) the compound of (1),
wherein the compound of formula (I) is a compound represented by the following formula:

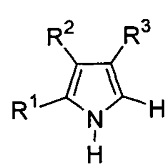

wherein $R^1$ is a lower alkoxyphenyl group or a lower alkoxy (halogeno) phenyl group,
$R^2$ is a pyridyl group, and
$R^3$ is a group of the following formula:

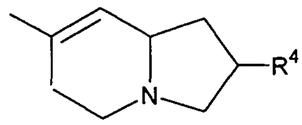

(wherein $R^4$ is a phenyl group), or a pharmacologically acceptable salt thereof, a pharmacologically acceptable ester thereof or other pharmacologically acceptable derivative thereof,
(36) the compound of (35),
wherein $R^1$ is a methoxyphenyl group or a fluoro(methoxy)phenyl group, or a pharmacologically acceptable salt thereof, a pharmacologically acceptable ester thereof or other pharmacologically acceptable derivative thereof,
(37) the compound of (1),
wherein the compound of formula (I) is a compound represented by the formula:

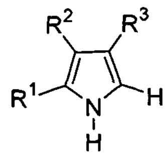

wherein $R^1$ is a 4-halogeno phenyl group,
$R^2$ is a pyridyl group, and
$R^3$ is a group of the following formula:

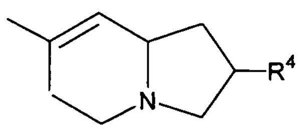

[wherein $R^4$ is a phenyl group substituted by a lower alkyl group other than a 4-methyl group, a phenyl group substituted by a lower alkoxy group other than a 4-methoxy group, a hydroxyphenyl group or a lower acyloxyphenyl group], or a pharmacologically acceptable salt thereof, a pharmacologically acceptable ester thereof or other pharmacologically acceptable derivative thereof,
(38) the compound of (39),
wherein $R^1$ is a 4-fluorophenyl group, and
$R^4$ is a 3-methylphenyl group, an ethylphenyl group, a butylphenyl group, a 3-methoxyphenyl group, an ethoxyphenyl group, a hydroxyphenyl group or an acetoxy group, or a pharmacologically acceptable salt thereof, a pharmacologically acceptable ester thereof or other pharmacologically acceptable derivative thereof,
(39) the compound of (1),
wherein the compound of formula (I) is a compound represented by the formula:

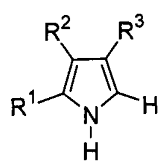

wherein $R^1$ is a phenyl group substituted by a halogen atom other than a 4-halogeno phenyl group,
$R^2$ is a pyridyl group, and
$R^3$ is a group of the following formula:

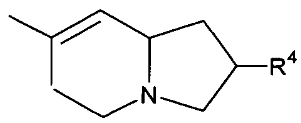

(wherein $R^4$ is a lower alkylphenyl group, a lower alkoxyphenyl group or a hydroxyphenyl group), or a pharmacologically acceptable salt thereof, a pharmacologically acceptable ester thereof or other pharmacologically acceptable derivative thereof,
(40) the compound of (39),
wherein $R^1$ is a chlorophenyl group or a difluorophenyl group and
$R^4$ is a methylphenyl group, an ethylphenyl group, a propylphenyl group, an isopropylphenyl group, a butylphenyl group, a methoxyphenyl group, an ethoxyphenyl group, a propoxyphenyl group or a hydroxyphenyl group, or a pharmacologically acceptable salt thereof, a pharmacologically acceptable ester thereof or other pharmacologically acceptable derivative thereof,
(41) the compound of (1),
which is selected from the following:

2-(4-fluorophenyl)-4-[(2S,8aS)-2-(3-methylphenyl)-1,2,3,5,6,8a-hexahydroindolizin-7-yl]-3-(pyridin-4-yl)-1H-pyrrole,

2-(3,4-difluorophenyl)-4-[(2S,8aS)-2-(4-methylphenyl)-1,2,3,5,6,8a-hexahydroindolizin-7-yl]-3-(pyridin-4-yl)-1H-pyrrole,

2-(3-chlorophenyl)-4-[(2S,8aS)-2-(4-methylphenyl)-1,2,3,5,6,8a-hexahydroindolizin-7-yl]-3-(pyridin-4-yl)-1H-pyrrole,

2-(3,4-difluorophenyl)-4-[(2S,8aS)-2-(4-methoxyphenyl)-1,2,3,5,6,8a-hexahydroindolizin-7-yl]-3-(pyridin-4-yl)-1H-pyrrole,

2-(4-fluorophenyl)-4-[(2S,8aS)-2-(3-methoxyphenyl)-1,2,3,5,6,8a-hexahydroindolizin-7-yl]-3-(pyridin-4-yl)-1H-pyrrole,

2-(3-chlorophenyl)-4-[(2S,8aS)-2-(4-methoxyphenyl)-1,2,3,5,6,8a-hexahydroindolizin -7-yl]-3-(pyridin-4-yl)-1H-pyrrole,

4-[(2S,8aS)-2-(4-ethoxyphenyl)-1,2,3,5,6,8a-hexahydroindolizin-7-yl]-2-(4-fluorophenyl)-3-(pyridin-4-yl)-1H-pyrrole,

2-(3,4-difluorophenyl)-4-[(2S,8aS)-2-(3-methoxyphenyl)-1,2,3,5,6,8a-hexahydroindolizin-7-yl]-3-(pyridin-4-yl)-1H-pyrrole,

2-(4-fluorophenyl)-4-[(2S,8aS)-2-(4-hydroxyphenyl)-1,2,3,5,6,8a-hexahydroindolizin-7-yl]-3-(pyridin-4-yl)-1H-pyrrole,

4-[(2S,8aS)-2-(4-acetoxyphenyl)-1,2,3,5,6,8a-hexahydroindolizin-7-yl]-2-(4-fluorophenyl)-3-(pyridin-4-yl)-1H-pyrrole,

2-(3,4-difluorophenyl)-4-[(2S,8aS)-2-(3-hydroxyphenyl)-1,2,3,5,6,8a-hexahydroindolizin-7-yl]-3-(pyridin-4-yl)-1H-pyrrole,

2-(4-fluoro-3-methoxyphenyl)-4-[(2S,8aS)-2-phenyl-1,2,3,5,6,8a-hexahydroindolizin-7-yl]-3-(pyridin-4-yl)-1H-pyrrole,

2-(3-methoxyphenyl)-4-[(2S,8aS)-2-phenyl-1,2,3,5,6,8a-hexahydroindolizin-7-yl]-3-(pyridin-4-yl)-1H-pyrrole),

4-[(2S,8aS)-2-(4-ethoxyphenyl)-1,2,3,5,6,8a-hexahydroindolizin-7-yl]-2-(3,4-difluorophenyl)-3-(pyridin-4-yl)-1H-pyrrole,

2-(4-fluorophenyl)-4-[(2S,8aS)-2-(3,4-dimethylphenyl)-1,2,3,5,6,8a-hexahydroindolizin-7-yl]-3-(pyridin-4-yl)-1H-pyrrole and

2-(3,4-difluorophenyl)-4-[(2S,8aS)-2-(3,4-dimethylphenyl)-1,2,3,5,6,8a-hexahydroindolizin-7-yl]-3-(pyridin-4-yl)-1H-pyrrole
or a pharmacologically acceptable salt thereof, a pharmacologically acceptable ester thereof or other pharmacologically acceptable derivative thereof.

Further, another object of the present invention is: (42) to provide a pharmaceutical composition containing a compound described in any one selected from (1) to (41), or a pharmacologically acceptable salt thereof, a pharmacologically acceptable ester thereof or other pharmacologically acceptable derivative thereof as an active ingredient, and preferably to provide

(43) the pharmaceutical composition described in (42) used for inhibiting the production of inflammatory cytokines,
(44) the pharmaceutical composition described in (42) used as an antifebrile/analgesic, an anti-inflammatory agent or an anti-virus agent,
(45) the pharmaceutical composition described in (42) used for preventing or treating rheumatoid arthritis,
(46) the pharmaceutical composition described in (42) used for preventing or treating osteoarthritis,
(47) the pharmaceutical composition described in (42) used for preventing or treating septicaemia,
(48) the pharmaceutical composition described in (42) used for preventing or treating diabetes [more preferably
(49) the pharmaceutical composition described in (48) wherein the diabetes is I type diabetes] and
(50) the pharmaceutical composition described in (42) used for preventing or treating psoriasis, Crohn's disease, ulcerative colitis or hepatitis.

Further, another object of the present invention is
(51)to provide a method for inhibiting the production of inflammatory cytokines [preferably a method for treating or removing pain and/or inflammation; a method for preventing or treating a viral disease; a method for preventing or treating rheumatoid arthritis; a method for preventing or treating osteoarthritis; a method for preventing or treating septicaemia; a method for preventing or treating diabetes (preferably I type diabetes); or a method for preventing or treating psoriasis, Crohn's disease, ulcerative colitis or hepatitis] comprising administering an effective amount of the compound described in any one selected from (1) to (41), or a pharmacologically acceptable salt thereof, a pharmacologically acceptable ester thereof or other pharmacologically acceptable derivative thereof to a mammal (preferably a human being).

The compound of the above formula (I) namely indicates any one of the following formulae:

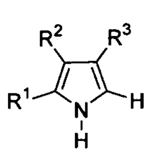 , 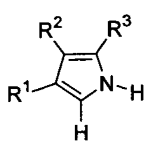 , 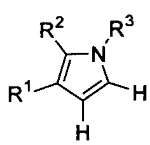 , 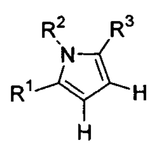 , 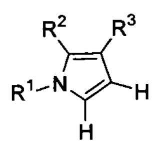

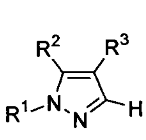 , 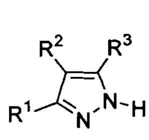 , 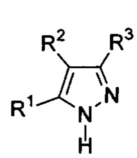 or 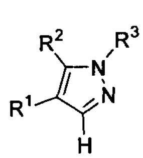

The preferred compounds among the compounds represented by the above formulae are those represented by any one of the following formulae:

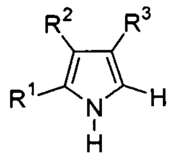 , 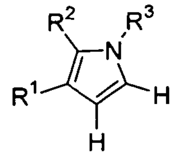 , 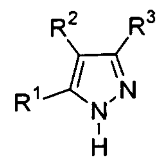 or 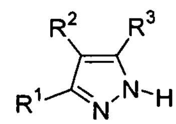

The particularly preferred compounds among the compounds represented by the above formulae are those represented by the following formulae:

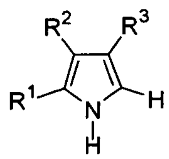 or 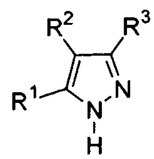

In the above formulae, $R^1$, $R^2$ and $R^3$ have the same meanings as defined above.

In the above formula (I), $R^3$ is preferably a group represented by the following formula in which $R^4$ substitutes the indolizine ring in a trans position:

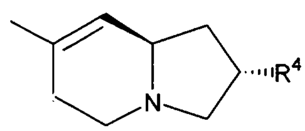

(wherein $R^4$ has the same meaning as defined above).

In the above formula (I), the "aryl group" in the definition of $R^1$ and $R^4$; the "aryl group" of the "aryl group substituted by group(s) selected from substituent group $\alpha$ and substituent group $\beta$" in the definition of $R^1$ and $R^4$; the "aryl group" of the "aryl group substituted by an aryl group" in the definition of $R^4$; and the "aryl group" of the "aryl group substituted by an aryloxy group" in the definition of $R^4$ is, for example, an aryl group having from 6 to 14 carbon atoms such as a phenyl, naphthyl, phenanthryl or anthracenyl group, and is preferably a phenyl or naphthyl group, most preferably phenyl.

Further, the above "aryl group" may be fused with a cycloalkyl group having from 3 to 10 carbon atoms, and such a group is, for example, a 5-indanyl group or the like.

The "aryl group substituted by group(s) selected from substituent group $\alpha$ and substituent group $\beta$" in the definition of $R^1$ and $R^4$ is preferably an aryl group substituted by from 1 to 4 group(s) selected from substituent group $\alpha$ and substituent group $\beta$, more preferably, an aryl group substituted by from 1 to 3 group(s) selected from substituent group $\alpha$ and substituent group $\beta$.

The specific examples preferably include 4-fluorophenyl, 3-fluorophenyl, 4-chlorophenyl, 3-chlorophenyl, 2,4-difluorophenyl, 3,4-difluorophenyl, 3,4-dichlorophenyl, 3,4,5-trifluorophenyl, 3-chloro-4-fluorophenyl, 3-difluoromethoxyphenyl, 3-trifluoromethoxyphenyl, 3-trifluoromethylphenyl, 3-methoxyphenyl, 3-cyanophenyl or 4-fluoro-3-methoxyphenyl group, more preferably 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl, 3-trifluoromethylphenyl, 3-methoxyphenyl, 3-cyanophenyl or 4-fluoro-3-methoxyphenyl group, further more preferably 4-fluorophenyl, 3-fluorophenyl, 3-chlorophenyl, 3,4-difluorophenyl, 3-chloro-4-fluorophenyl or 3-trifluoromethylphenyl group.

The "aryl group substituted by an aryl group" in the definition of $R^4$ is preferably an aryl group substituted by one aryl group, and the specific examples include 3-biphenyl, 4-biphenyl, 4-($\alpha$-naphthyl)phenyl or 4-($\beta$-naphthyl)phenyl group, preferably 4-biphenyl group.

The "aryloxy group" of the "aryl group substituted by an aryloxy group" in the definition of $R^4$ is a group in which an oxygen atom is bonded to the above "aryl group", and is preferably a phenoxy, naphthyloxy, phenanthryloxy or anthracenyloxy group, more preferably a phenoxy or naphthyloxy group, most preferably a phenoxy group.

The "aryl group substituted by an aryloxy group" in the definition of $R^4$ is an aryl group substituted by one aryloxy group, and the specific examples preferably include phenoxyphenyl or naphthyloxyphenyl group, most preferably phenoxyphenyl group.

The "heteroaryl group" in the definition of $R^1$; and the "heteroaryl group" of the "heteroaryl group substituted

by group(s) selected from substituent group α and substituent group β" in the definition of $R^1$ can be, for example, a 5- to 7-membered heteroaryl group containing from 1 to 3 sulfur atom(s), oxygen atom(s) and/or nitrogen atom(s), such as a furyl, thienyl, pyrrolyl, pyrazolyl, imidazolyl, oxazolyl, isoxazolyl, thiazolyl, isothiazolyl, triazolyl, tetrazolyl, thiadiazolyl, pyridyl, pyridazinyl, pyrimidinyl or pyrazinyl group, and is preferably a 5- or 6-membered heteroaryl group containing 1 or 2 sulfur atom(s), oxygen atom(s) and/or nitrogen atom(s), such as a furyl, thienyl, pyrrolyl, pyrazolyl, imidazolyl, oxazolyl, isoxazolyl, thiazolyl, isothiazolyl, pyridyl, pyridazinyl, pyrimidinyl or pyrazinyl group, more preferably a furyl, thienyl, pyridyl or pyrimidinyl group.

It should be noted that the above "heteroaryl group" may be fused with another cyclic group (for example, a cyclic group such as an aryl group or a cycloalkyl group having from 3 to 10 carbon atoms), and this group is, for example, an indolyl, benzofuranyl, benzothienyl, quinolyl, isoquinolyl, quinazolinyl, tetrahydroquinolyl or tetrahydroisoquinolyl group.

The "heteroaryl group substituted by group(s) selected from substituent group α and substituent group β" in the definition of $R^1$ is preferably a heteroaryl group substituted by from 1 to 3 group(s) selected from substituent group α and substituent group β, more preferably, a heteroaryl group substituted by 1 or 2 group(s) selected from substituent group α and substituent group β. The specific examples preferably include 5-fluoro-2-furyl, 4-chloro-2-thienyl, 5-difluoromethoxy-3-furyl, 5-trifluoromethyl-3-thienyl or 5-fluoro-2-oxazolyl group.

The "heteroaryl group having at least one nitrogen atom" in the definition of $R^2$ and the "heteroaryl group having at least one nitrogen atom" of the "heteroaryl group having at least one nitrogen atom, substituted by group(s) selected from substituent group α and substituent group β" can be a 5- to 7-membered heteroaryl group which contains at least one nitrogen atom and may further contain 1 or 2 sulfur atom(s), oxygen atom(s) and/or nitrogen atom(s) such as a pyrrolyl, pyrazolyl, imidazolyl, oxazolyl, isoxazolyl, thiazolyl, isothiazolyl, triazolyl, tetrazolyl, thiadiazolyl, pyridyl, pyridazinyl, pyrimidinyl or pyrazinyl group, and is preferably a 5- or 6-membered heteroaryl group which contains one nitrogen atom and may further contain one sulfur atom, oxygen atom or nitrogen atom, such as a pyrrolyl, pyrazolyl, imidazolyl, oxazolyl, isoxazolyl, thiazolyl, isothiazolyl, pyridyl, piridazinyl, pyrimidinyl or pyrazinyl group, more preferably a 5- or 6-membered heteroaryl group containing 1 or 2 nitrogen atom(s), such as an imidazolyl, pyridyl, pyridazinyl, pyrimidinyl or pyrazinyl group, particularly preferably a pyridyl or pyrimidinyl group, most preferably a 4-pyridyl or 4-pyrimidinyl group.

The "heteroaryl group having at least one nitrogen atom substituted by group(s) selected from substituent group α and substituent group β" in the definition of $R^2$ is preferably a group substituted by from 1 to 3 group(s) selected from substituent group α and substituent group β, more preferably a group substituted by 1 or 2 group(s) selected from substituent group α and substituent group β, further more preferably a group substituted by one group selected from substituent group α and substituent group β, particularly preferably a 4-pyridyl or 4-pyrimidinyl group wherein the 2-position is substituted by 1 group selected from substituent group α and substituent group β, most preferably a 4-pyridyl or a 4-pyrimidinyl group wherein the 2-position is substituted by a lower alkoxy group, a group having the formula: $-NR^aR^b$ (wherein $R^a$ and $R^b$ are the same or different and each represents a hydrogen atom, a lower alkyl group, a lower alkenyl group, a lower alkynyl group, an aralkyl group or a lower alkylsulfonyl group, or $R^a$ and $R^b$, together with the nitrogen atom to which they are bonded, form a heterocyclyl group) or a lower alkyl group substituted by a group of formula: $-NR^aR^b$ (wherein $R^a$ and $R^b$ have the same meanings as defined above). The specific examples preferably include 2-methoxy-4-pyridyl, 2-methoxy-4-pyrimidinyl, 2-amino-4-pyridyl, 2-amino-4-pyrimidinyl, 2-methylamino-4-pyridyl, 2-methylamino-4-pyrimidinyl, 2-benzylamino-4-pyridyl, 2-benzylamino-4-pyrimidinyl, 2-(α-methylbenzylamino)-4-pyridyl or 2-(α-methylbenzylamino)-4-pyrimidinyl group.

The "cycloalkyl group" in the definition of [substituent group β] is a cycloalkyl group having from 3 to 7 carbon atoms such as a cyclopropyl, cyclobutyl, cyclopentyl, cyclohexyl or cycloheptanyl group, and is preferably a cycloalkyl group having from 3 to 6 carbon atoms, more preferably a cyclopentyl or cyclohexyl group.

The "lower alkyl group" in the definition of $R^a$, $R^b$, $R^4$ and [substituent group β]; the "lower alkyl group" of the "lower alkyl group substituted by group(s) selected from substituent group α" in the definition of [substituent group β] can be, for example, a straight or branched alkyl group having from 1 to 6 carbon atoms such as a methyl, ethyl, propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl or 2-ethylbutyl group, and is preferably an alkyl group having from 1 to 4 carbon atoms, more preferably a methyl, ethyl, propyl, isopropyl or butyl group, particularly preferably a methyl, ethyl or propyl group.

The "lower alkenyl group" in the definition of $R^a$, $R^b$, and [substituent group β]; the lower alkenyl group of the "lower alkenyl group substituted by group(s) selected from substituent group α" in the definition of [substituent group β] can be, for example, a straight or branched alkenyl group having from 2 to 6 carbon atoms such as a vinyl, 2-propenyl, 1-methyl-2-propenyl, 2-methyl-2-propenyl, 2-ethyl-2-propenyl, 2-butenyl, 1-methyl-2-butenyl, 2-methyl-2-butenyl, 1-ethyl-2-butenyl, 3-butenyl, 1-methyl-3-butenyl, 2-methyl-3-butenyl, 1-ethyl-3-butenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl,

2-methyl-4-pentenyl, 2-hexenyl, 3-hexenyl, 4-hexenyl or 5-hexenyl group, and is preferably an alkenyl group having from 2 to 4 carbon atoms, more preferably an alkenyl group having 2 or 3 carbon atoms.

The "lower alkynyl group" in the definition of $R^a$, $R^b$, and [substituent group β]; the lower alkynyl group of the "lower alkynyl group substituted by group(s) selected from substituent group α" in the definition of [substituent group β] can be, for example, a straight or branched alkynyl group having from 2 to 6 carbon atoms such as an ethynyl, 2-propynyl, 1-methyl-2-propynyl, 2-butynyl, 1-methyl-2-butynyl, 1-ethyl-2-butynyl, 3-butynyl, 1-methyl-3-butynyl, 2-methyl-3-butynyl, 1-ethyl-3-butynyl, 2-pentynyl, 1-methyl-2-pentynyl, 3-pentynyl, 1-methyl-3-pentynyl, 2-methyl-3-pentynyl, 4-pentynyl, 1-methyl-4-pentynyl, 2-methyl-4-pentynyl, 2-hexynyl, 3-hexynyl, 4-hexynyl or 5-hexynyl group, and is preferably an alkynyl group having from 2 to 4 carbon atoms, more preferably an alkynyl group having 2 or 3 carbon atoms.

The "aralkyl group" in the definition of $R^a$, $R^b$ and [substituent group β] is a group in which the above "aryl group" is bonded to the above "lower alkyl group", and this group can be, for example, a benzyl, α-naphthylmethyl, β-naphthylmethyl, 1-phenethyl, 2-phenethyl, 2-(α-naphthyl)ethyl, 2-(β-naphthyl)ethyl, 3-phenylpropyl, 4-phenylbutyl, 5-phenylpentyl or 6-phenylhexyl group, and is preferably a benzyl group.

It should be noted that the aryl moiety of the above "aralkyl group" may be substituted by from 1 to 3 groups selected from the above "substituent group α" and "substituent group β", and this substituted aralkyl group is preferably an aralkyl group substituted by halogen atom, lower alkyl group or lower alkoxy group, such as a 2-fluorobenzyl, 3-fluorobenzyl, 4-fluorobenzyl, 2-chlorobenzyl, 3-chlorobenzyl, 4-chlorobenzyl, 2-methylbenzyl, 3-methylbenzyl, 4-methylbenzyl, 2-methoxybenzyl, 3-methoxybenzyl or 4-methoxybenzyl group.

Among the "aralkyl group" and the substituted aralkyl group, preferred are unsubstituted aralkyl groups or aralkyl groups substituted by halogen atom, lower alkyl group or lower alkoxy group, more preferred are unsubstituted aralkyl groups or aralkyl groups substituted by halogen atom or lower alkyl group, and most preferred are unsubstituted aralkyl groups.

The "lower alkylsulfonyl group" in the definition of $R^\alpha$, $R^\beta$, $R^a$ and $R^b$ is a group in which sulfonyl ($-SO_2-$) is bonded to the above "lower alkyl", and is preferably a straight or branched alkylsulfonyl group having from 1 to 4 carbon atoms, more preferably a methylsulfonyl, ethylsulfonyl, propylsulfonyl, isopropylsulfonyl or butylsulfonyl group, particularly preferably a methylsulfonyl, ethylsulfonyl or propylsulfonyl group.

The "lower aliphatic acyl group" in the definition of $R^\alpha$, $R^\beta$, $R^a$ and $R^b$ is a group in which a carbonyl group is bonded to the above "lower alkyl group", and is preferably a straight or branched acyl group having from 1 to 4 carbon atoms, more preferably an acyl group having from 1 to 3 carbon atoms, particularly preferably an acetyl group.

The heterocyclyl group which $R^a$ and $R^b$ form, together with the nitrogen atom to which they are bonded, is a 4- to 7-membered heterocyclyl group which contains one nitrogen atom and may further contain one oxygen atom, sulfur atom or nitrogen atom, and this group is, for example, a 1-azetidinyl, 1-pyrrolidinyl, 1-pyrrohnyl, 1-imidazolidinyl, 1-imidazolinyl, 1-pyrazolidinyl, 1-pyrazolinyl, 3-oxazolidinyl, 3-thiazolidinyl, 1-piperidyl, tetrahydropyridin-1-yl, dihydropyridin-1-yl, 1-piperazinyl, 4-morpholinyl, 4-thiomorpholinyl, 1-homopiperidyl, 8-azabicyclo[3.2.1]octan-8-yl, 8-azabicyclo[3.2.1]octen-8-yl, 9-azabicyclo[3.3.1]nonan-9-yl or 9-azabicyclo[3.3.1]nonen-9-yl group.

It should be noted that these groups may be fused with an aryl group or a heteroaryl group, and such a group is, for example, a tetrahydroquinolin-1-yl or tetrahydroisoquinolin-2-yl group.

The "halogen atom" in the definition of [substituent group α] can be a fluorine atom, a chlorine atom, a bromine atom or an iodine atom, and is preferably a fluorine atom or a chlorine atom.

The "lower alkoxy group" in the definition of $R^4$ and [substituent group α] is a group in which an oxygen atom is bonded to the above "lower alkyl group", and is preferably a straight or branched alkoxy group having from 1 to 4 carbon atoms, more preferably a methoxy, ethoxy, propoxy, isopropoxy or butoxy group, particularly preferably a methoxy, ethoxy or propoxy group.

The "halogeno lower alkoxy group" in the definition of [substituent group α] is a group in which 1 or 2 or more hydrogen atoms of the above "lower alkoxy group" are substituted by the above "halogen atom", and is preferably a halogeno lower alkoxy group having from 1 to 4 carbon atoms, more preferably a difluoromethoxy, trifluoromethoxy or 2,2,2-trifluoroethoxy group, particularly preferably a difluoromethoxy group.

The "lower acyloxy group" in the definition of [substituent group α] is a group in which an oxygen atom is bonded to the above "lower aliphatic acyl group", and is preferably a straight or branched acyloxy group having from 1 to 5 carbon atoms, more preferably an acetyloxy, propionyloxy, butyryloxy or pivaloyloxy group.

The "lower alkylthio group" in the definition of [substituent group α] is a group in which a sulfur atom is bonded to the above "lower alkyl group", and is preferably a straight or branched alkylthio group having from 1 to 4 carbon atoms, more preferably a methylthio, ethylthio, propylthio, isopropylthio or butylthio group, particularly preferably a methylthio, ethylthio or propylthio group.

The "halogeno lower alkylthio group" in the definition of [substituent group α] is a group in which 1 or 2 or more hydrogen atoms of the above "lower alkylthio group" are substituted by the above "halogen atom", and is preferably a halogeno lower alkylthio group having from 1 to 4 carbon atoms, more preferably a difluoromethylthio, trifluoromethylthio or 2,2,2-trifluoroethylthio group.

In the above formula (I), the group defined as $R^4$ is preferably a group arbitrarily selected from a hydrogen atom, a hydroxyl group, a lower alkyl group, a methoxy group, an ethoxy group, a propoxy group, a group of formula: $-NR^{\alpha}R^{\beta}$ (wherein $R^{\alpha}$ and $R^{\beta}$ are the same or different and each preferably represent(s) a hydrogen atom, a lower alkylsulfonyl group or a lower aliphatic acyl group), an aryl group, an aryl group substituted by group(s) selected from substituent group $\alpha$ and substituent group $\beta$, an aryl group substituted by a phenyl group and an aryl group substituted by a phenyloxy group,
more preferably a group optionally selected from a hydrogen atom, a lower alkyl group, a group of formula: $-NR^{\alpha}R^{\beta}$ (wherein $R^{\alpha}$ and $R^{\beta}$ are the same or different and each represent(s) a hydrogen atom, a lower alkylsulfonyl group or a lower aliphatic acyl group), an aryl group and an aryl group substituted by group(s) selected from substituent group $\alpha$ and substituent group $\beta$,
more preferably a hydrogen atom, a methyl group, an ethyl group, a propyl group, a butyl group, an amino group, a methanesulfonamido group, an acetamido group, a phenyl group or a phenyl group substituted by group(s) selected from substituent group $\alpha$ and substituent group $\beta$.

Among these, preferred is a hydrogen atom, a methyl group, an ethyl group, a propyl group, a butyl group, an amino group, a phenyl group or a phenyl group substituted by group(s) selected from substituent group $\alpha$ and substituent group $\beta$, more preferred is a methyl group, an ethyl group, a propyl group, a butyl group, a phenyl group; or a phenyl group substituted by 1 or 2 substituent(s) selected from the group consisting of a hydroxyl group, a halogen atom, a lower acyloxy group, a lower alkoxy group, a halogeno lower alkoxy group, a lower alkyl group and a halogeno lower alkyl group,
further more preferred is a methyl group, an ethyl group, a propyl group, a butyl group, a phenyl group; or a phenyl group substituted by 1 or 2 substituent(s) selected from the group consisting of a hydroxyl group, a fluorine atom, a chlorine atom, a bromine atom, a formyloxy group, an acetyloxy group, a propionyloxy group, a butyryloxy group, an isobutyryloxy group, a valeryloxy group, an isovaleryloxy group, a pivaloyloxy group, a methyl group, an ethyl group, a propyl group, a butyl group, a tert-butyl group, a difluoromethyl group, a trifluoromethyl group, a methoxy group, an ethoxy group, a propoxy group, a butoxy group, a tert-butoxy group, a difluoromethoxy group and a trifluoromethoxy group,
particularly preferred is a methyl group, an ethyl group, a propyl group, a butyl group, a phenyl group; or a phenyl group substituted by 1 or 2 substituent(s) selected from the group consisting of a hydroxyl group, a fluorine atom, a chlorine atom, an acetyloxy group, a methyl group, an ethyl group, a methoxy group and an ethoxy group.

Of the groups defined as [substituent group $\alpha$], "substituent group $\alpha^1$" represents the preferred groups, and this consists of a cyano group, a halogen atom, a lower alkoxy group, a halogeno lower alkoxy group and a group of formula: $-NR^aR^b$ (wherein one of $R^a$ and $R^b$ is a hydrogen atom or a lower alkyl group and the other is a hydrogen atom, a lower alkyl group or an aralkyl group). Among the groups defined in "substituent group $\alpha^1$", the preferred groups are a cyano group, a halogen atom, a lower alkoxy group and a halogeno lower alkoxy group.

Of the groups defined as [substituent group $\beta$], "substituent group $\beta^1$" represents the preferred groups, and this consists of a lower alkyl group, a halogeno lower alkyl group, a hydroxy lower alkyl group, a nitro lower alkyl group, an amino lower alkyl group, a lower alkylamino lower alkyl group, a di(lower alkyl)amino lower alkyl group and an aralkylamino lower alkyl group. Among the groups defined in "substituent group $\beta^1$", the preferred group is a halogeno lower alkyl group.

The "halogeno lower alkyl group" in the definition of "substituent group $\beta^1$" is a group in which 1 or 2 or more hydrogen atoms of the above "lower alkyl group" are substituted by the above "halogen atom", and is preferably a halogeno alkyl group having from 1 to 4 carbon atoms, more preferably a trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl or 2,2-dibromoethyl group, further more preferably a trifluoromethyl, trichloromethyl, difluoromethyl or fluoromethyl group, and most preferably a trifluoromethyl group.

The "hydroxy lower alkyl group" in the definition of "substituent group $\beta^1$" is a group in which 1 or 2 or more hydrogen atoms of the above "lower alkyl group" are substituted by the above "hydroxyl group", and is preferably a hydroxyalkyl group having from 1 to 4 carbon atoms, more preferably a hydroxymethyl, a 2-hydroxyethyl or a 3-hydroxypropyl group.

The "nitro lower alkyl group" in the definition of "substituent group $\beta^1$" is a group in which 1 or 2 or more hydrogen atoms of the above "lower alkyl group" are substituted by a nitro group, and is preferably a nitroalkyl group having from 1 to 4 carbon atoms, more preferably a nitromethyl, 2-nitroethyl or 3-nitropropyl group.

The "amino lower alkyl group", the "lower alkylamino lower alkyl group", the "di(lower alkyl)amino lower alkyl group" and the "aralkylamino lower alkyl group" in the definition of "substituent group $\beta^1$" are groups in which 1 or 2 or more hydrogen atoms of the above "lower alkyl group" are substituted by a group of formula: $-NR^cR^d$ (wherein one of $R^c$ and $R^d$ is the hydrogen atom or a lower alkyl group and the other is a hydrogen atom, a lower alkyl group or an aralkyl group), and are preferably a group in which the alkyl moiety is an alkyl group having from 1 to 4 carbon atoms,

more preferably an aminomethyl, 2-aminoethyl, 3-aminopropyl, methylaminomethyl, 2-(methylamino)ethyl, 3-(methylamino)propyl, ethylaminomethyl, 2-(ethylamino)ethyl, 3-(ethylamino)propyl, dimethylaminomethyl, 2-(dimethylamino)ethyl, 3-(dimethylamino)propyl, diethylaminomethyl, 2-(diethylamino)ethyl, 3-(diethylamino)propyl, benzylaminomethyl, 2-(benzylamino)ethyl or 3-(benzylamino)propyl group.

The "pharmacologically acceptable ester thereof or other pharmacologically acceptable derivative thereof" is a compound obtained by modifying a functional group (for example, a hydroxyl group, an amino group, a hydroxyaryl group or the like) possessed by a compound (I) of the present invention by a protecting group or the like according to conventional methods. For example, in the case where the compound of the present invention has a hydroxyl group, the "pharmacologically acceptable ester thereof or other pharmacologically acceptable derivative thereof' is obtained by protecting the hydroxyl group by a "general protecting group" or a "protecting group cleavable by a biological method such as hydrolysis in a living body".

Here, the "general protecting group" is a protecting group cleavable by a chemical method such as hydrogenolysis, hydrolysis, electrolysis and photolysis, and is preferably an "aliphatic acyl group" (preferably a lower aliphatic acyl group having from 1 to 6 carbon atoms) such as an alkanoyl group, e.g., formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3, 7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl and heneicosanoyl, a halogenated alkylcarbonyl group, e.g., chloroacetyl, dichloroacetyl, trichloroacetyl and trifluoroacetyl, a lower alkoxy alkylcarbonyl group, e.g. methoxyacetyl, or an unsaturated alkylcarbonyl group, e.g., acryloyl, propioloyl, methacryloyl, crotonoyl, isocrotonoyl and (E)-2-methyl-2-butenoyl; an "aromatic acyl group" such as an arylcarbonyl group, e.g., benzoyl, α-naphthoyl and β-naphthoyl, a halogenated arylcarbonyl group, e.g., 2-bromobenzoyl, 4-chlorobenzoyl and 2,4,6-trifluorobenzoyl, a lower alkylated arylcarbonyl group, e.g., 2,4,6-trimethylbenzoyl and 4-toluoyl, a lower alkoxylated arylcarbonyl group, e.g., 4-anisoyl, a nitrated arylcarbonyl group, e.g., 4-nitrobenzoyl and 2-nitrobenzoyl, a lower alkoxycarbonylated arylcarbonyl group, e.g., 2-(methoxycarbonyl)benzoyl, or an arylated arylcarbonyl group, e.g., 4-phenylbenzoyl; an "alkoxycarbonyl group" such as a lower alkoxycarbonyl group, e.g., methoxycarbonyl, ethoxycarbonyl, propoxycarbonyl, butoxycarbonyl, s-butoxycarbonyl, t-butoxycarbonyl and isobutoxycarbonyl, or a lower alkoxycarbonyl group substituted by halogen or tri-lower alkylsilyl group, e.g., 2,2,2-trichloroethoxycarbonyl and 2-trimethylsilylethoxycarbonyl; a "tetrahydropyranyl or tetrahydrothiopyranyl group" such as tetrahydropyran-2-yl, 3-bromotetrahydropyran-2-yl, 4-methoxytetrahydropyran-4-yl, tetrahydrothiopyran-2-yl and 4-methoxytetrahydrothiopyran-4-yl; a "tetrahydrofuranyl or tetrahydrothiofuranyl group" such as tetrahydrofuran-2-yl and tetrahydrothiofuran-2-yl; a "silyl group" such as a tri-lower alkylsilyl group, e.g., trimethylsilyl, triethylsilyl, isopropyldimethylsilyl, t-butyldimethylsilyl, methyldiisopropylsilyl, methyldi-t-butylsilyl and triisopropylsilyl, or a tri-lower alkylsilyl group substituted by 1 or 2 aryl groups, e.g., diphenylmethylsilyl, diphenylbutylsilyl, diphenylisopropylsilyl and phenyldiisopropylsilyl; an "alkoxymethyl group" such as a lower alkoxymethyl group, e.g., methoxymethyl, 1,1-dimethyl-1-methoxymethyl, ethoxymethyl, propoxymethyl, isopropoxymethyl, butoxymethyl and t-butoxymethyl, a lower alkoxylated lower alkoxymethyl group, e.g., 2-methoxyethoxymethyl, or a halogeno lower alkoxymethyl, e.g., 2,2,2-trichloroethoxymethyl and bis(2-chloroethoxy)methyl; a "substituted ethyl group" such as a lower alkoxylated ethyl group, e.g., 1-ethoxyethyl and 1-(isopropoxy)ethyl, or a halogenated ethyl group, e.g., 2,2,2-trichloroethyl; an "aralkyl group" such as a lower alkyl group substituted by from 1 to 3 aryl groups, e.g., benzyl, α-naphthylmethyl, β-naphthylmethyl, diphenylmethyl, triphenylmethyl, α-naphthyldiphenylmethyl and 9-anthrylmethyl, or a lower alkyl group substituted by from 1 to 3 aryl groups wherein the aryl ring is substituted by lower alkyl, lower alkoxy, nitro, halogen or cyano group, e.g., 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl and 4-cyanobenzyl; an "alkenyloxycarbonyl group" such as vinyloxycarbonyl and allyloxycarbonyl; or an "aralkyloxycarbonyl group" wherein the aryl ring may be substituted by 1 or 2 lower alkoxy or nitro groups such as benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl.

The "protecting group cleavable by a biological method such as hydrolysis in a living body" is a protecting group cleaved by a biological method such as hydrolysis in a human body to produce a free acid or a salt thereof, and whether a compound is such derivative or not can be determined by administering it to an experimental animal such as a rat or a mouse by intravenous injection, investigating a body fluid of the animal after administration, and detecting the original compound or a pharmacologically acceptable salt thereof.

This "protecting group cleavable by a biological method such as hydrolysis in a living body" can preferably be a 1-(acyloxy) "lower alkyl group" such as a 1-("lower aliphatic acyl"oxy) "lower alkyl group", e.g., formyloxymethyl, acetoxymethyl, dimethylaminoacetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl,

1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl and 1-pivaloyloxyhexyl, a 1-("cycloalkyl"carbonyloxy) "lower alkyl group", e.g., cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl and 1-cyclohexylcarbonyloxybutyl, or a 1-("aromatic acyl"oxy) "lower alkyl group", e.g., benzoyloxymethyl; a "carbonyloxyalkyl group" such as a (lower alkoxycarbonyloxy)alkyl group, e.g., methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, cyclohexyloxycarbonyloxymethyl, cyclohexyloxycarbonyloxy(cyclohexyl)methyl, 1-(methoxycarbonyloxy)ethyl, 1-(ethoxycarbonyloxy)ethyl, 1-(propoxycarbonyloxy)ethyl, 1-(isopropoxycarbonyloxy)ethyl, 1-(butoxycarbonyloxy)ethyl, 1-(isobutoxycarbonyloxy)ethyl, 1-(t-butoxycarbonyloxy)ethyl, 1-(pentyloxycarbonyloxy)ethyl, 1-(hexyloxycarbonyloxy)ethyl, 1-(cyclopentyloxycarbonyloxy) ethyl, 1-(cyclopentyloxycarbonyloxy)propyl, 1-(cyclohexyloxycarbonyloxy)propyl, 1-(cyclopentyloxycarbonyloxy)butyl, 1-(cylohexyloxycarbonyloxy)butyl, 1-(cyclohexyloxycarbonyloxy)ethyl, 1-(ethoxycarbonyloxy)propyl, 1-(methoxycarbonyloxy)propyl, 1-(ethoxycarbonyloxy)propyl, 1-(propoxycarbonyloxy)propyl, 1-(isoprnpoxycarbonyloxy)propyl, 1-(butoxycarbonyloxy)propyl, 1-(isobutoxycarbonyloxy)propyl, 1-(pentyloxycarbonyloxy)propyl, 1- (hexyloxycarbonyloxy) propyl, 1- (methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, 1-(isobutoxycarbonyloxy)butyl, 1-(methoxycarbonyloxy)pentyl, 1-(ethoxycarbonyloxy)pentyl, 1-(methoxycarbonyloxy)hexyl and 1-(ethoxycarbonyloxy)hexyl, or an oxodioxolenylmethyl group, e. g., (5-phenyl-2-oxo-1,3-dioxolen-4-yl)methyl,
[5-(4-methylphenyl)-2-oxo-1,3-dioxolen-4-yl]methyl,
[5-(4-methoxyphenyl-2-oxo-1,3-dioxolen-4-yl)methyl,
[5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)methyl, (5-methyl-2-oxo- l,3-dioxolen-4-yl)methyl, (5-ethyl-2-oxo-1,3-dioxolen-4-yl)methyl, (5-propyl-2-oxo-1,3-dioxolen-4-yl)methyl,
(5-isopropyl-2-oxo-1,3-dioxolen-4-yl)methyl and (5-butyl-2-oxo-1,3-dioxolen-4-yl)methyl; a "phthalidyl group" such as phthalidyl, dimethylphthalidyl and dimethoxyphthalidyl; the above "lower aliphatic acyl group"; the above "aromatic acyl group"; a "half ester salt residue of succinic acid"; a "phosphoric acid ester salt residue"; an "ester formation residue of an amino acid or the like"; a carbamoyl group; a carbamoyl group substituted by 1 or 2 lower alkyl groups; or a "1-(acyloxy)alkyloxycarbonyl group" such as pivaloyloxymethyloxycarbonyl; and is preferably a carbonyloxyalkyl group".

Further, even in the case where a compound (I) of the present invention has an amino group and/or a sulfonamide group, the compound can be made into a pharmacologically acceptable "derivative" by modifying the functional group. Such derivative is, for example, an amide derivative in which the above "aliphatic acyl group" or the above "aromatic acyl group" is bonded to the nitrogen atom of the amino group and/or the sulfonamide group possessed by the compound (I).

Where a compound (I) or a pharmacologically acceptable ester thereof or other pharmacologically acceptable derivative thereof has a basic group such as an amino group, the compound can be converted into a salt by reacting it with an acid, and in the case where a compound (I) or a pharmacologically acceptable ester thereof or other pharmacologically acceptable derivative thereof has an acidic group such as a hydroxyaryl group, the compound can be converted into a salt by reacting it with a base. The "pharmacologically acceptable salt thereof" indicates such salts.

The salt based on the basic group is preferably an inorganic acid salt such as a hydrohalide salt, e.g., hydrochloride, hydrobromide and hydroiodide, or nitrate, perchlorate, sulfate or phosphate; an organic acid salt such as a lower alkanesulfonate, e.g., methanesulfonate, trifluoromethanesulfonate and ethanesulfonate, an arylsulfonate, e.g., benzenesulfonate and p-toluenesulfonate, or acetate, malate, fumarate, succinate, citrate, ascorbate, tartrate, oxalate or maleate; or an amino acid salt such as glycinate, lysine salt, arginate, ornithinate, glutamate and aspartate.

On the other hand, the salt based on the acidic group is preferably a metal salt such as an alkali metal salt, e.g., sodium salt, potassium salt and lithium salt, an alkaline earth metal salt, e.g., calcium salt and magnesium salt, or aluminum salt or iron salt; an amine salt such as an inorganic salt, e.g. ammonium salt or an organic salt, e.g., t-octylamine salt, dibenzylamine salt, morpholine salt, glucosamate, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenethylamine salt, piperazine salt, tetramethylammonium salt and tris(hydroxymethyl)aminomethane salt; or an amino acid salt such as glycine salt, lysine salt, arginine salt, ornithine salt, glutamate and aspartate.

The compounds of general formula (I), or a pharmaceutically acceptable salt, ester or other derivative thereof of the present invention can sometimes take up water upon exposure to the atmosphere or when recrystallized to absorb water or to form a hydrate and such hydrates are also included within the scope of the present invention.

In the compounds of formula (I) of the present invention, geometrical isomers (cis-trans isomers or Z-E isomers) and optical isomers based on an asymmetric center in the molecule sometimes exist. In the compounds of the

present invention, all of these isomers and mixtures of these isomers are shown by a single formula, i.e., the formula (I), but the present invention includes all of these isomers and mixtures of these isomers in arbitrary ratio.

The compounds of the present invention are preferably the compounds shown in Table 1 to Table 4. The compounds of Table 1 to Table 4 have the formulae of compound (I-1) to the compound (I-4) respectively.

Table 1

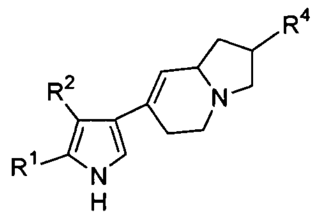

(I－1)

| Compound No. | $R^1$ | $R^2$ | $R^4$ |
|---|---|---|---|
| 1-1 | 3-F-Ph | 4-Pyr | 2-F-Ph |
| 1-2 | 3-F-Ph | 4-Pyr | 3-F-Ph |
| 1-3 | 3-F-Ph | 4-Pyr | 4-F-Ph |
| 1-4 | 3-F-Ph | 4-Pyr | 2,4-diF-Ph |
| 1-5 | 3-F-Ph | 4-Pyr | 3,4-diF-Ph |
| 1-6 | 3-F-Ph | 4-Pyr | 2-Cl-Ph |
| 1-7 | 3-F-Ph | 4-Pyr | 3-Cl-Ph |
| 1-8 | 3-F-Ph | 4-Pyr | 4-Cl-Ph |
| 1-9 | 3-F-Ph | 4-Pyr | 2,4-diCl-Ph |
| 1-10 | 3-F-Ph | 4-Pyr | 3,4-diCl-Ph |
| 1-11 | 3-F-Ph | 4-Pyr | 3-$CF_3$-Ph |
| 1-12 | 3-F-Ph | 4-Pyr | 4-$CF_3$-Ph |
| 1-13 | 3-F-Ph | 4-Pyr | 2-Me-Ph |
| 1-14 | 3-F-Ph | 4-Pyr | 3-Me-Ph |
| 1-15 | 3-F-Ph | 4-Pyr | 4-Me-Ph |
| 1-16 | 3-F-Ph | 4-Pyr | 3,4-diMe-Ph |
| 1-17 | 3-F-Ph | 4-Pyr | 3-Et-Ph |
| 1-18 | 3-F-Ph | 4-Pyr | 4-Et-Ph |
| 1-19 | 3-F-Ph | 4-Pyr | 3-Pr-Ph |
| 1-20 | 3-F-Ph | 4-Pyr | 4-Pr-Ph |
| 1-21 | 3-F-Ph | 4-Pyr | 3-Bu-Ph |
| 1-22 | 3-F-Ph | 4-Pyr | 4-Bu-Ph |
| 1-23 | 3-F-Ph | 4-Pyr | 4-tBu-Ph |
| 1-24 | 3-F-Ph | 4-Pyr | 3-Ph-Ph |
| 1-25 | 3-F-Ph | 4-Pyr | 4-Ph-Ph |
| 1-26 | 3-F-Ph | 4-Pyr | 2-MeO-Ph |

| | | | |
|---|---|---|---|
| 1-27 | 3-F-Ph | 4-Pyr | 3-MeO-Ph |
| 1-28 | 3-F-Ph | 4-Pyr | 4-MeO-Ph |
| 1-29 | 3-F-Ph | 4-Pyr | 3,4-diMeO-Ph |
| 1-30 | 3-F-Ph | 4-Pyr | 3-EtO-Ph |
| 1-31 | 3-F-Ph | 4-Pyr | 4-EtO-Ph |
| 1-32 | 3-F-Ph | 4-Pyr | 3-PrO-Ph |
| 1-33 | 3-F-Ph | 4-Pyr | 4-PrO-Ph |
| 1-34 | 3-F-Ph | 4-Pyr | 3-PhO-Ph |
| 1-35 | 3-F-Ph | 4-Pyr | 4-PhO-Ph |
| 1-36 | 3-F-Ph | 4-Pyr | 3-$OCHF_2$-Ph |
| 1-37 | 3-F-Ph | 4-Pyr | 4-$OCHF_2$-Ph |
| 1-38 | 3-F-Ph | 4-Pyr | 2-OH-Ph |
| 1-39 | 3-F-Ph | 4-Pyr | 3-OH-Ph |
| 1-40 | 3-F-Ph | 4-Pyr | 4-OH-Ph |
| 1-41 | 3-F-Ph | 4-Pyr | 3,4-diOH-Ph |
| 1-42 | 3-F-Ph | 4-Pyr | 3-$OCOCH_3$-Ph |
| 1-43 | 3-F-Ph | 4-Pyr | 3-$OCOC_2H_5$-Ph |
| 1-44 | 3-F-Ph | 4-Pyr | 3-$OCOC_3H_7$-Ph |
| 1-45 | 3-F-Ph | 4-Pyr | 4-$OCOCH_3$-Ph |
| 1-46 | 3-F-Ph | 4-Pyr | 4-$OCOC_2H_5$-Ph |
| 1-47 | 3-F-Ph | 4-Pyr | 4-$OCOC_3H_7$-Ph |
| 1-48 | 4-F-Ph | 4-Pyr | 2-F-Ph |
| 1-49 | 4-F-Ph | 4-Pyr | 3-F-Ph |
| 1-50 | 4-F-Ph | 4-Pyr | 3,4-diF-Ph |
| 1-51 | 4-F-Ph | 4-Pyr | 2-Cl-Ph |
| 1-52 | 4-F-Ph | 4-Pyr | 3-Cl-Ph |
| 1-53 | 4-F-Ph | 4-Pyr | 2,4-diCl-Ph |
| 1-54 | 4-F-Ph | 4-Pyr | 3,4-diCl-Ph |
| 1-55 | 4-F-Ph | 4-Pyr | 3-$CF_3$-Ph |
| 1-56 | 4-F-Ph | 4-Pyr | 2-Me-Ph |
| 1-57 | 4-F-Ph | 4-Pyr | 3-Me-Ph |
| 1-58 | 4-F-Ph | 4-Pyr | 3,4-diMe-Ph |
| 1-59 | 4-F-Ph | 4-Pyr | 3-Et-Ph |
| 1-60 | 4-F-Ph | 4-Pyr | 4-Et-Ph |
| 1-61 | 4-F-Ph | 4-Pyr | 3-Pr-Ph |
| 1-62 | 4-F-Ph | 4-Pyr | 4-Pr-Ph |

| | | | |
|---|---|---|---|
| 1-63 | 4-F-Ph | 4-Pyr | 3-Bu-Ph |
| 1-64 | 4-F-Ph | 4-Pyr | 4-Bu-Ph |
| 1-65 | 4-F-Ph | 4-Pyr | 4-tBu-Ph |
| 1-66 | 4-F-Ph | 4-Pyr | 3-Ph-Ph |
| 1-67 | 4-F-Ph | 4-Pyr | 4-Ph-Ph |
| 1-68 | 4-F-Ph | 4-Pyr | 2-MeO-Ph |
| 1-69 | 4-F-Ph | 4-Pyr | 3-MeO-Ph |
| 1-70 | 4-F-Ph | 4-Pyr | 3,4-diMeO-Ph |
| 1-71 | 4-F-Ph | 4-Pyr | 3-EtO-Ph |
| 1-72 | 4-F-Ph | 4-Pyr | 4-EtO-Ph |
| 1-73 | 4-F-Ph | 4-Pyr | 3-PrO-Ph |
| 1-74 | 4-F-Ph | 4-Pyr | 4-PrO-Ph |
| 1-75 | 4-F-Ph | 4-Pyr | 3-PhO-Ph |
| 1-76 | 4-F-Ph | 4-Pyr | 4-PhO-Ph |
| 1-77 | 4-F-Ph | 4-Pyr | 3-$OCHF_2$-Ph |
| 1-78 | 4-F-Ph | 4-Pyr | 4-$OCHF_2$-Ph |
| 1-79 | 4-F-Ph | 4-Pyr | 2-OH-Ph |
| 1-80 | 4-F-Ph | 4-Pyr | 3-OH-Ph |
| 1-81 | 4-F-Ph | 4-Pyr | 4-OH-Ph |
| 1-82 | 4-F-Ph | 4-Pyr | 3,4-diOH-Ph |
| 1-83 | 4-F-Ph | 4-Pyr | 3-$OCOCH_3$-Ph |
| 1-84 | 4-F-Ph | 4-Pyr | 3-$OCOC_2H_5$-Ph |
| 1-85 | 4-F-Ph | 4-Pyr | 3-$OCOC_3H_7$-Ph |
| 1-86 | 4-F-Ph | 4-Pyr | 4-$OCOCH_3$-Ph |
| 1-87 | 4-F-Ph | 4-Pyr | 4-$OCOC_2H_5$-Ph |
| 1-88 | 4-F-Ph | 4-Pyr | 4-$OCOC_3H_7$-Ph |
| 1-89 | 3-Cl-Ph | 4-Pyr | 2-F-Ph |
| 1-90 | 3-Cl-Ph | 4-Pyr | 3-F-Ph |
| 1-91 | 3-Cl-Ph | 4-Pyr | 4-F-Ph |
| 1-92 | 3-Cl-Ph | 4-Pyr | 2,4-diF-Ph |
| 1-93 | 3-Cl-Ph | 4-Pyr | 3,4-diF-Ph |
| 1-94 | 3-Cl-Ph | 4-Pyr | 2-Cl-Ph |
| 1-95 | 3-Cl-Ph | 4-Pyr | 3-Cl-Ph |
| 1-96 | 3-Cl-Ph | 4-Pyr | 4-Cl-Ph |
| 1-97 | 3-Cl-Ph | 4-Pyr | 2,4-diCl-Ph |
| 1-98 | 3-Cl-Ph | 4-Pyr | 3,4-diCl-Ph |

| | | | |
|---|---|---|---|
| 1-99 | 3-Cl-Ph | 4-Pyr | 3-$CF_3$-Ph |
| 1-100 | 3-Cl-Ph | 4-Pyr | 4-$CF_3$-Ph |
| 1-101 | 3-Cl-Ph | 4-Pyr | 2-Me-Ph |
| 1-102 | 3-Cl-Ph | 4-Pyr | 3-Me-Ph |
| 1-103 | 3-Cl-Ph | 4-Pyr | 4-Me-Ph |
| 1-104 | 3-Cl-Ph | 4-Pyr | 3,4-diMe-Ph |
| 1-105 | 3-Cl-Ph | 4-Pyr | 3-Et-Ph |
| 1-106 | 3-Cl-Ph | 4-Pyr | 4-Et-Ph |
| 1-107 | 3-Cl-Ph | 4-Pyr | 3-Pr-Ph |
| 1-108 | 3-Cl-Ph | 4-Pyr | 4-Pr-Ph |
| 1-109 | 3-Cl-Ph | 4-Pyr | 3-Bu-Ph |
| 1-110 | 3-Cl-Ph | 4-Pyr | 4-Bu-Ph |
| 1-111 | 3-Cl-Ph | 4-Pyr | 4-tBu-Ph |
| 1-112 | 3-Cl-Ph | 4-Pyr | 3-Ph-Ph |
| 1-113 | 3-Cl-Ph | 4-Pyr | 4-Ph-Ph |
| 1-114 | 3-Cl-Ph | 4-Pyr | 2-MeO-Ph |
| 1-115 | 3-Cl-Ph | 4-Pyr | 3-MeO-Ph |
| 1-116 | 3-Cl-Ph | 4-Pyr | 4-MeO-Ph |
| 1-117 | 3-Cl-Ph | 4-Pyr | 3,4-diMeO-Ph |
| 1-118 | 3-Cl-Ph | 4-Pyr | 3-EtO-Ph |
| 1-119 | 3-Cl-Ph | 4-Pyr | 4-EtO-Ph |
| 1-120 | 3-Cl-Ph | 4-Pyr | 3-PrO-Ph |
| 1-121 | 3-Cl-Ph | 4-Pyr | 4-PrO-Ph |
| 1-122 | 3-Cl-Ph | 4-Pyr | 3-PhO-Ph |
| 1-123 | 3-Cl-Ph | 4-Pyr | 4-PhO-Ph |
| 1-124 | 3-Cl-Ph | 4-Pyr | 3-$OCHF_2$-Ph |
| 1-125 | 3-Cl-Ph | 4-Pyr | 4-$OCHF_2$-Ph |
| 1-126 | 3-Cl-Ph | 4-Pyr | 2-OH-Ph |
| 1-127 | 3-Cl-Ph | 4-Pyr | 3-OH-Ph |
| 1-128 | 3-Cl-Ph | 4-Pyr | 4-OH-Ph |
| 1-129 | 3-Cl-Ph | 4-Pyr | 3,4-diOH-Ph |
| 1-130 | 3-Cl-Ph | 4-Pyr | 3-$OCOCH_3$-Ph |
| 1-131 | 3-Cl-Ph | 4-Pyr | 3-$OCOC_2H_5$-Ph |
| 1-132 | 3-Cl-Ph | 4-Pyr | 3-$OCOC_3H_7$-Ph |
| 1-133 | 3-Cl-Ph | 4-Pyr | 4-$OCOCH_3$-Ph |
| 1-134 | 3-Cl-Ph | 4-Pyr | 4-$OCOC_2H_5$-Ph |

| | | | |
|---|---|---|---|
| 1-135 | 3-Cl-Ph | 4-Pyr | 4-$OCOC_3H_7$-Ph |
| 1-136 | 3-$CF_3$-Ph | 4-Pyr | 2-F-Ph |
| 1-137 | 3-$CF_3$-Ph | 4-Pyr | 3-F-Ph |
| 1-138 | 3-$CF_3$-Ph | 4-Pyr | 3,4-diF-Ph |
| 1-139 | 3-$CF_3$-Ph | 4-Pyr | 2-Cl-Ph |
| 1-140 | 3-$CF_3$-Ph | 4-Pyr | 3-Cl-Ph |
| 1-141 | 3-$CF_3$-Ph | 4-Pyr | 2,4-diCl-Ph |
| 1-142 | 3-$CF_3$-Ph | 4-Pyr | 3,4-diCl-Ph |
| 1-143 | 3-$CF_3$-Ph | 4-Pyr | 3-$CF_3$-Ph |
| 1-144 | 3-$CF_3$-Ph | 4-Pyr | 2-Me-Ph |
| 1-145 | 3-$CF_3$-Ph | 4-Pyr | 3-Me-Ph |
| 1-146 | 3-$CF_3$-Ph | 4-Pyr | 3,4-diMe-Ph |
| 1-147 | 3-$CF_3$-Ph | 4-Pyr | 3-Et-Ph |
| 1-148 | 3-$CF_3$-Ph | 4-Pyr | 4-Et-Ph |
| 1-149 | 3-$CF_3$-Ph | 4-Pyr | 3-Pr-Ph |
| 1-150 | 3-$CF_3$-Ph | 4-Pyr | 4-Pr-Ph |
| 1-151 | 3-$CF_3$-Ph | 4-Pyr | 3-Bu-Ph |
| 1-152 | 3-$CF_3$-Ph | 4-Pyr | 4-Bu-Ph |
| 1-153 | 3-$CF_3$-Ph | 4-Pyr | 4-tBu-Ph |
| 1-154 | 3-$CF_3$-Ph | 4-Pyr | 3-Ph-Ph |
| 1-155 | 3-$CF_3$-Ph | 4-Pyr | 4-Ph-Ph |
| 1-156 | 3-$CF_3$-Ph | 4-Pyr | 2-MeO-Ph |
| 1-157 | 3-$CF_3$-Ph | 4-Pyr | 3-MeO-Ph |
| 1-158 | 3-$CF_3$-Ph | 4-Pyr | 3,4-diMeO-Ph |
| 1-159 | 3-$CF_3$-Ph | 4-Pyr | 3-EtO-Ph |
| 1-160 | 3-$CF_3$-Ph | 4-Pyr | 4-EtO-Ph |
| 1-161 | 3-$CF_3$-Ph | 4-Pyr | 3-PrO-Ph |
| 1-162 | 3-$CF_3$-Ph | 4-Pyr | 4-PrO-Ph |
| 1-163 | 3-$CF_3$-Ph | 4-Pyr | 3-PhO-Ph |
| 1-164 | 3-$CF_3$-Ph | 4-Pyr | 4-PhO-Ph |
| 1-165 | 3-$CF_3$-Ph | 4-Pyr | 3-$OCHF_2$-Ph |
| 1-166 | 3-$CF_3$-Ph | 4-Pyr | 4-$OCHF_2$-Ph |
| 1-167 | 3-$CF_3$-Ph | 4-Pyr | 2-OH-Ph |
| 1-168 | 3-$CF_3$-Ph | 4-Pyr | 3-OH-Ph |
| 1-169 | 3-$CF_3$-Ph | 4-Pyr | 4-OH-Ph |
| 1-170 | 3-$CF_3$-Ph | 4-Pyr | 3,4-diOH-Ph |

| | | | |
|---|---|---|---|
| 1-171 | 3-$CF_3$-Ph | 4-Pyr | 3-$OCOCH_3$-Ph |
| 1-172 | 3-$CF_3$-Ph | 4-Pyr | 3-$OCOC_2H_5$-Ph |
| 1-173 | 3-$CF_3$-Ph | 4-Pyr | 3-$OCOC_3H_7$-Ph |
| 1-174 | 3-$CF_3$-Ph | 4-Pyr | 4-$OCOCH_3$-Ph |
| 1-175 | 3-$CF_3$-Ph | 4-Pyr | 4-$OCOC_2H_5$-Ph |
| 1-176 | 3-$CF_3$-Ph | 4-Pyr | 4-$OCOC_3H_7$-Ph |
| 1-177 | 3,4-diF-Ph | 4-Pyr | 2-F-Ph |
| 1-178 | 3,4-diF-Ph | 4-Pyr | 3-F-Ph |
| 1-179 | 3,4-diF-Ph | 4-Pyr | 4-F-Ph |
| 1-180 | 3,4-diF-Ph | 4-Pyr | 2,4-diF-Ph |
| 1-181 | 3,4-diF-Ph | 4-Pyr | 3,4-diF-Ph |
| 1-182 | 3,4-diF-Ph | 4-Pyr | 2-Cl-Ph |
| 1-183 | 3,4-diF-Ph | 4-Pyr | 3-Cl-Ph |
| 1-184 | 3,4-diF-Ph | 4-Pyr | 4-Cl-Ph |
| 1-185 | 3,4-diF-Ph | 4-Pyr | 2,4-diCl-Ph |
| 1-186 | 3,4-diF-Ph | 4-Pyr | 3,4-diCl-Ph |
| 1-187 | 3,4-diF-Ph | 4-Pyr | 3-$CF_3$-Ph |
| 1-188 | 3,4-diF-Ph | 4-Pyr | 4-$CF_3$-Ph |
| 1-189 | 3,4-diF-Ph | 4-Pyr | 2-Me-Ph |
| 1-190 | 3,4-diF-Ph | 4-Pyr | 3-Me-Ph |
| 1-191 | 3,4-diF-Ph | 4-Pyr | 4-Me-Ph |
| 1-192 | 3,4-diF-Ph | 4-Pyr | 3,4-diMe-Ph |
| 1-193 | 3,4-diF-Ph | 4-Pyr | 3-Et-Ph |
| 1-194 | 3,4-diF-Ph | 4-Pyr | 4-Et-Ph |
| 1-195 | 3,4-diF-Ph | 4-Pyr | 3-Pr-Ph |
| 1-196 | 3,4-diF-Ph | 4-Pyr | 4-Pr-Ph |
| 1-197 | 3,4-diF-Ph | 4-Pyr | 3-Bu-Ph |
| 1-198 | 3,4-diF-Ph | 4-Pyr | 4-Bu-Ph |
| 1-199 | 3,4-diF-Ph | 4-Pyr | 4-tBu-Ph |
| 1-200 | 3,4-diF-Ph | 4-Pyr | 3-Ph-Ph |
| 1-201 | 3,4-diF-Ph | 4-Pyr | 4-Ph-Ph |
| 1-202 | 3,4-diF-Ph | 4-Pyr | 2-MeO-Ph |
| 1-203 | 3,4-diF-Ph | 4-Pyr | 3-MeO-Ph |
| 1-204 | 3,4-diF-Ph | 4-Pyr | 4-MeO-Ph |
| 1-205 | 3,4-diF-Ph | 4-Pyr | 3,4-diMeO-Ph |
| 1-206 | 3,4-diF-Ph | 4-Pyr | 3-EtO-Ph |

| | | | |
|---|---|---|---|
| 1-207 | 3,4-diF-Ph | 4-Pyr | 4-EtO-Ph |
| 1-208 | 3,4-diF-Ph | 4-Pyr | 3-PrO-Ph |
| 1-209 | 3,4-diF-Ph | 4-Pyr | 4-PrO-Ph |
| 1-210 | 3,4-diF-Ph | 4-Pyr | 3-PhO-Ph |
| 1-211 | 3,4-diF-Ph | 4-Pyr | 4-PhO-Ph |
| 1-212 | 3,4-diF-Ph | 4-Pyr | 3-$OCHF_2$-Ph |
| 1-213 | 3,4-diF-Ph | 4-Pyr | 4-$OCHF_2$-Ph |
| 1-214 | 3,4-diF-Ph | 4-Pyr | 2-OH-Ph |
| 1-215 | 3,4-diF-Ph | 4-Pyr | 3-OH-Ph |
| 1-216 | 3,4-diF-Ph | 4-Pyr | 4-OH-Ph |
| 1-217 | 3,4-diF-Ph | 4-Pyr | 3,4-diOH-Ph |
| 1-218 | 3,4-diF-Ph | 4-Pyr | 3-$OCOCH_3$-Ph |
| 1-219 | 3,4-diF-Ph | 4-Pyr | 3-$OCOC_2H_5$-Ph |
| 1-220 | 3,4-diF-Ph | 4-Pyr | 3-$OCOC_3H_7$-Ph |
| 1-221 | 3,4-diF-Ph | 4-Pyr | 4-$OCOCH_3$-Ph |
| 1-222 | 3,4-diF-Ph | 4-Pyr | 4-$OCOC_2H_5$-Ph |
| 1-223 | 3,4-diF-Ph | 4-Pyr | 4-$OCOC_3H_7$-Ph |
| 1-224 | 3-MeO-Ph | 4-Pyr | 2-F-Ph |
| 1-225 | 3-MeO-Ph | 4-Pyr | 3-F-Ph |
| 1-226 | 3-MeO-Ph | 4-Pyr | 4-F-Ph |
| 1-227 | 3-MeO-Ph | 4-Pyr | 2,4-diF-Ph |
| 1-228 | 3-MeO-Ph | 4-Pyr | 3,4-diF-Ph |
| 1-229 | 3-MeO-Ph | 4-Pyr | 2-Me-Ph |
| 1-230 | 3-MeO-Ph | 4-Pyr | 3-Me-Ph |
| 1-231 | 3-MeO-Ph | 4-Pyr | 4-Me-Ph |
| 1-232 | 3-MeO-Ph | 4-Pyr | 3,4-diMe-Ph |
| 1-233 | 3-MeO-Ph | 4-Pyr | 3-Et-Ph |
| 1-234 | 3-MeO-Ph | 4-Pyr | 4-Et-Ph |
| 1-235 | 3-MeO-Ph | 4-Pyr | 4-tBu-Ph |
| 1-236 | 3-MeO-Ph | 4-Pyr | 2-MeO-Ph |
| 1-237 | 3-MeO-Ph | 4-Pyr | 3-MeO-Ph |
| 1-238 | 3-MeO-Ph | 4-Pyr | 4-MeO-Ph |
| 1-239 | 3-MeO-Ph | 4-Pyr | 3,4-diMeO-Ph |
| 1-240 | 3-MeO-Ph | 4-Pyr | 2-OH-Ph |
| 1-241 | 3-MeO-Ph | 4-Pyr | 3-OH-Ph |
| 1-242 | 3-MeO-Ph | 4-Pyr | 4-OH-Ph |

| | | | |
|---|---|---|---|
| 1-243 | 3-MeO-Ph | 4-Pyr | 3,4-diOH-Ph |
| 1-244 | 3-MeO-Ph | 4-Pyr | 3-$OCOCH_3$-Ph |
| 1-245 | 3-MeO-Ph | 4-Pyr | 3-$OCOC_2H_5$-Ph |
| 1-246 | 3-MeO-Ph | 4-Pyr | 3-$OCOC_3H_7$-Ph |
| 1-247 | 3-MeO-Ph | 4-Pyr | 4-$OCOCH_3$-Ph |
| 1-248 | 3-MeO-Ph | 4-Pyr | 4-$OCOC_2H_5$-Ph |
| 1-249 | 3-MeO-Ph | 4-Pyr | 4-$OCOC_3H_7$-Ph |
| 1-250 | 3-MeO-Ph | 4-Pyr | Ph |
| 1-251 | 3-MeO-Ph | 4-Pyr | Me |
| 1-252 | 3-MeO-Ph | 4-Pyr | Et |
| 1-253 | 3-MeO-Ph | 4-Pyr | Pr |
| 1-254 | 3-MeO-Ph | 4-Pyr | Bu |
| 1-255 | 3-MeO-Ph | 4-Pyr | OH |
| 1-256 | 3-MeO-Ph | 4-Pyr | MeO |
| 1-257 | 3-MeO-Ph | 4-Pyr | EtO |
| 1-258 | 3-CN-Ph | 4-Pyr | 2-F-Ph |
| 1-259 | 3-CN-Ph | 4-Pyr | 3-F-Ph |
| 1-260 | 3-CN-Ph | 4-Pyr | 4-F-Ph |
| 1-261 | 3-CN-Ph | 4-Pyr | 2,4-diF-Ph |
| 1-262 | 3-CN-Ph | 4-Pyr | 3,4-diF-Ph |
| 1-263 | 3-CN-Ph | 4-Pyr | 2-Me-Ph |
| 1-264 | 3-CN-Ph | 4-Pyr | 3-Me-Ph |
| 1-265 | 3-CN-Ph | 4-Pyr | 4-Me-Ph |
| 1-266 | 3-CN-Ph | 4-Pyr | 3,4-diMe-Ph |
| 1-267 | 3-CN-Ph | 4-Pyr | 3-Et-Ph |
| 1-268 | 3-CN-Ph | 4-Pyr | 4-Et-Ph |
| 1-269 | 3-CN-Ph | 4-Pyr | 4-tBu-Ph |
| 1-270 | 3-CN-Ph | 4-Pyr | 2-MeO-Ph |
| 1-271 | 3-CN-Ph | 4-Pyr | 3-MeO-Ph |
| 1-272 | 3-CN-Ph | 4-Pyr | 4-MeO-Ph |
| 1-273 | 3-CN-Ph | 4-Pyr | 3,4-diMeO-Ph |
| 1-274 | 3-CN-Ph | 4-Pyr | 2-OH-Ph |
| 1-275 | 3-CN-Ph | 4-Pyr | 3-OH-Ph |
| 1-276 | 3-CN-Ph | 4-Pyr | 4-OH-Ph |
| 1-277 | 3-CN-Ph | 4-Pyr | 3,4-diOH-Ph |
| 1-278 | 3-CN-Ph | 4-Pyr | 3-$OCOCH_3$-Ph |

| | | | |
|---|---|---|---|
| 1-279 | 3-CN-Ph | 4-Pyr | 3-$OCOC_2H_5$-Ph |
| 1-280 | 3-CN-Ph | 4-Pyr | 3-$OCOC_3H_7$-Ph |
| 1-281 | 3-CN-Ph | 4-Pyr | 4-$OCOCH_3$-Ph |
| 1-282 | 3-CN-Ph | 4-Pyr | 4-$OCOC_2H_5$-Ph |
| 1-283 | 3-CN-Ph | 4-Pyr | 4-$OCOC_3H_7$-Ph |
| 1-284 | 3-CN-Ph | 4-Pyr | Ph |
| 1-285 | 3-CN-Ph | 4-Pyr | Me |
| 1-286 | 3-CN-Ph | 4-Pyr | Et |
| 1-287 | 3-CN-Ph | 4-Pyr | Pr |
| 1-288 | 3-CN-Ph | 4-Pyr | Bu |
| 1-289 | 3-CN-Ph | 4-Pyr | OH |
| 1-290 | 3-CN-Ph | 4-Pyr | MeO |
| 1-291 | 3-CN-Ph | 4-Pyr | EtO |
| 1-292 | 4-F-3-MeO-Ph | 4-Pyr | 2-F-Ph |
| 1-293 | 4-F-3-MeO-Ph | 4-Pyr | 3-F-Ph |
| 1-294 | 4-F-3-MeO-Ph | 4-Pyr | 4-F-Ph |
| 1-295 | 4-F-3-MeO-Ph | 4-Pyr | 2,4-diF-Ph |
| 1-296 | 4-F-3-MeO-Ph | 4-Pyr | 3,4-diF-Ph |
| 1-297 | 4-F-3-MeO-Ph | 4-Pyr | 2-Me-Ph |
| 1-298 | 4-F-3-MeO-Ph | 4-Pyr | 3-Me-Ph |
| 1-299 | 4-F-3-MeO-Ph | 4-Pyr | 4-Me-Ph |
| 1-300 | 4-F-3-MeO-Ph | 4-Pyr | 3,4-diMe-Ph |
| 1-301 | 4-F-3-MeO-Ph | 4-Pyr | 3-Et-Ph |
| 1-302 | 4-F-3-MeO-Ph | 4-Pyr | 4-Et-Ph |
| 1-303 | 4-F-3-MeO-Ph | 4-Pyr | 4-tBu-Ph |
| 1-304 | 4-F-3-MeO-Ph | 4-Pyr | 2-MeO-Ph |
| 1-305 | 4-F-3-MeO-Ph | 4-Pyr | 3-MeO-Ph |
| 1-306 | 4-F-3-MeO-Ph | 4-Pyr | 4-MeO-Ph |
| 1-307 | 4-F-3-MeO-Ph | 4-Pyr | 3,4-diMeO-Ph |
| 1-308 | 4-F-3-MeO-Ph | 4-Pyr | 2-OH-Ph |
| 1-309 | 4-F-3-MeO-Ph | 4-Pyr | 3-OH-Ph |
| 1-310 | 4-F-3-MeO-Ph | 4-Pyr | 4-OH-Ph |
| 1-311 | 4-F-3-MeO-Ph | 4-Pyr | 3,4-diOH-Ph |
| 1-312 | 4-F-3-MeO-Ph | 4-Pyr | 3-$OCOCH_3$-Ph |
| 1-313 | 4-F-3-MeO-Ph | 4-Pyr | 3-$OCOC_2H_5$-Ph |
| 1-314 | 4-F-3-MeO-Ph | 4-Pyr | 3-$OCOC_3H_7$-Ph |

| | | | |
|---|---|---|---|
| 1-315 | 4-F-3-MeO-Ph | 4-Pyr | 4-$OCOCH_3$-Ph |
| 1-316 | 4-F-3-MeO-Ph | 4-Pyr | 4-$OCOC_2H_5$-Ph |
| 1-317 | 4-F-3-MeO-Ph | 4-Pyr | 4-$OCOC_3H_7$-Ph |
| 1-318 | 4-F-3-MeO-Ph | 4-Pyr | Ph |
| 1-319 | 4-F-3-MeO-Ph | 4-Pyr | Me |
| 1-320 | 4-F-3-MeO-Ph | 4-Pyr | Et |
| 1-321 | 4-F-3-MeO-Ph | 4-Pyr | Pr |
| 1-322 | 4-F-3-MeO-Ph | 4-Pyr | Bu |
| 1-323 | 4-F-3-MeO-Ph | 4-Pyr | OH |
| 1-324 | 4-F-3-MeO-Ph | 4-Pyr | MeO |
| 1-325 | 4-F-3-MeO-Ph | 4-Pyr | EtO |
| 1-326 | 2,4-diF-Ph | 4-Pyr | 2-F-Ph |
| 1-327 | 2,4-diF-Ph | 4-Pyr | 3-F-Ph |
| 1-328 | 2,4-diF-Ph | 4-Pyr | 4-F-Ph |
| 1-329 | 2,4-diF-Ph | 4-Pyr | 2,4-diF-Ph |
| 1-330 | 2,4-diF-Ph | 4-Pyr | 3,4-diF-Ph |
| 1-331 | 2,4-diF-Ph | 4-Pyr | 2-Me-Ph |
| 1-332 | 2,4-diF-Ph | 4-Pyr | 3-Me-Ph |
| 1-333 | 2,4-diF-Ph | 4-Pyr | 4-Me-Ph |
| 1-334 | 2,4-diF-Ph | 4-Pyr | 3,4-diMe-Ph |
| 1-335 | 2,4-diF-Ph | 4-Pyr | 3-Et-Ph |
| 1-336 | 2,4-diF-Ph | 4-Pyr | 4-Et-Ph |
| 1-337 | 2,4-diF-Ph | 4-Pyr | 4-tBu-Ph |
| 1-338 | 2,4-diF-Ph | 4-Pyr | 2-MeO-Ph |
| 1-339 | 2,4-diF-Ph | 4-Pyr | 3-MeO-Ph |
| 1-340 | 2,4-diF-Ph | 4-Pyr | 4-MeO-Ph |
| 1-341 | 2,4-diF-Ph | 4-Pyr | 3,4-diMeO-Ph |
| 1-342 | 2,4-diF-Ph | 4-Pyr | 2-OH-Ph |
| 1-343 | 2,4-diF-Ph | 4-Pyr | 3-OH-Ph |
| 1-344 | 2,4-diF-Ph | 4-Pyr | 4-OH-Ph |
| 1-345 | 2,4-diF-Ph | 4-Pyr | 3,4-diOH-Ph |
| 1-346 | 2,4-diF-Ph | 4-Pyr | 3-$OCOCH_3$-Ph |
| 1-347 | 2,4-diF-Ph | 4-Pyr | 3-$OCOC_2H_5$-Ph |
| 1-348 | 2,4-diF-Ph | 4-Pyr | 3-$OCOC_3H_7$-Ph |
| 1-349 | 2,4-diF-Ph | 4-Pyr | 4-$OCOCH_3$-Ph |
| 1-350 | 2,4-diF-Ph | 4-Pyr | 4-$OCOC_2H_5$-Ph |

| | | | |
|---|---|---|---|
| 1-351 | 2,4-diF-Ph | 4-Pyr | 4-$OCOC_3H_7$-Ph |
| 1-352 | 2,4-diF-Ph | 4-Pyr | Ph |
| 1-353 | 2,4-diF-Ph | 4-Pyr | Me |
| 1-354 | 2,4-diF-Ph | 4-Pyr | Et |
| 1-355 | 2,4-diF-Ph | 4-Pyr | Pr |
| 1-356 | 2,4-diF-Ph | 4-Pyr | Bu |
| 1-357 | 2,4-diF-Ph | 4-Pyr | OH |
| 1-358 | 2,4-diF-Ph | 4-Pyr | MeO |
| 1-359 | 2,4-diF-Ph | 4-Pyr | EtO |
| 1-360 | 3,4,5-triF-Ph | 4-Pyr | 2-F-Ph |
| 1-361 | 3,4,5-triF-Ph | 4-Pyr | 3-F-Ph |
| 1-362 | 3,4,5-triF-Ph | 4-Pyr | 4-F-Ph |
| 1-363 | 3,4,5-triF-Ph | 4-Pyr | 2,4-diF-Ph |
| 1-364 | 3,4,5-triF-Ph | 4-Pyr | 3,4-diF-Ph |
| 1-365 | 3,4,5-triF-Ph | 4-Pyr | 2-Me-Ph |
| 1-366 | 3,4,5-triF-Ph | 4-Pyr | 3-Me-Ph |
| 1-367 | 3,4,5-triF-Ph | 4-Pyr | 4-Me-Ph |
| 1-368 | 3,4,5-triF-Ph | 4-Pyr | 3,4-diMe-Ph |
| 1-369 | 3,4,5-triF-Ph | 4-Pyr | 3-Et-Ph |
| 1-370 | 3,4,5-triF-Ph | 4-Pyr | 4-Et-Ph |
| 1-371 | 3,4,5-triF-Ph | 4-Pyr | 4-tBu-Ph |
| 1-372 | 3,4,5-triF-Ph | 4-Pyr | 2-MeO-Ph |
| 1-373 | 3,4,5-triF-Ph | 4-Pyr | 3-MeO-Ph |
| 1-374 | 3,4,5-triF-Ph | 4-Pyr | 4-MeO-Ph |
| 1-375 | 3,4,5-triF-Ph | 4-Pyr | 3,4-diMeO-Ph |
| 1-376 | 3,4,5-triF-Ph | 4-Pyr | 2-OH-Ph |
| 1-377 | 3,4,5-triF-Ph | 4-Pyr | 3-OH-Ph |
| 1-378 | 3,4,5-triF-Ph | 4-Pyr | 4-OH-Ph |
| 1-379 | 3,4,5-triF-Ph | 4-Pyr | 3,4-diOH-Ph |
| 1-380 | 3,4,5-triF-Ph | 4-Pyr | 3-$OCOCH_3$-Ph |
| 1-381 | 3,4,5-triF-Ph | 4-Pyr | 3-$OCOC_2H_5$-Ph |
| 1-382 | 3,4,5-triF-Ph | 4-Pyr | 3-$OCOC_3H_7$-Ph |
| 1-383 | 3,4,5-triF-Ph | 4-Pyr | 4-$OCOCH_3$-Ph |
| 1-384 | 3,4,5-triF-Ph | 4-Pyr | 4-$OCOC_2H_5$-Ph |
| 1-385 | 3,4,5-triF-Ph | 4-Pyr | 4-$OCOC_3H_7$-Ph |
| 1-386 | 3,4,5-triF-Ph | 4-Pyr | Ph |

| | | | |
|---|---|---|---|
| 1-387 | 3,4,5-triF-Ph | 4-Pyr | Me |
| 1-388 | 3,4,5-triF-Ph | 4-Pyr | Et |
| 1-389 | 3,4,5-triF-Ph | 4-Pyr | Pr |
| 1-390 | 3,4,5-triF-Ph | 4-Pyr | Bu |
| 1-391 | 3,4,5-triF-Ph | 4-Pyr | OH |
| 1-392 | 3,4,5-triF-Ph | 4-Pyr | MeO |
| 1-393 | 3,4,5-triF-Ph | 4-Pyr | EtO |
| 1-394 | 3-Cl-4-F-Ph | 4-Pyr | 2-F-Ph |
| 1-395 | 3-Cl-4-F-Ph | 4-Pyr | 3-F-Ph |
| 1-396 | 3-Cl-4-F-Ph | 4-Pyr | 4-F-Ph |
| 1-397 | 3-Cl-4-F-Ph | 4-Pyr | 2,4-diF-Ph |
| 1-398 | 3-Cl-4-F-Ph | 4-Pyr | 3,4-diF-Ph |
| 1-399 | 3-Cl-4-F-Ph | 4-Pyr | 2-Me-Ph |
| 1-400 | 3-Cl-4-F-Ph | 4-Pyr | 3-Me-Ph |
| 1-401 | 3-Cl-4-F-Ph | 4-Pyr | 4-Me-Ph |
| 1-402 | 3-Cl-4-F-Ph | 4-Pyr | 3,4-diMe-Ph |
| 1-403 | 3-Cl-4-F-Ph | 4-Pyr | 3-Et-Ph |
| 1-404 | 3-Cl-4-F-Ph | 4-Pyr | 4-Et-Ph |
| 1-405 | 3-Cl-4-F-Ph | 4-Pyr | 4-tBu-Ph |
| 1-406 | 3-Cl-4-F-Ph | 4-Pyr | 2-MeO-Ph |
| 1-407 | 3-Cl-4-F-Ph | 4-Pyr | 3-MeO-Ph |
| 1-408 | 3-Cl-4-F-Ph | 4-Pyr | 4-MeO-Ph |
| 1-409 | 3-Cl-4-F-Ph | 4-Pyr | 3,4-diMeO-Ph |
| 1-410 | 3-Cl-4-F-Ph | 4-Pyr | 2-OH-Ph |
| 1-411 | 3-Cl-4-F-Ph | 4-Pyr | 3-OH-Ph |
| 1-412 | 3-Cl-4-F-Ph | 4-Pyr | 4-OH-Ph |
| 1-413 | 3-Cl-4-F-Ph | 4-Pyr | 3,4-diOH-Ph |
| 1-414 | 3-Cl-4-F-Ph | 4-Pyr | 3-$OCOCH_3$-Ph |
| 1-415 | 3-Cl-4-F-Ph | 4-Pyr | 3-$OCOC_2H_5$-Ph |
| 1-416 | 3-Cl-4-F-Ph | 4-Pyr | 3-$OCOC_3H_7$-Ph |
| 1-417 | 3-Cl-4-F-Ph | 4-Pyr | 4-$OCOCH_3$-Ph |
| 1-418 | 3-Cl-4-F-Ph | 4-Pyr | 4-$OCOC_2H_5$-Ph |
| 1-419 | 3-Cl-4-F-Ph | 4-Pyr | 4-$OCOC_3H_7$-Ph |
| 1-420 | 3-Cl-4-F-Ph | 4-Pyr | Ph |
| 1-421 | 3-Cl-4-F-Ph | 4-Pyr | Me |
| 1-422 | 3-Cl-4-F-Ph | 4-Pyr | Et |

| | | | |
|---|---|---|---|
| 1-423 | 3-Cl-4-F-Ph | 4-Pyr | Pr |
| 1-424 | 3-Cl-4-F-Ph | 4-Pyr | Bu |
| 1-425 | 3-Cl-4-F-Ph | 4-Pyr | OH |
| 1-426 | 3-Cl-4-F-Ph | 4-Pyr | MeO |
| 1-427 | 3-Cl-4-F-Ph | 4-Pyr | EtO |
| 1-428 | 3-$OCHF_2$-Ph | 4-Pyr | 2-F-Ph |
| 1-429 | 3-$OCHF_2$-Ph | 4-Pyr | 3-F-Ph |
| 1-430 | 3-$OCHF_2$-Ph | 4-Pyr | 4-F-Ph |
| 1-431 | 3-$OCHF_2$-Ph | 4-Pyr | 2,4-diF-Ph |
| 1-432 | 3-$OCHF_2$-Ph | 4-Pyr | 3,4-diF-Ph |
| 1-433 | 3-$OCHF_2$-Ph | 4-Pyr | 2-Me-Ph |
| 1-434 | 3-$OCHF_2$-Ph | 4-Pyr | 3-Me-Ph |
| 1-435 | 3-$OCHF_2$-Ph | 4-Pyr | 4-Me-Ph |
| 1-436 | 3-$OCHF_2$-Ph | 4-Pyr | 3,4-diMe-Ph |
| 1-437 | 3-$OCHF_2$-Ph | 4-Pyr | 3-Et-Ph |
| 1-438 | 3-$OCHF_2$-Ph | 4-Pyr | 4-Et-Ph |
| 1-439 | 3-$OCHF_2$-Ph | 4-Pyr | 4-tBu-Ph |
| 1-440 | 3-$OCHF_2$-Ph | 4-Pyr | 2-MeO-Ph |
| 1-441 | 3-$OCHF_2$-Ph | 4-Pyr | 3-MeO-Ph |
| 1-442 | 3-$OCHF_2$-Ph | 4-Pyr | 4-MeO-Ph |
| 1-443 | 3-$OCHF_2$-Ph | 4-Pyr | 3,4-diMeO-Ph |
| 1-444 | 3-$OCHF_2$-Ph | 4-Pyr | 2-OH-Ph |
| 1-445 | 3-$OCHF_2$-Ph | 4-Pyr | 3-OH-Ph |
| 1-446 | 3-$OCHF_2$-Ph | 4-Pyr | 4-OH-Ph |
| 1-447 | 3-$OCHF_2$-Ph | 4-Pyr | 3,4-diOH-Ph |
| 1-448 | 3-$OCHF_2$-Ph | 4-Pyr | 3-$OCOCH_3$-Ph |
| 1-449 | 3-$OCHF_2$-Ph | 4-Pyr | 3-$OCOC_2H_5$-Ph |
| 1-450 | 3-$OCHF_2$-Ph | 4-Pyr | 3-$OCOC_3H_7$-Ph |
| 1-451 | 3-$OCHF_2$-Ph | 4-Pyr | 4-$OCOCH_3$-Ph |
| 1-452 | 3-$OCHF_2$-Ph | 4-Pyr | 4-$OCOC_2H_5$-Ph |
| 1-453 | 3-$OCHF_2$-Ph | 4-Pyr | 4-$OCOC_3H_7$-Ph |
| 1-454 | 3-$OCHF_2$-Ph | 4-Pyr | Ph |
| 1-455 | 3-$OCHF_2$-Ph | 4-Pyr | Me |
| 1-456 | 3-$OCHF_2$-Ph | 4-Pyr | Et |
| 1-457 | 3-$OCHF_2$-Ph | 4-Pyr | Pr |
| 1-458 | 3-$OCHF_2$-Ph | 4-Pyr | Bu |

| | | | |
|---|---|---|---|
| 1-459 | 3-$OCHF_2$-Ph | 4-Pyr | OH |
| 1-460 | 3-$OCHF_2$-Ph | 4-Pyr | MeO |
| 1-461 | 3-$OCHF_2$-Ph | 4-Pyr | EtO |
| 1-462 | Ph | 4-Pyr | $NH_2$ |
| 1-463 | Ph | 4-Pyr | $NHCOCH_3$ |
| 1-464 | Ph | 4-Pyr | $NHSO_2CH_3$ |
| 1-465 | 4-F-Ph | 4-Pyr | $NH_2$ |
| 1-466 | 4-F-Ph | 4-Pyr | $NHCOCH_3$ |
| 1-467 | 4-F-Ph | 4-Pyr | $NHSO_2CH_3$ |
| 1-468 | 3-F-Ph | 4-Pyr | $NH_2$ |
| 1-469 | 3-F-Ph | 4-Pyr | $NHCOCH_3$ |
| 1-470 | 3-F-Ph | 4-Pyr | $NHSO_2CH_3$ |
| 1-471 | 3-Cl-Ph | 4-Pyr | $NH_2$ |
| 1-472 | 3-Cl-Ph | 4-Pyr | $NHCOCH_3$ |
| 1-473 | 3-Cl-Ph | 4-Pyr | $NHSO_2CH_3$ |
| 1-474 | 3,4-diF-Ph | 4-Pyr | $NH_2$ |
| 1-475 | 3,4-diF-Ph | 4-Pyr | $NHCOCH_3$ |
| 1-476 | 3,4-diF-Ph | 4-Pyr | $NHSO_2CH_3$ |
| 1-477 | Ph | 4-Pyr | Ph |
| 1-478 | 4-F-Ph | 4-Pyr | Bu |
| 1-479 | 4-F-Ph | 4-Pyr | Ph |
| 1-480 | 4-F-Ph | 4-Pyr | 4-MeO-Ph |
| 1-481 | 4-F-Ph | 4-Pyr | 4-Me-Ph |
| 1-482 | 4-F-Ph | 4-Pyr | 4-F-Ph |
| 1-483 | 3-Cl-Ph | 4-Pyr | Ph |
| 1-484 | 3-$CF_3$-Ph | 4-Pyr | Ph |
| 1-485 | 3-$CF_3$-Ph | 4-Pyr | 4-MeO-Ph |
| 1-486 | 3-$CF_3$-Ph | 4-Pyr | 4-Me-Ph |
| 1-487 | 3,4-diF-Ph | 4-Pyr | Ph |

Table 2

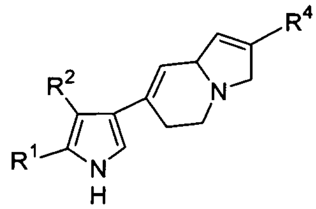

(I − 2)

| Compound No. | $R^1$ | $R^2$ | $R^4$ |
|---|---|---|---|
| 2-1 | 4-F-Ph | 4-Pyr | H |

Table 3

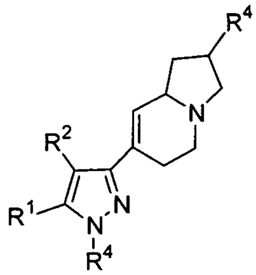

(I − 3)

| Compound No. | $R^1$ | $R^2$ | $R^4$ |
|---|---|---|---|
| 3-1 | 3-F-Ph | 4-Pyr | 2-F-Ph |
| 3-2 | 3-F-Ph | 4-Pyr | 3-F-Ph |
| 3-3 | 3-F-Ph | 4-Pyr | 4-F-Ph |
| 3-4 | 3-F-Ph | 4-Pyr | 2,4-diF-Ph |
| 3-5 | 3-F-Ph | 4-Pyr | 3,4-diF-Ph |
| 3-6 | 3-F-Ph | 4-Pyr | 2-Cl-Ph |
| 3-7 | 3-F-Ph | 4-Pyr | 3-Cl-Ph |
| 3-8 | 3-F-Ph | 4-Pyr | 4-Cl-Ph |
| 3-9 | 3-F-Ph | 4-Pyr | 2,4-diCl-Ph |
| 3-10 | 3-F-Ph | 4-Pyr | 3,4-diCl-Ph |
| 3-11 | 3-F-Ph | 4-Pyr | 3-$CF_3$-Ph |
| 3-12 | 3-F-Ph | 4-Pyr | 4-$CF_3$-Ph |
| 3-13 | 3-F-Ph | 4-Pyr | 2-Me-Ph |
| 3-14 | 3-F-Ph | 4-Pyr | 3-Me-Ph |

| | | | |
|---|---|---|---|
| 3-15 | 3-F-Ph | 4-Pyr | 4-Me-Ph |
| 3-16 | 3-F-Ph | 4-Pyr | 3,4-diMe-Ph |
| 3-17 | 3-F-Ph | 4-Pyr | 3-Et-Ph |
| 3-18 | 3-F-Ph | 4-Pyr | 4-Et-Ph |
| 3-19 | 3-F-Ph | 4-Pyr | 3-Pr-Ph |
| 3-20 | 3-F-Ph | 4-Pyr | 4-Pr-Ph |
| 3-21 | 3-F-Ph | 4-Pyr | 3-Bu-Ph |
| 3-22 | 3-F-Ph | 4-Pyr | 4-Bu-Ph |
| 3-23 | 3-F-Ph | 4-Pyr | 4-tBu-Ph |
| 3-24 | 3-F-Ph | 4-Pyr | 3-Ph-Ph |
| 3-25 | 3-F-Ph | 4-Pyr | 4-Ph-Ph |
| 3-26 | 3-F-Ph | 4-Pyr | 2-MeO-Ph |
| 3-27 | 3-F-Ph | 4-Pyr | 3-MeO-Ph |
| 3-28 | 3-F-Ph | 4-Pyr | 4-MeO-Ph |
| 3-29 | 3-F-Ph | 4-Pyr | 3,4-diMeO-Ph |
| 3-30 | 3-F-Ph | 4-Pyr | 3-EtO-Ph |
| 3-31 | 3-F-Ph | 4-Pyr | 4-EtO-Ph |
| 3-32 | 3-F-Ph | 4-Pyr | 3-PrO-Ph |
| 3-33 | 3-F-Ph | 4-Pyr | 4-PrO-Ph |
| 3-34 | 3-F-Ph | 4-Pyr | 3-PhO-Ph |
| 3-35 | 3-F-Ph | 4-Pyr | 4-PhO-Ph |
| 3-36 | 3-F-Ph | 4-Pyr | 3-$OCHF_2$-Ph |
| 3-37 | 3-F-Ph | 4-Pyr | 4-$OCHF_2$-Ph |
| 3-38 | 3-F-Ph | 4-Pyr | 2-OH-Ph |
| 3-39 | 3-F-Ph | 4-Pyr | 3-OH-Ph |
| 3-40 | 3-F-Ph | 4-Pyr | 4-OH-Ph |
| 3-41 | 3-F-Ph | 4-Pyr | 3,4-diOH-Ph |
| 3-42 | 3-F-Ph | 4-Pyr | 3-$OCOCH_3$-Ph |
| 3-43 | 3-F-Ph | 4-Pyr | 4-$OCOCH_3$-Ph |
| 3-44 | 3-F-Ph | 4-Pyr | 4-$OCOC_2H_5$-Ph |
| 3-45 | 3-F-Ph | 4-Pyr | 4-$OCOC_3H_7$-Ph |
| 3-46 | 4-F-Ph | 4-Pyr | 2-F-Ph |
| 3-47 | 4-F-Ph | 4-Pyr | 3-F-Ph |
| 3-48 | 4-F-Ph | 4-Pyr | 3,4-diF-Ph |
| 3-49 | 4-F-Ph | 4-Pyr | 2-Cl-Ph |
| 3-50 | 4-F-Ph | 4-Pyr | 3-Cl-Ph |

| | | | |
|---|---|---|---|
| 3-51 | 4-F-Ph | 4-Pyr | 2,4-diCl-Ph |
| 3-52 | 4-F-Ph | 4-Pyr | 3,4-diCl-Ph |
| 3-53 | 4-F-Ph | 4-Pyr | 3-$CF_3$-Ph |
| 3-54 | 4-F-Ph | 4-Pyr | 2-Me-Ph |
| 3-55 | 4-F-Ph | 4-Pyr | 3-Me-Ph |
| 3-56 | 4-F-Ph | 4-Pyr | 3,4-diMe-Ph |
| 3-57 | 4-F-Ph | 4-Pyr | 3-Et-Ph |
| 3-58 | 4-F-Ph | 4-Pyr | 4-Et-Ph |
| 3-59 | 4-F-Ph | 4-Pyr | 3-Pr-Ph |
| 3-60 | 4-F-Ph | 4-Pyr | 4-Pr-Ph |
| 3-61 | 4-F-Ph | 4-Pyr | 3-Bu-Ph |
| 3-62 | 4-F-Ph | 4-Pyr | 4-Bu-Ph |
| 3-63 | 4-F-Ph | 4-Pyr | 4-tBu-Ph |
| 3-64 | 4-F-Ph | 4-Pyr | 3-Ph-Ph |
| 3-65 | 4-F-Ph | 4-Pyr | 4-Ph-Ph |
| 3-66 | 4-F-Ph | 4-Pyr | 2-MeO-Ph |
| 3-67 | 4-F-Ph | 4-Pyr | 3-MeO-Ph |
| 3-68 | 4-F-Ph | 4-Pyr | 3,4-diMeO-Ph |
| 3-69 | 4-F-Ph | 4-Pyr | 3-EtO-Ph |
| 3-70 | 4-F-Ph | 4-Pyr | 4-EtO-Ph |
| 3-71 | 4-F-Ph | 4-Pyr | 3-PrO-Ph |
| 3-72 | 4-F-Ph | 4-Pyr | 4-PrO-Ph |
| 3-73 | 4-F-Ph | 4-Pyr | 3-PhO-Ph |
| 3-74 | 4-F-Ph | 4-Pyr | 4-PhO-Ph |
| 3-75 | 4-F-Ph | 4-Pyr | 3-$OCHF_2$-Ph |
| 3-76 | 4-F-Ph | 4-Pyr | 4-$OCHF_2$-Ph |
| 3-77 | 4-F-Ph | 4-Pyr | 2-OH-Ph |
| 3-78 | 4-F-Ph | 4-Pyr | 3-OH-Ph |
| 3-79 | 4-F-Ph | 4-Pyr | 4-OH-Ph |
| 3-80 | 4-F-Ph | 4-Pyr | 3,4-diOH-Ph |
| 3-81 | 4-F-Ph | 4-Pyr | 3-$OCOCH_3$-Ph |
| 3-82 | 4-F-Ph | 4-Pyr | 4-$OCOCH_3$-Ph |
| 3-83 | 4-F-Ph | 4-Pyr | 4-$OCOC_2H_5$-Ph |
| 3-84 | 4-F-Ph | 4-Pyr | 4-$OCOC_3H_7$-Ph |
| 3-85 | 3-Cl-Ph | 4-Pyr | 2-F-Ph |
| 3-86 | 3-Cl-Ph | 4-Pyr | 3-F-Ph |

| | | | |
|---|---|---|---|
| 3-87 | 3-Cl-Ph | 4-Pyr | 4-F-Ph |
| 3-88 | 3-Cl-Ph | 4-Pyr | 2,4-diF-Ph |
| 3-89 | 3-Cl-Ph | 4-Pyr | 3,4-diF-Ph |
| 3-90 | 3-Cl-Ph | 4-Pyr | 2-Cl-Ph |
| 3-91 | 3-Cl-Ph | 4-Pyr | 3-Cl-Ph |
| 3-92 | 3-Cl-Ph | 4-Pyr | 4-Cl-Ph |
| 3-93 | 3-Cl-Ph | 4-Pyr | 2,4-diCl-Ph |
| 3-94 | 3-Cl-Ph | 4-Pyr | 3,4-diCl-Ph |
| 3-95 | 3-Cl-Ph | 4-Pyr | 3-$CF_3$-Ph |
| 3-96 | 3-Cl-Ph | 4-Pyr | 4-$CF_3$-Ph |
| 3-97 | 3-Cl-Ph | 4-Pyr | 2-Me-Ph |
| 3-98 | 3-Cl-Ph | 4-Pyr | 3-Me-Ph |
| 3-99 | 3-Cl-Ph | 4-Pyr | 4-Me-Ph |
| 3-100 | 3-Cl-Ph | 4-Pyr | 3,4-diMe-Ph |
| 3-101 | 3-Cl-Ph | 4-Pyr | 3-Et-Ph |
| 3-102 | 3-Cl-Ph | 4-Pyr | 4-Et-Ph |
| 3-103 | 3-Cl-Ph | 4-Pyr | 3-Pr-Ph |
| 3-104 | 3-Cl-Ph | 4-Pyr | 4-Pr-Ph |
| 3-105 | 3-Cl-Ph | 4-Pyr | 3-Bu-Ph |
| 3-106 | 3-Cl-Ph | 4-Pyr | 4-Bu-Ph |
| 3-107 | 3-Cl-Ph | 4-Pyr | 4-tBu-Ph |
| 3-108 | 3-Cl-Ph | 4-Pyr | 3-Ph-Ph |
| 3-109 | 3-Cl-Ph | 4-Pyr | 4-Ph-Ph |
| 3-110 | 3-Cl-Ph | 4-Pyr | 2-MeO-Ph |
| 3-111 | 3-Cl-Ph | 4-Pyr | 3-MeO-Ph |
| 3-112 | 3-Cl-Ph | 4-Pyr | 4-MeO-Ph |
| 3-113 | 3-Cl-Ph | 4-Pyr | 3,4-diMeO-Ph |
| 3-114 | 3-Cl-Ph | 4-Pyr | 3-EtO-Ph |
| 3-115 | 3-Cl-Ph | 4-Pyr | 4-EtO-Ph |
| 3-116 | 3-Cl-Ph | 4-Pyr | 3-PrO-Ph |
| 3-117 | 3-Cl-Ph | 4-Pyr | 4-PrO-Ph |
| 3-118 | 3-Cl-Ph | 4-Pyr | 3-PhO-Ph |
| 3-119 | 3-Cl-Ph | 4-Pyr | 4-PhO-Ph |
| 3-120 | 3-Cl-Ph | 4-Pyr | 3-$OCHF_2$-Ph |
| 3-121 | 3-Cl-Ph | 4-Pyr | 4-$OCHF_2$-Ph |
| 3-122 | 3-Cl-Ph | 4-Pyr | 2-OH-Ph |

| | | | |
|---|---|---|---|
| 3-123 | 3-Cl-Ph | 4-Pyr | 3-OH-Ph |
| 3-124 | 3-Cl-Ph | 4-Pyr | 4-OH-Ph |
| 3-125 | 3-Cl-Ph | 4-Pyr | 3,4-diOH-Ph |
| 3-126 | 3-Cl-Ph | 4-Pyr | 3-$OCOCH_3$-Ph |
| 3-127 | 3-Cl-Ph | 4-Pyr | 4-$OCOCH_3$-Ph |
| 3-128 | 3-Cl-Ph | 4-Pyr | 4-$OCOC_2H_5$-Ph |
| 3-129 | 3-Cl-Ph | 4-Pyr | 4-$OCOC_3H_7$-Ph |
| 3-130 | 3-$CF_3$-Ph | 4-Pyr | 2-F-Ph |
| 3-131 | 3-$CF_3$-Ph | 4-Pyr | 3-F-Ph |
| 3-132 | 3-$CF_3$-Ph | 4-Pyr | 3,4-diF-Ph |
| 3-133 | 3-$CF_3$-Ph | 4-Pyr | 2-Cl-Ph |
| 3-134 | 3-$CF_3$-Ph | 4-Pyr | 3-Cl-Ph |
| 3-135 | 3-$CF_3$-Ph | 4-Pyr | 2,4-diCl-Ph |
| 3-136 | 3-$CF_3$-Ph | 4-Pyr | 3,4-diCl-Ph |
| 3-137 | 3-$CF_3$-Ph | 4-Pyr | 3-$CF_3$-Ph |
| 3-138 | 3-$CF_3$-Ph | 4-Pyr | 2-Me-Ph |
| 3-139 | 3-$CF_3$-Ph | 4-Pyr | 3-Me-Ph |
| 3-140 | 3-$CF_3$-Ph | 4-Pyr | 3,4-diMe-Ph |
| 3-141 | 3-$CF_3$-Ph | 4-Pyr | 3-Et-Ph |
| 3-142 | 3-$CF_3$-Ph | 4-Pyr | 4-Et-Ph |
| 3-143 | 3-$CF_3$-Ph | 4-Pyr | 3-Pr-Ph |
| 3-144 | 3-$CF_3$-Ph | 4-Pyr | 4-Pr-Ph |
| 3-145 | 3-$CF_3$-Ph | 4-Pyr | 3-Bu-Ph |
| 3-146 | 3-$CF_3$-Ph | 4-Pyr | 4-Bu-Ph |
| 3-147 | 3-$CF_3$-Ph | 4-Pyr | 4-tBu-Ph |
| 3-148 | 3-$CF_3$-Ph | 4-Pyr | 3-Ph-Ph |
| 3-149 | 3-$CF_3$-Ph | 4-Pyr | 4-Ph-Ph |
| 3-150 | 3-$CF_3$-Ph | 4-Pyr | 2-MeO-Ph |
| 3-151 | 3-$CF_3$-Ph | 4-Pyr | 3-MeO-Ph |
| 3-152 | 3-$CF_3$-Ph | 4-Pyr | 3,4-diMeO-Ph |
| 3-153 | 3-$CF_3$-Ph | 4-Pyr | 3-EtO-Ph |
| 3-154 | 3-$CF_3$-Ph | 4-Pyr | 4-EtO-Ph |
| 3-155 | 3-$CF_3$-Ph | 4-Pyr | 3-PrO-Ph |
| 3-156 | 3-$CF_3$-Ph | 4-Pyr | 4-PrO-Ph |
| 3-157 | 3-$CF_3$-Ph | 4-Pyr | 3-PhO-Ph |
| 3-158 | 3-$CF_3$-Ph | 4-Pyr | 4-PhO-Ph |

| | | | |
|---|---|---|---|
| 3-159 | 3-$CF_3$-Ph | 4-Pyr | 3-$OCHF_2$-Ph |
| 3-160 | 3-$CF_3$-Ph | 4-Pyr | 4-$OCHF_2$-Ph |
| 3-161 | 3-$CF_3$-Ph | 4-Pyr | 2-OH-Ph |
| 3-162 | 3-$CF_3$-Ph | 4-Pyr | 3-OH-Ph |
| 3-163 | 3-$CF_3$-Ph | 4-Pyr | 4-OH-Ph |
| 3-164 | 3-$CF_3$-Ph | 4-Pyr | 3,4-diOH-Ph |
| 3-165 | 3-$CF_3$-Ph | 4-Pyr | 3-$OCOCH_3$-Ph |
| 3-166 | 3-$CF_3$-Ph | 4-Pyr | 4-$OCOCH_3$-Ph |
| 3-167 | 3-$CF_3$-Ph | 4-Pyr | 4-$OCOC_2H_5$-Ph |
| 3-168 | 3-$CF_3$-Ph | 4-Pyr | 4-$OCOC_3H_7$-Ph |
| 3-169 | 3,4-diF-Ph | 4-Pyr | 2-F-Ph |
| 3-170 | 3,4-diF-Ph | 4-Pyr | 3-F-Ph |
| 3-171 | 3,4-diF-Ph | 4-Pyr | 4-F-Ph |
| 3-172 | 3,4-diF-Ph | 4-Pyr | 2,4-diF-Ph |
| 3-173 | 3,4-diF-Ph | 4-Pyr | 3,4-diF-Ph |
| 3-174 | 3,4-diF-Ph | 4-Pyr | 2-Cl-Ph |
| 3-175 | 3,4-diF-Ph | 4-Pyr | 3-Cl-Ph |
| 3-176 | 3,4-diF-Ph | 4-Pyr | 4-Cl-Ph |
| 3-177 | 3,4-diF-Ph | 4-Pyr | 2,4-diCl-Ph |
| 3-178 | 3,4-diF-Ph | 4-Pyr | 3,4-diCl-Ph |
| 3-179 | 3,4-diF-Ph | 4-Pyr | 3-$CF_3$-Ph |
| 3-180 | 3,4-diF-Ph | 4-Pyr | 4-$CF_3$-Ph |
| 3-181 | 3,4-diF-Ph | 4-Pyr | 2-Me-Ph |
| 3-182 | 3,4-diF-Ph | 4-Pyr | 3-Me-Ph |
| 3-183 | 3,4-diF-Ph | 4-Pyr | 4-Me-Ph |
| 3-184 | 3,4-diF-Ph | 4-Pyr | 3,4-diMe-Ph |
| 3-185 | 3,4-diF-Ph | 4-Pyr | 3-Et-Ph |
| 3-186 | 3,4-diF-Ph | 4-Pyr | 4-Et-Ph |
| 3-187 | 3,4-diF-Ph | 4-Pyr | 3-Pr-Ph |
| 3-188 | 3,4-diF-Ph | 4-Pyr | 4-Pr-Ph |
| 3-189 | 3,4-diF-Ph | 4-Pyr | 3-Bu-Ph |
| 3-190 | 3,4-diF-Ph | 4-Pyr | 4-Bu-Ph |
| 3-191 | 3,4-diF-Ph | 4-Pyr | 4-tBu-Ph |
| 3-192 | 3,4-diF-Ph | 4-Pyr | 3-Ph-Ph |
| 3-193 | 3,4-diF-Ph | 4-Pyr | 4-Ph-Ph |
| 3-194 | 3,4-diF-Ph | 4-Pyr | 2-MeO-Ph |

| | | | |
|---|---|---|---|
| 3-195 | 3,4-diF-Ph | 4-Pyr | 3-MeO-Ph |
| 3-196 | 3,4-diF-Ph | 4-Pyr | 4-MeO-Ph |
| 3-197 | 3,4-diF-Ph | 4-Pyr | 3,4-diMeO-Ph |
| 3-198 | 3,4-diF-Ph | 4-Pyr | 3-EtO-Ph |
| 3-199 | 3,4-diF-Ph | 4-Pyr | 4-EtO-Ph |
| 3-200 | 3,4-diF-Ph | 4-Pyr | 3-PrO-Ph |
| 3-201 | 3,4-diF-Ph | 4-Pyr | 4-PrO-Ph |
| 3-202 | 3,4-diF-Ph | 4-Pyr | 3-PhO-Ph |
| 3-203 | 3,4-diF-Ph | 4-Pyr | 4-PhO-Ph |
| 3-204 | 3,4-diF-Ph | 4-Pyr | 3-$OCHF_2$-Ph |
| 3-205 | 3,4-diF-Ph | 4-Pyr | 4-$OCHF_2$-Ph |
| 3-206 | 3,4-diF-Ph | 4-Pyr | 2-OH-Ph |
| 3-207 | 3,4-diF-Ph | 4-Pyr | 3-OH-Ph |
| 3-208 | 3,4-diF-Ph | 4-Pyr | 4-OH-Ph |
| 3-209 | 3,4-diF-Ph | 4-Pyr | 3,4-diOH-Ph |
| 3-210 | 3,4-diF-Ph | 4-Pyr | 3-$OCOCH_3$-Ph |
| 3-211 | 3,4-diF-Ph | 4-Pyr | 4-$OCOCH_3$-Ph |
| 3-212 | 3,4-diF-Ph | 4-Pyr | 4-$OCOC_2H_5$-Ph |
| 3-213 | 3,4-diF-Ph | 4-Pyr | 4-$OCOC_3H_7$-Ph |
| 3-214 | 3-MeO-Ph | 4-Pyr | 2-F-Ph |
| 3-215 | 3-MeO-Ph | 4-Pyr | 3-F-Ph |
| 3-216 | 3-MeO-Ph | 4-Pyr | 4-F-Ph |
| 3-217 | 3-MeO-Ph | 4-Pyr | 2,4-diF-Ph |
| 3-218 | 3-MeO-Ph | 4-Pyr | 3,4-diF-Ph |
| 3-219 | 3-MeO-Ph | 4-Pyr | 2-Me-Ph |
| 3-220 | 3-MeO-Ph | 4-Pyr | 3-Me-Ph |
| 3-221 | 3-MeO-Ph | 4-Pyr | 4-Me-Ph |
| 3-222 | 3-MeO-Ph | 4-Pyr | 3,4-diMe-Ph |
| 3-223 | 3-MeO-Ph | 4-Pyr | 3-Et-Ph |
| 3-224 | 3-MeO-Ph | 4-Pyr | 4-Et-Ph |
| 3-225 | 3-MeO-Ph | 4-Pyr | 4-tBu-Ph |
| 3-226 | 3-MeO-Ph | 4-Pyr | 2-MeO-Ph |
| 3-227 | 3-MeO-Ph | 4-Pyr | 3-MeO-Ph |
| 3-228 | 3-MeO-Ph | 4-Pyr | 4-MeO-Ph |
| 3-229 | 3-MeO-Ph | 4-Pyr | 3,4-diMeO-Ph |
| 3-230 | 3-MeO-Ph | 4-Pyr | 2-OH-Ph |

| | | | |
|---|---|---|---|
| 3-231 | 3-MeO-Ph | 4-Pyr | 3-OH-Ph |
| 3-232 | 3-MeO-Ph | 4-Pyr | 4-OH-Ph |
| 3-233 | 3-MeO-Ph | 4-Pyr | 3,4-diOH-Ph |
| 3-234 | 3-MeO-Ph | 4-Pyr | 3-$OCOCH_3$-Ph |
| 3-235 | 3-MeO-Ph | 4-Pyr | 4-$OCOCH_3$-Ph |
| 3-236 | 3-MeO-Ph | 4-Pyr | 4-$OCOC_2H_5$-Ph |
| 3-237 | 3-MeO-Ph | 4-Pyr | 4-$OCOC_3H_7$-Ph |
| 3-238 | 3-MeO-Ph | 4-Pyr | Ph |
| 3-239 | 3-MeO-Ph | 4-Pyr | H |
| 3-240 | 3-MeO-Ph | 4-Pyr | Me |
| 3-241 | 3-MeO-Ph | 4-Pyr | Et |
| 3-242 | 3-MeO-Ph | 4-Pyr | Pr |
| 3-243 | 3-MeO-Ph | 4-Pyr | Bu |
| 3-244 | 3-MeO-Ph | 4-Pyr | OH |
| 3-245 | 3-MeO-Ph | 4-Pyr | MeO |
| 3-246 | 3-MeO-Ph | 4-Pyr | EtO |
| 3-247 | 3-CN-Ph | 4-Pyr | 2-F-Ph |
| 3-248 | 3-CN-Ph | 4-Pyr | 3-F-Ph |
| 3-249 | 3-CN-Ph | 4-Pyr | 4-F-Ph |
| 3-250 | 3-CN-Ph | 4-Pyr | 2,4-diF-Ph |
| 3-251 | 3-CN-Ph | 4-Pyr | 3,4-diF-Ph |
| 3-252 | 3-CN-Ph | 4-Pyr | 2-Me-Ph |
| 3-253 | 3-CN-Ph | 4-Pyr | 3-Me-Ph |
| 3-254 | 3-CN-Ph | 4-Pyr | 4-Me-Ph |
| 3-255 | 3-CN-Ph | 4-Pyr | 3,4-diMe-Ph |
| 3-256 | 3-CN-Ph | 4-Pyr | 3-Et-Ph |
| 3-257 | 3-CN-Ph | 4-Pyr | 4-Et-Ph |
| 3-258 | 3-CN-Ph | 4-Pyr | 4-tBu-Ph |
| 3-259 | 3-CN-Ph | 4-Pyr | 2-MeO-Ph |
| 3-260 | 3-CN-Ph | 4-Pyr | 3-MeO-Ph |
| 3-261 | 3-CN-Ph | 4-Pyr | 4-MeO-Ph |
| 3-262 | 3-CN-Ph | 4-Pyr | 3,4-diMeO-Ph |
| 3-263 | 3-CN-Ph | 4-Pyr | 2-OH-Ph |
| 3-264 | 3-CN-Ph | 4-Pyr | 3-OH-Ph |
| 3-265 | 3-CN-Ph | 4-Pyr | 4-OH-Ph |
| 3-266 | 3-CN-Ph | 4-Pyr | 3,4-diOH-Ph |

| | | | |
|---|---|---|---|
| 3-267 | 3-CN-Ph | 4-Pyr | 3-$OCOCH_3$-Ph |
| 3-268 | 3-CN-Ph | 4-Pyr | 4-$OCOCH_3$-Ph |
| 3-269 | 3-CN-Ph | 4-Pyr | 4-$OCOC_2H_5$-Ph |
| 3-270 | 3-CN-Ph | 4-Pyr | 4-$OCOC_3H_7$-Ph |
| 3-271 | 3-CN-Ph | 4-Pyr | Ph |
| 3-272 | 3-CN-Ph | 4-Pyr | H |
| 3-273 | 3-CN-Ph | 4-Pyr | Me |
| 3-274 | 3-CN-Ph | 4-Pyr | Et |
| 3-275 | 3-CN-Ph | 4-Pyr | Pr |
| 3-276 | 3-CN-Ph | 4-Pyr | Bu |
| 3-277 | 3-CN-Ph | 4-Pyr | OH |
| 3-278 | 3-CN-Ph | 4-Pyr | MeO |
| 3-279 | 3-CN-Ph | 4-Pyr | EtO |
| 3-280 | 4-F-3-MeO-Ph | 4-Pyr | 2-F-Ph |
| 3-281 | 4-F-3-MeO-Ph | 4-Pyr | 3-F-Ph |
| 3-282 | 4-F-3-MeO-Ph | 4-Pyr | 4-F-Ph |
| 3-283 | 4-F-3-MeO-Ph | 4-Pyr | 2,4-diF-Ph |
| 3-284 | 4-F-3-MeO-Ph | 4-Pyr | 3,4-diF-Ph |
| 3-285 | 4-F-3-MeO-Ph | 4-Pyr | 2-Me-Ph |
| 3-286 | 4-F-3-MeO-Ph | 4-Pyr | 3-Me-Ph |
| 3-287 | 4-F-3-MeO-Ph | 4-Pyr | 4-Me-Ph |
| 3-288 | 4-F-3-MeO-Ph | 4-Pyr | 3,4-diMe-Ph |
| 3-289 | 4-F-3-MeO-Ph | 4-Pyr | 3-Et-Ph |
| 3-290 | 4-F-3-MeO-Ph | 4-Pyr | 4-Et-Ph |
| 3-291 | 4-F-3-MeO-Ph | 4-Pyr | 4-tBu-Ph |
| 3-292 | 4-F-3-MeO-Ph | 4-Pyr | 2-MeO-Ph |
| 3-293 | 4-F-3-MeO-Ph | 4-Pyr | 3-MeO-Ph |
| 3-294 | 4-F-3-MeO-Ph | 4-Pyr | 4-MeO-Ph |
| 3-295 | 4-F-3-MeO-Ph | 4-Pyr | 3,4-diMeO-Ph |
| 3-296 | 4-F-3-MeO-Ph | 4-Pyr | 2-OH-Ph |
| 3-297 | 4-F-3-MeO-Ph | 4-Pyr | 3-OH-Ph |
| 3-298 | 4-F-3-MeO-Ph | 4-Pyr | 4-OH-Ph |
| 3-299 | 4-F-3-MeO-Ph | 4-Pyr | 3,4-diOH-Ph |
| 3-300 | 4-F-3-MeO-Ph | 4-Pyr | 3-$OCOCH_3$-Ph |
| 3-301 | 4-F-3-MeO-Ph | 4-Pyr | 4-$OCOCH_3$-Ph |
| 3-302 | 4-F-3-MeO-Ph | 4-Pyr | 4-$OCOC_2H_5$-Ph |

| | | | |
|---|---|---|---|
| 3-303 | 4-F-3-MeO-Ph | 4-Pyr | 4-OCOC$_3$H$_7$-Ph |
| 3-304 | 4-F-3-MeO-Ph | 4-Pyr | Ph |
| 3-305 | 4-F-3-MeO-Ph | 4-Pyr | H |
| 3-306 | 4-F-3-MeO-Ph | 4-Pyr | Me |
| 3-307 | 4-F-3-MeO-Ph | 4-Pyr | Et |
| 3-308 | 4-F-3-MeO-Ph | 4-Pyr | Pr |
| 3-309 | 4-F-3-MeO-Ph | 4-Pyr | Bu |
| 3-310 | 4-F-3-MeO-Ph | 4-Pyr | OH |
| 3-311 | 4-F-3-MeO-Ph | 4-Pyr | MeO |
| 3-312 | 4-F-3-MeO-Ph | 4-Pyr | EtO |
| 3-313 | 2,4-diF-Ph | 4-Pyr | 2-F-Ph |
| 3-314 | 2,4-diF-Ph | 4-Pyr | 3-F-Ph |
| 3-315 | 2,4-diF-Ph | 4-Pyr | 4-F-Ph |
| 3-316 | 2,4-diF-Ph | 4-Pyr | 2,4-diF-Ph |
| 3-317 | 2,4-diF-Ph | 4-Pyr | 3,4-diF-Ph |
| 3-318 | 2,4-diF-Ph | 4-Pyr | 2-Me-Ph |
| 3-319 | 2,4-diF-Ph | 4-Pyr | 3-Me-Ph |
| 3-320 | 2,4-diF-Ph | 4-Pyr | 4-Me-Ph |
| 3-321 | 2,4-diF-Ph | 4-Pyr | 3,4-diMe-Ph |
| 3-322 | 2,4-diF-Ph | 4-Pyr | 3-Et-Ph |
| 3-323 | 2,4-diF-Ph | 4-Pyr | 4-Et-Ph |
| 3-324 | 2,4-diF-Ph | 4-Pyr | 4-tBu-Ph |
| 3-325 | 2,4-diF-Ph | 4-Pyr | 2-MeO-Ph |
| 3-326 | 2,4-diF-Ph | 4-Pyr | 3-MeO-Ph |
| 3-327 | 2,4-diF-Ph | 4-Pyr | 4-MeO-Ph |
| 3-328 | 2,4-diF-Ph | 4-Pyr | 3,4-diMeO-Ph |
| 3-329 | 2,4-diF-Ph | 4-Pyr | 2-OH-Ph |
| 3-330 | 2,4-diF-Ph | 4-Pyr | 3-OH-Ph |
| 3-331 | 2,4-diF-Ph | 4-Pyr | 4-OH-Ph |
| 3-332 | 2,4-diF-Ph | 4-Pyr | 3,4-diOH-Ph |
| 3-333 | 2,4-diF-Ph | 4-Pyr | 3-OCOCH$_3$-Ph |
| 3-334 | 2,4-diF-Ph | 4-Pyr | 4-OCOCH$_3$-Ph |
| 3-335 | 2,4-diF-Ph | 4-Pyr | 4-OCOC$_2$H$_5$-Ph |
| 3-336 | 2,4-diF-Ph | 4-Pyr | 4-OCOC$_3$H$_7$-Ph |
| 3-337 | 2,4-diF-Ph | 4-Pyr | Ph |
| 3-338 | 2,4-diF-Ph | 4-Pyr | H |

| | | | |
|---|---|---|---|
| 3-339 | 2,4-diF-Ph | 4-Pyr | Me |
| 3-340 | 2,4-diF-Ph | 4-Pyr | Et |
| 3-341 | 2,4-diF-Ph | 4-Pyr | Pr |
| 3-342 | 2,4-diF-Ph | 4-Pyr | Bu |
| 3-343 | 2,4-diF-Ph | 4-Pyr | OH |
| 3-344 | 2,4-diF-Ph | 4-Pyr | MeO |
| 3-345 | 2,4-diF-Ph | 4-Pyr | EtO |
| 3-346 | 3,4,5-triF-Ph | 4-Pyr | 2-F-Ph |
| 3-347 | 3,4,5-triF-Ph | 4-Pyr | 3-F-Ph |
| 3-348 | 3,4,5-triF-Ph | 4-Pyr | 4-F-Ph |
| 3-349 | 3,4,5-triF-Ph | 4-Pyr | 2,4-diF-Ph |
| 3-350 | 3,4,5-triF-Ph | 4-Pyr | 3,4-diF-Ph |
| 3-351 | 3,4,5-triF-Ph | 4-Pyr | 2-Me-Ph |
| 3-352 | 3,4,5-triF-Ph | 4-Pyr | 3-Me-Ph |
| 3-353 | 3,4,5-triF-Ph | 4-Pyr | 4-Me-Ph |
| 3-354 | 3,4,5-triF-Ph | 4-Pyr | 3,4-diMe-Ph |
| 3-355 | 3,4,5-triF-Ph | 4-Pyr | 3-Et-Ph |
| 3-356 | 3,4,5-triF-Ph | 4-Pyr | 4-Et-Ph |
| 3-357 | 3,4,5-triF-Ph | 4-Pyr | 4-tBu-Ph |
| 3-358 | 3,4,5-triF-Ph | 4-Pyr | 2-MeO-Ph |
| 3-359 | 3,4,5-triF-Ph | 4-Pyr | 3-MeO-Ph |
| 3-360 | 3,4,5-triF-Ph | 4-Pyr | 4-MeO-Ph |
| 3-361 | 3,4,5-triF-Ph | 4-Pyr | 3,4-diMeO-Ph |
| 3-362 | 3,4,5-triF-Ph | 4-Pyr | 2-OH-Ph |
| 3-363 | 3,4,5-triF-Ph | 4-Pyr | 3-OH-Ph |
| 3-364 | 3,4,5-triF-Ph | 4-Pyr | 4-OH-Ph |
| 3-365 | 3,4,5-triF-Ph | 4-Pyr | 3,4-diOH-Ph |
| 3-366 | 3,4,5-triF-Ph | 4-Pyr | 3-$OCOCH_3$-Ph |
| 3-367 | 3,4,5-triF-Ph | 4-Pyr | 4-$OCOCH_3$-Ph |
| 3-368 | 3,4,5-triF-Ph | 4-Pyr | 4-$OCOC_2H_5$-Ph |
| 3-369 | 3,4,5-triF-Ph | 4-Pyr | 4-$OCOC_3H_7$-Ph |
| 3-370 | 3,4,5-triF-Ph | 4-Pyr | Ph |
| 3-371 | 3,4,5-triF-Ph | 4-Pyr | H |
| 3-372 | 3,4,5-triF-Ph | 4-Pyr | Me |
| 3-373 | 3,4,5-triF-Ph | 4-Pyr | Et |
| 3-374 | 3,4,5-triF-Ph | 4-Pyr | Pr |

| | | | |
|---|---|---|---|
| 3-375 | 3,4,5-triF-Ph | 4-Pyr | Bu |
| 3-376 | 3,4,5-triF-Ph | 4-Pyr | OH |
| 3-377 | 3,4,5-triF-Ph | 4-Pyr | MeO |
| 3-378 | 3,4,5-triF-Ph | 4-Pyr | EtO |
| 3-379 | 3-Cl-4-F-Ph | 4-Pyr | 2-F-Ph |
| 3-380 | 3-Cl-4-F-Ph | 4-Pyr | 3-F-Ph |
| 3-381 | 3-Cl-4-F-Ph | 4-Pyr | 4-F-Ph |
| 3-382 | 3-Cl-4-F-Ph | 4-Pyr | 2,4-diF-Ph |
| 3-383 | 3-Cl-4-F-Ph | 4-Pyr | 3,4-diF-Ph |
| 3-384 | 3-Cl-4-F-Ph | 4-Pyr | 2-Me-Ph |
| 3-385 | 3-Cl-4-F-Ph | 4-Pyr | 3-Me-Ph |
| 3-386 | 3-Cl-4-F-Ph | 4-Pyr | 4-Me-Ph |
| 3-387 | 3-Cl-4-F-Ph | 4-Pyr | 3,4-diMe-Ph |
| 3-388 | 3-Cl-4-F-Ph | 4-Pyr | 3-Et-Ph |
| 3-389 | 3-Cl-4-F-Ph | 4-Pyr | 4-Et-Ph |
| 3-390 | 3-Cl-4-F-Ph | 4-Pyr | 4-tBu-Ph |
| 3-391 | 3-Cl-4-F-Ph | 4-Pyr | 2-MeO-Ph |
| 3-392 | 3-Cl-4-F-Ph | 4-Pyr | 3-MeO-Ph |
| 3-393 | 3-Cl-4-F-Ph | 4-Pyr | 4-MeO-Ph |
| 3-394 | 3-Cl-4-F-Ph | 4-Pyr | 3,4-diMeO-Ph |
| 3-395 | 3-Cl-4-F-Ph | 4-Pyr | 2-OH-Ph |
| 3-396 | 3-Cl-4-F-Ph | 4-Pyr | 3-OH-Ph |
| 3-397 | 3-Cl-4-F-Ph | 4-Pyr | 4-OH-Ph |
| 3-398 | 3-Cl-4-F-Ph | 4-Pyr | 3,4-diOH-Ph |
| 3-399 | 3-Cl-4-F-Ph | 4-Pyr | 3-$OCOCH_3$-Ph |
| 3-400 | 3-Cl-4-F-Ph | 4-Pyr | 4-$OCOCH_3$-Ph |
| 3-401 | 3-Cl-4-F-Ph | 4-Pyr | 4-$OCOC_2H_5$-Ph |
| 3-402 | 3-Cl-4-F-Ph | 4-Pyr | 4-$OCOC_3H_7$-Ph |
| 3-403 | 3-Cl-4-F-Ph | 4-Pyr | Ph |
| 3-404 | 3-Cl-4-F-Ph | 4-Pyr | H |
| 3-405 | 3-Cl-4-F-Ph | 4-Pyr | Me |
| 3-406 | 3-Cl-4-F-Ph | 4-Pyr | Et |
| 3-407 | 3-Cl-4-F-Ph | 4-Pyr | Pr |
| 3-408 | 3-Cl-4-F-Ph | 4-Pyr | Bu |
| 3-409 | 3-Cl-4-F-Ph | 4-Pyr | OH |
| 3-410 | 3-Cl-4-F-Ph | 4-Pyr | MeO |

| | | | |
|---|---|---|---|
| 3-411 | 3-Cl-4-F-Ph | 4-Pyr | EtO |
| 3-412 | 3-$OCHF_2$-Ph | 4-Pyr | 2-F-Ph |
| 3-413 | 3-$OCHF_2$-Ph | 4-Pyr | 3-F-Ph |
| 3-414 | 3-$OCHF_2$-Ph | 4-Pyr | 4-F-Ph |
| 3-415 | 3-$OCHF_2$-Ph | 4-Pyr | 2,4-diF-Ph |
| 3-416 | 3-$OCHF_2$-Ph | 4-Pyr | 3,4-diF-Ph |
| 3-417 | 3-$OCHF_2$-Ph | 4-Pyr | 2-Me-Ph |
| 3-418 | 3-$OCHF_2$-Ph | 4-Pyr | 3-Me-Ph |
| 3-419 | 3-$OCHF_2$-Ph | 4-Pyr | 4-Me-Ph |
| 3-420 | 3-$OCHF_2$-Ph | 4-Pyr | 3,4-diMe-Ph |
| 3-421 | 3-$OCHF_2$-Ph | 4-Pyr | 3-Et-Ph |
| 3-422 | 3-$OCHF_2$-Ph | 4-Pyr | 4-Et-Ph |
| 3-423 | 3-$OCHF_2$-Ph | 4-Pyr | 4-tBu-Ph |
| 3-424 | 3-$OCHF_2$-Ph | 4-Pyr | 2-MeO-Ph |
| 3-425 | 3-$OCHF_2$-Ph | 4-Pyr | 3-MeO-Ph |
| 3-426 | 3-$OCHF_2$-Ph | 4-Pyr | 4-MeO-Ph |
| 3-427 | 3-$OCHF_2$-Ph | 4-Pyr | 3,4-diMeO-Ph |
| 3-428 | 3-$OCHF_2$-Ph | 4-Pyr | 2-OH-Ph |
| 3-429 | 3-$OCHF_2$-Ph | 4-Pyr | 3-OH-Ph |
| 3-430 | 3-$OCHF_2$-Ph | 4-Pyr | 4-OH-Ph |
| 3-431 | 3-$OCHF_2$-Ph | 4-Pyr | 3,4-diOH-Ph |
| 3-432 | 3-$OCHF_2$-Ph | 4-Pyr | 3-$OCOCH_3$-Ph |
| 3-433 | 3-$OCHF_2$-Ph | 4-Pyr | 4-$OCOCH_3$-Ph |
| 3-434 | 3-$OCHF_2$-Ph | 4-Pyr | 4-$OCOC_2H_5$-Ph |
| 3-435 | 3-$OCHF_2$-Ph | 4-Pyr | 4-$OCOC_3H_7$-Ph |
| 3-436 | 3-$OCHF_2$-Ph | 4-Pyr | Ph |
| 3-437 | 3-$OCHF_2$-Ph | 4-Pyr | H |
| 3-438 | 3-$OCHF_2$-Ph | 4-Pyr | Me |
| 3-439 | 3-$OCHF_2$-Ph | 4-Pyr | Et |
| 3-440 | 3-$OCHF_2$-Ph | 4-Pyr | Pr |
| 3-441 | 3-$OCHF_2$-Ph | 4-Pyr | Bu |
| 3-442 | 3-$OCHF_2$-Ph | 4-Pyr | OH |
| 3-443 | 3-$OCHF_2$-Ph | 4-Pyr | MeO |
| 3-444 | 3-$OCHF_2$-Ph | 4-Pyr | EtO |
| 3-445 | 3-F-Ph | 4-Pyr | 3-$OCOC_2H_5$-Ph |
| 3-446 | 3-F-Ph | 4-Pyr | 3-$OCOC_3H_7$-Ph |

| | | | |
|---|---|---|---|
| 3-447 | 4-F-Ph | 4-Pyr | 3-$OCOC_2H_5$-Ph |
| 3-448 | 4-F-Ph | 4-Pyr | 3-$OCOC_3H_7$-Ph |
| 3-449 | 3-Cl-Ph | 4-Pyr | 3-$OCOC_2H_5$-Ph |
| 3-450 | 3-Cl-Ph | 4-Pyr | 3-$OCOC_3H_7$-Ph |
| 3-451 | 3-$CF_3$-Ph | 4-Pyr | 3-$OCOC_2H_5$-Ph |
| 3-452 | 3-$CF_3$-Ph | 4-Pyr | 3-$OCOC_3H_7$-Ph |
| 3-453 | 3,4-$F_2$-Ph | 4-Pyr | 3-$OCOC_2H_5$-Ph |
| 3-454 | 3,4-$F_2$-Ph | 4-Pyr | 3-$OCOC_3H_7$-Ph |
| 3-455 | 3-MeO-Ph | 4-Pyr | 3-$OCOC_2H_5$-Ph |
| 3-456 | 3-MeO-Ph | 4-Pyr | 3-$OCOC_3H_7$-Ph |
| 3-457 | 3-CN-Ph | 4-Pyr | 3-$OCOC_2H_5$-Ph |
| 3-458 | 3-CN-Ph | 4-Pyr | 3-$OCOC_3H_7$-Ph |
| 3-459 | 4-F-3-MeO-Ph | 4-Pyr | 3-$OCOC_2H_5$-Ph |
| 3-460 | 4-F-3-MeO-Ph | 4-Pyr | 3-$OCOC_3H_7$-Ph |
| 3-461 | 2,4-$F_2$-Ph | 4-Pyr | 3-$OCOC_2H_5$-Ph |
| 3-462 | 2,4-$F_2$-Ph | 4-Pyr | 3-$OCOC_3H_7$-Ph |
| 3-463 | 3,4,5-$F_3$-Ph | 4-Pyr | 3-$OCOC_2H_5$-Ph |
| 3-464 | 3,4,5-$F_3$-Ph | 4-Pyr | 3-$OCOC_3H_7$-Ph |
| 3-465 | 3-Cl-4-F-Ph | 4-Pyr | 3-$OCOC_2H_5$-Ph |
| 3-466 | 3-Cl-4-F-Ph | 4-Pyr | 3-$OCOC_3H_7$-Ph |
| 3-467 | 3-$OCHF_2$-Ph | 4-Pyr | 3-$OCOC_2H_5$-Ph |
| 3-468 | 3-$OCHF_2$-Ph | 4-Pyr | 3-$OCOC_3H_7$-Ph |
| 3-469 | 4-F-Ph | 4-Pyr | $NH_2$ |
| 3-470 | 4-F-Ph | 4-Pyr | $NHCOCH_3$ |
| 3-471 | 4-F-Ph | 4-Pyr | $NHSO_2CH_3$ |
| 3-472 | 3-F-Ph | 4-Pyr | $NH_2$ |
| 3-473 | 3-F-Ph | 4-Pyr | $NHCOCH_3$ |
| 3-474 | 3-F-Ph | 4-Pyr | $NHSO_2CH_3$ |
| 3-475 | 3-Cl-Ph | 4-Pyr | $NH_2$ |
| 3-476 | 3-Cl-Ph | 4-Pyr | $NHCOCH_3$ |
| 3-477 | 3-Cl-Ph | 4-Pyr | $NHSO_2CH_3$ |
| 3-478 | 3,4-diF-Ph | 4-Pyr | $NH_2$ |
| 3-479 | 3,4-diF-Ph | 4-Pyr | $NHCOCH_3$ |
| 3-480 | 3,4-diF-Ph | 4-Pyr | $NHSO_2CH_3$ |
| 3-481 | 4-F-Ph | 4-Pyr | $NH_2$ |
| 3-482 | 4-F-Ph | 4-Pyr | $NHCOCH_3$ |

| | | | |
|---|---|---|---|
| 3-483 | 4-F-Ph | 4-Pyr | $NHSO_2CH_3$ |
| 3-484 | 3-F-Ph | 4-Pyr | $NH_2$ |
| 3-485 | 3-F-Ph | 4-Pyr | $NHCOCH_3$ |
| 3-486 | 3-F-Ph | 4-Pyr | $NHSO_2CH_3$ |
| 3-487 | 3-Cl-Ph | 4-Pyr | $NH_2$ |
| 3-488 | 3-Cl-Ph | 4-Pyr | $NHCOCH_3$ |
| 3-489 | 3-Cl-Ph | 4-Pyr | $NHSO_2CH_3$ |
| 3-490 | 3,4-diF-Ph | 4-Pyr | $NH_2$ |
| 3-491 | 3,4-diF-Ph | 4-Pyr | $NHCOCH_3$ |
| 3-492 | 3,4-diF-Ph | 4-Pyr | $NHSO_2CH_3$ |
| 3-493 | Ph | 4-Pyr | H |
| 3-494 | 4-F-Ph | 4-Pyr | Pr |
| 3-495 | 4-F-Ph | 4-Pyr | Bu |
| 3-496 | 4-F-Ph | 4-Pyr | Ph |
| 3-497 | 4-F-Ph | 4-Pyr | 4-Me-Ph |
| 3-498 | 3,4-diF-Ph | 4-Pyr | H |
| 3-499 | 3,4-diF-Ph | 4-Pyr | Ph |

Table 4

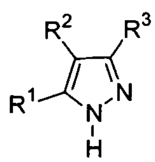

(I−4)

| Compound No. | $R^1$ | $R^2$ | $R^3$ |
|---|---|---|---|
| 4-25 | 4-F-Ph | 4-Pyr | 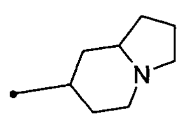 |

In the above table, "Bu" represents butyl, "t-Bu" represents t-butyl, "Et" represents ethyl, "Me" represents methyl, "Ph" represents phenyl, "Pr" represents propyl, and "Pyr" represents pyridyl.

Further, the compounds referred to in the above Table 3 and Table 4 exhibit tautomerism as shown below.

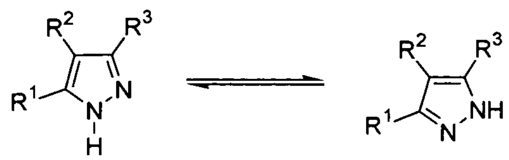

The exemplified compounds in the above Table 3 and Table 4 include both types of compounds.

In the above Table 1 to Table 4, the preferred compounds are the exemplified compounds No.:

1-1 to 1-228, 1-230 to 1-235, 1-237 to 1-239, 1-241 to 1-262, 1-264 to 1-269, 1-271 to 1-273, 1-275 to 1-296, 1-298 to 1-303, 1-305 to 1-307, 1-309 to 1-330, 1-332 to 1-337, 1-339 to 1-341, 1-343 to 1-364, 1-366 to 1-371, 1-373 to 1-375, 1-377 to 1-398, 1-400 to 1-405, 1-407 to 1-409, 1-411 to 1-432, 1-434 to 1-439, 1-441 to 1-443, 1-445 to 1-476, 1-477 to 1-487 and

3-1 to 3-218, 3-220 to 3-225, 3-227 to 3-229, 3-231 to 3-251, 3-253 to 3-258, 3-260 to 3-262, 3-264 to 3-284, 3-286 to 3-291, 3-293 to 3-295, 3-297 to 3-317, 3-319 to 3-324, 3-326 to 3-328, 3-330 to 3-350, 3-352 to 3-357, 3-359 to 3-361, 3-363 to 3-383, 3-385 to 3-390, 3-392 to 3-394, 3-396 to 3-416, 3-418 to 3-423, 3-425 to 3-427, 3-429 to 3-492, 3-493 to 3-499.

The more preferred compounds are the exemplified compounds No.:

1-1 to 1-5, 1-7 to 1-12, 1-14 to 1-25, 1-27 to 1-37, 1-39 to 1-50, 1-52 to 1-55, 1-57 to 1-67, 1-69 to 1-78, 1-80 to 1-93, 1-95 to 1-100, 1-102 to 1-113, 1-115 to 1-125, 1-127 to 1-138, 1-140 to 1-143, 1-145 to 1-155, 1-157 to 1-166, 1-168 to 1-181, 1-183 to 1-188, 1-190 to 1-201, 1-203 to 1-213, 1-215 to 1-228, 1-230 to 1-235, 1-237 to 1-239, 1-24 to 1-250, 1-258 to 1-262, 1-264 to 1-269, 1-271 to 1-273, 1-275 to 1-284, 1-292 to 1-296, 1-298 to 1-303, 1-305 to 1-307, 1-309 to 1-318, 1-326 to 1-330, 1-332 to 1-337, 1-339 to 1-341, 1-343 to 1-352, 1-360 to 1-364, 1-366 to 1-371, 1-373 to 1-375, 1-377 to 1-386, 1-394 to 1-398, 1-400 to 1-405, 1-407 to 1-409, 1-411 to 1-420, 1-428 to 1-432, 1-434 to 1-439, 1-441 to 1-443, 1-445 to 1-454, 1-462 to 1-476, 1-477 to 1-487,

3-1 to 3-5, 3-7 to 3-12, 3-14 to 3-25, 3-27 to 3-37, 3-39 to 3-48, 3-50 to 3-53, 3-55 to 3-65, 3-67 to 3-76, 3-78 to 3-89, 3-91 to 3-96, 3-98 to 3-109, 3-111 to 3-121, 3-123 to 3-137, 3-139 to 3-149, 3-151 to 3-160, 3-162 to 3-173, 3-175 to 3-180, 3-182 to 3-193, 3-195 to 3-205, 3-207 to 3-218, 3-220 to 3-225, 3-227 to 3-229, 3-231 to 3-238, 3-247 to 3-251, 3-253 to 3-258, 3-260 to 3-262, 3-264 to 3-271, 3-280 to 3-284, 3-286 to 3-291, 3-293 to 3-295, 3-297 to 3-304, 3-313 to 3-317, 3-319 to 3-324, 3-326 to 3-328, 3-330 to 3-337, 3-346 to 3-350, 3-352 to 3-357, 3-359 to 3-361, 3-363 to 3-370, 3-379 to 3-383, 3-385 to 3-390, 3-392 to 3-394, 3-396 to 3-403, 3-412 to 3-416, 3-418 to 3-423, 3-425 to 3-427, 3-429 to 3-436, 3-445 to 3-492, 3-493 to 3-499.

Further more preferred compounds are the exemplified compounds No.:

1-1 to 1-5, 1-7 to 1-8, 1-10 to 1-12, 1-14 to 1-25, 1-27 to 1-37, 1-39 to 1-40, 1-42 to 1-50, 1-52, 1-54 to 1-55, 1-57 to 1-67, 1-69 to 1-78, 1-80 to 1-93, 1-95 to 1-96, 1-98 to 1-100, 1-102 to 1-113, 1-115 to 1-125, 1-127 to 1-138, 1-140, 1-142 to 1-1432, 1-145 to 1-155, 1-157 to 1-166, 1-168 to 1-181, 1-183 to 1-184, 1-186 to 1-188, 1-190 to 1-201, 1-203 to 1-213, 1-215 to 1-223, 1-250, 1-284, 1-318, 1-420, 1-477 to 1-487,

3-1 to 3-5, 3-7 to 3-12, 3-14 to 3-25, 3-27 to 3-37, 3-39 to 3-48, 3-50, 3-52 to 3-53, 3-55 to 3-65, 3-67 to 3-76, 3-78 to 3-89, 3-91 to 3-92, 3-94 to 3-96, 3-98 to 3-109, 3-111 to 3-121, 3-123 to 3-134, 3-136 to 3-137, 3-139 to 3-149, 3-151 to 3-160, 3-162 to 3-173, 3-175 to 3-176, 3-178 to 3-180, 3-182 to 3-193, 3-195 to 3-205, 3-207 to 3-213, 3-403, 3-445 to 3-454, 3-493 to 3-499.

The particularly preferred compounds are, for example,

2-(4-fluorophenyl)-4-[(2S,8aS)-2-(3-methylphenyl)-1,2,3,5,6,8a-hexahydroindolizin-7-yl]-3-(pyridin-4-yl)-1H-pyrrole (Exemplary Compound No. 1-57),

2-(4-fluorophenyl)-4-[(2S,8aS)-2-(3,4-dimethylphenyl)-1,2,3,5,6,8a-hexahydroindolizin-7-yl]-3-(pyridin-4-yl)-1H-pyrrole (Exemplary Compound No. 1-58),

4-[(2S,8aS)-2-(4-ethylphenyl)-1,2,3,5,6,8a-hexahydroindolizin-7-yl]-2-(4-fluorophenyl)-3-(pyridin-4-yl)-1H-pyrrole (Exemplary Compound No. 1-60),

2-(4-fluorophenyl)-4-[(2S,8aS)-2-(3-methoxyphenyl)-1,2,3,5,6,8a-hexahydroindolizin-7-yl]-3-(pyridin-4-yl)-1H-pyrrole (Exemplary Compound No. 1-69),

4-[(2S,8aS)-2-(4-ethoxyphenyl)-1,2,3,5,6,8a-hexahydroindolizin-7-yl]-2-(4-fluorophenyl)-3-(pyridin-4-yl)-1H-pyrrole (Exemplary Compound No. 1-72),

2-(4-fluorophenyl)-4-[(2S,8aS)-2-(4-hydroxyphenyl)-1,2,3,5,6,8a-hexahydroindolizin-7-yl]-3-(pyridin-4-yl)-1H-pyrrole (Exemplary Compound No. 1-81),

4-[(2S,8aS)-2-(4-acetoxyphenyl)-1,2,3,5,6,8a-hexahydroindolizin-7-yl]-2-(4-fluorophenyl)-3-(pyridin-4-yl)-1H-pyrrole (Exemplary Compound No. 1-86),

2-(3-chlorophenyl)-4-[(2S,8aS)-2-(4-methylphenyl)-1,2,3,5,6,8a-hexahydroindolizin-7-yl]-3-(pyridin-4-yl)-1H-pyrrole (Exemplary Compound No. 1-103),

2-(3-chlorophenyl)-4-[(2S,8aS)-2-(4-methoxyphenyl)-1,2,3,5,6,8a-hexahydroindolizin-7-yl]-3-(pyridin-4-yl)-1H-pyrrole (Exemplary Compound No. 1-116),
2-(3,4-difluorophenyl)-4-[(2S,8aS)-2-(3,4-dimethylphenyl)-1,2,3,5,6,8a-hexahydroindolizin-7-yl]-3-(pyridin-4-yl)-1H-pyrrole (Exemplary Compound No. 1-192),
2-(3,4-difluorophenyl)-4-[(2S,8aS)-2-(3-methoxyphenyl)-1,2,3,5,6,8a-hexahydroindolizin-7-yl]-3-(pyridin-4-yl)-1H-pyrrole (Exemplary Compound No. 1-203),
2-(3,4-difluorophenyl)-4-[(2S,8aS)-2-(4-methoxyphenyl)-1,2,3,5,6,8a-hexahydroindolizin-7-yl]-3-(pyridin-4-yl)-1H-pyrrole (Exemplary Compound No. 1-204),
4-[(2S,8aS)-2-(4-ethoxyphenyl)-1,2,3,5,6,8a-hexahydroindolizin-7-yl]-2-(3,4-difluorophenyl)-3-(pyridin-4-yl)-1H-pyrrole (Exemplary Compound No. 1-207),
2-(3,4-difluorophenyl)-4-[(2S,8aS)-2-(3-hydroxyphenyl)-1,2, 3, 5,6,8a-hexahydroindoli zin-7-yl]-3-(pyridin-4-yl)-1H-pyrrole (Exemplary Compound No. 1-215),
2-(3-methoxyphenyl)-4-[(2S,8aS)-2-phenyl-1,2,3,5,6,8a-hexahydroindolizin-7-yl]-3-(pyridin-4-yl)-1H-pyrrole (Exemplary Compound No. 1-250),
2-(4-fluoro-3-methoxyphenyl)-4-[(2S,8aS)-2-phenyl-1,2,3,5,6,8a-hexahydroindolizin-7-yl]-3-(pyridin-4-yl)-1H-pyrrole (Exemplary Compound No. 1-318),
2-(3-chloro-4-fluorophenyl)-4-[(2S,8aS)-2-phenyl-1,2,3,5,6,8a-hexahydroindolizin-7-yl]-3-(pyridin-4-yl)-1H-pyrrole (Exemplary Compound No. 1-420),
2-phenyl-4-[(2S,8aS)-2-phenyl-1,2,3,5,6,8a-hexahydroindolizin-7-yl]-3-(pyridin-4-yl)-1H-pyrrole (Exemplary Compound No. 1-477),
2-(4-fluorophenyl)-4-[(2S,8aS)-2-(4-methoxyphenyl)-1,2,3,5,6,8a-hexahydroindolizin-7-yl]-3-(pyridin-4-yl)-1H-pyrrole (Exemplary Compound No. 1-480),
2-(3-chlorophenyl)-4-[(2S,8aS)-2-phenyl-1,2,3,5,6,8a-hexahydroindolizin-7-yl]-3-(pyridin-4-yl)-1H-pyrrole (Exemplary Compound No. 1-483),
4-[(2S,8aS)-2-phenyl-1,2,3,5,6,8a-hexahydroindolizin-7-yl]-3-(pyridin-4-yl)-2-(3-trifluoromethylphenyl)-1H-pyrrole (Exemplary Compound No. 1-484),
4-[(2S,8aS)-2-(4-methylphenyl)-1,2,3,5,6,8a-hexahydroindolizin-7-yl]-3-(pyridin-4-yl)-2-(3-trifluoromethylphenyl)-1H-pyrrole (Exemplary Compound No. 1-486),
4-[(2S,8aS)-2-butyl-1,2,3, 5,6,8a-hexahydroindolizin-7-yl]-2-(4-fluorophenyl)-3-(pyridin-4-yl)-1H-pyrrole (Exemplary Compound No. 1-478),
2-(4-fluorophenyl)-4-[(2S,8aS)-2-phenyl-1,2,3,5,6,8a-hexahydroindolizin-7-yl]-3-(pyridin-4-yl)-1H-pyrrole (Exemplary Compound No. 1-479),
2-(4-fluorophenyl)-4-[(2S,8aS)-2-(4-methylphenyl)-1,2,3,5,6,8a-hexahydroindolizin-7-yl]-3-(pyridin-4-yl)-1H-pyrrole (Exemplary Compound No. 1-481),
2-(4-fluorophenyl)-4-[(2R,8aS)-2-(4-fluorophenyl)-1,2,3,5,6,8a-hexahydroindolizin-7-yl]-3-(pyridin-4-yl)-1H-pyrrole (Exemplary Compound No. 1-482),
2-(3,4-difluorophenyl)-4-[(2S,8aS)-2-phenyl-1,2,3,5,6,8a-hexahydroindolizin-7-yl]-3-(pyridin-4-yl)-1H-pyrrole (Exemplary Compound No. 1-487),
2-(3,4-difluorophenyl)-4-[(2S,8aS)-2-(4-methylphenyl)-1,2,3,5,6,8a-hexahydroindolizin-7-yl]-3-(pyridin-4-yl)-1H-pyrrole (Exemplary Compound No. 1-191).
4-[(2S,8aS)-2-(4-ethylphenyl)-1,2,3,5,6,8a-hexahydroindolizin-7-yl]-2-(3,4-difluorophenyl)-3-(pyridin-4-yl)-1H-pyrrole (Exemplary Compound No. 1-194),
5-(3-chloro-4-fluorophenyl)-3-[(2S,8aS)-2-phenyl-1,2,3,5,6,8a-hexahydroindolizin-7-yl]-4-(pyridin-4-yl)pyrazole (Exemplary Compound No. 3-403),
5-(4-fluorophenyl)-3-[(2S,8aS)-2-propyl-1,2,3,5,6,8a-hexahydroindolizin-7-yl]-4-(pyridin-4-yl)pyrazole (Exemplary Compound No. 3-494),
3- [(2S,8aS)-2-butyl-1,2,3,5,6,8a-hexahydroindolizin-7-yl]-5-(4-fluorophenyl)-4-(pyridin-4-yl)pyrazole (Exemplary Compound No. 3-495),
5-(4-fluorophenyl)-3-[(2S,8aS)-2-phenyl-1,2,3,5,6,8a-hexahydroindolizin-7-yl]-4-(pyridin-4-yl)pyrazole (Exemplary Compound No. 3-496) and
5-(4-fluorophenyl)-3-[(2S,8aS)-2-(4-methylphenyl)-1,2,3,5,6,8a-hexahydroindolizin-7-yl]-4-(pyridin-4-yl)pyrazole (Exemplary Compound No. 3-497).

[Mode for carrying out Invention]

The compounds of formula (I) can be easily prepared according to Process A to Process D as shown below.
Process A is a method for the preparation of a compound in which $R^3$ is bonded to a carbon atom on ring A of the compound of the formula (I).

(Process A)

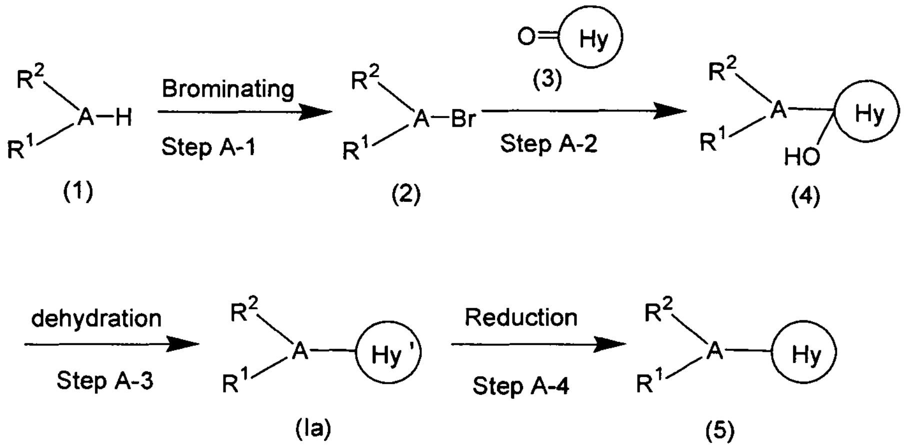


(1) (2) (4) (Ia) (5)

In the above formulae, A, $R^1$ and $R^2$ have the same meanings as defined above. Cyclic group Hy represents a group (indolidizine group) in which the group of the above formula (II) is saturated and cyclic group Hy' represents a group of the above formula (II).

Step A-1 is to prepare bromo cyclic compound (2) by brominating cyclic compound (1) using a brominating agent (for example, N-bromosuccinimide or the like), and Step A-2 is to prepare condensed group substituted cyclic compound (4) by lithiation of bromo cyclic compound (2) and reaction with heterocyclyl ketone (3). Step A-1 and Step A-2 can be carried out according to a method described in detail in Brian L. Bray et al., J. Org. Chem., vol.55, 6317-6318 (1990).

Further, compound (4) can be also prepared by direct lithiation of cyclic compound (1) by a method similar to the method described in L. Revesz et al., Bioorg. Med. Chem. Ltt., 10, 1261-1264 (2000), and reaction of the thus obtained compound with heterocyclyl ketone (3).

Step A-3 is to prepare compound (Ia) of the present invention by carrying out a dehydration reaction of condensed group substituted cyclic compound (4). The dehydration reaction is usually carried out in the presence of an acid catalyst such as sulfuric acid, a solid catalyst such as alumina or a halogenating agent such as thionyl chloride [these reactions are described in detail, for example, in G.H. Coleman & H.F. Johnstone, Org. Synth., I, 183 (1941), R.L. Sawyer & D.W Andrus, Org. Synth., III, 276 (1955) and J.S. Lomas et al., Tetrahedron Lett., 599 (1971)]. Further, the dehydration reaction of the present step can be also accomplished by reaction using a trialkylsilane such as triethylsilane, tripropylsilane or tributylsilane, and trifluoroacetic acid (for example, Francis A. Carey & Henry S. Tremper, J. Am. Chem. Soc., 91, 2967-2972 (1969).

Step A-4 is to prepare a compound by reduction of a double bond of the compound (Ia) of the present invention and can be carried out, for example, according to the method described in S.M. Kerwin et al., J. Org. Chem., 52, 1686 (1987), T. Hudlicky et al., J. org. Chem., 52, 4641 (1987), etc.

Further, in the above Process A, in the case where a compound in which A is pyrrole is synthesized, a compound in which the nitrogen atom at the 1-position of pyrrole is previously protected is subjected to Step A-1, and it is desirable to synthesize such a compound by deprotecting it after Step A-2 or Step A-3.

The protection of the nitrogen atom at the 1-position of pyrrole can be carried out, for example, by dissolving the pyrrole compound corresponding to the compound (1) in 300 ml of tetrahydrofuran, adding butyl lithium and triisopropylsilyl triflate thereto under ice-cooling (for example -78°C), and stirring it at room temperature. The deprotection reaction is usually carried out in a solvent in the presence of a base and can be carried out, for example, by adding saturated aqueous sodium hydrogencarbonate solution to the protected compound.

Further, in the above Process A, it is desirable that compound (3) is a compound in which the nitrogen atom in the indolizine ring of the formula (II) is protected, and the deprotection is carried out, if desired, after a required step is carried out. As this protection reaction and deprotection reaction, deprotection reactions (for example, the reactions described in T.W. Greene et al., Protective Groups in Organic Synthesis, John Willey & Sons, Inc.) conventionally and generally used in organic synthesis can be adopted.

(Process B)

Process B is a process for the preparation of a compound in which $R^3$ is bonded to a nitrogen atom of the cyclic group A.

<Process B>

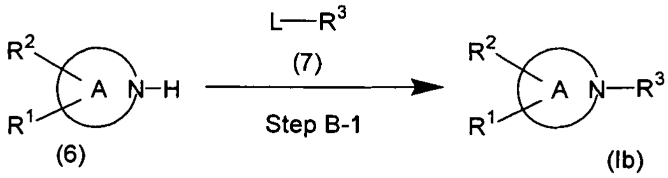


(6)

(Ib)

In the above formulae, A, $R^1$, $R^2$ and $R^3$ have the same meanings as defined above and L represents a leaving group.

The "leaving group" in the definition of L usually represents a group eliminated as a nucleophilic residue and can be, for example, a halogen atom such as fluorine, chlorine, bromine and iodine; a lower alkanesulfonyloxy group such as methanesulfonyloxy and ethanesulfonyloxy; a halogeno lower alkanesulfonyloxy group such as trifluoromethanesulfonyloxy and pentafluoroethanesulfonyloxy; or an arylsulfonyloxy group such as benzenesulfonyloxy, p-toluenesulfonyloxy and p-nitrobenzenesulfonyloxy, and is preferably a halogen atom, particularly preferably a bromine atom.

Step B-1 is to prepare compound (Ib) of the present invention by reacting compound (6) with heterocyclyl compound (7). The present reaction is usually carried out in a solvent in the presence or absence of a base.

The solvent to be employed here can be, for example, an alcohol such as methanol, ethanol, propanol and isopropanol; an ether such as diethyl ether, diisopropyl ether, tetrahydrofuran and dioxane; an aprotic polar solvent such as dimethylformamide, dimethylacetamide and dimethyl sulfoxide; a nitrile such as acetonitrile; an ester such as methyl acetate and ethyl acetate; an aromatic hydrocarbon such as benzene, toluene and xylene; or an aliphatic hydrocarbon such as pentane, hexane and heptane, and is preferably an alcohol, further preferably methanol or ethanol.

The base to be employed here can be, for example, an alkali metal alkoxide such as sodium methoxide, sodium ethoxide and potassium t-butoxide; an alkali metal hydride such as sodium hydride and lithium hydride; an alkali metal hydroxide such as sodium hydroxide and potassium hydroxide; an alkali metal carbonate such as sodium carbonate and potassium carbonate; or an amine such as triethylamine, tributylamine, pyridine, picoline and 1,8-diazabicyclo[5.4.0]-7-undecene, and is preferably an amine, further preferably triethylamine, pyridine or 1,8-diazabicyclo [5.4.0]-7-undecene.

The reaction is usually carried out at from -20°C to 150°C, preferably from 0°C to 100°C. The reaction time is usually from 10 minutes to 48 hours, preferably from 30 minutes to 12 hours.

Of compounds (I) of the present invention, compounds (Ic) in which $R^2$ is a heteroaryl group having at least one nitrogen atom and substituted by $NR^aR^b$ can be also prepared by the following (Process C).

<Process C>

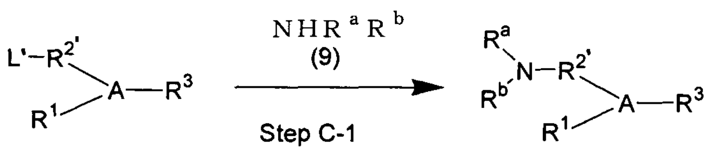


(8)

(Ic)

In the above formulae, A, $R^1$, $R^3$, $R^a$ and $R^b$ have the same meanings as defined above, L' represents a leaving group, the group $-R^{2'}-L'$ represents a "heteroaryl group having at least one nitrogen atom" having a leaving group (group L') (for example, 2-methanesulfonylpyrimidin-4-yl, 2-methanesulfonylpyridin-4-yl, etc.) and the "heteroaryl group having at least one nitrogen atom" represents a group similar to the "heteroaryl group having at least one nitrogen atom" in the definition of $R^2$.

The leaving group in the definition of L' represents a group similar to the leaving group in the definition of L; a lower alkylsulfonyl group such as methanesulfonyl, ethanesulfonyl, propanesulfonyl and butanesulfonyl; or an arylsulfonyl group such as benzenesulfonyl, p-toluenesulfonyl and p-nitrobenzenesulfonyl, and is preferably a lower alkylsulfonyl group, more preferably methanesulfonyl.

Step C-1 is to prepare compound (Ic) of the present invention by converting the leaving group to $NR^aR^b$ by reacting compound (8) with amine compound (9). The present reaction is carried out similarly to Step B-1.

The compounds which are the starting materials in the above Process A to Process C, i.e., the compounds

(1), (3), (6), (7) and (9), are publicly known compounds themselves, or the compounds are easily obtained by treating publicly known compounds according to publicly known methods. Compound (8) can be easily synthesized by carrying out reactions similar to the above Process A to Process C starting from publicly known compounds.

For example, with respect to compound (1), compounds in which A is pyrrole can be synthesized according to EP1070711, etc., and compounds in which A is pyrazole can be synthesized according to WO0031063, WO9958523, WO0039116 and WO9531451.

Further, compound (3) can be synthesized according to any one of the following (Process D) to (Process H).

Compound (7) can be synthesized according to the following (Process K).

<Process D>

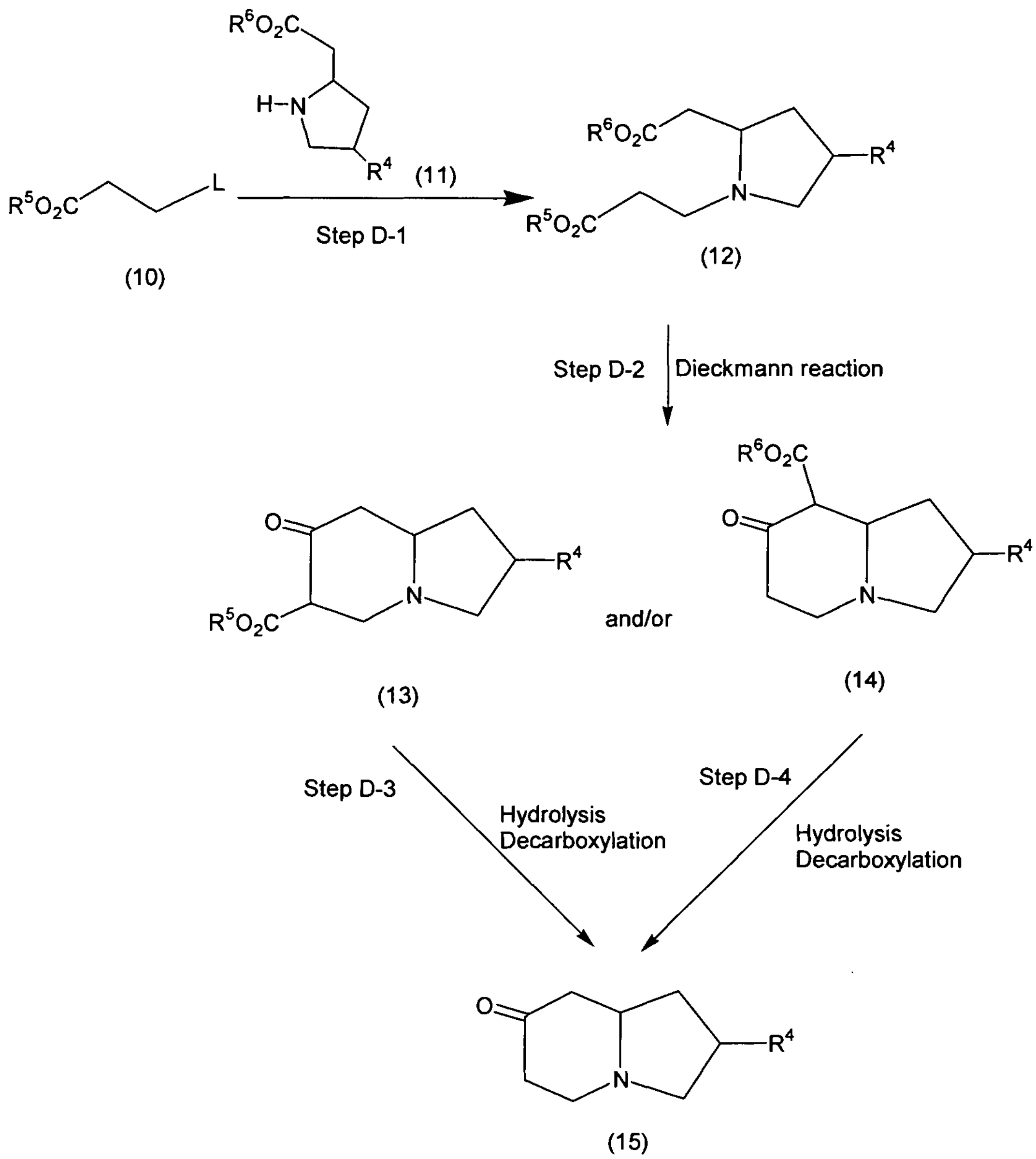


(11) (10) (12)

(13) and/or (14)

(15)

In the above formulae, L and $R^4$ have the same meanings as defined above and $R^5$ and $R^6$ are the same or different and each represents the above "lower alkyl group" or the above "aralkyl group".

Step D-1 is to prepare cyclic amine diester compound (12) by adding cyclic amino acid ester compound (11) to carboxylic acid ester compound (10) having a leaving group (L).

The present reaction is usually carried out in a solvent in the presence or absence of a base.

The solvent to be employed here can be, for example, an alcohol such as methanol, ethanol, propanol and isopropanol; an ether such as diethyl ether, diisopropyl ether, tetrahydrofuran and dioxane; an aprotic polar solvent such as dimethylformamide, dimethylacetamide and dimethyl sulfoxide; a nitrile such as acetonitrile; an ester such as methyl acetate and ethyl acetate; an aromatic hydrocarbon such as benzene, toluene and xylene; or an aliphatic hydrocarbon such as pentane, hexane and heptane.

The base to be employed here can be, for example, an alkali metal alkoxide such as sodium methoxide, sodium ethoxide and potassium t-butoxide; an alkali metal hydride such as sodium hydride and lithium hydride; an alkali metal hydroxide such as sodium hydroxide and potassium hydroxide; an alkali metal carbonate such as sodium carbonate and potassium carbonate; or an amine such as triethylamine, tributylamine, pyridine, picoline and 1,8-diazabicyclo[5.4.0]-7-undecene.

The reaction is usually carried out at from -20°C to 150°C, preferably from 0°C to 100°C. The reaction time is usually from 10 minutes to 48 hours, preferably from 30 minutes to 12 hours.

Next, after cyclic amine diester compound (12) is subjected to a Dieckmann reaction (Step D-2) to prepare a keto ester compound [compound (13) and/or compound (14)], from each compound respectively, the desired cyclic amino ketone compound [compound (15)] is prepared by carrying out a hydrolysis and decarboxylation reaction (Step D-3 and Step D-4).

The reactions adopted in the above Step D-2 to Step D-4 can be carried out according to a method described in J.R. Harrison et al., J. Chem. Soc., Perkin Trans. 1, 1999, 3623-3631, and Step D-3 and Step D-4 are carried out, for example, as follows.

The reactions of Step D-3 and Step D-4 are usually carried out in the presence or absence of a solvent and in the presence or absence of an acid or a base.

The solvent to be employed here can be water, or a mixture of water and an organic solvent (for example, an aliphatic hydrocarbon such as pentane, hexane and heptane; an aromatic hydrocarbon such as benzene, toluene and xylene; a halogenated hydrocarbon such as dichloromethane, chloroform, carbon tetrachloride and dichloroethane; an ether such as diethyl ether, diisopropyl ether, tetrahydrofuran and dioxane; an alcohol such as methanol, ethanol, propanol, isopropanol, butanol, s-butanol, isobutanol and t-butanol; an aprotic polar solvent such as N,N-dimethylformamide, N,N-dimethylacetamide and dimethyl sulfoxide; a nitrile such as acetonitrile; or an ester such as methyl acetate and ethyl acetate), and is preferably water, or a mixture of water and an alcohol or an ether.

The acid to be employed here is not particularly limited so long as it is used as an acid in conventional hydrolysis reactions, and can be, for example, a mineral acid such as hydrochloric acid, sulfuric acid and phosphoric acid; a carboxylic acid such as formic acid, acetic acid, propionic acid and trifluoroacetic acid; or a sulfonic acid such as methanesulfonic acid and ethanesulfonic acid. Of these, a mineral acid or a carboxylic acid is preferably used, and hydrochloric acid, sulfuric acid, formic acid or acetic acid is more preferably used.

Further, the progression of the present reaction is promoted by the addition of the acid.

The base to be employed here is not particularly limited so long as it is used as a base in conventional hydrolysis reactions, and can be, for example, an alkali metal hydride such as sodium hydride and lithium hydride; an alkali metal hydroxide such as sodium hydroxide and potassium hydroxide; an alkali metal carbonate such as sodium carbonate and potassium carbonate; or an amine such as triethylamine, tributylamine, pyridine, picoline and 1,8-diazabicyclo[5.4.0]-7-undecene. Of these, an alkali metal hydroxide is preferably used, and sodium hydroxide or potassium hydroxide is more preferably used.

The reaction is usually carried out at from -20°C to 150°C, preferably from 0°C to 100°C. The reaction time is usually from 10 minutes to 48 hours, preferably from 30 minutes to 12 hours.

<Process E>

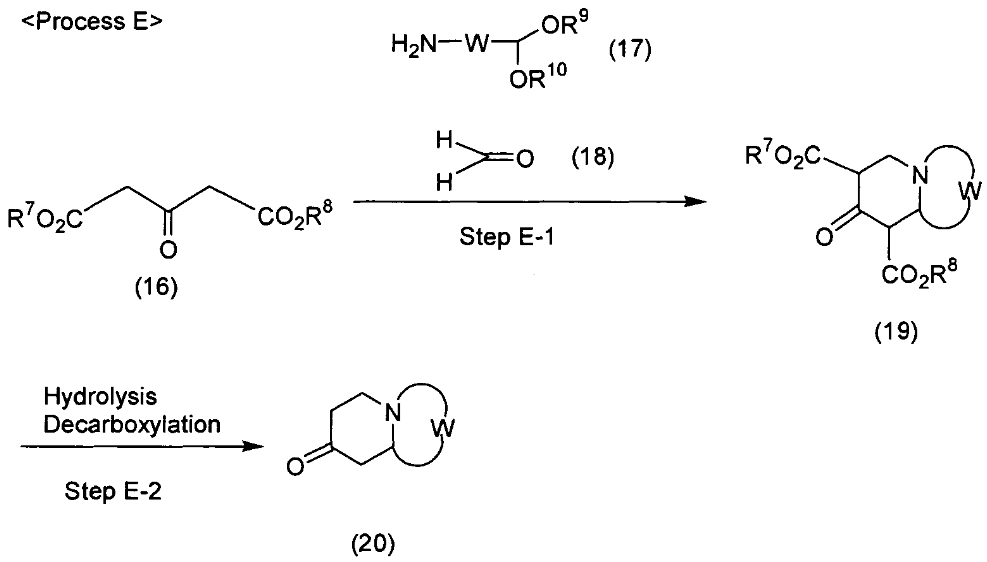


In the above formulae, $R^7$ and $R^8$ are the same or different and each represents the above "lower alkyl group" or the above "aralkyl group", $R^9$ and $R^{10}$ are the same or different and each represents the above "lower alkyl group", or $R^9$ and $R^{10}$ together represent the above "lower alkylene group", W represents a group represented by the formula: [$-CH_2-CH(R^4)-CH_2-$] and $R^4$ has the same meaning as defined above.

Step E-1 and Step E-2 can be carried out according to a reaction described in detail in O. Pollet et al., Heterocycles, 43, 1391 (1996) or Anet et al., Austral. J. Scient. Res., <A>3, 635-640 (1950).

<Process F>

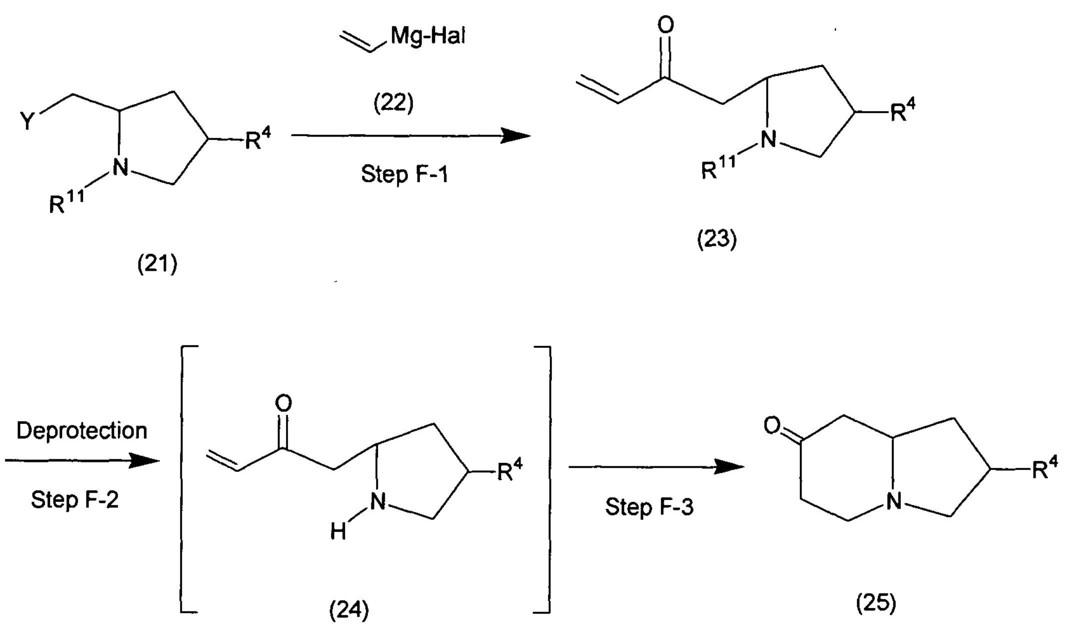


In the above formulae, $R^4$ has the same meaning as defined above, $R^{11}$ represents a protecting group of an amino group, Hal represents a halogen atom (preferably a chlorine atom, a bromine atom or an iodine atom), and Y represents a halogeno carbonyl group (for example, -CO-Cl, -CO-Br or -CO-I), an N-lower alkoxy-N-lower alkylcarbamoyl group (for example, N-methoxy-N-methylcarbamoyl, N-ethoxy-N-methylcarbamoyl, N-ethyl-N-methoxycarbamoyl, etc.) or a cyano group.

The "protecting group of an amino group" in the definition of $R^{11}$ means a protecting group of an amino group conventionally used in organic synthesis, and this group can be, for example, the above "aliphatic acyl group", the above "aromatic acyl group", the above "silyl group", the above "aralkyl group", the above "alkoxycarbonyl group", the above "alkenyloxycarbonyl group" or the above "aralkyloxycarbonyl group".

Step F-1 is to prepare α,β-unsaturated ketone (23) by reacting cyclic amino acid derivative (21) with a vinylic Grignard reagent (22). In the present step, the well known reaction for synthesizing ketones from carboxylic acid derivatives and Grignard reagents can be adopted, and the present step is, for example, carried out according to the methods described in detail in H.R. Snyder et al., Org. Synth., III, 798 (1955); J. Cason et al., J. Org. Chem., 26, 1768 (1961); G.H. Posner et al., J. Am. Chem. Soc., 94, 5106 (1972); and G.H. Posner, Org. React., 19, 1 (1972).

Next, the desired cyclic amino ketone compound (25) can be prepared by removing the protecting group ($R^{11}$) from the nitrogen atom of the α,β-unsaturated ketone (23) to make the free amine (24) (Step F-2), and ring-closure of the thus obtained compound (Step F-3). In Step F-2, deprotection reactions (for example, the reactions described in T.W. Greene et al., Protective Groups in Organic Synthesis, John Willey & Sons, Inc.) conventionally and generally used in organic synthesis can be adopted, and a deprotection reaction under neutral or acidic conditions is preferably adopted. After this deprotection reaction, the compound (24) produced is immediately subjected to ring-closure to produce the desired amino ketone compound (25). In Step F-2, in the case where the deprotection is carried out under acidic conditions, the amino ketone compound (25) is immediately produced by neutralizing the reaction solution.

<Process G>

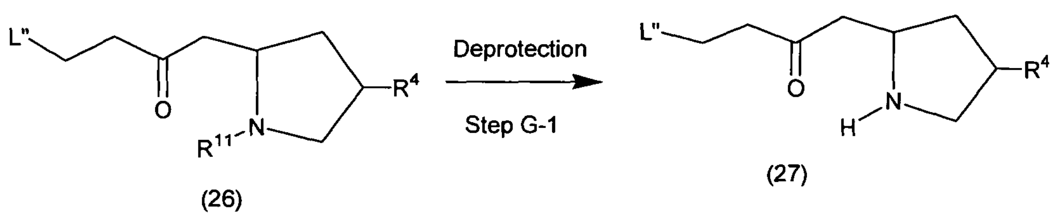


(26)

(27)

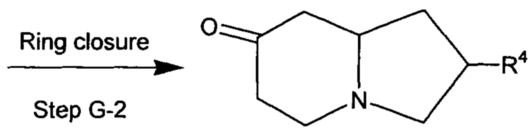


(28)

In the above formulae, $R^4$ and $R^{11}$ have the same meanings as defined above, L" represents the leaving group in the definition of L, the above "lower alkylsulfonyl group", the above "arylsulfonyl group", or a halogeno lower alkylsulfonyl group (for example, trifluoromethanesulfonyl, pentafluoroethanesulfonyl, etc.).

Step G-1 and Step G-2 are to prepare the desired amino ketone compound (28) by removing the protecting group ($R^{11}$) from ketone compound (26) having a leaving group, to obtain the free amine (27), and then ring-closing the free amine, and are carried out similarly to Step F-2 and Step F-3. Further, compound (26), i.e., the starting material in the present Process, is publicly known or can be prepared according to publicly known methods (for example, the method described in S.W Goldstein et al., J. Org. Chem., 57, 1179-1190 (1992), B. Achille et al., J. Comb. Chem., 2, 337-340 (2000), etc.) from publicly known compounds.

<Process H>

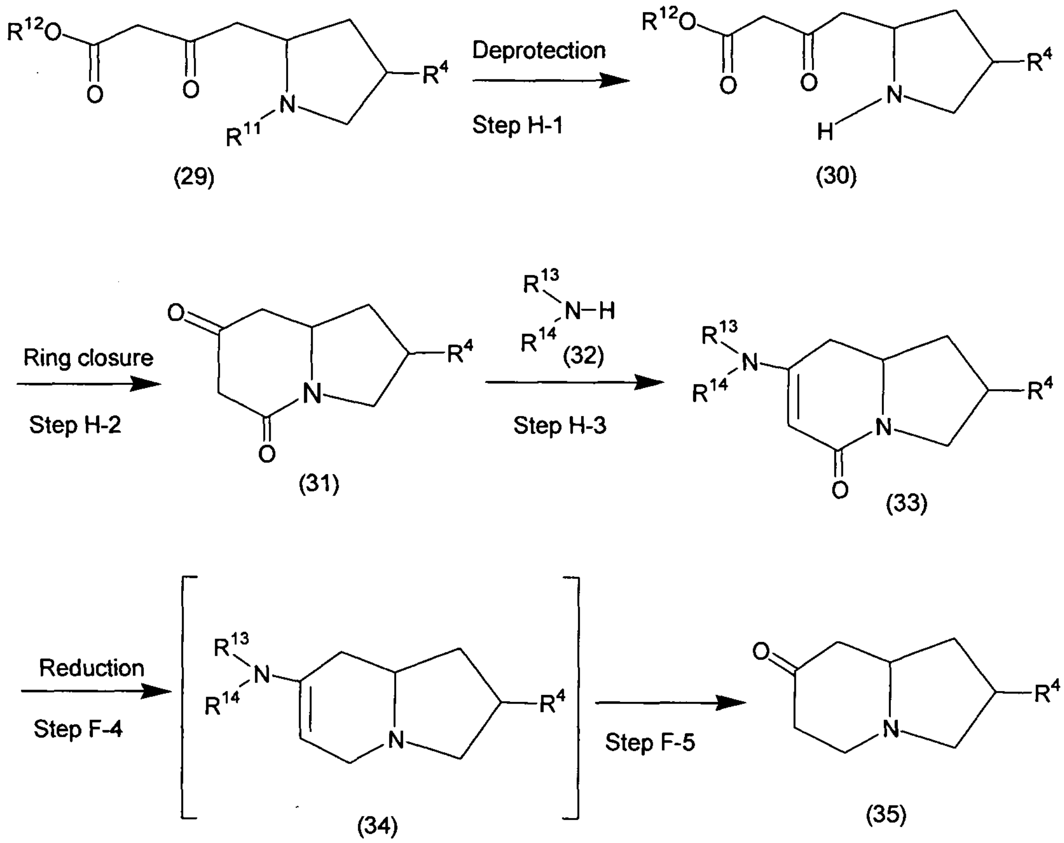


(29) (30) (31) (32) (33) (34) (35)

In the above formulae, $R^4$ and $R^{11}$ have the same meanings as defined above, $R^{12}$ represents a hydrogen atom or a protecting group of a carboxyl group, $R^{13}$ and $R^{14}$ are the same or different and each represents a hydrogen atom, the above "lower alkyl group" or the above "aralkyl group", or $R^{13}$ and $R^{14}$, together with the nitrogen atom to which they are bonded, form a 5- or 6-membered heterocyclyl group which contains one nitrogen atom and may further contain one oxygen atom, sulfur atom or nitrogen atom, such as piperidyl, piperazinyl, morpholinyl and thiomorpholinyl.

The "protecting group of a carboxyl group" in the definition of $R^{12}$ represents a protecting group of a carboxyl group conventionally used in the field of organic synthesis, and this protecting group can be, for example, the above "lower alkyl group", the above "lower alkenyl group" or the above "aralkyl group", and is preferably the above "lower alkyl group" or the above "aralkyl group".

Step H-1 and Step H-2 are to prepare ketolactam compound (31) by first removing the protecting group ($R^{11}$) from $\alpha$-keto acid compound (29) having a leaving group, to obtain the free amine (30), and then ring-closing the free amine, and are carried out by a method similar to the method mentioned as Step F-2 and Step F-3.

Step H-3 is to prepare cyclic enamino lactam compound (33) by reacting ketolactam compound (31) and secondary amine compound (32). In the present step, methods for the synthesis of enamines generally used in the field of organic synthesis can be adopted, and the present step is carried out, for example, according to the method described in G. Stork et al., J. Am. Chem. Soc., 85, 207 (1963) and is carried out, for example, as follows.

The reaction is usually carried out in a solvent in the presence or absence of an acid.

The solvent to be employed here can include, for example, an aliphatic hydrocarbon such as pentane, hexane and heptane; an aromatic hydrocarbon such as benzene, toluene and xylene; a halogenated hydrocarbon such as dichloromethane, chloroform, carbon tetrachloride and dichloroethane; an ether such as diethyl ether, diisopropyl ether, tetrahydrofuran and dioxane; an alcohol such as methanol, ethanol, propanol, isopropanol, butanol, s-butanol, isobutanol and t-butanol; an aprotic polar solvent such as N,N-dimethylformamide, N,N-dimethylacetamide and dimethyl sulfoxide; a nitrile such as acetonitrile; or an ester such as methyl acetate and ethyl acetate. Of these, an ether is preferably used.

The acid to be employed here can be an inorganic acid such as hydrochloric acid, hydrobromic acid, sulfuric acid, perchloric acid and phosphoric acid; or an organic acid such as acetic acid, formic acid, oxalic acid, methanesulfonic acid, para-toluenesulfonic acid, trifluoroacetic acid and trifluoromethane sulfonic acid, and is preferably sulfuric acid, hydrochloride or para-toluenesulfonic acid.

In the present step, the reaction can be carried out efficiently by removing the produced water by adding molecular sieves or using a water separation device (for example, Dean Stark Water Separator: manufactured by Aldrich).

The reaction is usually carried out at from -20°C to 150°C, preferably from 0°C to 100°C. The reaction time is usually from 10 minutes to 48 hours, preferably from 30 minutes to 12 hours.

Step H-4 is to prepare cyclic enamine compound (34) by reducing cyclic enamino lactam compound (33). In the present step, reduction reactions for producing an amine from an amide generally used in the field of organic synthesis can be adopted, and the step is carried out according to the method described in S. Cortes et al., J. Org. Chem., 48, 2246 (1983); Y. Tsuda et al., Synthesis, 652 (1977); H.C. Brown et al., J. Am. Chem. Soc., 86, 3566 (1964) and R.J. Sundberg et al., J. org. Chem., 46, 3730 (1981), and is carried out, for example, as follows.

The present reaction is usually carried out in a solvent in the presence of a reducing agent.

The reducing agent to be employed here can be, for example, a hydride reagent such as an alkali metal borohydride, e.g., sodium borohydride and lithium borohydride, or an aluminum hydride compound, e.g., lithium aluminum hydride and lithium triethoxide aluminum hydride; a combination of a Lewis acid such as aluminum chloride, tin tetrachloride and titanium tetrachloride and the above "hydride reagent"; or a boron compound such as diborane. Of these, lithium aluminum hydride is preferably used.

As the solvent, nonpolar solvents can be used, and aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; and ethers such as diethyl ether, diisopropyl ether, tetrahydrofuran and dioxane are preferably used. Of these, ethers are more preferably used.

The reaction is usually carried out at from -20°C to 150°C, preferably from 0°C to 100°C. The reaction time is usually from 10 minutes to 48 hours, preferably from 30 minutes to 12 hours.

Step H-5 is to prepare cyclic aminoketone compound (35) by hydrolyzing cyclic enamine compound (34). The reaction is carried out by contacting cyclic enamine compound (34) with water in the presence or absence of a solvent and in the presence or absence of an acid or a base.

The solvent can include water or a mixed solvent of water and an organic solvent (for example, an aliphatic hydrocarbon such as pentane, hexane and heptane; an aromatic hydrocarbon such as benzene, toluene and xylene; a halogenated hydrocarbon such as dichloromethane, chloroform, carbon tetrachloride and dichloroethane; an ether such as diethyl ether, diisopropyl ether, tetrahydrofuran and dioxane; an alcohol such as methanol, ethanol, propanol, isopropanol, butanol, s-butanol, isobutanol and t-butanol; an aprotic polar solvent such as N,N-dimethylformamide, N, N-dimethylacetamide and dimethyl sulfoxide; a nitrile such as acetonitrile; or an ester such as methyl acetate and ethyl acetate). Of these, water, or a mixed solvent of water and an alcohol or an ether is preferably used.

The acid to be employed here is not particularly limited so long as it is used as an acid in conventional hydrolysis reactions, and can be, for example, a mineral acid such as hydrochloric acid, sulfuric acid and phosphoric acid; a carboxylic acid such as formic acid, acetic acid, propionic acid and trifluoroacetic acid; or a sulfonic acids such as methanesulfonic acid and ethanesulfonic acid. Of these, hydrochloric acid, sulfuric acid or acetic acid is preferably used.

The progression of the present reaction is promoted by the addition of the acid.

The base to be employed here is not particularly limited so long as it is used as a base in conventional hydrolysis reactions, and can be, for example, an alkali metal hydride such as sodium hydride and lithium hydride; an alkali metal hydroxide such as sodium hydroxide and potassium hydroxide; an alkali metal carbonate such as sodium carbonate and potassium carbonate; or an amine such as triethylamine, tributylamine, pyridine, picoline and 1,8-diazabicyclo[5.4.0]-7-undecene. Of these, sodium hydroxide or potassium hydroxide is preferably used.

The reaction is usually carried out at from -20°C to 150°C, preferably from 0°C to 100°C. The reaction time is usually from 10 minutes to 48 hours, preferably from 30 minutes to 12 hours.

Compound (33), i.e., the intermediate in the preparation of cyclic amino ketone compound (35), can be also prepared by the following (Process I).

<Process I>

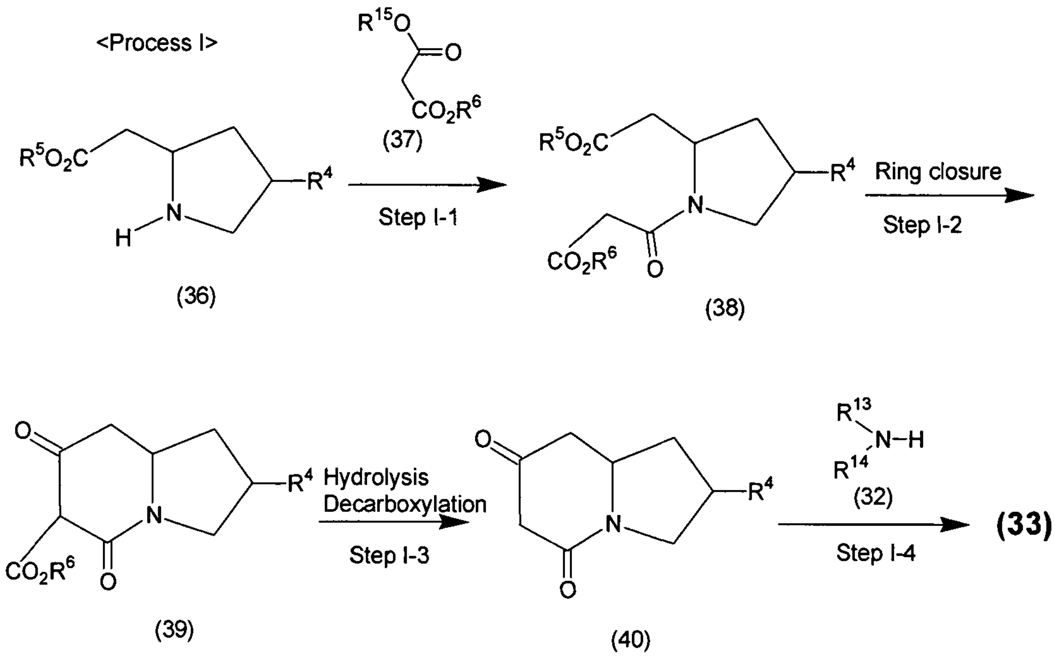


In the above formulae, $R^4$, $R^5$, $R^6$, $R^{13}$ and $R^{14}$ have the same meanings as defined above, and $R^{15}$ represents a hydrogen atom or a protecting group of a carboxyl group.

The "protecting group of a carboxyl group" in the definition of $R^{15}$ means a protecting group of a carboxyl group conventionally used in organic synthesis, and this group can preferably include the above "lower alkyl group" and the above "aralkyl group".

Step I-1 is to prepare amino diester compound (38) by reacting cyclic amino acid ester compound (36) with malonic acid derivative (37) or a reactive derivative thereof. In the present step, amidation reactions generally used in the field of organic synthesis can be adopted, and the step is carried out, for example, as shown in the following (a), (b) and (c).

(a) In the case where $R^{15}$ is a hydrogen atom, the reaction is carried out in a solvent in the presence of a condensing agent and in the presence or absence of a base.

The solvent to be employed here can include aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethylacetamide and dimethyl sulfoxide; nitriles such as acetonitrile; esters such as methyl acetate and ethyl acetate; water; or mixed solvents of these. Of these, a halogenated hydrocarbon, an ether or an ester is preferably used, and dichloromethane, tetrahydrofuran or ethyl acetate is more preferably used.

The condensing agent can include, for example, dicyclohexylcarbodiimide, 1-ethyl-3-(3-dimethylaminopropyl) carbodiimide or N,N'-carbonyldiimidazole.

The base to be employed here can include, for example, alkali metal alkoxides such as sodium methoxide, sodium ethoxide and potassium t-butoxide; alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0]-7-undecene. Of these, an amine is preferably used and triethylamine, pyridine or 1,8-diazabicyclo [5.4.0]-7-undecene is more preferably used.

The reaction is usually carried out at from -20°C to 150°C, preferably from 0°C to 100°C. The reaction time is usually from 10 minutes to 48 hours, preferably from 30 minutes to 12 hours.

Further, in the case where $R^{15}$ is a hydrogen atom, the reaction can be also carried out according to the method described in (c), after compound (37) is converted into a reactive derivative.

(b) In the case where $R^{15}$ is a protecting group of a carboxyl group (preferably the above "lower alkyl group" or the above "aralkyl group"), the reaction is accomplished by heating in the presence or absence of a solvent.

In the case where the reaction is carried out in a solvent, a similar solvent to the solvents described in (a) can be used, and it is preferable that the reaction temperature is from 30°C to 100°C, preferably in a range of ±5°C of the boiling point of the solvent used. Most preferably, the reaction is carried out by heating the reaction solution under reflux.

In the case where a solvent is not used, the reaction can be carried out by mixing compound (36) and compound (37) and heating them. The reaction temperature is from 30°C to 150°C, preferably from 50°C to 120°C.

The reaction time is usually from 10 minutes to 48 hours, preferably from 30 minutes to 12 hours.

(c) In the case where a reactive derivative of compound (37) is used, the "reactive derivative" represents an acid halide, a mixed acid anhydride, an active ester or an active amide, and the reaction is carried out in a solvent in the presence of a condensing agent and in the presence or absence of a base.

The "acid halide" can be obtained by reacting compound (37) in which $R^{15}$ is hydrogen with a halogenating agent (for example, thionyl chloride, oxalyl chloride, etc.); the "mixed acid anhydride" can be obtained by reacting compound (37) in which $R^{15}$ is hydrogen with an acid halide (for example, methyl chlorocarbonate, ethyl chlorocarbonate, etc.); the "active ester" can be obtained by reacting compound (37) in which $R^{15}$ is hydrogen with a hydroxy compound (for example, N-hydroxysuccinimide, N-hydroxyphthalimide, etc.) in the presence of the "condensing agent" mentioned in (a); and the "active amide" (for example, the Weinreb amide) can be obtained by reacting compound (37) in which $R^{15}$ is hydrogen with an N-lower alkoxy-N-loweralkylhydroxylamine (for example, N-methoxy-N-methylhydroxylamine) in the presence of the "condensing agent" mentioned in (a). Each reaction is carried out by applying reaction conditions generally used in conventional organic synthesis chemistry.

As the solvent, condensing agent and base, the solvents, condensing agents and bases described in (a) can be used.

The reaction is usually carried out at from -20°C to 150°C, preferably from 0°C to 100°C. The reaction time is usually from 10 minutes to 48 hours, preferably from 30 minutes to 12 hours.

In Step I-2 and Step I-3, amide diester compound (38) is subjected to a Dieckman reaction to prepare ketollactam ester compound (39), and then a hydrolysis and decarboxylation reaction are carried out to prepare ketolactam compound (40). Step I-2 and Step I-3 are carried out similarly to Step D-2 and Step D-3.

Step I-4 is to prepare cyclic enamino lactam compound (34) by reacting ketolactam compound (40) with secondary amine compound (32), and the step is carried out similarly to Step H-3.

Further, compound (39), i.e., the intermediate in the above (Process I) can be also prepared by the following (Process J).

<Process J>

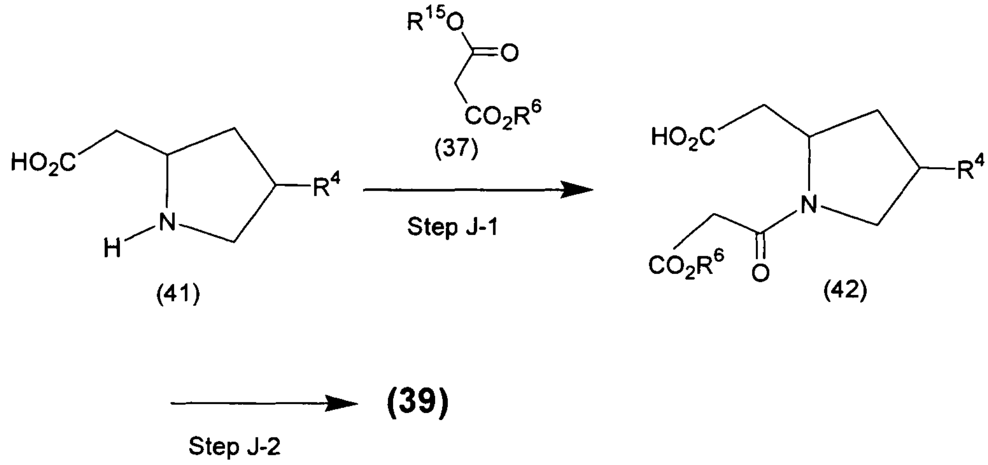


(37) (41) (42) (39)

In the above formulae, $R^4$, $R^6$ and $R^{15}$ have the same meanings as defined above.

Step J-1 is to prepare amide monoester compound (42) by reacting cyclic amino acid (41) with malonic acid derivative (37) or a reactive derivative thereof, and is carried out similarly to (a), (b) and (c) of Step I-1.

Step J-2 is to prepare ketolactam ester compound (39) by fusing the carboxyl group of amide monoester compound (42) with an active methylene group in the molecule to close the ring. In the present step, compound (42) is used as such, or compound (42) can be used by converting it into a reactive derivative.

(a) In the case where compound (42) is used as such, the reaction is carried out in a solvent in the presence of a

condensing agent and in the presence or absence of a base.

The solvent to be employed here can include halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethylacetamide and dimethyl sulfoxide; nitriles such as acetonitrile; esters such as methyl acetate and ethyl acetate; water; or mixed solvents of these. Of these, a halogenated hydrocarbon, an ether or an ester is preferably used, and dichloromethane, tetrahydrofuran or ethyl acetate is more preferably used.

The condensing agent can include, for example, dicyclohexylcarbodiimide, 1-ethyl-3-(3-dimethylaminopropyl) carbodiimide or N,N'-carbonyldiimidazole.

The base to be employed here can include, for example, alkali metal alkoxides such as sodium methoxide, sodium ethoxide and potassium t-butoxide; alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0]-7-undecene. Of these, an amine is preferably used, and triethylamine, pyridine or 1,8-diazabicyclo[5.4.0]-7-undecene is more preferably used.

The reaction is usually carried out at from -20°C to 150°C, preferably from 0°C to 100°C. The reaction time is usually from 10 minutes to 48 hours, preferably from 30 minutes to 12 hours.

(b) In the case where compound (42) is made into a reactive derivative and this is used, the reactive derivative can include an acid halide, a mixed acid anhydride, an active ester, or an active amide.

The acid halide can be obtained by reacting compound (42) with a halogenating agent (for example, thionyl chloride, oxalyl chloride, etc.); the mixed acid anhydride can be obtained by reacting compound (42) with an acid halide (for example, methyl chlorocarbonate, ethyl chlorocarbonate, etc.); the active ester can be obtained by reacting compound (42) with a hydroxy compound (for example, N-hydroxysuccinimide, N-hydroxyphthalimide, etc.) in the presence of the "condensing agent" mentioned in (a); and the "active amide" (for example, the Weinreb amide) can be obtained by reacting compound (42) with an N-lower alkoxy-N-lower alkylhydroxylamine (for example, N-methoxy-N-methylhydroxylamine, etc.) in the presence of the "condensing agent" mentioned in (a). Each reaction is carried out by applying reaction conditions generally used in conventional organic synthesis chemistry.

The ring closure reaction of the reactive derivative is usually carried out in a solvent in the presence or absence of a base.

As the solvent, condensing agent and base, the solvents, condensing agents and bases described in (a) can be used.

The reaction is usually carried out at from -20°C to 150°C, preferably from 0°C to 100°C. The reaction time is usually from 10 minutes to 48 hours, preferably from 30 minutes to 12 hours.

<Process K>

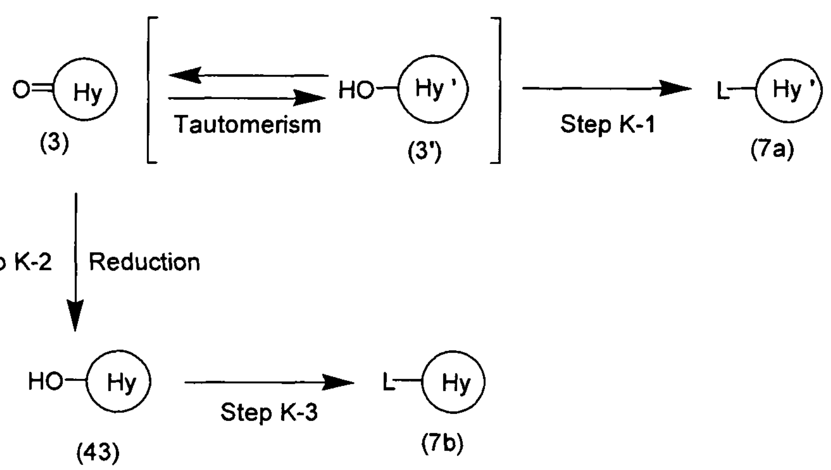


In the above formulae, Hy, Hy' and L have the same meanings as defined above.

Step K-1 is to prepare compound (7a) by converting the hydroxyl group of the compound (3'), i.e., the tautomer of the heterocyclyl ketone (3), into a leaving group, and the present reaction is carried out by reacting compound (3') with a halogenating agent (for example, a fluorinating agent such as diethylamino sulfur trifluoride (DAST); a chlorinating

agent such as thionyl chloride, phosphorus trichloride, phosphorus pentachloride, phosphorus oxychloride, triphenylphosphine/carbon tetrachloride; a brominating agent such as hydrobromic acid, thionyl bromide, phosphorus tribromide and triphenylphosphine/carbon tetrabromide; or an iodinating agent such as hydroiodic acid and phosphorus triiodide), a sulfonyl halide (for example, methanesulfonyl chloride, p-toluenesulfonyl chloride, etc.) or a sulfonic anhydride (for example, trifluoromethanesulfonic anhydride, etc.).

Step K-2 is to prepare heterocyclyl alcohol (43) by reducing heterocyclyl ketone (3), and can be carried out by employing a reduction reaction using a hydride reagent such as an alkali metal borohydride, e.g., sodium borohydride and lithium borohydride; an aluminum hydride compound, e.g., lithium aluminum hydride and lithium triethoxide aluminum hydride; sodium tellurium hydride; an organic aluminum hydride based reducing agent, e.g., sodium diisobutyl aluminum hydride and di(methoxyethoxy) aluminum dihydride, or by catalytic reduction with hydrogen. The reaction is carried out, for example, according to the method described in detail in J. Dale, J. Chem. Soc., 910 (1961) and F. G. Bordwell et al., J. Org. Chem., 33, 3385 (1968).

Step K-3 is to prepare compound (7b) by converting the hydroxyl group of heterocyclyl alcohol (43) into a leaving group, and is carried out similarly to the method described in Step K-1.

Further, $R^3$, i.e., a part of the structure of the compound of formula (I), can have various substituents ($R^4$). The $R^4$ group can be converted into other substituents in the respective steps, and, for example, can be converted according to conventional methods as follows.

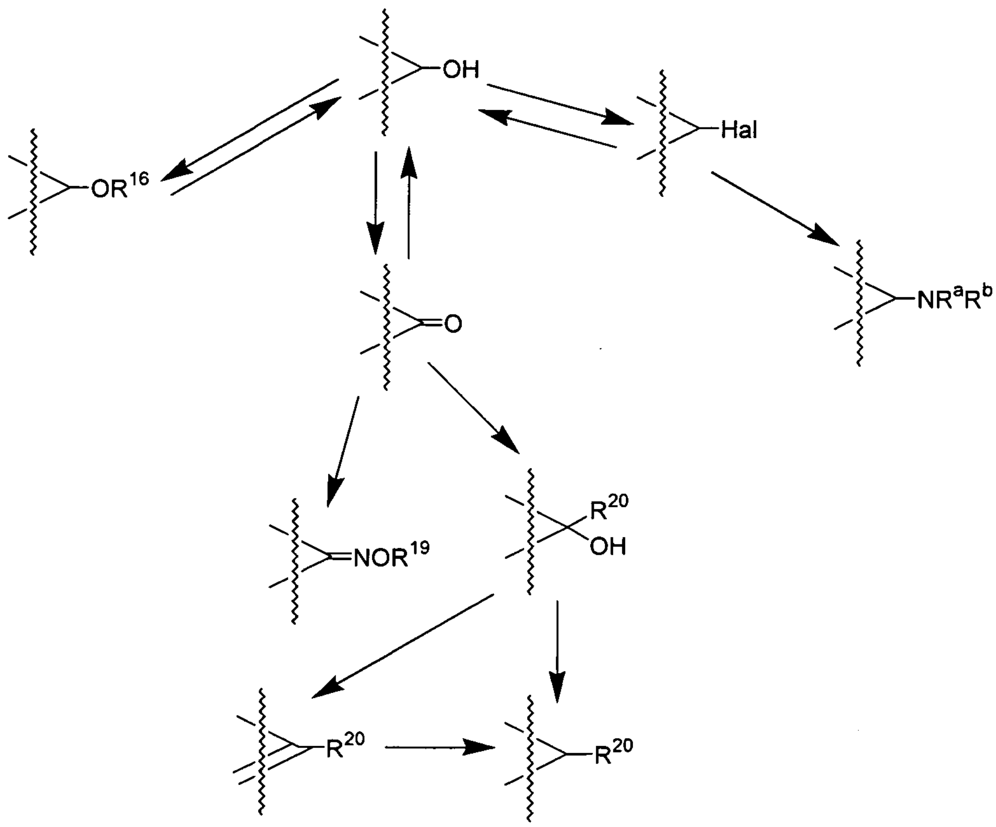

In the above formulae, $R^a$, $R^b$ and Hal have the same meanings as defined above, $R^{16}$ represents the above "lower alkyl group" or the above "halogeno lower alkyl group", "aryl group" or "aryl group substituted by group(s) selected from substituent group α and substituent group β", $R^{17}$'s is the same or different and each represent the above "lower alkyl group" or the above "halogeno lower alkyl group" or two $R^{17}$'s together represent a lower alkylene group, $R^{18}$ represents the above "lower alkyl group", $R^{19}$ represents a hydrogen atom or the above "lower alkyl group", $R^{20}$ represents the "lower alkyl group", the "lower alkenyl group", the "lower alkynyl group", the "aralkyl group", the "cycloalkyl group", the "lower alkyl group substituted by group(s) selected from substituent group α", the "lower alkenyl group substituted by group(s) selected from substituent group α" or the "alkynyl group substituted by group(s) selected from substituent group α" in the definition of substituent group β, or the "aryl group" or the "aryl group substituted by group (s) selected from substituent group α and substituent group β" in the definition of $R^4$.

Further, in the case where $R^4$ is a halogen atom, a hydroxyl group or a cyano group, the substituent can be replaced with a hydrogen atom according to conventional methods by carrying out a reduction reaction after a double

bond is formed as follows.

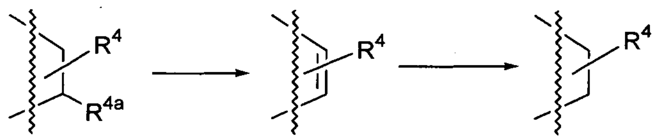

In the above formulae, $R^4$ has the same meaning as defined above and $R^{4a}$ represents a halogen atom, a hydroxyl group or a cyano group.

Compounds in which $R^4$ is an aryl group having a hydroxyl group can be also prepared by cleaving the ether bond (carbon-oxygen bond) of a compound in which $R^4$ is an aryl group having a lower alkoxy group (in the following formulae, $R^4$ is represented by $R^{4b}$-O-$R^{23}$) as indicated.

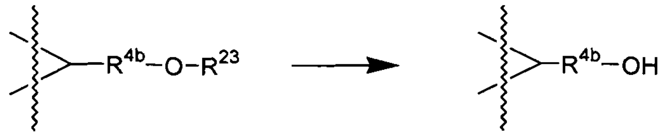

In the above formulae, $R^{4b}$ represents the above "arylene group" and $R^{23}$ represents the above "lower alkyl group".

The cleavage reaction of the above ether bond is carried out, for example, according to the method described in D. Landini et al., Synthesis, 771 (1978), C.A. Smith and J.B. Grutzer, J. Org. Chem., 41 367 (1976), J.P. Marsh and L. Goodman, J. Org. Chem., 30, 2491 (1965), E.G. Paul and P.S.C. Wang, J. Org. Chem., 44, 2307 (1979), J.F.W. McOmie and D.E. West, Org. Synth., V, 412 (1973), C.F. Carvalho and M.V Sargent, J. Chem. Soc., Chem. Commun., 1198 (1982), etc. using a compound in which $R^4$ is an aryl group having a lower alkoxy group and a Bronsted acid (for example, hydrobromic acid, hydroiodic acid, trifluoroacetic acid, etc.) or a Lewis acid (aluminum trichloride, boron tribromide, boron trichloride, etc.).

After completion of the respective reactions, the desired compound is collected from the reaction mixture according to conventional methods.

For example, after the reaction mixture is appropriately neutralized or, in the case where insolubles exist, they are removed by filtration, water and an immiscible organic solvent such as ethyl acetate is added, and, after washing with water or the like, the organic layer containing the desired compound is separated, and it is dried with anhydrous magnesium sulfate or the like, and the solvent is distilled off. Thereby, the desired compound is obtained.

The thus obtained desired compound can be isolated and purified, if necessary, by appropriately combining conventional methods, for example, recrystalization, reprecipitation, or by methods usually used in the isolation and purification of organic compounds, for example, adsorption column chromatography using a carrier such as silica gel, alumina, or florisil (a magnesium-silica gel); methods using a synthetic adsorption agent such as partition column chromatography using a carrier such as Sephadex LH-20 (manufactured by Pharmacia Co.), Amberlite XAD-11 (manufactured by Rohm & Haas) or Diaion HP-20 (manufactured by Mitsubishi Kagaku Co.), or methods using ion exchange chromatography or normal phase/reverse phase column chromatography (preferably high performance liquid chromatography) with silica gel or alkylated silica gel, and eluting with an appropriate eluent.

The bicyclic unsaturated tertiary amine compounds of the present invention exhibit excellent inhibition of inflammatory cytokine production, indicating excellent characteristics as preventive or therapeutic agents for diseases mediated by inflammatory cytokines in warm blooded animals (particularly human beings). Such pharmaceutical compositions are, for example, analgesic/anti-inflammatory agents, anti-virus agents and preventative or therapeutic agents for rheumatoid arthritis, osteoarthritis, allergic diseases, asthma, septicaemia, psoriasis, osteoporosis, autoimmune diseases (for example, systemic lupus erythematosus, ulcerative colitis, Crohn's disease, etc.) diabetes, nephritis, hepatitis, tumors, ischemic heart disease, Alzheimer's disease and arterial sclerosis, preferably analgesic/anti-inflammatory agents, and preventative or therapeutic agents for rheumatoid arthritis, osteoarthritis, allergic diseases, septicaemia, psoriasis, osteoporosis, chronic ulcerative colitis, diabetes, hepatitis, arterial sclerosis and Crohn's disease, particularly analgesic/anti-inflammatory agents, and preventative or therapeutic agents for rheumatoid arthritis, osteoarthritis, septicaemia, psoriasis, Crohn's disease, chronic ulcerative colitis, diabetes (preferably I type diabetes) and hepatitis.

The administration route of the compounds of formula (I) of the present invention, the pharmacologically acceptable salts thereof or the pharmacologically acceptable esters thereof or the pharmacologically acceptable derivatives thereof is, for example, oral administration by tablets, capsules, granules, powders or syrups, or parenteral administration by injection or suppository. These preparations are prepared by well-known methods using additives such as excipients, lubricants, binders, disintegrating agents, stabilizers, corrigents and diluents.

Here, the excipients are organic excipients such as sugar derivatives, e.g., lactose, sucrose, glucose, mannitol and sorbitol; starch derivatives, e.g., corn starch, potato starch, α-starch, dextrin and carboxymethyl starch; cellulose derivatives, e.g., crystalline cellulose, low substituted hydroxypropyl cellulose, hydroxypropylmethyl cellulose, carboxymethyl cellulose, calcium carboxymethyl cellulose and internally cross-linked sodium carboxymethyl cellulose; gum arabic; dextran; and pullulan; and inorganic excipients such as silicate derivatives, e.g., light anhydrous silicic acid, synthetic aluminum silicate and magnesium metasilicate aluminate; phosphates, e.g. calcium phosphate; carbonates, e.g., calcium carbonate; and sulfates, e.g., calcium sulfate.

The lubricant is, for example, stearic acid; a metal stearate such as calcium stearate and magnesium stearate; talc; colloidal silica; a wax such as beesgum and spermaceti; boric acid; adipic acid; a sulfate such as sodium sulfate; glycol; fumaric acid; sodium benzoate; DL-leucine; sodium salt of a fatty acid; a lauryl sulfate such as sodium lauryl sulfate and magnesium lauryl sulfate; a silicic acid such as anhydrous silicic acid and silicic hydrate; or the above starch derivative.

The binder is, for example, polyvinylpyrrolidone, Macrogol or a compound similar to the above excipients.

The disintegrating agent is, for example, a compound similar to the above excipients or a chemically modified starch/cellulose such as sodium cross carmellose, sodium carboxymethyl starch, and cross-linked polyvinylpyrrolidone.

The stabilizer is, for example, a paraoxybenzoate such as methyl paraben and propyl paraben; an alcohol such as chlorobutanol, benzyl alcohol and phenylethyl alcohol; benzalkonium chloride; a phenol such as phenol and cresol; thimerosal; dehydroacetic acid; or sorbic acid.

The corrigent is, for example, a sweetener, a sour agent or a perfume usually used.

The amount used of the compound of formula (I) of the present invention or the pharmacologically acceptable salt, ester or other derivative is varied depending on the symptoms, the age of the patient, the administration method, etc. For example, in the case of the oral administration, it is desirable to administer 0.1 mg (preferably 0.5 mg) as a lower limit and 2000 mg (preferably 500 mg) as an upper limit per day once or by dividing the dose several times for an adult human depending on the symptoms. In the case of intravenous administration, it is desirable to administer 0.01 mg (preferably 0.05 mg) as a lower limit and 200 mg (preferably 50 mg) as an upper limit per day once or by dividing the dose several times for an adult human depending on the symptoms.

[Best mode for carrying out the invention]

The present invention is further illustrated in detail by referring to Examples, Reference Examples, Preparation Examples and Test Examples below, but it is not limited thereto.

[Examples]

(Example 1)

4-[(2S,8aS)-2-Butyl-1,2,3,5,6,8a-hexahydroindolizin-7-yl]-2-(4-fluorophenyl)-3-(pyri din-4-yl)-1H-pyrrole (Exemplary compound No. 1-478)

1) 4-Ethoxycarbonyl-2-(4-fluorophenyl)-3-(pyridin-4-yl)-1H-pyrrole

To 240 ml of tetrahydrofuran were added 36 ml (54.7 mmol) of 1.53M butyl lithium/hexane solution, and at -45°C a solution of 15.90 g (54.7 mmol) of α-(p-toluenesulfonyl)-4-fluorobenzyl isocyanide in 120 ml of tetrahydrofuran. After stirring at the same temperature for 10 minutes, 25.00 g (273 mmol) of 95% lithium bromide was added thereto, and the mixture was stirred for 30 minutes, and to this mixture was added a solution of 8.73 g (49.2 mmol) of 3-(4-pyridyl) acrylic acid ethyl ester in 120 ml of tetrahydrofuran. After stirring at the same temperature for 1 hour, the reaction vessel was removed from the cooling bath, and this mixture was stirred for further 1 hour. 500 ml of water was added to the mixture, and the reaction product was extracted with ethyl acetate. The organic layer was washed with water, dried over anhydrous sodium sulfate and concentrated under reduced pressure. The resulting solid was washed with diethyl ether to obtain 13.61 g of the title compound as a pale yellow powder (yield: 89%).
$^1$H-NMR Spectrum (500 MHz, $CDCl_3$) δ ppm:
8.84 (1H, br.s), 8.51 (2H, d, J = 7Hz), 7.58 (1H, d, J = 3Hz), 7.21 (2H, d, J = 6Hz), 7.11 (2H, dd, J = 9Hz, 5Hz), 6.97 (2H, t, J = 9Hz), 4.18 (2H, quartet, J = 7Hz), 1.20 (3H, t, J = 7Hz).

2) 2-(4-Fluorophenyl)-3-(pyridin-4-yl)-1H-pyrrole

In a mixture of 90 ml of acetic acid, 30 ml of sulfuric acid and 60 ml of water was dissolved 15.00 g (48.3 mmol) of 4-ethoxycarbonyl-2-(4-fluorophenyl)-3-(pyridin-4-yl)-1H-pyrrole obtained in 1) and the mixture was stirred at 100°C for 16 hours. After cooling the mixture to room temperature, the mixture was made alkaline by adding 10% aqueous sodium hydroxide solution and the reaction product was extracted with ethyl acetate. The organic layer was washed with water, dried over anhydrous sodium sulfate and concentrated under reduced pressure to obtain 11.40 g of the title compound as a slightly red powder (yield: 99%).
$^1$H-NMR Spectrum (500 MHz, $CDCl_3$) δ ppm:
9.78 (1H, br.s), 8.42 (2H, d, J = 7Hz), 7.37 (2H, dd, J = 9Hz, 5Hz), 7.22 (2H, d, J = 6Hz), 7.06 (2H, t, J = 9Hz), 6.90 (1H, t, J = 3Hz), 6.47 (1H, t, J = 3Hz).

3) 2-(4-Fluorophenyl)-3-(pyridin-4-yl)-1-triisopropylsilyl-1H-pyrrole

In 300 ml of tetrahydrofuran was dissolved 11.30 g (47.4 mmol) of 2-(4-fluorophenyl)-3-(pyridin-4-yl)-1H-pyrrole obtained in 2), and to this solution was added at -78°C 31 ml (47.4 mmol) of 1.57N butyl lithium/hexane solution. The mixture was stirred for 10 minutes, and to this mixture was added at the same temperature 13.4 ml (49.8 mmol) of triisopropylsilyl triflate. The reaction vessel was removed from the cooling bath and the mixture was stirred at room temperature for 30 minutes. To the reaction mixture were added 200 ml of water and 300 ml of saturated aqueous sodium hydrogencarbonate solution, and the reaction product was extracted with ethyl acetate. The organic layer was washed with water, dried over anhydrous sodium sulfate and concentrated under reduced pressure to obtain 18.70 g of the title compound as a reddish violet oil (yield: quantitative).
$^1$H-NMR Spectrum (500 MHz, DMSO-$d_6$) δ ppm:
8.25 (2H, d, J = 6Hz), 7.39 (2H, dd, J = 9Hz, 6Hz), 7.28 (2H, t, J = 9Hz), 7.00 (1H, d, J = 3Hz), 6.91(2H, d, J = 7Hz), 6.71 (1H, d, J = 3Hz), 1.15-1.05 (3H, m), 0.98 (18H, d, J = 8Hz).

4) 4-Bromo-2-(4-fluorophenyl)-3-(pyridin-4-yl)-1-triisopropylsilyl-1H-pyrrole

In 300 ml of tetrahydrofuran was dissolved 18.70 g (47.4 mmol) of 2-(4-fluorophenyl)-3-(pyridin-4-yl)-1-triisopropylsilyl-1H-pyrrole obtained in 3), and to this solution was gradually added at -78°C a suspension of 8.61 g (47.4 mmol) of N-bromosuccinimide in 100 ml of tetrahydrofuran, and the mixture was stirred at the same temperature for 6 hours. After removing the reaction vessel from the cooling bath, it was stirred at room temperature for 1 hour, and 400 ml of hexane was added thereto and insolubles were filtered off. The filtrate was concentrated under reduced pressure and the residue was subjected to silica gel column chromatography (solvent; hexane:ethyl acetate = 2:1) to obtain 9.57 g of the title compound as pale yellow prism crystals (yield: 43%).
$^1$H-NMR, Spectrum (500 MHz, DMSO-$d_6$) δ ppm:
8.36 (2H, d, J = 6Hz), 7.34 (2H, dd, J = 9Hz, 6Hz), 7.18 (2H, t, J = 9Hz), 7.12 (1H, s), 7.04(2H, d, J = 6Hz), 1.16-1.08 (3H, m), 0.99 (18H, d, J = 8Hz).

5) 4-[(2S,8aS)-2-Butyl-1,2,3,5,6,8a-hexahydroindolizin-7-yl]-2-(4-fluorophenyl)-3-(pyridin-4-yl)-1H-pyrrole

In 60 ml of tetrahydrofuran was dissolved 3.00 g (6.34 mmol) of 4-bromo-2-(4-fluorophenyl)-3-(pyridin-4-yl)-1-triisopropylsilyl-1H-pyrrole obtained in 4), and to this solution was added at -78°C 4.36 ml (6.97 mmol) of 1.6M butyl lithium/hexane solution, and, after stirring the mixture for 10 minutes, at the same temperature 1.48 g (7.60 mmol) of (2S,8aS)-2-butyl-1,2,3,5,6,7,8,8a-octahydroindolizin-7-one was added thereto and the mixture was stirred at -78°C for 2 hours and at room temperature for 1 hour. The reaction mixture was added to saturated aqueous sodium hydrogencarbonate solution, the reaction product was extracted with ethyl acetate, and the organic layer was washed with water, dried over anhydrous sodium sulfate, and concentrated under reduced pressure.

The residue was dissolved in 40 ml of dichloroethane and to this solution was added 1.95 ml (25.3 mmol) of trifluoroacetic acid. The mixture was heated at reflux for 1 hour, and then the reaction mixture was concentrated under reduced pressure. The residue was then dissolved in 30 ml of tetrahydrofuran and to this solution was added 25.3 ml (25.3 mmol) of 1M tetrabutyl ammonium fluoride/tetrahydrofuran solution, and, after stirring of the mixture at room temperature for 10 minutes, water was added to the reaction mixture, which was acidified with 1N aqueous hydrochloric acid, and then extracted with ethyl acetate. The aqueous layer was made alkaline by adding sodium carbonate, and then the reaction product was extracted with ethyl acetate, and the organic layer was washed with water, dried over anhydrous sodium sulfate, and concentrated under reduced pressure. The residue was subjected to silica gel column chromatography (solvent; ethyl acetate:methanol:isopropylamine = 88:7:1) to obtain 747 mg of the title compound (Rf value: 0.33) as a pale brown powder (yield: 11%).

Melting Point: 197-200°C (dec.)
$^1$H-NMR Spectrum (400 MHz, DMSO-$d_6$) δ ppm:
11.38 (1H, s), 8.43 (2H, d, J = 6Hz), 7.18-7.09 (6H, m), 6.90 (1H, s), 5.12 (1H, s), 3.21 (1H, s), 2.92-2.87 (1H, m), 2.67-2.60 (2H, m), 2.42-2.38 (1H, m), 2.31-2.23 (1H, m), 2.00-1.89 (3H, m), 1.38-1.16 (6H, m), 0.89-0.81 (3H, m), 0.79-0.72 (1H, m).

(Example 2)

2-(4-Fluorophenyl)-4-[(2S,8aS)-2-phenyl-1,2,3,5,6,8a-hexahydroindolizin-7-yl]-3-(py ridin-4-yl)-1H-pyrrole (Exemplary compound No. 1-479)

A reaction similar to that of Example 1-5) using (2S,8aS)-2-phenyl-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 20:1:1) were carried out to obtain 616 mg of the title compound (Rf value: 0.50) as a pale yellow powder (yield: 43%).
Melting Point: 227-228°C (dec.)
$^1$H-NMR Spectrum (400 MHz, DMSO-$d_6$) δ ppm:
11.41-11.40 (1H, br.s), 8.46 (2H, d, J = 6Hz,), 7.30-7.25 (4H, m), 7.20-7.10 (7H, m), 6.96 (1H, d, J = 3Hz), 5.21-5.20 (1H, br.s), 3.50-3.49 (1H, m), 3.33-3.30 (1H, m), 3.23-3.18 (1H, m), 2.92-2.86 (1H, m), 2.75-2.63 (2H, m), 2.34-2.29 (1H, m), 2.08-2.04 (1H, m) 1.88-1.72 (2H, m).

(Example 3)

2-(4-Fluorophenyl)-4-[(2R,8aS)-2-(4-fluorophenyl)-1,2,3,5,6,8a-hexahydroindolizin-7 -yl]-3-(pyridin-4-yl)-1H-pyrrole (Exemplary compound No. 1-482)

A reaction similar to that of Example 1-5) using (2R,8aS)-2-(4-fluorophenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 100:10:1) were carried out to obtain 1.76 g of the title compound (Rf value: 0.20) as a pale brown powder (yield: 61%).
Melting Point: 211-213°C (dec.)
$^1$H-NMR Spectrum (400 MHz, DMSO-$d_6$) δ ppm:
11.41 (1H, br.s), 8.36 (2H, d, J = 6Hz), 7.27 (2H, dd, J = 9Hz, 6Hz), 7.21-7.08 (8H, m), 6.95 (1H, d, J = 3Hz), 5.23-5.18 (1H, m), 3.45-3.37 (1H, m), 3.32-3.21 (1H, m), 3.02-2.87 (2H, m), 2.80-2.66 (2H, m), 2.39-2.26 (2H, m),2.04-1.95 (1H, m), 1.25-1.15 (1H, m).

(Example 4)

2-(4-Fluorophenyl)-4-[(2R,8aS)-2-(4-methylphenyl)-1,2,3,5,6,8a-hexahydroindolizin-7-yl]-3-(pyridin-4-yl)-1H-pyrrole (Exemplary compound No. 1-481)

A reaction similar to that of Example 1-5) using (2R,8aS)-2-(4-methylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 20:1:1) were carried out to obtain 130 mg of the title compound (Rf value: 0.35) as a white powder (yield: 47%).
Melting Point: 214-217°C (dec.)
$^1$H-NMR Spectrum (400 MHz, DMSO-$d_6$) δ ppm:
11.39 (1H, br.s), 8.36 (2H, d, J = 4Hz), 7.20-7.09 (10H, m), 6.95 (1H, d, J = 3Hz), 5.21 (1H, s), 3.46-3.12 (3H, m), 3.01-2.93 (1H, m), 2.90 (1H, t, J = 8Hz), 2.75 (1H, t, J = 8Hz), 2.76-2.66 (1H, m), 2.38-2.21 (1H, m), 2.28 (3H, s), 2.03-1.93 (1H, m), 1.28-1.13 (1H, m).

(Example 5)

3-[(2S,8aS)-2-Butyl-1,2,3,5,6,8a-hexahydroindolizin-7-yl]-5-(4-fluorophenyl)-4-(pyri din-4-yl)pyrazole (Exemplary compound No. 3-495)

1) 3-Bromo-5-(4-fluorophenyl)-4-(pyridin-4-yl)pyrazole

In 60 ml of dimethylformamide was dissolved 6.0 g (25 mmol) of 5-(4-fluorophenyl)-4-(pyridin-4-yl)pyrazole (WO 00/31063), and to this solution was added 8.93 g (50 mmol) of N-bromosuccinimide, and the mixture was stirred at room temperature for 3 days. Water was added to the reaction mixture, and the crystals which precipitated were filtered off and washed with diethyl ether to obtain 5.73 g of the title compound as a white powder (yield: 72%).
$^1$H-NMR Spectrum (400 MHz, $CDCl_3$-DMSO-$d_6$) δ ppm:
8.56 (2H, d, J = 5Hz), 7.33 (2H, dd, J = 8Hz, 5Hz), 7.24 (2H, d, J = 5Hz), 7.05 (2H, t, J = 8Hz)$_o$.

2) 3-[(2S,8aS)-2-Butyl-7-hydroxy-1,2,3,5,6,7,8,8a-octahydroindolizin]-5-(4-fluoro phenyl)-4-(pyridin-4-yl)pyrazole

In 51 ml of tetrahydrofuran was dissolved 1.63 g (5.12 mmol) of 3-bromo-5-(4-fluorophenyl)-4-(pyridin-4-yl) pyrazole obtained in 1), and to this solution was added at -78°C a solution of 8.05 ml (12.80 mmol) of 1.6M butyl lithium/ hexane. After stirring of the mixture for 10 minutes, at the same temperature 1.25 g (6.40 mmol) of (2S,8aS)-2-butyl-1,2,3,5,6,7,8,8a-octahydroindolizin-7-one was added thereto, and the mixture was stirred at -78°C for 2 hours and at room temperature for 1 hour. The reaction mixture was added to saturated aqueous sodium hydrogencarbonate solution and the mixture was extracted with ethyl acetate. The organic layer was washed with water, dried over anhydrous sodium sulfate and concentrated under reduced pressure to obtain the title compound (crude product) as a brown oil.

3) 3-[(2S,8aS)-2-Butyl-1,2,3,5,6,8a-hexahydroindolizin-7-yl]-5-(4-fluorophenyl) -4-(pyridin-4-yl)pyrazole

In 45 ml of concentrated hydrochloric acid was dissolved 3-[(2S,8aS)-2-butyl-7-hydroxy-1,2,3,5,6,7,8,8a-octahydroindolizin]-5-(4-fluorophenyl)-4-(pyridin-4-yl)pyrazole (the total amount of the crude product) obtained in 2) and the solution was refluxed with heating for 8 hours, neutralized with 1N aqueous sodium hydroxide solution, and extracted with ethyl acetate. The organic layer was washed with water, dried over anhydrous sodium sulfate, and concentrated under reduced pressure. The resulting residue was subjected to silica gel column chromatography (solvent; ethyl acetate:methanol:isopropylamine = 20:1:1) to obtain 506 mg of the title compound (Rf value: 0.20) as a brown amorphous solid (yield: 24%).
$^1$H-NMR Spectrum (500 MHz, $CDCl_3$) δ ppm:
8.54 (2H, d, J=8Hz), 7.28 (2H, dd, J = 9Hz, 5Hz), 7.14 (2H, d, J=8Hz), 6.99 (2H, t, J = 9Hz), 5.84-5.79 (1H, br.s), 3.55-3.46 (1H, m), 3.06-2.97 (1H, m), 2.93-2.81 (2H, m), 2.52-2.45 (1H, m), 2.43-2.33 (1H, m), 2.22-2.07 (3H, m), 1.47-1.20 (6H, m), 1.10-1.00 (1H, m), 0.90 (3H, t, J = 7Hz).

(Example 6)

5-(4-Fluorophenyl)-3-[(2S,8aS)-2-propyl-1,2,3,5,6,8a-hexahydroindolizin-7-yl]-4-(pyr idin-4-yl)-pyrazole (Exemplary compound No. 3-494)

A reaction similar to that of Example 5-2) using (2S,8aS)-2-propyl-1,2,3,5,6,7,8,8a-octahydroindolizin-7-one instead of (2S,8aS)-2-butyl-1,2,3,5,6,7,8,8a-octahydroindolizin-7-one was carried out and the resulting product was then subjected to a dehydration reaction similar to that of Example 5-3), and to silica gel column chromatography (solvent; ethyl acetate:methanol:isopropylamine = 20:1:1), to obtain 432 mg of the title compound (Rf value: 0.18) as a pale brown amorphous solid (yield: 20%).
$^1$H-NMR Spectrum (500 MHz, $CDCl_3$) δ ppm:
8.54 (2H, d, J = 8Hz), 7.28 (2H, dd, J = 9Hz, 5Hz), 7.14 (2H, d, J = 8Hz), 7.00 (2H, t, J = 9Hz), 5.83-5.78 (1H, br.s), 3.54-3.47 (1H, m), 3.06-2.98 (1H, m), 2.93-2.81 (2H, m), 2.51-2.44 (1H, m), 2.43-2.33 (2H, m), 1.43-1.23 (5H, m), 1.09-1.00 (1H, m), 0.91 (3H, t, J = 7Hz).

(Example 7)

5-(4-Fluorophenyl)-3-[(2S,8aS)-2-phenyl-1,2,3,5,6,8a-hexahydroindolizin-7-yl]-4-(py ridin-4-yl)pyrazole (Exemplary compound No. 3-496)

A reaction similar to that of Example 5-2) using (2S,8aS)-2-phenyl-1,2,3,5,6,7,8,8a-octahydroindolizin-7-one instead of (2S,8aS)-2-butyl-1,2,3,5,6,7,8,8a-octahydroindolizin-7-one was carried out and the resulting product was then subjected to a dehydration reaction similar to that of Example 5-3), and to silica gel column chromatography (solvent; ethyl acetate:methanol:isopropylamine = 20:1:1), to obtain 185 mg of the title compound (Rf value: 0.20) as a pale brown powder (yield: 11%).
Melting Point: 199-200°C
$^1$H-NMR Spectrum (500 MHz, $CDCl_3$) δ ppm:
8.57 (2H, d, J = 8Hz), 7.33-7.19 (7H, m), 7.18 (2H, d, J = 8Hz), 7.00 (2H, t, J = 9Hz), 5.93-5.89 (1H, br.s), 3.71-3.61 (1H, m), 3.52-3.42 (1H, m), 3.38-3.31 (1H, m), 3.07-2.99 (1H, m), 2.92-2.80 (2H, m), 2.54-2.40 (1H, m), 2.34-2.20 (1H, m), 2.14-2.00 (2H, m)$_o$.

(Example 8)

2-(3,4-Difluorophenyl)-4-[(2S,8aS)-2-phenyl-1,2,3,5,6,8a-hexahydroindolizin-7-yl]-3-(pyridin-4-yl)-1H-pyrrole (Exemplary compound No. 1-487)

A reaction similar to that of Example 1-5) using 4-bromo-2-(3,4-difluorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-phenyl-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:isopropylamine = 20:1) were carried out to obtain 240 mg of the title compound (Rf value: 0.40) as a pale brown powder (yield: 17%).
Melting Point: 235-240°C (dec.)
$^1$H-NMR Spectrum (400 MHz, DMSO-$d_6$) δ ppm:
11.51 (1H, br.s), 8.50-8.49 (2H, dd, J = 6Hz, 1Hz), 7.38-7.13 (8H, m), 7.01-7.00 (1H, d, J = 3Hz), 6.94-6.90 (1H, m), 5.20 (1H, s), 3.49 (1H, m), 3.38-3.16 (2H, m), 2.93-2.84 (1H, m), 2.76-2.51 (3H, m), 2.30-2.26 (1H, m),2.08-2.03 (1H, m), 1.87-1.80 (1H, m), 1.77-1.66 (1H, m).

(Example 9)

2-(4-Fluorophenyl)-4-[(2S,8aS)-2-(4-methylphenyl)-1,2,3,5,6,8a-hexahydroindolizin-7-yl]-3-(pyridin-4-yl)-1H-pyrrole (Exemplary compound No. 1-481)

A reaction similar to that of Example 1-5) using (2S,8aS)-2-(4-methylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 20:1:1) were carried out to obtain 670 mg of the title compound (Rf value: 0.40) as a white powder (yield: 48%).
Melting Point: 220-222°C (dec.)
$^1$H-NMR Spectrum (500 MHz, DMSO-$d_6$) δ ppm:
11.39 (1H, br.s), 8.45 (2H, d, J = 5Hz), 7.20-7.04 (10H, m),
6.95 (1H, d, J = 3Hz), 5.21 (1H, s), 3.50-3.44 (1H, m), 3.30-3.23 (1H, m), 3.18 (1H, t, J = 9Hz), 2.92-2.83 (1H, m), 2.74-2.65 (1H, m), 2.59 (1H, dd, J = 10Hz, 8Hz), 2.35-2.22 (1H, m), 2.25 (3H, s), 2.10-2.01 (1H, m), 1.84-1.76 (1H, m), 1.76-1.68 (1H, m).

(Example 10)

2-(3-Chlorophenyl)-4-[(2S,8aS)-2-phenyl-1,2,3,5,6,8a-hexahydroindolizin-7-yl]-3-(py ridin-4-yl)-1H-pyrrole (Exemplary compound No. 1-483)

A reaction similar to that of Example 1-5) using 4-bromo-2-(3-chlorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-phenyl-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:dichlorometane = 20:20:1) were carried out to obtain 440 mg of the title compound (Rf value: 0.10) as a pale brown powder (yield: 21%).

Melting Point: 186-190°C (dec.)
$^1$H-NMR Spectrum (400 MHz, $CDCl_3$) δ ppm:
8.60 (1H, br.s), 8.51 (2H, d, J = 6Hz), 7.32-7.14 (10H, m), 6.99-6.96 (1H, m), 6.88 (1H, d, J=3Hz), 5.48 (1H, d, J = 1Hz), 3.63-3.55 (1H, m), 3.49-3.40 (1H, m), 3.32 (1H, dd, J = 10Hz, 8Hz), 3.03-2.97 (1H, m), 2.86-2.77 (2H, m), 2.45-2.33 (1H, m), 2.21-2.11 (1H, m), 2.07-1.93 (2H, m).

(Example 11)

2-(4-Fluorophenyl)-4-[(2S,8aS)-2-(3-methylphenyl)-1,2,3,5,6,8a-hexahydroindolizin-7-yl]-3-(pyridin-4-yl)-1H-pyrrole (Exemplary compound No. 1-57)

A reaction similar to that of Example 1-5) using (2S,8aS)-2-(3-methylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 40:4:1) were carried out to obtain 146 mg of the title compound (Rf value: 0.35) as an orange powder (yield: 12%).
Melting Point: 204-206°C
$^1$H-NMR Spectrum (500 MHz, DMSO-$d_6$) δ ppm:
8.46 (2H, d, J = 6Hz), 7.19-7.10 (7H, m), 7.07 (1H, s),
7.04 (1H, d, J = 8Hz), 6.97 (1H, d, J = 7Hz), 6.95 (1H, d, J = 3Hz), 5.22 (1H, s), 3.50-3.44 (1H, m), 3.30-3.28 (1H, m), 3.19 (1H, t, J = 9Hz), 2.90-2.86 (1H, m), 2.73-2.68 (1H, m), 2.63 (1H, t, J = 9Hz), 2.56-2.44 (1H, m), 2.34-2.28 (1H, m), 2.27 (3H, s), 2.10-2.02 (1H, m), 1.86-1.80 (1H, m), 1.77-1.71 (1H, m).

(Example 12)

2-(3,4-Difluorophenyl)-4-[(2S,8aS)-2-(4-methylphenyl)-1,2,3,5,6,8a-hexahydroindoli zin-7-yl]-3-(pyridin-4-yl)-1H-pyrrole (Exemplary compound No. 1-191)

A reaction similar to that of Example 1-5) using 4-bromo-2-(3,4-difluorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-(4-methylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 20:1:1) were carried out to obtain 420 mg of the title compound (Rf value: 0.35) as a pale brown powder (yield: 42%).
Melting Point: 197-200°C (dec.)
$^1$H-NMR Spectrum (400 MHz, $CDCl_3$) δ ppm:
8.52 (2H, d, J = 6Hz), 8.30 (1H, br.s), 7.19 (2H, d, J = 6Hz), 7.15 (2H, d, J = 8Hz), 7.11 (2H, d, J = 8Hz), 7.07 (1H, dd, J = 18Hz, 8Hz), 6.98 (1H, ddd, J = 11Hz, 7Hz, 2Hz), 6.88-6.84 (1H, m), 6.87 (1H, d, J = 3Hz), 5.47 (1H, m), 3.64-3.53 (1H, m), 3.42 (1H, quintet, J = 8Hz), 3.29 (1H, t, J = 10Hz), 3.00 (1H, dt, J = 12Hz, 4Hz), 2.86-2.75 (2H, m), 2.44-2.34 (1H, m), 2.32 (3H, s), 2.20-2.10 (1H, m), 2.05-1.90 (2H, m).

(Example 13)

2-(3-Chlorophenyl)-4-[(2S,8aS)-2-(4-methylphenyl)-1,2,3,5,6,8a-hexahydroindolizin-7-yl]-3-(pyridin-4-yl)-1H-pyrrole (Exemplary compound No. 1-103)

A reaction similar to that of Example 1-5) using 4-bromo-2-(3-chlorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-(4-methylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 40:4:1) were carried out to obtain 238 mg of the title compound (Rf value: 0.35) as a pale orange powder (yield: 23%).
Melting Point: 187-192°C (dec.)
$^1$H-NMR Spectrum (400 MHz, $CDCl_3$) δ ppm:
8.50 (2H, d, J = 6Hz), 7.29-7.21 (3H, m), 7.19 (1H, s), 7.17 (1H, s), 7.14 (2H, d, J = 8Hz), 7.07 (2H, d, J = 8Hz), 7.06-7.02 (1H, m), 7.01 (1H, d, J = 3Hz), 5.20 (1H, s), 3.49-3.42 (1H, m), 3.29-3.23 (1H, m), 3.17 (1H, t, J = 9Hz), 2.91-2.83 (1H, m), 2.73-2.27 (1H, m), 2.25 (3H, s), 2.10-2.01 (1H, m), 1.83-1.77 (1H, m), 1.74-1.67 (1H, m).

(Example 14)

5-(4-Fluorophenyl)-3-[(2S,8aS)-2-(4-methylphenyl)-1,2,3,5,6,8a-hexahydroindolizin-7-yl]-4-(pyridin-4-yl)-pyrazole (Exemplary compound No. 3-497)

A reaction similar to that of Example 5-2) using (2S,8aS)-2-(4-methylphenyl)-1,2,3,5,6,7,8,8a-octahydroindolizin-7-one instead of (2S,8aS)-2-butyl-1,2,3,5,6,7,8,8a-octahydroindolizin-7-one was carried out, and the resulting product was then subjected to a dehydration reaction similar to that of Example 5-3), and to silica gel column chromatography (solvent; ethyl acetate:methanol:isopropylamine = 20:2:1), to obtain 540 mg of the title compound (Rf value: 0.26) as a pale yellow powder (yield: 32%).
Melting Point: 222-225°C
$^1$H-NMR Spectrum (400 MHz, $CDCl_3$) δ ppm:
8.56 (2H, d, J = 6Hz), 7.29 (2H, dd, J = 9Hz, 5Hz), 7.17 (2H, d, J = 6Hz), 7.14 (2H, d, J = 8Hz), 7.11 (2H, d, J=8Hz), 7.00 (2H, t, J = 9Hz), 5.92 (1H, s), 3.70-3.60 (1H, m), 3.44 (1H, quintet, J = 8Hz), 3.33 (1H, dd, J = 10Hz, 8Hz), 3.02 (1H, dt, J = 12Hz, 4Hz), 2.90-2.79 (2H, m), 2.52-2.36 (1H, m), 2.32 (3H, s), 2.32-2.20 (1H, m), 2.12-1.98 (2H, m).

(Example 15)

4-[(2S,8aS)-2-(4-Methylphenyl)-1,2,3,5,6,8a-hexahydroindolizin-7-yl]-3-(pyridin-4-yl)-2-(3-trifluoromethylphenyl)-1H-pyrrole (Exemplary compound No. 1-486)

A reaction similar to that of Example 1-5) using 4-bromo-3-(pyridin-4-yl)-2-(3-trifluoromethylphenyl)-1-triisopropylsilyl-1H-pyrrole instead of 4-bromo-2-(4-fluorophenyl)-3-(pyridin-4-yl)-1-triisopropylsilyl-1H-pyrrole and (2S, 8aS)-2-(4-methylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 20:1:1) were carried out to obtain 149 mg of the title compound (Rf value: 0.45) as a pale yellow powder (yield: 52%).
Melting Point: 217-220°C (dec.)
$^1$H-NMR Spectrum (500 MHz, $CDCl_3$) δ ppm:
8.53 (2H, d, J = 6Hz), 8.42 (1H, br.s), 7.52-7.47 (2H, m),
7.36 (1H, t, J = 8Hz), 7.28-7.24 (1H, m), 7.21 (2H, d, J = 6Hz), 7.16 (2H, d, J = 8Hz), 7.12 (2H, d, J = 8Hz), 6.92 (1H, d, J = 3Hz), 5.49 (1H, m), 3.66-3.54 (1H, m), 3.43 (1H, quintet, J = 8Hz), 3.31 (1H, t, J = 10Hz), 3.06-2.98 (1H, m), 2.88-2.75 (2H, m), 2.46-2.36 (1H, m), 2.33 (3H, s), 2.22-2.12 (1H, m), 2.06-1.88 (2H, m).

(Example 16)

2-(4-Fluorophenyl)-4-[(2S,8aS)-2-(4-methoxyphenyl)-1,2,3,5,6,8a-hexahydroindolizi n-7-yl]-3-(pyridin-4-yl)-1H-pyrrole (Exemplary compound No. 1-480)

A reaction similar to that of Example 1-5) using (2S,8aS)-2-(4-methoxyphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 20:1:1) were carried out to obtain 576 mg of the title compound (Rf value: 0.18) as a slightly brown powder (yield: 43%).
Melting Point: 236-238°C
$^1$H-NMR Spectrum (500 MHz, DMSO-$d_6$) δ ppm:
11.43-11.37 (1H, br.s), 8.45 (2H, d, J = 8Hz), 7.20-7.09 (8H, m), 6.95 (1H, d, J = 3Hz), 6.83 (1H, d, J = 9Hz), 5.22-5.19 (1H, br.s), 3.71 (3H, s), 3.50-3.43 (1H, m), 3.32-3.24 (1H, m), 3.20-3.13 (1H, m), 2.91-2.83 (1H, m), 2.73-2.67 (1H, m), 2.58 (1H, dd, J = 10Hz, 8Hz), 2.34-2.27 (1H, m), 2.09-2.00 (1H, m), 1.83-1.68 (2H, m).

(Example 17)

2-(4-Fluorophenyl)-4-[(2S,8aS)-2-(2-methoxyphenyl)-1,2,3,5,6,8a-hexahydroindolizi n-7-yl]-3-(pyridin-4-yl)-1H-pyrrole (Exemplary compound No. 1-68)

A reaction similar to that of Example 1-5) using (2S,8aS)-2-(2-methoxyphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 20:1:1) were carried out to obtain 235 mg of the title compound (Rf value: 0.20) as a slightly brown powder (yield: 21%).
Melting Point: 225-226°C

$^1$H-NMR Spectrum (500 MHz, DMSO-$d_6$) δ ppm:
11.43-11.35 (1H, br.s), 9.44 (2H, d, J = 8Hz), 7.27-7.23 (1H, m), 7.20-7.09 (7H, m), 6.96-6.92 (2H, m), 6.90-6.85 (1H, m), 5.24-5.21 (1H, br.s), 3.79 (3H, s), 3.71-3.63 (1H, m), 3.52-3.45 (1H, m), 3.16-3.09 (1H, m), 2.90-2.83 (1H, m), 2.73-2.67 (1H, m), 2.62 (1H, dd, J = 10Hz, 8Hz), 2.33-2.23 (1H, m), 2.09-2.01 (1H, m), 1.88-1.81 (1H, m), 1.72-1.64 (1H, m).

(Example 18)

2-(3,4-Difluorophenyl)-4-[(2S,8aS)-2-(4-methoxyphenyl)-1,2,3,5,6,8a-hexahydroindo lizin-7-yl]-3-(pyridin-4-yl)-1H-pyrrole (Exemplary compound No. 1-204)

A reaction similar to that of Example 1-5) using 4-bromo-2-(3,4-difluorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-(4-methoxyphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 100:10:1) were carried out to obtain 469 mg of the title compound (Rf value: 0.22) as a pale brown powder (yield: 48%).
Melting Point: 188-190°C (dec.)
$^1$H-NMR Spectrum (400 MHz, DMSO-$d_6$) δ ppm:
11.50 (1H, br.s), 8.49 (2H, d, J = 6Hz), 7.39-7.30 (1H, m), 7.25-7.09 (5H, m), 7.00 (1H, d, J = 3Hz), 6.95-6.88 (1H, m), 6.83 (2H, d, J = 9Hz), 5.21-5.18 (1H, m), 3.71 (3H, s), 3.49-3.42 (1H, m), 3.35-3.22 (1H, m), 3.19-3.12 (1H, m), 2.91-2.84 (1H, m), 2.74-2.64 (1H, m), 2.61-2.54 (1H, m), 2.36-2.24 (1H, m), 2.09-1.99 (1H, m), 1.82-1.64 (2H, m).

(Example 19)

2-(4-Fluorophenyl)-4-[(2S,8aS)-2-(3-methoxyphenyl)-1,2,3,5,6,8a-hexahydroindolizi n-7-yl]-3-(pyridin-4-yl)-1H-pyrrole (Exemplary compound No. 1-69)

A reaction similar to that of Example 1-5) using (2S,8aS)-2-(3-methoxyphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 100:10:1) were carried out to obtain 423 mg of the title compound (Rf value: 0.26) as a pale brown powder (yield: 45%).
Melting Point: 204-206°C (dec.)
$^1$H-NMR Spectrum (400 MHz, DMSO-$d_6$) δ ppm:
11.41 (1H, br.s), 8.46 (2H, d, J = 6Hz), 7.22-7.09 (7H, m), 6.95 (1H, d, J = 2Hz), 6.87-6.79 (2H, m), 6.74 (1H, dd, J = 8Hz, 2Hz), 5.23-5.19 (1H, m), 3.73 (3H, s), 3.53-3.45 (1H, m), 3.37-3.27 (1H, m), 3.23-3.15 (1H, m), 2.92-2.83 (1H, m), 2.75-2.59 (2H, m), 2.36-2.25 (1H, m), 2.11-2.01 (1H, m), 1.89-1.80 (1H, m), 1.78-1.68 (1H, m).

(Example 20)

4-[(2S,8aS)-2-Phenyl-1,2,3,5,6,8a-hexahydroindolizin-7-yl]-3-(pyridin-4-yl)-2-(3-trifl uoromethylphenyl)-1H-pyrrole (Exemplary compound No. 1-484)

A reaction similar to that of Example 1-5) using 4-bromo-3-(pyridin-4-yl)-2-(3-trifluoromethylphenyl)-1-triisopropylsilyl-1H-pyrrole instead of 4-bromo-2-(4-fluorophenyl)-3-(pyridin-4-yl)-1-triisopropylsilyl-1H-pyrrole and (2S, 8aS)-2-phenyl-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; dichloromethane:methanol = 20:1) were carried out to obtain 35 mg of the title compound (Rf value: 0.13) as a yellow powder (yield: 12%).
Melting Point: 202-205°C (dec.)
$^1$H-NMR Spectrum (400 MHz, DMSO-$d_6$) δ ppm:
11.65 (1H, s), 8.51 (2H, d, J = 5Hz), 7.62-7.45 (3H, m), 7.41 (1H, d, J = 2Hz), 7.32-7.26 (4H, m), 7.20-7.17 (3H, m), 7.06 (1H, s), 5.22 (1H, s), 3.50 (1H, s), 3.21 (2H, t, J = 9Hz), 2.91-2.88 (1H, m), 2.78-2.70 (1H, m), 2.65-2.61 (1H, m), 2.35-2.31 (1H, m), 2.08 (1H, d, J = 16Hz), 1.88-1.83 (1H, m), 1.81-1.70 (1H, m).

(Example 21)

4-[(2S,8aS)-2-(4-Methoxyphenyl)-1,2,3,5,6,8a-hexahydroindolizin-7-yl]-3-(pyridin-4-yl)-2-(3-trifluoromethylphenyl)-1H-pyrrole (Exemplary compound No. 1-485)

A reaction similar to that of Example 1-5) using 4-bromo-3-(pyridin-4-yl)-2-(3-trifluoromethylphenyl)-1-triisopropylsilyl-1H-pyrrole instead of 4-bromo-2-(4-fluorophenyl)-3-(pyridin-4-yl)-1-triisopropylsilyl-1H-pyrrole and (2S, 8aS)-2-(4-methoxypheny)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; dichloromethane:methanol = 20:1) were carried out to obtain 103 mg of the title compound (Rf value: 0.05) as a yellow powder (yield: 35%). Melting Point: 182-185°C (dec.)
$^1$H-NMR Spectrum (400 MHz, DMSO-$d_6$) δ ppm:
11.64 (1H, s), 8.50 (2H, d, J = 6Hz), 7.53-7.46 (3H, m),
7.41 (1H, d, J = 7Hz), 7.20-7.18 (3H, m), 7.16 (1H, s), 7.06 (1H, d, J = 3Hz), 6.84 (2H, d, J = 9Hz), 5.21 (1H, s), 3.71 (3H, s), 3.48-3.37 (1H, m), 3.19-3.14 (1H, m), 2.90-2.87 (1H, m), 2.71-2.67 (1H, m), 2.60-2.54 (1H, m), 2.34-2.30 (1H, m), 2.09-2.08 (2H, m), 1.79-1.77 (1H, m), 1.71-1.69 (1H, m).

(Example 22)

2-(3,4-Difluorophenyl)-4-[(2S,8aS)-2-(3-methylphenyl)-1,2,3,5,6,8a-hexahydroindoli zin-7-yl]-3-(pyridin-4-yl)-1H-pyrrole (Exemplary compound No. 1-190)

A reaction similar to that of Example 1-5) using 4-bromo-2-(3,4-difluorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-(3-methylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 20:1:1) were carried out to obtain 178 mg of the title compound (Rf value: 0.68) as a pale brown powder (yield: 24%).
Melting Point: 179-182°C
$^1$H-NMR Spectrum (400 MHz, $CDCl_3$) δ ppm:
8.88 (1H, br.s), 8.51 (2H, d, J = 6Hz), 7.24-7.14 (3H, m), 7.10-6.93 (5H, m), 6.91-6.80 (2H, m), 5.47 (1H, s), 3.64-3.54 (1H, m), 3.42 (1H, quintet, J = 8Hz), 3.29 (1H, t, J = 9Hz), 3.00 (1H, dt, J = 12Hz, 4Hz), 2.88-2.74 (2H, m), 2.44-2.10 (2H, m), 2.33 (3H, s), 2.07-1.89 (2H, m).

(Example 23)

2-(3-Chlorophenyl)-4-[(2S,8aS)-2-(4-methoxyphenyl)-1,2,3,5,6,8a-hexahydroindolizi n-7-yl]-3-(pyridin-4-yl)-1H-pyrrole (Exemplary compound No. 1-116)

A reaction similar to that of Example 1-5) using 4-bromo-2-(3-chlorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-(4-methoxyphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:isopropylamine = 20:1) were carried out to obtain 90 mg of the title compound (Rf value: 0.38) as a grayish white powder (yield: 12%).
Melting Point: 215-217°C (dec.)
$^1$H-NMR Spectrum (400 MHz, DMSO-$d_6$) δ ppm:
11.53 (1H, br.s), 8.50-8.48 (2H, dd, J = 4Hz, 1Hz), 7.29-7.16 (7H, m), 7.05-7.01 (2H, m), 6.85-6.82 (2H, d, J = 9Hz), 5.19 (1H, s), 3.71 (3H, s), 3.49-3.43 (1H, m), 3.29-3.24 (1H, t, J = 7Hz), 3.19-3.15 (1H, t, J = 9Hz), 2.91-2.88 (1H, m), 2.75-2.69 (1H, m), 2.62-2.57 (1H, m), 2.33-2.29 (1H, m), 2.08-2.04 (1H, m), 1.83-1.67 (2H, m).

(Example 24)

4-[(2S,8aS)-2-(4-t-butylphenyl)-1,2,3,5,6,8a-hexahydroindolizin-7-yl]-2-(4-fluorophe nyl)-3-(pyridin-4-yl)-1H-pyrrole (Exemplary compound No. 1-65)

A reaction similar to that of Example 1-5) using (2S,8aS)-2-(4-t-butylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:acetic acid = 40:4:1) were carried out to obtain 436 mg of the title compound (Rf value: 0.20) as a grayish white powder (yield: 34%).

Melting Point: 226-228°C
$^1$H-NMR Spectrum (400 MHz, $CDCl_3$) δ ppm:
8.49 (2H, d, J = 6Hz), 8.32 (1H, br.s), 7.32 (2H, d, J = 8Hz), 7.20-7.18 (4H, m), 7.14 (2H, dd, J = 9Hz, 5Hz), 6.98 (2H, t, J = 9Hz), 6.86 (1H, d, J = 2Hz), 5.49 (1H, s), 3.60 (1H, m), 3.44 (1H, quintet, J = 8Hz), 3.30 (1H, t, J = 8Hz), 3.00 (1H, dt, J = 12Hz, 4Hz), 2.88-2.77 (2H, m), 2.44-2.35 (1H, m), 2.20-2.12 (1H, m), 2.06-1.92 (2H, m), 1.31 (9H, s).

(Example 25)

2-Phenyl-4-[(2S,8aS)-2-phenyl-1,2,3,5,6,8a-hexahydroindolizin-7-yl]-3-(pyridin-4-yl) -1H-pyrrole (Exemplary compound No. 1-477)

A reaction similar to that of Example 1-5) using 4-bromo-2-phenyl-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-phenyl-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:dichloromethane = 10:10:1) were carried out to obtain 250 mg of the title compound (Rf value: 0.20) as a pale yellow powder (yield: 31%).
Melting Point: 155-158°C (dec.)
$^1$H-NMR Spectrum (400 MHz, $CDCl_3$) δ ppm:
8.49 (2H, dd, J =5Hz, 2Hz), 8.33 (1H, br.s), 7.32-7.24 (5H, m), 7.21 (2H, dd, J = 5Hz, 2Hz), 7.20-7.15 (5H, m), 6.87 (1H, d, J = 3Hz), 5.49 (1H, d, J = 2Hz), 3.67-3.58 (1H, m), 3.50-3.40 (1H, m), 3.32 (1H, dd, J = 10Hz, 8Hz), 3.05-2.97 (1H, m), 2.90-2.78 (2H, m), 2.48-2.33 (1H, m), 2.22-2.13 (1H, m), 2.08-1.95 (2H, m).

(Example 26)

5-(3,4-Difluorophenyl)-3-[(8aS)-1,2,3,5,6,8a-hexahydroindolizin-7-yl]-4-(pyridin-4-yl)-pyrazole (Exemplary compound No. 3-498)

A reaction similar to that of Example 5-2) using 3-bromo-5-(3,4-difluorophenyl)-4-(pyridin-4-yl)pyrazole instead of 3-bromo-5-(4-fluorophenyl)-4-(pyridin-4-yl)pyrazole and (8aS)-1,2,3,5,6,7,8,8a-octahydroindolizin-7-one instead of (2S,8aS)-2-butyl-1,2,3,5,6,7,8,8a-octahydroindolizin-7-one was carried out, and the resulting product was then subjected to a dehydration reaction similar to that of Example 5-3), and to silica gel column chromatography (solvent; ethyl acetate:methanol:dichloromethane = 5:5:1), to obtain 460 mg of the title compound (Rf value: 0.10) as a pale yellow powder (yield: 14%).
Melting Point: 105-110°C (dec.)
$^1$H-NMR Spectrum (400 MHz, $CDCl_3$) δ ppm:
8.56 (2H, d, J = 6Hz), 7.25-7.19 (1H, m), 7.14 (2H, d, J = 6Hz), 7.10-6.95 (2H, m), 5.94 (1H, s), 3.35-3.28 (1H, m), 3.00-2.86 (2H, m), 2.83-2.68 (2H, m), 2.35-2.10 (2H, m), 2.05-1.65 (3H, m), 1.56-1.43 (1H, m).

(Example 27)

5-(3,4-Difluorophenyl)-3-[(2S,8aS)-2-phenyl-1,2,3,5,6,8a-hexahydroindolizin-7-yl]-4-(pyridin-4-yl)-pyrazole (Exemplary compound No. 3-499)

A reaction similar to that of Example 5-2) using 3-bromo-5-(3,4-difluorophenyl)-4-(pyridin-4-yl)pyrazole instead of 3-bromo-5-(4-fluorophenyl)-4-(pyridin-4-yl)pyrazole and (2S,8aS)-2-phenyl-1,2,3,5,6,7,8,8a-octahydroindolizin-7-one instead of (2S,8aS)-2-butyl-1,2,3,5,6,7,8,8a-octahydroindolizin-7-one was carried out, and the resulting product was then subjected to a dehydration reaction similar to that of Example 5-3), and to silica gel column chromatography (solvent; ethyl acetate:methanol:dichloromethane = 5:5:1), to obtain 472 mg of the title compound (Rf value: 0.10) as a pale brown powder (yield: 15%).
Melting Point: 113-115°C (dec.)
$^1$H-NMR Spectrum (400 MHz, $CDCl_3$) δ ppm:
8.58 (2H, d, J = 6Hz), 7.33-7.16 (6H, m), 7.18 (2H, d, J = 6Hz), 7.11-6.99 (2H, m), 5.96 (1H, s), 3.70-3.61 (1H, m), 3.51-3.44 (1H, m), 3.36 (1H, dd, J = 11Hz, 8Hz), 3.04-2.98 (1H, m), 2.89 (1H, dd, J = 11Hz, 8Hz), 2.87-2.80 (1H, m), 2.45-2.33 (1H, m), 2.29-2.19 (1H, m), 2.15-1.95 (2H, m).

(Example 28)

3-[(8aS)-1,2,3,5,6,8a-Hexahydroindolizin-7-yl]-5-phenyl-4-(pyridin-4-yl)-pyrazole (Exemplary compound No. 3-493)

A reaction similar to that of Example 5-2) using 3-bromo-5-phenyl-4-(pyridin-4-yl)pyrazole instead of 3-bromo-5-(4-fluorophenyl)-4-(pyridin-4-yl)pyrazole and (8aS)-1,2,3,5,6,7,8,8a-octahydroindolizin-7-one instead of (2S,8aS)-2-butyl-1,2,3,5,6,7,8,8a-octahydroindolizin-7-one was carried out, and the resulting product was then subjected to a dehydration reaction similar to that of Example 5-3), and to silica gel column chromatography (solvent; ethyl acetate: methanol:dichloromethane = 5:5:1), to obtain 308 mg of the title compound (Rf value: 0.10) as a white powder (yield: 13%).
Melting Point: 98-101°C (dec.)
$^1$H-NMR Spectrum (400 MHz, $CDCl_3$) δ ppm:
8.52 (2H, d, J = 5Hz), 7.30 (5H, s), 7.17 (2H, d, J = 5Hz), 5.85 (1H, s), 3.36-3.28 (1H, m), 3.03-2.96 (1H, m), 2.95-2.88 (1H, m), 2.82-2.70 (2H, m), 2.50-2.35 (1H, m), 2.31-2.19 (1H, m), 2.00-1.70 (3H, m), 1.52-1.42 (1H, m).

(Example 29)

4-[(2S,8aS)-2-(4-t-Butylphenyl)-1,2,3,5,6,8a-hexahydroindolizin-7-yl]-2-(3,4-difluoro phenyl)-3-(pyridin-4-yl)-1H-pyrrole (Exemplary compound No. 1-199)

A reaction similar to that of Example 1-5) using 4-bromo-2-(3,4-difluorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-(4-t-butylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol = 10:1) were carried out to obtain 327 mg of the title compound (Rf value: 0.20) as a brown powder (yield: 28%).
Melting Point: 218-220°C
$^1$H-NMR Spectrum (500 MHz, $CDCl_3$) δ ppm:
8.52 (2H, d, J = 6Hz), 8.48 (1H, br.s), 7.32 (2H, d, J = 9Hz), 7.19-7.17 (4H, m), 7.06 (1H, dd, J = 10Hz, 8Hz), 7.00-6.96 (1H, m), 6.87-6.85 (2H, m), 5.48 (1H, s), 3.59 (1H, br.s), 3.46-3.39 (1H, m), 3.29 (1H, dd, J = 11Hz, 9Hz), 2.99 (1H, dt, J = 12Hz, 5Hz), 2.86-2.77 (2H, m), 2.42-2.36 (1H, m), 2.17-2.14 (1H, m), 2.05-1.94 (2H, m), 1.31 (9H, s).

(Example 30)

4-[(2S,8aS)-2-(4-Ethoxyphenyl)-1,2,3,5,6,8a-hexahydroindolizin-7-yl]-2-(4-fluorophe nyl)-3-(pyridin-4-yl)-1H-pyrrole (Exemplary compound No. 1-72)

A reaction similar to that of Example 1-5) using (2S,8aS)-2-(4-ethoxyphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 100:10:1) were carried out to obtain 612 mg of the title compound (Rf value: 0.23) as a pale brown powder (yield: 55%).
Melting Point: 182-184°C (dec.)
$^1$H-NMR Spectrum (400 MHz, DMSO-$d_6$) δ ppm:
11.41 (1H, br.s), 8.45 (2H, d, J = 6Hz), 7.22-7.08 (8H, m), 6.95 (1H, d, J = 3Hz), 6.82 (2H, d, J = 9Hz), 5.22-5.19 (1H, m), 3.97 (2H, quartet, J = 7Hz), 3.51-3.43 (1H, m), 3.35-3.23 (1H, m), 3.20-3.12 (1H, m), 2.92-2.84 (1H, m), 2.75-2.64 (1H, m), 2.62-2.54 (1H, m), 2.36-2.25 (1H, m), 2.10-2.00 (1H, m), 1.83-1.67 (2H, m), 1.30 (3H, t, J = 7Hz).

(Example 31)

2-(3,4-Difluorophenyl)-4-[(2S,8aS)-2-(3-methoxyphenyl)-1,2,3,5,6,8a-hexahydroindo lizin-7-yl]-3-(pyridin-4-yl)-1H-pyrrole (Exemplary compound No. 1-203)

A reaction similar to that described in Example 1-5) using 4-bromo-2-(3,4-difluorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-(3-methoxyphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 100:10:1) were carried out to obtain 787 mg of the title compound (Rf value: 0.25) as a pale brown powder (yield: 48%).
Melting Point: 177-179°C (dec.)

$^1$H-NMR Spectrum (400 MHz, DMSO-$d_6$) δ ppm:
11.51 (1H, br.s), 8.50 (2H, d, J = 6Hz), 7.40-7.30 (1H, m), 7.25-7.11 (4H, m), 7.00 (1H, d, J = 3Hz), 6.96-6.89 (1H, m), 6.87-6.78 (2H, m), 6.73 (1H, dd, J = 8Hz, 2Hz), 5.23-5.18 (1H, m), 3.73 (3H, s), 3.52-3.43 (1H, m), 3.37-3.25 (1H, m), 3.22-3.13 (1H, m), 2.92-2.83 (1H, m), 2.76-2.58 (2H, m), 2.36-2.24 (1H, m), 2.11-1.99 (1H, m), 1.88-1.79 (1H, m), 1.77-1.66 (1H, m).

(Example 32)

2-(4-Fluorophenyl)-4-[(2S,8aS)-2-(4-hydroxyphenyl)-1,2,3,5,6,8a-hexahydroindolizin-7-yl]-3-(pyridin-4-yl)-1H-pyrrole (Exemplary compound No. 1-81)

In 10 ml of dichloromethane was suspended 150 mg (0.32 mmol) of the compound of Example 16, 2-(4-fluorophenyl)-4-[(2S,8aS)-2-(4-methoxyphenyl)-1,2,3,5,6,8a-hexahydroindolizin -7-yl]-3-(pyridin-4-yl)-1H-pyrrole, and to this suspension was added a solution of 4.83 ml (4.83 mmol) of boron tribromide in dichloromethane (concentration; 1M), and the mixture was stirred at room temperature overnight. Water was added to the reaction mixture and the reaction product was extracted with dichloromethane. The organic layer was washed with water, dried over anhydrous sodium sulfate and the solvent was distilled off under reduced pressure. The residue was subjected to silica gel column chromatography (solvent; ethyl acetate:methanol:isopropylamine = 20:1:1) to obtain 33 mg of the title compound (Rf value: 0.20) as a slightly brown powder (yield: 23%).
Melting Point: 245-246°C
$^1$H-NMR Spectrum (500 MHz, DMSO-$d_6$) δ ppm:
11.62-11.55 (1H, br.s), 9.38-9.32 (1H, br.s), 8.49 (2H, d, J = 8Hz), 7.24-7.08 (9H, m), 6.74 (2H, d, J = 8Hz), 5.07-5.00 (1H, br.s), 4.36-4.21 (1H, m), 3.67-3.55 (1H, m), 3.52-3.40 (1H, m), 3.40-3.18 (3H, m), 2.65-2.55 (1H, m), 2.53-2.43 (1H, m), 2.09-1.98 (1H, m), 1.90-1.81 (1H, m).

(Example 33)

2-(4-Fluorophenyl)-4-[(2S,8aS)-2-(2-hydroxyphenyl)-1,2,3,5,6,8a-hexahydroindolizin-7-yl]-3-(pyridin-4-yl)-1H-pyrrole (Exemplary compound No. 1-79)

A reaction similar to that of Example 32 using the compound of Example 17, 2-(4-fluorophenyl)-4-[(2S,8aS)-2-(2-methoxyphenyl)-1,2,3,5,6,8a-hexahydroindolizin -7-yl]-3-(pyridin-4-yl)-1H-pyrrole, instead of 2-(4-fluorophenyl)-4-[(2S,8aS)-2-(4-methoxyphenyl)-1,2,3,5,6,8a-hexahydroindolizin -7-yl]-3-(pyridin-4-yl)-1H-pyrrole was carried out to obtain 77 mg of the title compound as a yellow powder (yield: 53%).
Melting Point: 195-196°C
$^1$H-NMR Spectrum (500 MHz, DMSO-$d_6$) δ ppm:
11.66-11.49 (1H, br.s), 9.82-9.77 (1H, br.s), 8.50 (2H, d, J = 8Hz), 7.27-7.07 (9H, m), 6.88 (1H, d, J = 8Hz), 6.81 (1H, t, J = 7Hz), 5.13-5.09 (1H, br.s), 4.42-4.34 (1H, m), 3.87-3.77 (1H, m), 3.68-3.60 (1H, m), 3.50-3.30 (3H, m), 2.65-2.48 (2H, m), 2.27-2.18 (1H, m), 1.97-1.89 (1H, m).

(Example 34)

4-[(2S,8aS)-2-(4-Acetoxyphenyl)-1,2,3,5,6,8a-hexahydroindolizin-7-yl]-2-(4-fluorophenyl)-3-(pyridin-4-yl)-1H-pyrrole (Exemplary compound No. 1-86)

In 1 ml of pyridine was dissolved 45 mg (0.10 mmol) of the compound of Example 32, 2-(4-fluorophenyl)-4-[(2S,8aS)-2-(4-hydroxyphenyl)-1,2,3,5,6,8a-hexahydroindolizin-7-yl]-3-(pyridin-4-yl)-1H-pyrrole, and to this solution was added 100 mg (0.98 mmol) of acetic anhydride. The resulting mixture was left to stand at room temperature for 1 hour. The reaction mixture was concentrated under reduced pressure, and the residue was made weakly alkaline by adding saturated aqueous sodium hydrogencarbonate solution, and the reaction product was extracted with ethyl acetate. The organic layer was washed with water and dried over anhydrous sodium sulfate, and the solvent was distilled off under reduced pressure. 30% Ethanol was added to the residue, and the precipitated solids were filtered off to obtain 31 mg of the title compound as a slightly brown powder (yield: 63%).
Melting Point: 195-196°C
$^1$H-NMR Spectrum (500 MHz, DMSO-$d_6$) δ ppm:
11.43-11.36 (1H, br.s), 8.46 (2H, d, J = 8Hz), 7.30 (2H, d, J = 8Hz), 7.22-7.08 (6H, m), 7.02 (2H, d, J = 8Hz), 6.96 (1H, d, J = 3Hz), 5.23-5.18 (1H, br.s), 3.53-3.47 (1H, m), 3.40-3.30 (1H, m), 3.23-3.17 (1H, m), 2.92-2.84 (1H, m), 2.76-2.68 (1H, m), 2.62 (1H, dd, J = 10Hz, 7Hz), 2.38-2.25 (1H, m), 2.10-2.00 (1H, m), 1.87-1.80 (1H, m), 1.79-1.72 (1H, m).

(Example 35)

2-(3,4-Difluorophenyl)-4-[(2S,8aS)-2-(3-hydroxyphenyl)-1,2,3,5,6,8a-hexahydroindol izin-7-yl]-3-(pyridin-4-yl)-1H-pyrrole (Exemplary compound No. 1-215)

A reaction similar to that of Example 32 using the compound of Example 31, 2-(3,4-difluorophenyl)-4-[(2S, 8aS)-2-(3-methoxyphenyl)-1,2,3,5,6,8a-hexahydroindol izin-7-yl]-3-(pyridin-4-yl)-1H-pyrrole, instead of 2-(4-fluorophenyl)-4-[(2S,8aS)-2-(4-methoxyphenyl)-1,2,3,5,6,8a-hexahydroindolizin -7-yl]-3-(pyridin-4-yl)-1H-pyrrole was carried out to obtain 31 mg of the title compound as a pale yellow powder (yield: 15%).
Melting Point: 170-172°C (dec.)
$^1$H-NMR Spectrum (400 MHz, DMSO-$d_6$) δ ppm:
11.52 (1H, br.s), 9.25 (1H, s), 8.50 (2H, d, J = 6Hz), 7.40-7.30 (1H, m), 7.23-7.12 (3H, m), 7.06 (1H, t, J = 8Hz), 7.02 (1H, d, J = 3Hz), 6.95-6.88 (1H, m), 6.71-6.63 (2H, m), 6.60-6.53 (1H, m), 5.21-5.15 (1H, m), 3.57-3.45 (1H, m), 3.35-3.15 (2H, m), 2.97-2.87 (1H, m), 2.80-2.60 (2H, m), 2.37-2.24 (1H, m), 2.13-2.02 (1H, m), 1.86-1.77 (1H, m), 1.76-1.66 (1H, m).

(Example 36)

2-(3-Cyanophenyl)-4-[(2S,8aS)-2-phenyl-1,2,3,5,6,8a-hexahydroindolizin-7-yl]-3-(py ridin-4-yl)-1H-pyrrole (Exemplary compound No. 1-284)

A reaction similar to that of Example 1-5) using 4-bromo-2-(3-cyanophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-phenyl-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:isopropylamine = 40:1) were carried out to obtain 58 mg of the title compound (Rf value: 0.15) as a pale ruby-color powder (yield: 3%).
Melting Point: 182-185°C
$^1$H-NMR Spectrum (400 MHz, DMSO-$d_6$) δ ppm:
11.66 (1H, br.s), 8.51-8.50 (2H, dd, J = 4Hz, 1Hz), 7.69-7.59 (2H, m), 7.47-7.43 (1H, t, J = 8Hz), 7.39-7.35 (1H, m), 7.28-7.24 (4H, m), 7.20-7.14 (3H, m), 7.07-7.06 (1H, d, J = 3Hz), 5.22 (1H, s), 3.51-3.47 (1H, m), 3.33-3.29 (1H, m), 3.22-3.17 (1H, m), 2.90-2.86 (1H, m), 2.75-2.68 (1H, m), 2.64-2.60 (1H, m), 2.35-2.27 (1H, m), 2.08-2.03 (1H, m), 1.86-1.81 (1H, m), 1.77-1.69 (1H, m).

(Example 37)

2-(4-Fluoro-3-methoxyphenyl)-4-[(2S,8aS)-2-phenyl-1,2,3,5,6,8a-hexahydroindolizin -7-yl]-3-(pyridin-4-yl)-1H-pyrrole (Exemplary compound No. 1-318)

A reaction similar to that of Example 1-5) using 4-bromo-2-(4-fluoro-3-methoxyphenyl)-3-(pyridin-4-yl)-1-triisopropylsilyl-1H-pyrrol e instead of 4-bromo-2-(4-fluorophenyl)-3-(pyridin-4-yl)-1-triisopropylsilyl-1H-pyrrole and (2S, 8aS)-2-phenyl-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:isopropylamine = 30:1) were carried out to obtain 390 mg of the title compound (Rf value: 0.42) as a pale yellow powder (yield: 22%).
Melting Point: 190-193°C (dec.)
$^1$H-NMR Spectrum (400 MHz, DMSO-$d_6$) δ ppm:
11.42 (1H, br.s), 8.49-8.48 (2H, dd, J = 4Hz, 2Hz), 7.30-7.25 (4H, m), 7.19-7.08 (4H, m), 6.98-6.97 (1H, d, J = 3Hz), 6.92-6.90 (1H, dd, J = 8Hz, 2Hz), 6.70-6.67 (1H, m), 5.22 (1H, s), 3.63 (3H, s), 3.51-3.47 (1H, m), 3.36-3.29 (1H, m), 3.22-3.18 (1H, m), 2.92-2.86 (1H, m), 2.74-2.68 (1H, m), 2.64-2.60 (1H, dd, J = 10Hz, 7Hz),2.36-2.28 (1H, m), 2.09-2.03 (1H, m), 1.87-1.83 (1H, m), 1.81-1.71 (1H, m).

(Example 38)

2-(3-Methoxyphenyl)-4-[(2S,8aS)-2-phenyl-1,2,3,5,6,8a-hexahydroindolizin-7-yl]-3-(pyridin-4-yl)-1H-pyrrole (Exemplary compound No. 1-250)

A reaction similar to that of Example 1-5) using 4-bromo-2-(3-methoxyphenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-phenyl-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 20:1:1) were carried out to obtain 315 mg of the title compound (Rf value: 0.25) as a brown powder (yield: 25%).
Melting Point: 141-144°C
$^1$H-NMR Spectrum (400 MHz, DMSO-$d_6$) δ ppm:
11.39 (1H, s), 8.48 (2H, d, J = 7Hz), 7.30-7.26 (4H, m), 7.19-7.14 (4H, m), 6.96 (1H, d, J = 2Hz), 6.77-6.72 (3H, m), 5.21 (1H, s), 3.61 (3H, s), 3.52-3.48 (1H, m), 3.20 (1H, t, J = 9Hz), 2.92-2.86 (1H, m), 2.75-2.68 (1H, m), 2.65-2.61 (1H, m), 2.35-2.28 (1H, m), 2.07-2.04 (1H, m), 1.99 (1H, s), 1.87-1.81 (1H, m), 1.77-1.70 (1H, m).

(Example 39)

4-[(2S,8aS)-2-(4-Ethoxyphenyl)-1,2,3,5,6,8a-hexahydroindolizin-7-yl]-2-(3,4-difluoro phenyl)-3-(pyridin-4-yl)-1H-pyrrole (Exemplary compound No. 1-207)

A reaction similar to that of Example 1-5) using 4-bromo-2-(3,4-difluorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-(4-ethoxyphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:isopropylamine = 100:10:1) were carried out to obtain 300 mg of the title compound (Rf value: 0.20) as a pale brown powder (yield: 40%).
Melting Point: 176-178°C (dec.)
$^1$H-NMR Spectrum (400 MHz, DMSO-$d_6$) δ ppm:
11.50 (1H, br.s), 8.49 (2H, d, J = 6Hz), 7.39-7.30 (1H, m), 7.22-7.10 (5H, m), 7.00 (1H, d, J = 3Hz), 6.95-6.88 (1H, m), 6.81 (2H, d, J = 8Hz), 5.21-5.18 (1H, m), 3.98 (2H, quartet, J = 7Hz), 3.50-3.42 (1H, m), 3.33-3.22 (1H, m), 3.19-3.12 (1H, m), 2.92-2.83 (1H, m), 2.74-2.64 (1H, m), 2.57 (1H, dd, J = 10Hz, 7Hz), 2.35-2.24 (1H, m), 2.10-1.98 (1H, m), 1.82-1.63 (2H, m), 1.30 (3H, t, J = 7Hz).

(Example 40)

2-(4-Fluorophenyl)-4-[(2S,8aS)-2-(3,4-dimethylphenyl)-1,2,3,5,6,8a-hexahydroindoli zin-7-yl]-3-(pyridin-4-yl)-1H-pyrrole (Exemplary compound No. 1-58)

A reaction similar to that of Example 1-5) using (2S,8aS)-2-(3,4-dimethylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:dichloromethane = 20:20:1) were carried out to obtain 143 mg of the title compound (Rf value: 0.10) as a pale orange-color powder (yield: 30%).
Melting Point: 212-214°C (dec.)
$^1$H-NMR Spectrum (400 MHz, $CDCl_3$) δ ppm:
8.49 (2H, d, J = 6Hz), 8.34 (1H, br.s), 7.19 (2H, d, J = 6Hz), 7.14 (2H, dd, J = 9Hz, 5Hz), 7.06 (1H, d, J = 8Hz), 7.02 (1H, s), 7.00-6.95 (3H, m), 6.85 (1H, d, J = 3Hz), 5.49 (1H, s), 3.62-3.55 (1H, m), 3.46-3.35 (1H, m), 3.32-3.26 (1H, m), 3.03-2.97 (1H, m), 2.87-2.76 (2H, m), 2.46-2.33 (1H, m), 2.25 (3H, s), 2.23 (3H, s), 2.23-2.12 (1H, m),2.05-1.92 (2H, m).

(Example 41)

2-(3,4-Difluorophenyl)-4-[(2S,8aS)-2-(3,4-dimethylphenyl)-1,2,3,5,6,8a-hexahydroin dolizin-7-yl]-3-(pyridin-4-yl)-1H-pyrrole (Exemplary compound No. 1-192)

A reaction similar to that of Example 1-5) using 4-bromo-2-(3,4-difluorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-(3,4-dimethylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:dichloromethane = 20:20:1) were carried out to obtain 110 mg of the title compound (Rf value: 0.10) as an orange-color powder (yield: 22%).
Melting Point: 165-170°C (dec.)
$^1$H-NMR Spectrum (400 MHz, $CDCl_3$) δ ppm:
8.52 (2H, d, J = 6Hz), 8.43 (1H, br.s), 7.19 (2H, d, J = 6Hz), 7.10-6.95 (5H, m), 6.90-6.83 (2H, m), 5.47 (1H, s), 3.61-3.53 (1H, m), 3.44-3.33 (1H, m), 3.31-3.25 (1H, m), 3.03-2.94 (1H, m), 2.86-2.75 (2H, m), 2.45-2.33 (1H, m), 2.25 (3H, s), 2.23 (3H, s), 2.23-2.10 (1H, m), 2.03-1.90 (2H, m).

(Example 42)

4-[(2S,8aS)-2-(4-Biphenyl)-1,2,3,5,6,8a-hexahydroindolizin-7-yl]-2-(4-fluorophenyl)-3-(pyridin-4-yl)-1H-pyrrole (Exemplary compound No. 1-67)

A reaction similar to that of Example 1-5) using (2S,8aS)-2-(4-biphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol = 10:1) were carried out to obtain 259 mg of the title compound (Rf value: 0.25) as a brown powder (yield: 52%).
Melting Point: 190-193°C (dec.)
$^1$H-NMR Spectrum (500 MHz, $CDCl_3$) δ ppm:
8.50 (2H, d, J = 6Hz), 8.30 (1H, br.s), 7.58 (2H, d, J = 8Hz), 7.53 (2H, d, J = 8Hz), 7.43 (2H, t, J = 8Hz), 7.35-7.30 (3H, m), 7.19 (2H, d, J = 6Hz), 7.14 (2H, dd, J = 9Hz, 6Hz), 6.98 (2H, t, J = 8Hz), 6.87 (1H, d, J = 2Hz), 5.50 (1H, s), 3.68-3.59 (1H, m), 3.50 (1H, quintet, J = 9Hz), 3.35 (1H, t, J = 9Hz), 3.03 (1H, dt, J = 12Hz, 5Hz), 2.92-2.80 (2H, m), 2.47-2.36 (1H, m), 2.23-2.14 (1H, m), 2.11-1.96 (2H, m).

(Example 43)

4-[(2R,8aS)-2-Amino-1,2,3, 5,6,8a-hexahydroindolizin-7-yl]-2-(4-fluorophenyl)-3-(pyr idin-4-yl)-1H-pyrrole (Exemplary compound No. 1-465)

1) 4-[(2R,8aS)-2-Azido-1,2,3,5,6,8a-hexahydroindolizin-7-yl]-2-(4-fluorophenyl)-3-(pyridin-4-yl)-1H-pyrrole

A reaction similar to that of Example 1-5) using (2R,8aS)-2-azido-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol = 10:1) were carried out to obtain 11 mg of the title compound (Rf value: 0.27) as a brown oil (yield: 5%).
$^1$H-NMR Spectrum (500 MHz, $CD_3OD$) δ ppm:
8.38 (2H, d, J = 5Hz), 7.23 (2H, d, J=7Hz), 7.18 (2H, dd, J = 9Hz, 7Hz), 7.00 (2H, t, J = 9Hz), 5.31 (1H, s), 4.31-4.24 (1H, m), 3.94 (1H, quintet, J = 7Hz), 3.68-3.60 (1H, m), 3.23 (1H, dd, J = 12Hz, 7Hz), 2.99-2.82 (2H, m), 2.38-2.28 (1H, m), 2.27-2.17 (1H, m), 2.05-1.97 (1H, m), 1.90-1.81 (1H, m).

2) 4-[(2R,8aS)-2-Amino-1,2,3,5,6,8a-hexahydroindolizin-7-yl]-2-(4-fluorophenyl)-3-(pyridin-4-yl)-1H-pyrrole

In 4 ml of tetrahydrofuran was dissolved 8 mg (0.02 mmol) of 4-[(2R,8aS)-2-azido-1,2,3,5,6,8a-hexahydroindolizin-7-yl]-2-(4-fluorophenyl)-3-(pyri din-4-yl)-1H-pyrrole obtained in 1) and to this solution were added 10 μl of water and 13 mg (0.048 mmol) of triphenylphosphine, and the mixture was stirred at 50°C for 10 hours. The reaction mixture was dried under reduced pressure and the solid residue was washed with diethyl ether to obtain 3 mg of the title compound as a pale brown powder (yield: 40%).
$^1$H-NMR Spectrum (500 MHz, $CD_3OD$) δ ppm:
8.40 (2H, d, J = 6Hz), 7.24 (2H, d, J = 6Hz), 7.18 (2H, dd, J = 9Hz, 5Hz), 7.01 (2H, t, J = 9Hz), 5.24 (1H, s), 4.60-4.53 (1H, m), 4.31 (1H, quintet, J = 7Hz), 3.93-3.75 (1H, m), 3.16-3.00 (2H, m), 2.98-2.83 (1H, m), 2.52-2.42 (1H, m), 2.37-2.26 (1H, m), 1.98-1.81 (2H, m).

(Example 44)

4-[(2S,8aS)-2-(4-Ethylphenyl)-1,2,3,5,6,8a-hexahydroindolizin-7-yl]-2-(4-fluorophen yl)-3-(pyridin-4-yl)-1H-pyrrole (Exemplary compound No. 1-60)

A reaction similar to that of Example 1-5) using (2S,8aS)-2-(4-ethylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:dichloromethylene = 5:5:1) were carried out to obtain 215 mg of the title compound (Rf value: 0.20) as a pale pink powder (yield: 28%).
Melting Point: 215-218°C (dec.)
$^1$H-NMR Spectrum (400 MHz, $CDCl_3$) δ ppm:
8.49 (2H, d, J = 6Hz), 8.39 (1H, br.s), 7.18 (2H, d, J = 6Hz), 7.18-7.09 (6H, m), 6.97 (2H, t, J = 9Hz), 6.86 (1H, d, J = 3Hz), 5.49 (1H, d, J = 2Hz), 3.64-3.55 (1H, m), 3.47-3.38 (1H, m), 3.30 (1H, dd, J = 10Hz, 8Hz), 3.03-2.95 (1H, m), 2.88-2.76 (2H, m), 2.62 (2H, dd, J = 15Hz, 8Hz), 2.45-2.34 (1H, m), 2.21-2.12 (1H, m), 2.06-1.92 (2H, m), 1.23 (3H, t,

J = 8Hz).

(Example 45)

4-[(2S,8aS)-2-(4-Ethylphenyl)-1,2,3,5,6,8a-hexahydroindolizin-7-yl]-2-(3,4-difluorophenyl)-3-(pyridin-4-yl)-1H-pyrrole (Exemplary compound No. 1-194)

A reaction similar to that of Example 1-5) using 4-bromo-2-(3,4-difluorophenyl)-3-(pyridin-4-yl)-1-triisopropylsilyl-1H-pyrrole instead of 4-bromo-2-(4-fluorophenyl)-3-(pyridin-4-yl)-1-triisopropylsilyl-1H-pyrrole and (2S,8aS)-2-(4-ethylphenyl)-1,2,3,5,6,7,8,8a-octahydroindolizin-7-one instead of (2S,8aS)-2-butyl-1,2,3,5,6,7,8,8a-octahydroindolizin-7-one and silica gel column chromatography (solvent; ethyl acetate:methanol:dichloromethane = 5:5:1) were carried out to obtain 175 mg of the title compound (Rf value: 0.20) as a pink powder (yield: 22%).
Melting Point: 200-203°C (dec.)
$^1$H-NMR Spectrum (400 MHz, $CDCl_3$) δ ppm:
8.52 (2H, d, J = 6Hz), 8.35 (1H, br.s), 7.19 (2H, d, J = 6Hz), 7.19-7.12 (4H, m),7.06 (1H, dd, J = 19Hz, 8Hz), 7.01-6.95 (1H, m), 6.89-6.84 (1H, m),6.87 (1H, d, J = 3Hz), 5.48 (1H, d, J = 2Hz), 3.62-3.52 (1H, m), 3.47-3.38 (1H, m), 3.29 (1H, dd, J = 10Hz, 8Hz), 3.03-2.96 (1H, m), 2.87-2.75 (2H, m),2.62 (2H, dd, J = 15Hz, 8Hz), 2.44-2.33 (1H, m), 2.20-2.10 (1H, m), 2.05-1.90 (2H, m), 1.23 (3H, t, J = 8Hz).

(Example 46)

2-(3-Chloro-4-fluorophenyl)-4-[(2S,8aS)-2-phenyl-1,2,3,5,6,8a-hexahydroindolizin-7-yl]-3-(pyridin-4-yl)-1H-pyrrole (Exemplary compound No. 1-420)

1) 4-Bromo-1-(t-butyl)diphenylsilyl-2-(3-chloro-4-fluorophenyl)-3-(pyridin-4 -yl)-1H-pyrrole

The reactions of Examples 1-1) and 2) were carried out successively using α-(p-toluenesulfonyl)-(3-chloro-4-fluorobenzyl)isocyanide instead of α-(p-toluenesulfonyl)-4-fluorobenzylisocyanide as a starting material to obtain 2-(3-chloro-4-fluorophenyl)-3-(pyridin-4-yl)-1H-pyrrole as a slightly pink powder (yield: 78%).

Then 29.6 g (109 mmol) of this powder was dissolved in 300 ml of tetrahydrofuran and the solution was added dropwise to a suspension of 9.50 g (218 mmol) of 55% sodium hydride/600 ml of tetrahydrofuran under ice cooling while stirring and the mixture was stirred at room temperature for 30 minutes. To the reaction mixture was added 56.7 ml (218 mmol) of (t-butyl)diphenylsilyl chloride and the resulting mixture was stirred at room temperature for 1 hour. Then, the reaction was quenched by adding ice water and the tetrahydrofuran was distilled off under reduced pressure. The reaction product was extracted by adding ethyl acetate to the residue, and the organic layer was washed with water, dried over anhydrous sodium sulfate and concentrated under reduced pressure. The residue was subjected to silica gel column chromatography (solvent; ethyl acetate) to obtain 45.2 g of 1-(t-butyl)diphenylsilyl-2-(3-chloro-4-fluorophenyl)-3-(pyridin-4-yl)-1H-pyrrole as a white powder (yield: 81%).

Then 45.2 g (88.4 mmol) of this powder was dissolved in 900 ml of toluene, and to this solution were added 14.3 ml of pyridine and 33.9 g (106.1 mmol) of pyridine perbromide hydrobromide, and the mixture was stirred at room temperature overnight. The reaction mixture was filtered and the filtrate was concentrated under reduced pressure. The residue was subjected to silica gel column chromatography (solvent; ethyl acetate:hexane = 1:2) to obtain 42.3 g of the title compounds as a white powder (yield: 81%).
$^1$H-NMR Spectrum (400 MHz, DMSO-$d_6$) δ ppm:
8.36 (2H, d, J = 6Hz), 7.46-7.29 (11H, m), 7.03 (2H, d, J = 6Hz), 6.72-6.66 (1H, m), 6.56-6.53 (1H, m), 6.53-6.47 (1H, m), 1.06 (9H, s).

2) 2-(3-Chloro-4-fluorophenyl)-4-[(2S,8aS)-2-phenyl-1,2,3,5,6,8a-hexahydroindoli zin-7-yl]-3-(pyridin-4-yl)-1H-pyrrole

In 54 ml of tetrahydrofuran was dissolved 2.70 g (4.65 mmol) of 4-bromo-1-(t-butyl)diphenylsilyl-2-(3-chloro-4-fluorophenyl)-3-(pyridin-4-yl)-1H-pyr role obtained in 1), and to this solution was added dropwise a solution of 3.30 ml (5.11 mmol) of 1.57M butyl lithium/hexane at -78°C. The mixture was stirred at the same temperature for 15 minutes. To this reaction mixture was slowly added dropwise a separately prepared solution containing 1.00 g (4.65 mmol) of (2S,8aS)-2-phenyl-1,2,3,5,6,7,8,8a-octahydroindolizin-7-one and 237 mg (5.58 mmol) of lithium chloride in 14 ml of tetrahydrofuran at -78°C, and the mixture was stirred at the same temperature for 1 hour. To the reaction mixture were added successively 0.66 ml (10.23 mmol) of methanesulfonic acid, 54 ml of dichloromethane, 1.50 ml (18.59 mmol) of pyridine, 0.54 ml (7.44 mmol) of thionyl chloride, and 15 ml of methanol. Then, the cooling bath was removed and stirring was continued until the mixture returned to room temperature. The solvent was distilled off under reduced

pressure, and 15 ml of tetrahydrofuran, 15 ml of methanol, and 15 ml of water were added to the residue, and further 11.2 ml of 25% aqueous sodium hydroxide solution was added thereto. The mixture was stirred at room temperature for 3 hours. The solvent was distilled off and the reaction product was extracted by adding 100 ml of ethyl acetate and 50 ml of water to the residue and the organic layer was washed with water, dried over anhydrous sodium sulfate and concentrated under reduced pressure. The residue was subjected to silica gel column chromatography (solvent; ethyl acetate:methanol:isopropylamine = 20:1:1) to obtain 1.00 g of the title compound as a brown powder (yield: 46%).
Melting Point: 158-163°C (dec.)
$^1$H-NMR Spectrum (400 MHz, DMSO-$d_6$) δ ppm:
11.52 (1H, s), 8.50 (2H, d, J = 6Hz), 7.37-7.25 (6H, m), 7.19-7.17 (3H, m), 7.07-7.04 (1H, m), 7.01 (1H, d, J = 2Hz), 5.21 (1H, s), 3.51-3.46 (1H, m), 3.35-3.28 (1H, m), 3.22-3.17 (1H, m), 2.90-2.87 (1H, m), 2.73-2.69 (1H, m), 2.64-2.62 (1H, m), 2.33-2.27 (1H, m), 2.08-2.03 (1H, m), 1.87-1.80 (1H, m), 1.77-1.70 (1H, m).

(Example 47)

5-(3-Chloro-4-fluorophenyl)-3-[(2S,8aS)-2-phenyl-1,2,3,5,6,8a-hexahydroindolizin-7-yl]-4-(pyridin-4-yl)-pyrazole (Exemplary compound No. 3-403)

A reaction similar to that of Example 5-2) using 3-bromo-5-(3-chloro-4-fluorophenyl)-4-(pyridin-4-yl)pyrazole instead of 3-bromo-5-(4-fluorophenyl)-4-(pyridin-4-yl)pyrazole and (2S,8aS)-2-phenyl-1,2,3,5,6,7,8,8a-octahydroindolizin-7-one instead of (2S,8aS)-2-butyl-1,2,3,5,6,7,8,8a-octahydroindolizin-7-one was carried out, and the resulting product was subjected to a dehydration reaction similar to that of Example 5-3), and to silica gel column chromatography (solvent; ethyl acetate:methanol:dichloromethane = 10:10:1), to obtain 60 mg of the title compound (Rf value: 0.25) as a yellow powder (yield: 2%).
Melting Point: 179-180°C
$^1$H-NMR Spectrum (400 MHz, $CDCl_3$) δ ppm:
8.59 (2H, d, J = 6Hz), 7.49 (1H, d, J = 5Hz), 7.33-7.29 (2H, m), 7.26-7.21 (3H, m), 7.18 (2H, d, J = 6Hz), 7.13-7.09 (1H, m), 7.04 (1H, t, J = 9Hz), 5.97 (1H, s), 3.68-3.64 (1H, m), 3.50-3.44 (1H, m), 3.38-3.33 (1H, m), 3.05-3.00 (1H, m), 2.90-2.82 (2H, m), 2.43-2.36 (1H, m), 2.24-2.21 (1H, m), 2.11-2.05 (2H, m).

(Reference Example 1)

(2R,8aS)-2-Methoxy-1,2,3,5,6,7,8,8a-octahydroindolizin-7-one

1) (2S,4R)-1-Benzyloxycarbonyl-2-cyanomethyl-4-methoxypyrrolidine

In 20 ml of tetrahydrofuran was dissolved 2.00 g (7.16 mmol) of (2S,4R)-1-benzyloxycarbonyl-4-methoxyproline and the solution was stirred at 0°C. To this solution was added 17.9 ml (17.9 mmol) of 1M borane-tetrahydrofuran complex solution in tetrahydrofuran, and the mixture was stirred at the same temperature for 1 hour and further at room temperature for 3 hours. The reaction mixture was cooled again to 0°C, methanol was carefully added thereto and the mixture was concentrated under reduced pressure. Saturated sodium hydrogencarbonate was added to the residue, the mixture was extracted with ethyl acetate, and the organic layer was washed with water, dried over anhydrous magnesium sulfate, and concentrated under reduced pressure to obtain 1.82 g of a brown oil (reduction product: alcohol).

This oil was dissolved in 25 ml of dichloromethane, and to this solution was added 1.13 ml (8.14 mmol) of triethylamine. 0.58 ml (7.46 mmol) of methanesulfonyl chloride was added to the resulting mixture under ice cooling and stirring, and the mixture was stirred at the same temperature for 30 minutes. To the reaction mixture was added saturated aqueous sodium hydrogencarbonate solution, and the mixture was extracted with dichloromethane. The organic layer was washed with water, dried over anhydrous magnesium sulfate and concentrated under reduced pressure to obtain 2.19 g of a brown oil (mesylate).

Then this oil was dissolved in 22 ml of dimethyl sulfoxide and to this solution was added 0.31 g (6.32 mmol) of sodium cyanide. The mixture was stirred at 100°C for 30 minutes. Saturated aqueous sodium hydrogencarbonate solution was added to the reaction mixture, and the mixture was extracted with ethyl acetate. The organic layer was washed with water, dried over anhydrous sodium sulfate and concentrated under reduced pressure to obtain 1.70 g of the title compound as a slightly brown oil (yield: 88%).
$^1$H-NMR Spectrum (400 MHz, $CDCl_3$) δ ppm:
7.43-7.28 (5H, m), 5.26-5.08 (2H, m), 4.20-4.10 (1H, m), 4.03-3.93 (1H, m), 3.90 (0.4H, d, J = 12Hz), 3.74 (0.6H, d, J = 12Hz), 3.56-3.44 (1H, m), 3.31 (1.2H, s), 3.30 (1.8H, s), 3.16 (0.6H, dd, J=17Hz, 6Hz), 2.80 (0.4H, dd, J=17Hz, 6Hz), 2.76-2.58 (1H, m), 2.39-2.30 (1H, m), 2.08-1.97 (1H, m).

2) (2S,4R)-1-Benzyloxycarbonyl-2-(3-ethoxycarbonyl-2-oxopropyl)-4-methoxypyrroli dine

In 600 ml of tetrahydrofuran was suspended 57.19 g (875 mmol) of zinc powder and the suspension was heated to 90°C. To the suspension was added 0.5 ml (4.51 mmol) of ethyl bromoacetate and the mixture was refluxed with heating for 1 hour. To the reaction mixture were further added a solution of 30.00 g (109 mmol) of (2S,4R)-1-benzyloxycarbonyl-2-cyanomethyl-4-methoxypyrrolidine obtained in 1) in 30 ml of tetrahydrofuran and 84.9 ml (766 mmol) of ethyl bromoacetate, and the mixture was heated at reflux for 1.5 hours. The mixture was cooled to room temperature and filtered, and the filtrate was concentrated under reduced pressure. The residue was dissolved in ethyl acetate and mixed thoroughly with saturated aqueous sodium hydrogencarbonate solution and separated. The organic layer was washed with water, dried over anhydrous magnesium sulfate and concentrated under reduced pressure. The residue was dissolved in a mixture of 200 ml of dioxane and 100 ml of 1N aqueous hydrochloric acid solution and left to stand at room temperature for 3 hours. Water was added to the reaction mixture and the mixture was extracted with ethyl acetate. The organic layer was washed with water and concentrated under reduced pressure, and the resulting residue was subjected to silica gel column chromatography (solvent; ethyl acetate:hexane = 2:3) to obtain 28.23 g of the title compound as a pale yellow oil (yield: 71%).
$^1$H-NMR Spectrum (400 MHz, $CDCl_3$) δ ppm:
7.43-7.27 (5H, m), 5.24-5.06 (2H, m), 4.33-4.10 (3H, m), 3.93-3.86 (1H, m), 3.78 (0.4H, d, J = 12Hz), 3.65 (0.6H, d, J = 12Hz), 3.52-3.24 (3.6H, m), 3.29 (3H, s), 3.14-3.05 (0.4H, m), 2.80-2.62 (1H, m), 2.42-2.32 (1H, m), 1.84-1.73 (1H, m), 1.34-1.21 (3H, m).

3) (2R,8aS)-2-Methoxy-1,2,3,5,6,7,8,8a-octahydroindolizin-7-one

In 150 ml of ethyl acetate was dissolved 15.00 g (41.3 mmol) of (2S,4R)-1-benzyloxycarbonyl-2-(3-ethoxycarbonyl-2-oxopropyl)-4-methoxypyrrolidin e obtained in 2), and to this solution were added 3.79 ml (45.4 mmol) of pyrrolidine, 1.50 g of molecular sieve (MS4A) and 3.75 g of 20% palladium hydroxide on carbon. The mixture was stirred at room temperature under a hydrogen atmosphere for 2 hours. The reaction mixture was filtered, the filtrate was concentrated under reduced pressure, and the resulting residue was dissolved in 150 ml of tetrahydrofuran. This solution was added dropwise to a suspension of 4.70 g (124 mmol) of lithium aluminum hydride in 100 ml of tetrahydrofuran under ice cooling and stirring, and the mixture was stirred at room temperature for 18 hours.
The reaction mixture was cooled to 0°C, and 19 ml of 4% aqueous sodium hydroxide solution was carefully added thereto, followed by the addition of 250 ml of ethanol, and the mixture was filtered. The filtrate was concentrated under reduced pressure and the resulting residue was subjected to alumina column chromatography (solvent; ethyl acetate) to obtain 4.13 g of the title compound as a slightly brown powder (yield: 59%).
$^1$H-NMR Spectrum (400 MHz, $CDCl_3$) δ ppm:
4.12-4.04 (1H, m), 3.54 (1H, dd, J = 10Hz, 7Hz), 3.34-3.24 (1H, m), 3.29 (3H, s), 2.63-2.30 (5H, m), 2.29-2.19 (2H, m), 2.00 (1H, ddd, J = 13Hz, 6Hz, 1Hz), 1.79-1.67 (1H, m).

(Reference Example 2)

(2R,8aS)-2-(t-Butyldimethylsilyloxy)-1,2,3,5,6,7,8,8a-octahydroindolizin-7-one

1) (2R,8aS)-2-Hydroxy-1,2,3,5,6,7,8,8a-octahydroindolizin-7-one

In 26 ml of 47% aqueous hydrobromic acid solution was dissolved 2.63 g (15.5 mmol) of (2R,8aS)-2-methoxy-1,2,3,5,6,7,8,8a-octahydroindolizin-7-one obtained in Reference Example 1 and the solution was stirred at 100°C for 8 hours, cooled to 0°C, neutralized by the addition of sodium carbonate, and concentrated under reduced pressure.
Ethanol was added to the residue and the insolubles were filtered off. The filtrate was concentrated under reduced pressure and the residue was subjected to alumina column chromatography (solvent; ethyl acetate:methanol = 39:1) to obtain 1.30 g of the title compound as a pale yellow oil (yield: 52%).
$^1$H-NMR Spectrum (400 MHz, $CDCl_3$) δ ppm:
4.63-4.56 (1H, m), 3.58 (1H, dd, J = 10Hz, 7Hz), 3.31-3.24 (1H, m), 2.72-2.44 (4H, m), 2.39-2.32 (1H, m), 2.29-2.20 (2H, m), 1.97-1.70 (3H, m).

2) (2R,8aS)-2-(t-Butyldimethylsilyloxy)-1,2,3,5,6,7,8,8a-octahydroindolizin-7-one

(2R,8aS)-2-Hydroxy-1,2,3,5,6,7,8,8a-octahydroindolizin-7-one obtained in 1) was dissolved in 30 ml of dichloromethane. To this solution were added 1.70 g (24.9 mmol) of imidazole and 1.88 g (12.5 mmol) of t-butyldimethylsilyl chloride and the mixture was stirred at room temperature for 20 hours. Water was added to the reaction mixture, the

mixture was extracted with dichloromethane, and the organic layer was washed with water and concentrated under reduced pressure. The resulting residue was subjected to alumina column chromatography (solvent; hexane:ethyl acetate = 9:1) to obtain 1.98 g of the title compound as a colorless oil (yield 88%).
$^1$H-NMR Spectrum (400 MHz, $CDCl_3$) δ ppm:
4.56-4.48 (1H, m), 3.50-3.42 (1H, m), 3.30-3.23 (1H, m), 2.69-2.32 (5H, m), 2.30-2.17 (2H, m), 1.90-1.78 (2H, m), 0.88 (9H, s), 0.06 (3H, s), 0.05 (3H, s)$_o$.

(Reference Example 3)

(2S,8aS)-2-Chloro-1,2,3,5,6,7,8,8a-octahydroindolizin-7-one

In 45 ml of carbon tetrachloride was dissolved 1.55 g (10.0 mmol) of (2R,8aS)-2-hydroxy-1,2,3,5,6,7,8,8a-octahydroindolizin-7-one obtained in Reference Example 2-1), and to this solution was added 3.93g (15.0 mmol) of triphenylphosphine. The mixture was heated at reflux for 5 hours. The solvent was distilled off under reduced pressure and the residue was subjected to alumina column chromatography (solvent; ethyl acetate:hexane = 1:1) to obtain 1.52 g of the title compound as a pale yellow oil (yield: 88%).
$^1$H-NMR Spectrum (400 MHz, $CDCl_3$) δ ppm:
4.47-4.40 (1H, m), 3.39-3.31 (2H, m), 2.78-2.63 (3H, m), 2.57-2.42 (2H, m), 2.42-2.26 (3H, m), 1.94 (1H, ddd, J = 14Hz, 10Hz, 5Hz).

(Reference Example 4)

(8aS)-2,2-Difluoro-1,2,3,5,6,7,8,8a-octahydroindolizin-7-one

The reactions of Reference Examples 1-1), 2) and 3) were successively carried out using (2S)-1-benzyloxycarbonyl-4,4-difluoroproline instead of (2S,4R)-1-benzyloxycarbonyl-4-methoxyproline as the starting material to obtain the title compound as a pale yellow oil (total yield: 14%).
$^1$H-NMR Spectrum (400 MHz, $CDCl_3$) δ ppm:
3.55-3.45 (1H, m), 3.33-3.24 (1H, m), 2.72-2.33 (8H, m), 2.17-2.00 (1H, m).

(Reference Example 5)

(±)-6,7,8,9,9a,10-Tetrahydropyrido[1,2-a]indol-8-one

1) 1-Benzyloxycarbonylindoline-2-methanol

In 450 ml of tetrahydrofuran was dissolved 33.0 g (106 mmol) of 1-benzyloxycarbonylindoline-2-carboxylic acid methyl ester and to this solution was added 4.6 g (212 mmol) of lithium borohydride in three portions, and the mixture was stirred at room temperature for 5 hours. Then, ice was added to the reaction mixture and the mixture was stirred for 1 hour and extracted with ethyl acetate. The organic layer was washed with water, dried over anhydrous magnesium sulfate and concentrated under reduced pressure. The residue was subjected to silica gel column chromatography (ethyl acetate:hexane = 2:5) to obtain 25.0 g of the title compound as a colorless oil (yield: 83%).
$^1$H-NMR Spectrum (400 MHz, $CDCl_3$) δ ppm:
7.47-7.28 (6H, m), 7.19-7.10 (2H, m), 6.97 (1H, t, J = 7Hz), 5.30 (2H, s), 4.72-4.53 (2H, m), 3.82-3.63 (2H, m), 3.33 (1H, dd, J = 16Hz, 10Hz), 3.00-2.77 (1H, m).

2) 1-Benzyloxycarbonyl-2-cyanomethylindoline

Methanesulfonylation and cyanation were carried out similarly to Reference Example 1-1) using 1-benzyloxycarbonylindoline-2-methanol obtained in 1) to give the title compound as an orange-color oil (yield: 65%).
$^1$H-NMR Spectrum (400 MHz, $CDCl_3$) δ ppm:
7.48-7.32 (6H, m), 7.23-7.16 (2H, m), 7.02 (1H, t, J=7Hz), 5.31 (2H, s), 4.81-4.68 (1H, m), 3.50 (1H, dd, J = 16Hz, 10Hz), 3.01 (1H, d, J=16Hz), 2.99-2.50 (2H, m).

3) 1-Benzyloxycarbonyl-2-(3-ethoxycarbonyl-2-oxopropyl)indoline

A reaction similar to Reference Example 1-2) was carried out using 1-benzyloxycarbonyl-2-cyanomethylindoline obtained in 2) instead of (2S,4R)-1-benzyloxycarbonyl-2-cyanomethyl-4-methoxypyrrolidine to give the title compound as a yellow oil (yield: 47%).
$^{1}$H-NMR Spectrum (400 MHz, $CDCl_3$) δ ppm:
7.45-4.32 (6H, m), 7.22-7.13 (2H, m), 6.97 (1H, t, J = 7Hz), 5.28 (2H, s), 4.92-4.84 (1H, m), 4.22-4.12 (2H, m), 3.46 (1H, dd, J = 16Hz, 9Hz), 3.43-3.31 (2H, m), 2.84 (1H, dd, J = 6Hz, 4Hz), 2.80 (1H, dd, J = 6Hz, 4Hz), 2.74 (1H, dd, J = 16Hz, 2Hz), 1.29 (3H, t, J = 7Hz).

4) 2-(3-Ethoxycarbonyl-2-oxopropyl)indoline

A hydrolysis reaction (debenzylation reaction) with hydrogen gas/palladium hydroxide on carbon similar to Reference Example 1-3) was carried out using 1-benzyloxycarbonyl-2-(3-ethoxycarbonyl-2-oxopropyl)indoline obtained in 3) to give the title compound as an orange-color oil (quantitative yield).
$^{1}$H-NMR Spectrum (400 MHz, $CDCl_3$) δ ppm:
7.08 (1H, d, J = 7Hz), 6.99 (1H, t, J = 7Hz), 6.68 (1H, t, J = 7Hz), 6.57 (1H, d, J = 7Hz), 4.28-4.16 (1H, m), 4.12-4.01 (2H, m), 3.39-3.18 (4H, m), 3.03-2.81 (1H, m), 2.81-2.71 (1H, m), 1.32-1.21 (3H, m).

5) (±)-6,7,8,9,9a,10-Tetrahydropyrido[1,2-a]indol-8-one

In 140 ml of dichloromethane wad dissolved 7.0 g (28.3 mmol) of 2-(3-ethoxycarbonyl-2-oxopropyl)indoline obtained in 4), and to this solution was added 14.1 ml (56.6 mmol) of 4N hydrochloric acid/dioxane solution. The mixture was stirred at room temperature for 2 hours. The reaction mixture was concentrated under reduced pressure, and the residue was dissolved in 140 ml of ethanol. To this solution was added 2.6 ml (31.1 mmol) of pyrrolidine, and the mixture was stirred at room temperature for 5 hours. The reaction mixture was concentrated under reduced pressure and the residue was dissolved in 100 ml of tetrahydrofuran. To this solution was added under ice cooling 3.18 g (84.9 mmol) of lithium aluminum hydride, and the mixture was stirred at room temperature for 18 hours.

The reaction mixture was cooled to 0°C and 13 ml of 4% aqueous sodium hydroxide solution was carefully added thereto, followed by the addition of 150 ml of ethanol. The mixture was filtered. The filtrate was concentrated under reduced pressure and the resulting residue was subjected to alumina column chromatography (solvent; ethyl acetate:hexane = 1:5) to obtain 720 mg of the title compounds as a pale yellow oil (yield: 14%).
$^{1}$H-NMR Spectrum (400 MHz, $CDCl_3$) δ ppm:
7.20-7.10 (2H, m), 6.61-6.52 (2H, m), 3.20-3.09 (2H, m), 2.74-2.33 (7H, m).

(Reference Example 6)

(2R,8aS)-2-Phenyl-1,2,3,5,6,7,8,8a-octahydroindolizin-7-one

1) (S)-1-Benzyloxycarbonyl-2-hydroxymethyl-4-phenyl-3-pyrroline

The methyl ester of (S)-1-benzyloxycarbonyl-4-phenyl-3-pyrrolin-2-carboxylic acid [J. Med. Chem., 31, 1148-1160 (1988)] was reduced with lithium borohydride in a similar manner to Reference Example 5-1) to obtain the title compound as a pale yellow powder (yield: 78%).
$^{1}$H-NMR Spectrum (400 MHz, $CDCl_3$) δ ppm:
7.47-7.27 (10H, m), 6.11-6.07 (0.2H, m), 6.03-5.99 (0.8H, m), 5.23 (2H, dd, J = 16Hz, 12Hz), 5.00-4.94 (0.8H, m), 4.85-4.80 (0.2H, m), 4.77-4.70 (0.2H, m), 4.65 (0.8H, dt, J = 15Hz, 2Hz), 4.56 (1H, ddd, J = 15Hz, 5Hz, 2Hz), 4.26 (1H, br.s), 3.94-3.83 (1H, m), 3.80-3.74 (0.2H, m), 3.72 (0.8H, dd, 12Hz, 7Hz).

2) (S)-1-Benzyloxycarbonyl-2-cyanomethyl-4-phenyl-3-pyrroline

Methanesulfonylation and cyanation were carried in a manner similar to Reference 1-1) using (S)-1-benzyloxycarbony-2-hydroxymethyl-4-phenyl-3-pyrroline obtained in 1) to give the title compound as a pale yellow powder (yield: 80%).
$^{1}$H-NMR Spectrum (400 MHz, $CDCl_3$) δ ppm:
7.47-7.29 (10H, m), 6.17-6.11 (1H, m), 5.25 (1H, d, J = 12Hz), 5.18 (1H, d, J = 12Hz),5.01-4.90 (1H, m), 4.77-4.60 (2H, m), 3.10 (0.7H, dd, J = 17Hz, 6Hz), 2.90 (0.7H, dd, J = 17Hz, 3Hz), 2.86-2.73 (0.6H, m).

3) (2S,4R)-1-Benzyloxycarbonyl-2-cyanomethyl-4-phenylpyrrolidine

In 150 ml of ethyl acetate was dissolved 11.60 g (36.4 mmol) of (S)-1-benzyloxycarbonyl-2-cyanomethyl-4-phenyl-3-pyrroline obtained in 2), and to this solution was added 2.32 g of 20% palladium hydroxide on carbon. The

mixture was stirred at room temperature under a hydrogen atmosphere for 7 hours. The reaction mixture was filtered, the filtrate was concentrated under reduced pressure, and the residue was subjected to silica gel column chromatography (solvent; hexane:ethyl acetate= 4:1) to obtain 8.18 g of the title compound as a pale brown oil (yield: 70%).
$^1$H-NMR Spectrum (400 MHz, $CDCl_3$) δ ppm:
7.44-7.17 (10H, m), 5.26-5.09 (2H, m), 4.32-4.06 (2H, m), 3.50-3.41 (1H, m), 3.37-3.26 (1H, m), 3.21 (0.7H, dd, J = 17Hz, 6Hz), 2.93 (0.3Hz, dd, J = 17Hz, 6Hz), 2.88-2.59 (2H, m), 2.19-2.07 (1H, m).

4) (2S,4R)-1-Benzyloxycarbonyl-2-(3-ethoxycarbonyl-2-oxopropyl)-4-phenylpyrroli dine

The reaction was carried out in a manner similar to Reference Example 1-2) using (2S,4R)-1-benzyloxycarbonyl-2-cyanomethyl-4-phenylpyrrolidine obtained in 3) instead of (2S,4R)-1-benzyloxycarbonyl-2-cyanomethyl-4-methoxypyrrolidine to give the title compound as a pale yellow oil (yield: 72%).
$^1$H-NMR Spectrum (400 MHz, $CDCl_3$) δ ppm:
7.43-7.17 (10H, m), 5.23-5.07 (2H, m), 4.31-4.10 (4H, m), 3.55-3.20 (5H, m), 2.87-2.67 (2H, m), 1.85-1.74 (1H, m), 1.32-1.21 (3H, m).

5) (2R,8aS)-2-Phenyl-1,2,3,5,6,7,8,8a-octahydroindolizin-7-one

The reaction was carried out in a manner similar to Reference Example 1-3) using (2S,4R)-1-benzyloxycarbonyl-2-(3-ethoxycarbonyl-2-oxopropyl)-4-phenylpyrrolidine obtained in 4) instead of (2S,4R)-1-benzyloxycarbonyl-2-(3-ethoxycarbonyl-2-oxopropyl)-4-methoxypyrrolidin e to give the title compound as a pale yellow oil (yield: 27%).
$^1$H-NMR Spectrum (400 MHz, $CDCl_3$) δ ppm:
7.42-7.14 (5H, m), 3.42-3.28 (2H, m), 3.18 (1H, dd, J = 9Hz, 3Hz), 2.77-2.63 (2H, m), 2.58-2.30 (6H, m), 1.70-1.58 (1H, m).

(Reference Example 7)

(8aS)-2,2-Ethylenedioxy-1,2,3,5,6,7,8,8a-octahydroindolizin-7-one

1) (S)-1-Benzyloxycarbonyl-4,4-ethylenedioxy-2-hydroxymethylpyrrolidine

(S)-1-Benzyloxycarbonyl-4,4-ethylenedioxyproline methyl ester was reduced with lithium borohydride in a manner similar to Reference Example 5-1) to obtain the title compound as a colorless oil (yield: 85%).
$^1$H-NMR Spectrum (400 MHz, $CDCl_3$) δ ppm:
7.42-7.29 (5H, m), 5.14 (2H, s), 4.22-4.08 (1H, m), 4.02-3.88 (4H, m), 3.82-3.62 (2H, m), 3.59 (1H, d, J = 12Hz), 3.47 (1H, d, J = 12Hz), 2.27-2.18 (1H, m), 1.90-1.82 (1H, m).

2) (S)-1-Benzyloxycarbonyl-2-cyanomethyl-4,4-ethylenedioxypyrrolidine

Methanesulfonylation and cyanation were carried in a manner similar to Reference Example 1-1) using (S)-1-benzyloxycarbonyl-4,4-ethylenedioxy-2-hydroxymethylpyrrolidine obtained in 1) to give the title compound as a colorless oil (yield: 88%).
$^1$H-NMR Spectrum (400 MHz, $CDCl_3$) δ ppm:
7.43-7.28 (5H, m), 5.22-5.08 (2H, m), 4.30-4.21 (1H, m), 4.06-3.88 (4H, m), 3.62-3.44 (2H, m), 3.00-2.72 (2H, m), 2.41-2.29 (1H, m), 2.17-2.10 (1H, m).

3) (8aS)-2,2-Ethylenedioxy-1,2,3,5,6,7,8,8a-octahydroindolizin-7-one

Reactions similar to those of Reference Examples 1-2) and 1-3) were successively carried out using (S)-1-benzyloxycarbonyl-2-cyanomethyl-4,4-ethylenedioxypyrrolidine obtained in 2) instead of (2S,4R)-1-benzyloxycarbonyl-2-cyanomethyl-4-methoxypyrrolidine as the starting material, to give the title compound as a white powder (total yield: 17%).
$^1$H-NMR Spectrum (400 MHz, $CDCl_3$) δ ppm:
4.06-3.84 (4H, m), 3.32-3.24 (1H, m), 3.22 (1H, d, J = 10Hz), 2.73-2.61 (1H, m), 2.58-2.32 (6H, m), 2.23 (1H, dd, J = 13Hz, 6Hz), 1.89 (1H, dd, J = 13Hz, 10Hz).

(Reference Example 8)

(8aS)-2-Methyl-1,2,3,5,6,7,8,8a-octahydroindolizin-7-one

1) (S)-1-Benzyloxycarbonyl-4-methylideneproline methyl ester

In 100 ml of diethyl ether was suspended 1.41 g (12.6 mmol) of potassium t-butoxide, and to this suspension was added 4.80 g (13.4 mmol) of methyltriphenylphosphonium bromide. The mixture was stirred at 5°C for 15 minutes and a solution of 2.50 g (9.0 mmol) of (S)-1-benzyloxycarbonyl-4-oxoproline methyl ester in 30 ml of diethyl ether was added thereto. The resulting mixture was stirred at 35°C for 3 hours. To the reaction mixture was added under ice cooling 50 ml of saturated aqueous ammonium chloride solution, and the mixture was separated. The organic layer was washed with water, dried over anhydrous magnesium sulfate and concentrated under reduced pressure, and the residue was subjected to silica gel column chromatography (solvent; ethyl acetate:hexane = 1:3) to obtain 1.80 g of the title compound as a pale yellow oil (yield: 72%).
$^1$H-NMR Spectrum (400 MHz, $CDCl_3$) δ ppm:
7.40-7.23 (5H, m), 5.21-4.95 (4H, m), 4.60-4.50 (1H, m), 4.20-4.09 (2H, m), 3.74 (1.5H, s), 3.60 (1.5H, s), 3.07-2.91 (1H, m), 2.65 (1H, d, J = 16Hz).

2) (2S)-4-Methylproline methyl ester

In 50 ml of methanol was dissolved 1.80 g (6.5 mmol) of (S)-1-benzyloxycarbonyl-4-methylidene proline methyl ester obtained in 1), and to this solution was added 180 mg of 10% palladium on carbon, followed by stirring at room temperature under a hydrogen atmosphere for 2 hours. The reaction mixture was filtered and the filtrate was concentrated under reduced pressure to obtain 0.93 g of the title compound as a pale yellow oil (quantitative yield).
$^1$H-NMR Spectrum (400 MHz, $CDCl_3$) δ ppm:
3.82 (1H, t, J = 8Hz), 3.74 (3H, s), 3.08 (1H, dd, J = 10Hz, 7Hz), 2.60 (1H, dd, J = 10Hz, 8Hz), 2.33 (1H, dt, J = 12Hz, 8Hz), 2.28-2.15 (1H, m), 1.44-1.37 (1H, m), 1.27 (1H, dd, J = 14Hz, 7Hz), 1.02 (3H, d, J=7Hz).

3) (2S)-1-Benzyloxycarbonyl-2-hydroxymethyl-4-methylpyrrolidine

In 20 ml of toluene was dissolved 0.93 g (6.5 mmol) of (2S)-4-methylproline methyl ester obtained in 2), and to this solution were added a solution of 1.89 g (22.5 mmol) of sodium hydrogencarbonate in 20 ml of water and 1.54 ml (10.8 mmol) of benzyl chloroformate. The mixture was stirred at room temperature overnight. The reaction mixture was extracted by adding ethyl acetate, and the organic layer was washed with water and concentrated under reduced pressure to obtain 1.78 g of (2S)-1-benzyloxycarbonyl-4-methylproline methyl ester as a pale yellow oil (yield 99%). Then reduction with lithium borohydride was carried out in a manner similar to Reference Example 5-1) using the whole amount of this compound to obtain 1.07 g of the title compound as a pale yellow oil (yield: 66%).
$^1$H-NMR Spectrum (400 MHz, $CDCl_3$) δ ppm:
7.42-7.29 (5H, m), 5.14 (2H, br.s), 5.07-4.90 (1H, m), 4.08-3.87 (1H, m), 3.86-3.40 (4H, m), 2.90-2.65 (1H, m), 2.40-2.05 (2H, m), 1.02 (3H, d, J = 6Hz).

4) (2S)-1-Benzyloxycarbonyl-2-cyanomethyl-4-methylpyrrolidine

Methanesulfonylation and cyanation were carried in a manner similar to Reference Example 1-1) using (2S)-1-benzyloxycarbonyl-2-hydroxymethyl-4-methylpyrrolidine obtained in 3) to give the title compound as a colorless oil (yield: 70%).
$^1$H-NMR Spectrum (400 MHz, $CDCl_3$) δ ppm:
7.50-7.30 (5H, m), 5.25-5.05 (2H, m), 4.20-3.78 (1.8H, m), 3.70-3.62 (0.2H, m), 3.15-2.88 (1.4H, m), 2.84-2.67 (1.2H, m), 2.62-2.50 (0.4H, m), 2.45-2.30 (0.8H, m), 2.23-2.00 (1H, m), 1.89-1.77 (0.2H, m), 1.60-1.49 (1H, m), 1.10-1.03 (3H, m).

5) (2S)-1-Benzyloxycarbonyl-2-(3-ethoxycarbonyl-2-oxopropyl)-4-methylpyrrolidine

The reaction was carried out in a manner similar to Reference Example 1-2) using (2S)-1-benzyloxycarbonyl-2-cyanomethyl-4-methylpyrrolidine obtained in 4) instead of (2S,4R)-1-benzyloxycarbonyl-2-cyanomethyl-4-methoxypyrrolidine to give the title compound as a pale yellow oil (yield: 66%).
$^1$H-NMR Spectrum (400 MHz, $CDCl_3$) δ ppm:
7.43-7.28 (5H, m), 5.20-4.99 (2H, m), 4.27-4.03 (3H, m), 3.56-3.40 (1.6H, m), 3.37-3.25 (0.4H, m), 3.00-2.89 (0.2H,

m), 2.89-2.75 (0.8H, m), 2.75-2.56 (1H, m), 2.50-2.22 (1H, m), 2.20-2.05 (1H, m), 1.32-1.15 (4H, m), 1.08 (0.6H, d, J = 6Hz), 1.02 (2.4H, d, J = 6Hz).

6) (8aS)-2-Methyl-1,2,3,5,6,7,8,8a-octahydroindolizin-7-one

The reaction was carried out in a manner similar to Reference Example 1-3) using (2S)-1-benzyloxycarbonyl-2-(3-ethoxycarbonyl-2-oxopropyl)-4-methylpyrrolidine obtained in 5) instead of (2S,4R)-1-benzyloxycarbonyl-2-(3-ethoxycarbonyl-2-oxopropyl)-4-methoxypyrrolidin e to give the title compound as a colorless oil (yield: 34%).
$^1$H-NMR Spectrum (400 MHz, $CDCl_3$) δ ppm:
3.34-3.22 (1.2H, m), 2.77 (0.8H, dd, J = 9Hz, 3Hz), 2.68-2.55 (1H, m), 2.51-2.43 (2H, m), 2.39-2.24 (5H, m), 2.20-2.10 (1H, m), 1.87-1.75 (0.8H, m), 1.57-1.51 (0.2H, m), 1.14 (2.4H, d, J = 7Hz), 1.04 (0.6H, d, J = 7Hz).

(Reference Example 9)

(8aS)-8-Methyl-1,2,3,5,6,7,8,8a-octahydroindolizin-7-one

1) (2S)-1-Methylmalonyl-2-(1-carboxyethyl)pyrrolidine

In 60 ml of dichloromethane was dissolved 2.43 g (17.0 mmol) of (2S)-2-(1-carboxyethyl)pyrrolidine [Tetrahedron Lett. 40, 2891-2894 (1999)] and to this solution was added 2.61 ml (18.7 mmol) of triethylamine. To the mixture was added dropwise under ice cooling and stirring 2.01 ml (18.7 mmol) of methylmalonyl chloride, and the mixture was stirred at the same temperature for 15 minutes. After the mixture was further stirred at room temperature for 30 minutes, saturated aqueous sodium hydrogencarbonate solution was added to this reaction mixture, and the aqueous layer was made pH 2 by the addition of concentrated hydrochloric acid, and extracted with a mixture of dichloromethane:isopropanol = 4:1. The organic layer was washed with water, dried over anhydrous magnesium sulfate and concentrated under reduced pressure to obtain 4.47 g of the title compound as a pale brown oil (quantitative yield).
$^1$H-NMR Spectrum (500 MHz, $CDCl_3$) δ ppm:
4.55-4.47 (0.4H, m), 4.34-4.25 (0.6H, m), 3.76 (2.4H, s), 3.59-3.42 (4H, m), 3.25-3.16 (0.6H, m), 2.14-1.80 (5H, m), 1.18 (1.8H, d, J = 7Hz), 1.08 (1.2H, d, J = 7Hz).

2) (8aS)-6-Methoxycarbonyl-8-methyl-1,2,3,5,6,7,8,8a-octahydroindolizin-5,7-dione

In 120 ml of tetrahydrofuran was dissolved 4.47 g (17.0 mmol) of (2S)-1-methylmalonyl-2-(1-carboxyethyl) pyrrolidine obtained in 1) and to this solution was added 4.13 g (25.5 mmol) of carbonyldiimidazole. The mixture was stirred at room temperature for 30 minutes, 3.81 ml (25.5 mmol) of 1,8-diazabicyclo[5.4.0]-7-undecene was added thereto, and the mixture was further stirred for 1 hour. The reaction mixture was concentrated under reduced pressure, and to the residue were added dichloromethane and aqueous 1N hydrochloric acid solution to separate the mixture. The organic layer was washed with water, dried over anhydrous magnesium sulfate and concentrated under reduced pressure to obtain 4.16 g of the title compound as a brown oil (quantitative yield).
$^1$H-NMR Spectrum (500 MHz, $CDCl_3$) δ ppm:
3.90 (3H, s), 3.89-3.82 (1H, m), 3.73-3.64 (1H, m), 3.53-3.32 (2H, m), 2.67-2.52 (2H, m), 2.33-2.25 (0.5H, m), 2.07-1.96 (1.5H, m), 1.87-1.74 (1H, m), 1.68-1.55 (1H, m), 1.43 (1.5H, d, J = 7Hz), 1.12 (1.5H, d, J = 7Hz).

3) (8aS)-8-Methyl-1,2,3,5,6,7,8,8a-octahydroindolizin-5,7-dione

In 40 ml of 10% aqueous acetic acid solution was dissolved 4.16 g (17.0 mmol) of (8aS)-6-methoxycarbonyl-8-methyl-1,2,3,5,6,7,8,8a-octahydroindolizin-5,7-dione obtained in 2), and the solution was stirred at 110°C for 30 minutes and then cooled to room temperature. The reaction solution was made alkaline by the addition of saturated aqueous sodium hydrogencarbonate solution and extracted with a mixture of dichloromethane: isopropanol = 4:1. The organic layer was washed with water, dried over anhydrous magnesium sulfate and concentrated under reduced pressure to obtain 2.62 g of the title compound as a brown oil (yield: 92%).
$^1$H-NMR Spectrum (500 MHz, $CDCl_3$) δ ppm:
4.03-3.97 (0.3H, m), 3.74-3.41 (2.7H, m), 3.27 (1.4H, s), 3.26 (0.6H, s), 2.73-2.67 (0.3H, m), 2.38-2.32 (0.7H, m), 2.30-2.23 (0.7H, m), 2.14-2.01 (1.3H, m), 1.96-1.86 (1H, m), 1.73-1.63 (1H, m), 1.77 (2.1H, d, J = 7Hz), 1.08 (0.9H, d, J = 7Hz).

4) (8aS)-8-Methyl-7-(1-pyrrolidinyl)-1,2,3,5,8,8a-hexahydroindolizin-5-one

In 30 ml of ethanol was dissolved 2.62 g (15.7 mmol) of (8aS)-8-methyl-1,2,3,5,6,7,8,8a-octahydroindolizin-5,7-dione obtained in 3) and to this solution was added 2.62 ml (31.4 mmol) of pyrrolidine. The mixture was stirred at 80°C for 30 minutes, and then the solvent and excess pyrrolidine were distilled off under reduced pressure to obtain 3.67 g of the title compound as a brown oil (quantitative yield).
$^1$H-NMR Spectrum (500 MHz, $CDCl_3$) δ ppm:
4.52 (1H, s), 3.80-3.65 (2H, m), 3.44-3.34 (1H, m), 3.33-3.17 (4H, m), 2.61-2.43 (1H, m), 2.02-1.89 (6H, m), 1.88-1.72 (4H, m), 1.01 (3H, d, J = 7Hz).

5) (8aS)-8-Methyl-1,2,3,5,6,7,8,8a-octahydroindolizin-7-one

In 50 ml of tetrahydrofuran was dissolved 3.67 g (15.7 mmol) of (8aS)-8-methyl-7-(1-pyrrolidinyl)-1,2,3,5,8,8a-hexahydroindolizin-5-one obtained in 4), and to this solution was added under ice cooling and stirring 1.79 g (47.1 mmol) of lithium aluminum hydride in portions. The mixture was stirred at room temperature overnight, and to the reaction mixture was added 7.22 ml of 1N aqueous sodium hydroxide solution. Ethanol was then added, and the insolubles were filtered off. The filtrate was concentrated under reduced pressure and the residue was subjected to alumina column chromatography (solvent; ethyl acetate) to obtain 1.69 g of the title compound as a pale yellow oil (yield: 70%).
$^1$H-NMR Spectrum (500 MHz, $CDCl_3$) δ ppm:
3.36-3.29 (1H, m), 3.18-3.13 (1H, m), 2.72-2.63 (1H, m), 2.40-2.30 (3H, m), 2.26-2.17 (1H, m), 2.05-1.91 (3H, m), 1.87-1.78 (1H, m), 1.64-1.55 (2H, m), 1.01 (3H, d, J=7Hz).

(Reference Example 10)

(2S,8aS)-2-Methoxy-1,2,3,5,6,7,8,8a-octahydroindolizin-7-one

1) (2S,4S)-4-Methoxy-1-methylmalonyl homoproline

In 100 ml of dichloromethane was suspended 4.40 g (22.5 mmol) of (2S,4S)-4-methoxy-homoproline hydrochloride, and to this suspension was added 7.52 ml (54 mmol) of triethylamine. To the mixture was added dropwise under ice cooling and stirring 2.66 ml (24.8 mmol) of methylmalonyl chloride, and the mixture was stirred at the same temperature for 15 minutes. The mixture was further stirred at room temperature for 2 hours, and saturated aqueous sodium hydrogencarbonate solution was added to the reaction mixture. The mixture was separated and the aqueous layer was made pH 2 by the addition of concentrated hydrochloric acid, and extracted with a mixture of dichloromethane: isopropanol = 4:1. The organic layer was washed with water, dried over anhydrous magnesium sulfate and concentrated under reduced pressure to obtain 5.83 g of the title compound as a brown oil (yield: quantitative).
$^1$H-NMR Spectrum (500 MHz, $CDCl_3$) δ ppm:
4.51-4.44 (0.8H, m), 4.36-4.30 (0.2H, m), 4.05-3.97 (1H, m), 3.78-3.73 (2.6H, m), 3.67-3.52 (2.4H, m), 3.48-3.38 (2H, m), 3.35-3.30 (3H, m), 3.14-3.08 (0.8H, m), 3.03 (0.2H, dd, J = 16Hz, 9Hz), 2.83-2.77 (0.2H, m), 2.69 (0.8H, dd, J = 16Hz, 9Hz), 2.21-2.12 (3H, m).

2) (2S,8aS)-2-Methoxy-6-methoxycarbonyl-1,2,3,5,6,7,8,8a-octahydroindolizin-5,7-dione

In 90 ml of tetrahydrofuran was suspended 5.83 g (22.5 mmol) of (2S,4S)-4-methoxy-1-methylmalonyl homoproline obtained in 1), and to this suspension was added 4.02 g (24.8 mmol) of carbonyldiimidazole. After the mixture was stirred at room temperature for 30 minutes, 3.71 ml (24.8 mmol) of 1,8-diazabicyclo[5.4.0]-7-undecene was added thereto, and the mixture was further stirred for 1 hour. The reaction mixture was concentrated under reduced pressure, and to the residue were added dichloromethane and aqueous 1N hydrochloric acid solution to separate the mixture. The organic layer was washed with water, dried over anhydrous magnesium sulfate and concentrated under reduced pressure to obtain 5.21 g of the title compound as a brown oil (yield: quantitative).
$^1$H-NMR Spectrum (500 MHz, $CDCl_3$) δ ppm:
4.02-3.96 (1H, m), 3.90 (3H, s), 3.87-3.79 (1H, m), 3.78-3.72 (1H, m), 3.55 (1H, dd, J = 13Hz, 6Hz), 3.39-3.29 (4H, m), 2.74 (1H, dd, J = 17Hz, 13Hz), 2.59 (1H, dd, J = 13Hz, 4Hz), 2.49-2.42 (1H, m), 1.84-1.77 (1H, m).

3) (2S,8aS)-2-Methoxy-1,2,3,5,6,7,8,8a-octahydroindolizine-5,7-dione

In 50 ml of 10% aqueous acetic acid solution was dissolved 5.21 g (22.5 mmol) of (2S,8aS)-2-methoxy-6-methoxycarbonyl-1,2,3,5,6,7,8,8a-octahydroindolizine-5,7-dio ne obtained in 2), and the solution was stirred at 110°C for 1 hour and then cooled to room temperature. The reaction mixture was made alkaline by the addition of saturated aqueous sodium hydrogencarbonate solution and extracted with a mixture of dichloromethane:isopropanol = 4:1. The organic layer was washed with water, dried over anhydrous magnesium sulfate and concentrated under reduced pressure to obtain 3.38 g of the title compound as a pale brown oil (yield: 82%).
$^1$H-NMR Spectrum (500 MHz, $CDCl_3$) δ ppm:
4.06-3.90 (3H, m), 3.52 (1H, dd, J = 12Hz, 5Hz), 3.37-3.28 (5H, m), 2.73 (1H, dd, J = 17Hz, 3Hz), 2.55-2.43 (2H, m), 1.95-1.87 (1H, m).

4) (2S,8aS)-2-Methoxy-7-(1-pyrrolidinyl)-1,2,3,5,8,8a-hexahydroindolizin-5-one

In 34 ml of ethanol was dissolved 3.38 g (18.4 mmol) of (2S,8aS)-2-methoxy-1,2,3,5,6,7,8,8a-octahydroindolizine-5,7-dione obtained in 3), and to this solution was added 3.07 ml (36.8 mmol) of pyrrolidine. The mixture was left to stand at room temperature for 30 minutes, and the solvent and excess pyrrolidine were distilled off under reduced pressure to obtain 4.26 g of the title compound as a brown oil (yield: 98%).
$^1$H-NMR Spectrum (500 MHz, $CDCl_3$) δ ppm:
4.60 (1H, s), 4.05-3.96 (1H, m), 3.77-3.65 (2H, m), 3.57 (1H, dd, J = 12Hz, 5Hz), 3.34 (3H, s), 3.33-3.16 (4H, m), 2.87 (1H, dd, J = 16Hz, 5Hz), 2.50-2.37 (2H, m), 2.02-1.83 (4H, m), 1.80-1.70 (1H, m).

5) (2S,8aS)-2-Methoxy-1,2,3,5,6,7,8,8a-octahydroindolizin-7-one

In 50 ml of tetrahydrofuran was dissolved 4.26 g (18.1 mmol) of (2S,8aS)-2-methoxy-7-(1-pyrrolidinyl)-1,2,3,5,8,8a-hexahydroindolizin-5-one obtained in 4), and to this solution was added under ice cooling and stirring 2.10 g (55 mmol) of lithium aluminum hydride. The mixture was stirred at room temperature for 3 hours, and to the reaction mixture was added carefully 8.40 ml of 1N aqueous sodium hydroxide solution. Ethanol was then added, and the insolubles were filtered off. The filtrate was concentrated under reduced pressure and the residue was subjected to alumina column chromatography (solvent; ethyl acetate) to obtain 1.60 g of the title compounds as a slightly brown oil (yield: 51%).
$^1$H-NMR Spectrum (500 MHz, $CDCl_3$) δ ppm:
3.96-3.90 (1H, m), 3.35-3.21 (2H, m), 3.32 (3H, s), 2.75-2.65 (1H, m), 2.55-2.17 (5H, m), 1.63-1.53 (2H, m), 1.32-1.20 (1H, m).

(Reference Example 11)

(8aS)-2-Methylidene-1,2,3,5,6,7,8,8a-octahydroindolizin-7-one

1) (S)-1-Benzyloxycarbonyl-2-cyanomethyl-4-methylidene pyrrolidine

Reduction, methanesulfonylation and cyanation were carried in a manner similar to Reference Examples 5-1) and 5-2) using (S)-1-benzyloxycarbony-4-methylidene proline methyl ester obtained in Reference Example 8-1) to give the title compound as a colorless oil (yield: 61%).
$^1$H-NMR Spectrum (400 MHz, $CDCl_3$) δ ppm:
7.42-7.30 (5H, m), 5.20-5.02 (4H, m), 4.36-4.27 (1H, m), 4.22-3.93 (2H, m), 2.92 (1H, dd, J = 16Hz, 9Hz), 2.80-2.38 (3H, m).

2) (S)-1-(t-Butoxycarbonyl)-4-methylidene homoproline ethyl ester

In 100 ml of ethanol was dissolved 7.00 g (27.3 mmol) of (S)-1-benzyloxycarbonyl-2-cyanomethyl-4-methylidene pyrrolidine obtained in 1), and this solution was stirred at room temperature for 1 hour while passing hydrogen chloride gas through the solution and further stirred at 80°C for 2 hours. The ethanol was distilled off and the residue was dissolved in 100 ml of water. The aqueous layer was washed with ethyl acetate and then 50 ml of dioxane was added to the aqueous layer. This solution was neutralized by adding triethylamine and a further 3.80 ml (27.3 mmol) of triethylamine was added thereto. Then 8.95 g (41.0 mmol) of di-t-butyl dicarbonate was added and the mixture was stirred at room temperature for 2 hours. The reaction mixture was extracted with ethyl acetate and the organic layer was washed with water, dried over anhydrous magnesium sulfate, and concentrated under reduced pressure. The residue was subjected to silica gel column chromatography (solvent; ethyl acetate:hexane = 1:3) to obtain 3.40 g of the title compound as a colorless oil (yield: 46%).
$^1$H-NMR Spectrum (400 MHz, $CDCl_3$) δ ppm:

5.01 (2H, br.s), 4.43-4.21 (1H, m), 4.12 (2H, dd, J = 14Hz, 7Hz), 4.08-3.90 (1H, m), 3.84 (1H, dd, J = 15Hz, 2Hz), 2.88-2.55 (2H, m), 2.41-2.29 (2H, m), 1.49 (9H, s), 1.30-1.18 (3H, m).

3) (S)-4-Methylidene homoproline trifluoroacetate

In 35 ml of ethanol was dissolved 3.40 g (12.6 mmol) of (S)-1-(t-butoxycarbonyl)-4-methylidene homoproline ethyl ester obtained in 2), and to this solution was added 18.9 ml (18.9 mmol) of 1N aqueous sodium hydroxide solution. The mixture was stirred at room temperature for 2 hours and the reaction mixture was concentrated under reduced pressure. To the residue were added ethyl acetate and water to separate the mixture. The organic layer was washed with water, dried over anhydrous magnesium sulfate, and concentrated under reduced pressure. The residue was dissolved in 30 ml of dichloromethane and to this solution was added 9.7 ml (126 mmol) of trifluoroacetic acid. The mixture was stirred at room temperature for 2 hours and concentrated under reduced pressure to obtain 3.08 g of the title compound as a white powder (yield: 96%).
$^1$H-NMR Spectrum (400 MHz, $CDCl_3$) δ ppm:
5.22-5.12 (2H, m), 4.03-3.88 (2H, m), 3.80-3.30 (3H, m), 2.91-2.79 (2H, m).

4) (8aS)-2-Methylidene-1,2,3,5,6,7,8,8a-octahydroindolizin-7-one

Reactions similar to those of Reference Examples 10-1), 10-2), 10-3), 10-4) and 10-5) were successively carried out using (S)-4-methylidene homoproline trifluoroacetate obtained in 3) instead of (2S,4S)-4-methoxyhomoproline hydrochloride, to give the title compound as a yellow oil (yield: 39%).
$^1$H-NMR Spectrum (400 MHz, $CDCl_3$) δ ppm:
4.97 (1H, br.s), 4.94 (1H, br.s), 3.72 (1H, d, J=13Hz), 3.34-3.25 (1H, m), 3.00-2.91 (1H, m), 2.70-2.20 (8H, m).

(Reference Example 12)

(2S,8aS)-2-Methylthio-1,2,3,5,6,7,8,8a-octahydroindolizin-7-one

1) (2S,4R)-1-Benzyloxycarbonyl-2-hydroxymethyl-4-(p-toluenesulfonyloxy)pyrrol dine

a) In 600 ml of dichloromethane was dissolved 77.70 g (245 mmol) of (2S,4R)-1-benzyloxycarbonyl-4-hydroxyproline methyl ester, and to this solution were successively added 37.5 ml (270 mmol) of triethylamine, 49.04 g (257 mmol) of p-toluenesulfonyl chloride and 2.99 g (24.5 mmol) of 4-dimethylaminopyridine. The mixture was stirred at room temperature overnight. The reaction mixture was concentrated under reduced pressure and to the residue were added ethyl acetate and water. The organic layer was washed with water, dried over anhydrous magnesium sulfate, and concentrated under reduced pressure. The residue was subjected to silica gel column chromatography (solvent; hexane:ethyl acetate = 1:1) to obtain 106.18 g of (2S,4R)-1-benzyloxycarbonyl-4-(p-toluenesulfonyloxy)proline methyl ester as a yellow oil.

b) Reduction of 106.18 g (245 mmol) of (2S,4R)-1-benzyloxycarbonyl-4-(p-toluenesulfonyloxy)proline methyl ester obtained in a) was carried out in a manner similar to Reference Example 5-1) to give 104.98 g of the title compound as a yellow oil (yield: quantitative).

2) (2S,4S)-1-Benzyloxycarbonyl-2-(t-butyldimethylsilyloxy)methyl-4-acetylthiopyrro lidine

a) In 610 ml of dichloromethane was dissolved 104.98 g (245 mmol) of (2S,4R)-1-benzyloxycarbonyl-2-hydroxymethyl-4-(p-toluenesulfonyloxy)pyrrolidine obtained in 1), and to this solution were added successively 37.4 ml (269 mmol) of triethylamine, 38.76 g (257 mmol) of t-butyldimethylsilyl chloride and 2.99 g (24.5 mmol) of 4-dimethylaminopyridine. The mixture was stirred at room temperature overnight. The reaction mixture was concentrated under reduced pressure and to the residue were added ethyl acetate and water. The organic layer was washed with water, dried over anhydrous magnesium sulfate, and concentrated under reduced pressure. The residue was subjected to silica gel column chromatography (solvent; hexane:ethyl acetate = 1:1) to obtain 119.34 g of (2S,4R)-1-benzyloxycarbonyl-2-(t-butyldimethylsilyloxy)methyl-4-(p-toluenesulfonyl oxy)pyrrolidine as a yellow oil (yield: 94%).

b) In 245 ml of dimethylformamide was dissolved 127.17 g (245 mmol) of (2S,4R)-1-benzyloxycarbonyl-2-(t-butyldimethylsilyloxy)methyl-4-(p-toluenesulfonyl oxy)pyrrolidine obtained in a), and to this solution was added 29.34 g (257 mmol) of potassium thioacetate. The mixture was stirred at 60°C for 1.5 hours. After cooling the mixture to room temperature, water was added thereto, and the mixture was extracted with ethyl acetate. The organic layer was washed with water, dried over anhydrous magnesium sulfate, and concentrated under reduced pressure. The residue was subjected to silica gel column chromatography (solvent; hexane:ethyl acetate = 8:1) to obtain 91.67 g of the title compound as an orange-color oil (yield: 88%).

3) (2S,4S)-1-Benzyloxycarbonyl-2-(t-butyldimethylsilyloxy)methyl-4-methylthio pyrrolidine

In 166 ml of methanol was dissolved 35.26 g (83.2 mmol) of (2S,4S)-1-benzyloxycarbonyl-2-(t-butyldimethylsilyloxy)methyl-4-acetylthiopyrrolid ine obtained in 2), and to this solution was added 6.22 ml (99.9 mmol) of methyl iodide. Then, 149 ml (41.6 mmol) of 28% sodium methoxide/methanol solution was added to the mixture at 0°C and the mixture was stirred at the same temperature for 1 hour. The reaction mixture was concentrated under reduced pressure and to the residue were added ethyl acetate and water. The organic layer was washed with water, dried over anhydrous magnesium sulfate and concentrated under reduced pressure. The residue was subjected to silica gel column chromatography (solvent; hexane:ethyl acetate = 9:1) to obtain 30.74 g of the title compound as a yellow oil (yield: 93%).

4) (2S,4S)-1-Benzyloxycarbonyl-2-hydroxymethyl-4-methylthiopyrrolidine

In 155 ml of tetrahydrofuran was dissolved 30.74 g (77.7 mmol) of (2S,4S)-1-benzyloxycarbonyl-2-(t-butyldimethylsilyloxy)methyl-4-methylthiopyrroli dine obtained in 3), and to this solution was added 85.5 ml (85.5 mmol) of 1M tetrabutylammonium fluoride/tetrahydrofuran solution. The mixture was stirred at room temperature overnight. The reaction mixture was concentrated under reduced pressure, and to the residue were added ethyl acetate and water. The organic layer was washed with water, dried over anhydrous magnesium sulfate, and concentrated under reduced pressure. The residue was subjected to silica gel column chromatography (solvent; hexane:ethyl acetate = 1:1) to obtain 21.87 g of the title compound as a pale yellow oil (yield: quantitative).

5) (2S,4S)-1-Benzyloxycarbonyl-2-cyanomethyl-4-methylthio pyrrolidine

Methanesulfonylation and cyanation were carried in a manner similar to Reference Example 1-1) using (2S, 4S)-1-benzyloxycarbonyl-2-hydroxymethyl-4-methylthiopyrrolidine obtained in 4) to give the title compound as a yellow oil (yield: 52%).

6) (2S,4S)-4-Methylthio homoproline hydrochloride

To 7.43 g (25.6 mmol) of (2S,4S)-1-benzyloxycarbonyl-2-cyanomethyl-4-methylthiopyrrolidine obtained in 5) was added 37 ml of 35% hydrochloric acid, and the mixture was stirred at 80°C overnight. The reaction mixture was concentrated under reduced pressure. After the reside was washed with ethyl acetate, ethanol was added to the residue and the insolubles were filtered off. The filtrate was concentrated under reduced pressure to give 5.39 g of the title compound as a brown oil (yield: quantitative).

7) (2S,8aS)-2-Methylthio-1,2,3,5,6,7,8,8a-octahydroindolizin-7-one

Reactions similar to those of Reference Examples 10-1), 10-2), 10-3), 10-4) and 10-5) were successively carried out using (2S,4S)-4-methylthiohomoproline hydrochloride obtained in 6) instead of (2S,4S)-4-methoxyhomoproline hydrochloride to give the title compound as a yellow oil (yield: 37%).
$^1$H-NMR Spectrum (400 MHz, $CDCl_3$) δ ppm:
3.32-3.23 (2H, m), 3.17 (1H, dd, J = 10Hz, 2Hz), 2.72-2.59 (2H, m), 2.51-2.40 (2H, m), 2.37-2.31 (4H, m), 2.15 (3H, s), 1.52-1.45 (1H, m).

(Reference Example 13)

(2S,8aS)-2-Ethylthio-1,2,3,5,6,7,8,8a-octahydroindolizin-7-one

Reactions similar to those of Reference Examples 12-3), 12-4), 12-5), 12-6) and 12-7) were successively carried out using ethyl iodide instead of methyl iodide to give the title compound as a yellow oil (yield: 8%).

$^1$H-NMR Spectrum (400 MHz, $CDCl_3$) δ ppm:
3.36-3.28 (2H, m), 3.16 (1H, dd, J = 10Hz, 2Hz), 2.74-2.57 (4H, m), 2.52-2.41 (2H, m), 2.38-2.27 (4H, m), 1.53-1.46 (1H, m), 1.28 (3H, t, J = 7Hz).

(Reference Example 14)

(2S,8aS)-2-Butylthio-1,2,3,5,6,7,8,8a-octahydroindolizin-7-one

Reactions similar to those of Reference Examples 12-3), 12-4), 12-5), 12-6) and 12-7) were successively carried out using butyl bromide instead of methyl iodide to give the title compound as a brown oil (yield: 13%).

(Reference Example 15)

(2S,8aS)-2-Methylsulfonyl-1,2,3,5,6,7,8,8a-octahydroindolizin-7-one

In 15 ml of methanol was dissolved 1.14 g (6.15 mmol) of (2S,8aS)-2-methylthio-1,2,3,5,6,7,8,8a-octahydroindolizin-7-one obtained in Reference Example 12, and to this solution was added 3 ml of an aqueous solution of 4.6 ml (9.23 mmol) of 2N sulfuric acid with 101 mg (0.31 mmol) of sodium tungstate dihydrate. To the mixture was added dropwise at 55°C 1.35 ml (12.3 mmol) of 30% hydrogen peroxide solution. The mixture was stirred at the same temperature for 1 hour, 30 ml of saturated aqueous sodium hydrogencarbonate solution was added thereto, and the mixture was extracted with dichloromethane. The organic layer was washed with water, dried over anhydrous magnesium sulfate, and concentrated under reduced pressure. The residue was subjected to alumina column chromatography (solvent; ethyl acetate) to give 945 mg of the title compound as a yellow oil (yield: 71%).
$^1$H-NMR Spectrum (400 MHz, $CDCl_3$) δ ppm:
3.62-3.55 (2H, m), 3.63-3.32 (1H, m), 2.93 (3H, s), 2.69-2.56 (3H, m), 2.52-2.43 (2H, m), 2.41-2.33 (3H, m), 2.02-1.94 (1H, m).

(Reference Example 16)

6-Spirocyclopropan-[(8aS)-1,2,3,5,6,7,8,8a-octahydroindolizin]-7-one

1) 1-Ethoxycarbonyl-1-[(S)-2-ethoxycarbonylmethylpyrrolidin-1-yl]methylcyclopro pane

In 200 ml of ethanol was dissolved 12.74 g (43.7 mmol) of (S)-1-benzyloxycarbonylhomoproline ethyl ester, and to this solution was added 4.25 g of 10% palladium on carbon. The mixture was stirred at room temperature under a hydrogen atmosphere for 2 hours. The reaction mixture was filtered, the filtrate was concentrated under reduced pressure, and the residue was dissolved in 150 ml of ethanol. To this solution was added under ice cooling and stirring 6.84 g (48.1 mmol) of 1-ethoxycarbonyl-1-formylcyclopropane, and, after the mixture was stirred at 0°C for 1 hour, 1.92 g (30.6 mmol) of sodium cyanotrihydroborate was added thereto. The mixture was stirred at room temperature for 2 days, and 300 ml of water was added thereto, followed by extraction with ethyl acetate. The organic layer was washed with water, dried over anhydrous magnesium sulfate, and concentrated under reduced pressure. The residue was subjected to silica gel column chromatography (solvent; hexane:ethyl acetate = 7:3) to give 3.07 g of the title compound as a pale brown oil (yield: 25%).
$^1$H-NMR Spectrum (400 MHz, $CDCl_3$) δ ppm:
4.21-4.07 (4H, m), 3.24 (1H, d, J=13Hz), 3.22-3.12 (1H, m), 2.82-2.68 (2H, m), 2.28-2.13 (3H, m), 2.03-1.90 (1H, m), 1.82-1.64 (2H, m), 1.56-1.45 (1H, m), 1.36-1.21 (7H, m), 1.12-1.03 (1H, m), 0.84-0.70 (2H, m).

2) 6-Spirocyclopropane-[(8aS)-8-ethoxycarbonyl-1,2,3,5,6,7,8,8a-octahydroindo lizin]-7-one

In 20 ml of toluene was suspended 518 mg (11.9 mmol) of 55% sodium hydride, and to this suspension was added 3 drops of methanol through a Pasteur pipette. The mixture was heated to 130°C. To the reaction mixture was added 3.06 g (10.8 mmol) of 1-ethoxycarbonyl-1-[(S)-2-ethoxycarbonylmethylpyrrolidin-1-yl]methylcyclopropane obtained in 1), and the mixture was heated at reflux for 10 minutes. After cooling the mixture to 0°C, a saturated brine solution was added thereto, and the mixture was extracted with ethyl acetate. The organic layer was washed with water, dried over anhydrous magnesium sulfate, and concentrated under reduced pressure. The residue was subjected to silica gel column chromatography (solvent; ethyl acetate:methanol = 9:1) to give 2.02 g of the title compound as a colorless oil (yield: 79%).
$^1$H-NMR Spectrum (400 MHz, $CDCl_3$) δ ppm:

4.31-4.15 (2H, m), 3.32 (1H, d, J = 11Hz), 3.15 (1H, td, J = 9Hz, 3Hz), 2.92-2.82 (2H, m), 2.67 (1H, d, J = 11Hz), 2.29 (1H, q, J = 9Hz), 2.12-1.80 (3H, m), 1.71-1.55 (2H, m), 1.29 (3H, t, J = 7Hz), 1.09-1.02 (1H, m), 0.99-0.92 (1H, m), 0.69-0.62 (1H, m).

3) 6-Spirocyclopropane-[(8aS)-1,2,3,5,6,7',8,8a-octahydroindolizin]-7-one

In 1 ml of ethanol was dissolved 50 mg (0.21 mmol) of 6-spirocyclopropane-[(8aS)-8-ethoxycarbonyl-1,2,3,5,6,7,8,8a-octahydroindolizin]-7-one obtained in 2), and to this solution was added 1 ml of 1N aqueous sodium hydroxide solution, followed by heating at reflux for 1 hour. The reaction mixture was concentrated under reduced pressure, and the residue was subjected to alumina column chromatography (solvent; ethyl acetate) to give 10 mg of the title compound as a colorless oil (yield: 29%).
$^1$H-NMR Spectrum (400 MHz, $CDCl_3$) δ ppm:
3.17 (1H, td, J = 9Hz, 3Hz), 2.76 (1H, d, J = 12Hz), 2.69 (1H, d, J = 12Hz), 2.67 (1H, dd, J = 16Hz, 3Hz), 2.56-2.44 (1H, m), 2.34 (1H, dd, J = 16Hz, 12Hz), 2.30-2.18 (1H, m), 2.12-1.93 (2H, m), 1.90-1.78 (1H, m), 1.64-1.50 (2H, m), 1.08-1.00 (1H, m), 0.96-0.88 (1H, m), 0.67-0.58 (1H, m).

(Reference Example 17)

(8aS)-2,2-Dimethyl-1,2,3,5,6,7,8,8a-octahydroindolizin-7-one

1) (S)-1-Benzyloxycarbonyl-4,4-dimethyl-2-hydroxymethylpyrrolidine

In 500 ml of tetrahydrofuran was suspended 25.72 g (678 mmol) of lithium aluminum hydride, and to this suspension was added dropwise under ice cooling and stirring a solution of 32.35 g (226 mmol) of (S)-4,4-dimethyl-2-hydroxymethyl-5-oxopyrrolidine in 300 ml of tetrahydrofuran at 8°C to 17°C over 20 minutes. The mixture was heated at reflux for 7 hours and then cooled to 0°C. 103 ml of 4% aqueous sodium hydroxide solution was added carefully thereto, and the insolubles were filtered off. The filtrate was concentrated under reduced pressure, the residue was dissolved in 500 ml of dichloromethane, and to this solution was added 40.94 ml (294 mmol) of triethylamine. Then, to the mixture was added under ice cooling and stirring 38.71 ml (271 mmol) of benzyloxycarbonyl chloride, and the mixture was stirred at the same temperature for 1 hour and at room temperature for a further 1 hour. To the reaction mixture was added saturated aqueous sodium hydrogencarbonate solution and the mixture was extracted with dichloromethane. The organic layer was washed with water, dried over anhydrous magnesium sulfate, and concentrated under reduced pressure. The residue was subjected to silica gel column chromatography (solvent; hexane:ethyl acetate = 1:1) to give 43.97 g of the title compound as a colorless oil (yield: 74%).
$^1$H-NMR Spectrum (400 MHz, $CDCl_3$) δ ppm:
7.42-7.29 (5H, m), 5.20-5.10 (2H, m), 4.89 (1H, dd, J = 9Hz, 3Hz), 4.14-4.05 (1H, m), 3.73-3.59 (2H, m), 3.41 (1H, d, J = 11Hz), 3.06 (1H, d, J = 11Hz), 1.80 (1H, ddd, J = 13Hz, 7Hz, 1Hz), 1.33 (1H, dd, J = 12Hz, 10Hz), 1.08 (3H, s), 1.02 (3H, s).

2) (8aS)-2,2-Dimethyl-1,2,3,5,6,7,8,8a-octahydroindolizin-7-one

Reactions similar to those of Reference Examples 12-5), 12-6) and 12-7) were successively carried out using (S)-1-benzyloxycarbonyl-4,4-dimethyl-2-hydroxymethylpyrrolidine obtained in 1) instead of (2S,4S)-1-benzyloxycarbonyl-2-hydroxymethyl-4-methythiopyrrolidine to give the title compound as a brown oil (yield: 36%).
$^1$H-NMR Spectrum (400 MHz, $CDCl_3$) δ ppm:
3.26-3.19 (1H, m), 2.89 (1H, d, J = 9Hz), 2.68-2.57 (1H, m), 2.49-2.25 (5H, m), 2.07 (1H, d, J = 9Hz), 1.73 (1H, dd, J = 12Hz, 6Hz), 1.40 (1H, dd, 12Hz, 10Hz), 1.20 (3H, s), 1.07 (3H, s).

(Reference Example 18)

(±)-2-Spirocyclopentane-1,2,3,5,6,7,8,8a-octahydroindolizin-7-one

1) 1-Cyano-1-formylmethylcyclopentane diethylacetal

In 500 ml of tetrahydrofuran was dissolved 22.00 ml (157 mmol) of diisopropylamine and to this solution was added dropwise under ice cooling 100 ml (157 mmol) of 1.57M n-butyl lithium/hexane solution. The mixture was stirred at the same temperature for 30 minutes. The reaction vessel was transferred to a dry ice-acetone bath and 14.89 ml (143 mmol) of cyclopentanecarbonitrile was added dropwise thereto. After the mixture was stirred at -78°C for 15

minutes, a solution of 27.31 ml (157 mmol) of hexamethylphosphonic amide in 50 ml of tetrahydrofuran was added dropwise thereto, and after stirring at the same temperature for 30 minutes, 23.62 ml (157 mmol) of bromoacetaldehyde diethylacetal was added dropwise thereto. The mixture was stirred at -78°C for 2 hours and at room temperature for a further 20 hours. Ice water was added to the reaction mixture and the mixture was extracted with ethyl acetate. The organic layer was washed with water, dried over anhydrous magnesium sulfate and concentrated under reduced pressure. The residue was subjected to silica gel column chromatography (solvent; hexane:ethyl acetate = 19:1) to give 23.92 g of the title compound as a colorless oil (yield: 79%).
$^1$H-NMR Spectrum (400 MHz, $CDCl_3$) δ ppm:
4.75 (1H, t, J = 5Hz), 3.76-3.66 (2H, m), 3.61-3.52 (2H, m), 2.22-2.13 (2H, m), 1.93 (2H, d, J = 5Hz), 1.91-1.63 (6H, m), 1.23 (6H, t, J = 7Hz).

2) 1-Aminomethyl-1-formylmethylcyclopentane diethylacetal

In 460 ml of tetrahydrofuran was suspended 12.33 g (325 mmol) of lithium aluminum hydride and to this suspension was added dropwise under ice cooling and stirring 8.66 ml (162 mmol) of concentrated sulfuric acid over 10 minutes. The mixture was stirred at 0°C for 1 hour and 22.88 g (108 mmol) of 1-cyano-1-formylmethylcyclopentane diethylacetal obtained in 1) was added in portions thereto, and the mixture was stirred at room temperature for 2 hours. The reaction mixture was again cooled to 0°C, and 49.3 ml of 4% aqueous sodium hydroxide solution was carefully added thereto and the insolubles were filtered off. The filtrate was concentrated under reduced pressure to give 18.69 g of the title compound as a pale yellow oil (yield: 80%).
$^1$H-NMR Spectrum (400 MHz, $CDCl_3$) δ ppm:
4.51 (1H, t, J = 5Hz), 3.70-3.60 (2H, m), 3.54-3.41 (2H, m), 2.50 (2H, s), 1.70 (2H, d, J = 5Hz), 1.69-1.34 (10H, m), 1.21 (6H, t, J = 7Hz).

3) (±)-2-Spirocyclopentane-1,2,3,5,6,7,8,8a-octahydroindolizin-7-one

In 400 ml of diethyl ether was dissolved 18.68 (86.7 mmol) of 1-aminomethyl-1-formylmethylcyclopentane diethyl acetal obtained in 2), and to this solution was added under ice cooling and stirring 8.67 ml (104 mmol) of methyl vinyl ketone. The mixture was stirred at room temperature for 24 hours and extracted with 200 ml of 3N hydrochloric acid. The aqueous layer was stirred at 100°C for 3 hours, cooled to room temperature, made alkaline with sodium hydrogencarbonate, and extracted with ethyl acetate. The organic layer was washed with water, dried over anhydrous magnesium sulfate, and concentrated under reduced pressure. The residue was subjected to alumina column chromatography (solvent; hexane:ethyl acetate= 9:1) to give 5.75 g of the title compound as a colorless oil (yield: 34%).
$^1$H-NMR Spectrum (400 MHz, $CDCl_3$) δ ppm:
3.27-3.21 (1H, m), 3.03 (1H, d, J = 9Hz), 2.69-2.59 (1H, m), 2.47 (1H, dt, J = 13Hz, 2Hz), 2.42-2.26 (4H, m), 2.19 (1H, d, J = 9Hz), 1.86 (1H, dd, J = 12Hz, 5Hz), 1.80-1.46 (9H, m).

(Reference Example 19)

(8aS)-2-Methyl-3,5,6,7,8,8a-hexahydroindolizin-7-one

1) (S)-2-Carboxymethyl-4-methyl-3-pyrroline hydrochloride

To 24.00 g (93.6 mmol) of (S)-1-benzyloxycarbonyl-2-cyanomethyl-4-methylidenepyrrolidine obtained in Reference Example 11-1 was added 150 ml of concentrated hydrochloric acid and the mixture was stirred at 80°C overnight and concentrated under reduced pressure. Ethyl acetate was added to the residue to separate the mixture and the aqueous layer was concentrated under reduced pressure to give 16.60 g of the title compound as a white powder (yield: quantitative).
$^1$H-NMR Spectrum (400 MHz, DMSO-$d_6$) δ ppm:
5.47-5.40 (1H, m), 4.60-4.47 (1H, m), 4.15-3.75 (2H, m), 3.60-3.35 (2H, m), 1.74 (3H, m).

2) (8aS)-2-Methyl-3,5,6,7,8,8a-hexahydroindolizin-7-one

Reactions similar to those of Reference Examples 10-1), 10-2), 10-3), 10-4) and 10-5) were successively carried out using (S)-2-carboxymethyl-4-methyl-3-pyrroline hydrochloride obtained in 1) instead of (2S,4S)-4-methoxyhomoproline hydrochloride to give the title compound as an orange-color oil (yield: 10%).
$^1$H-NMR Spectrum (400 MHz, $CDCl_3$) δ ppm:
5.44-5.38 (1H, m), 3.75-3.67 (1H, m), 3.58 (1H, dd, J = 13Hz, 3Hz), 3.48-3.39 (1H, m), 3.30-3.21 (1H, m), 3.01-2.92

(1H, m), 2.63-2.49 (2H, m), 2.45-2.35 (2H, m), 1.79 (3H, s).

(Reference Example 20)

(2R,8aS)-2-Ethoxy-1,2,3,5,6,7,8,8a-octahydroindolizin-7-one

Reactions similar to those of Reference Examples 1-1), 1-2) and 1-3) were successively carried out using (2S,4R)-1-benzyloxycarbonyl-4-ethoxyproline instead of (2S,4R)-1-benzyloxycarbonyl-4-methoxyproline to give the title compound as an orange-color oil (yield: 5%).
$^1$H-NMR Spectrum (400 MHz, $CDCl_3$) δ ppm:
4.21-4.13 (1H, m), 3.60-3.38 (3H, m), 3.28 (1H, dd, J = 11Hz, 7Hz), 2.66-2.20 (7H, m), 1.99 (1H, dd, J = 13Hz, 6Hz), 1.82-1.70 (1H, m), 1.20 (3H, t, J = 7Hz).

(Reference Example 21)

(8aS)-2,2-Propylenedioxy-1,2,3,5,6,7,8,8a-octahydroindolizin-7-one

Reactions similar to those of Reference Examples 7-1), 7-2) and 7-3) were successively carried out using (S)-1-benzyloxycarbonyl-4,4-propylenedioxyproline methyl ester instead of (S)-1-benzyloxycarbonyl-4,4-ethylenedioxyproline methyl ester to give the title compound as a pale yellow powder (yield: 16%).
$^1$H-NMR Spectrum (400 MHz, $CDCl_3$) δ ppm:
4.00-3.82 (4H, m), 3.51 (1H, d, J = 10Hz), 3.33-3.25 (1H, m), 2.74-2.62 (1H, m), 2.57-2.48 (2H, m), 2.43-2.31 (5H, m), 1.86-1.62 (2H, m), 1.82 (1H, dd, J = 13Hz, 10Hz).

(Reference Example 22)

(8aS)-2,2-(2',2'-Dimethylpropylenedioxy)-1,2,3,5,6,7,8,8a-octahydroindolizin-7-one

Reactions similar to those of Reference Examples 7-1), 7-2) and 7-3) were successively carried out using (S)-1-benzyloxycarbonyl-4,4-(2',2'-dimethylpropylenedioxy)proline methyl ester instead of (S)-1-benzyloxycarbonyl-4,4-ethylenedioxyproline methyl ester to give the title compound as a pale yellow powder (yield: 19%).
$^1$H-NMR Spectrum (400 MHz, $CDCl_3$) δ ppm:
3.57-3.41 (5H, m), 3.31-3.24 (1H, m), 2.73-2.62 (1H, m), 2.57-2.46 (2H, m), 2.42-2.31 (5H, m), 1.82 (1H, dd, J = 13Hz, 10Hz), 1.00 (3H, s), 0.96 (3H, s).

(Reference Example 23)

(2S,8aS)-2-Phenoxy-1,2,3,5,6,7,8,8a-octahydroindolizin-7-one

1) (2S,4S)-1-Benzyloxycarbonyl-4-phenoxyhomoproline benzyl ester

In 300 ml of tetrahydrofuran was dissolved 18.29 g (49.5 mmol) of (2S,4R)-1-benzyloxycarbonyl-4-hydroxyhomoproline benzyl ester and to this solution were added 6.53 ml (74.3 mmol) of phenol and 19.48 g (74.3 mmol) of triphenylphosphine, and the mixture was cooled to 0°C. At the same temperature under stirring 11.69 ml (74.3 mmol) of diethyl azodicarboxylate (DEAD) was added dropwise thereto and the mixture was stirred at room temperature overnight. The reaction mixture was concentrated under reduced pressure, and diethyl ether was added to the residue and the insolubles were filtered off. The filtrate was washed successively with saturated aqueous sodium hydrogencarbonate solution and water, dried over anhydrous magnesium sulfate, and concentrated under reduced pressure. The residue was subjected to silica gel column chromatography (solvent; hexane:ethyl acetate = 3:1) to give 11.14 g of the title compound as a colorless oil (yield: 51%).
$^1$H-NMR Spectrum (400 MHz, $CDCl_3$) δ ppm:
7.40-7.21 (12H, m), 6.97 (1H, t, J = 7Hz), 6.84 (2H, d, J = 8Hz), 5.21-4.99 (4H, m), 4.93-4.83 (1H, m), 4.48-4.38 (1H, m), 3.84-3.68 (2H, m), 3.24-3.15 (0.6H, m), 3.04-2.95 (0.4H, m), 2.92-2.76 (1H, m), 2.39-2.30 (1H, m), 2.26-2.17 (1H, m).

2) (2S,4S)-4-Phenoxyhomoproline hydrochloride

In 220 ml of tetrahydrofuran was dissolved 11.09 g (24.9 mmol) of (2S,4s)-1-benzyloxycarbonyl-4-phenoxyhomoproline benzyl ester obtained in 1), and to this solution was added 2.22 g of 20% palladium hydroxide on carbon. The mixture was stirred at room temperature under a hydrogen atmosphere for 6 hours. To the reaction mixture was added 6.85 ml (27.4 mmol) of 4N hydrochloric acid/dioxane solution, the mixture was filtered, and the filtrate was concentrated under reduced pressure to give 6.39 g of the title compound as a pale brown powder (yield: quantitative).
$^1$H-NMR Spectrum (400 MHz, DMSO-$d_6$) δ ppm:
12.70 (1H, br.s), 7.33 (2H, dd, J = 8Hz, 7Hz), 6.99 (1H, t, J = 7Hz), 6.96 (2H, d, J = 8Hz), 5.16-5.09 (1H, m), 3.98-3.87 (1H, m), 3.50 (1H, dd, J = 13Hz, 5Hz), 3.42-3.28 (2H, m), 2.89 (1H, dd, J = 18Hz, 8Hz), 2.80 (1H, dd, J = 18Hz, 6Hz), 2.64-2.54 (1H, m), 1.91-1.81 (1H, m).

3) (2S,8aS)-2-Phenoxy-1,2,3,5,6,7,8,8a-octahydroindolizin-7-one

Reactions similar to those of Reference Examples 10-1), 10-2), 10-3), 10-4) and 10-5) were successively carried out using (2S,4S)-4-phenoxyhomoproline hydrochloride obtained in 2) instead of (2S,4S)-4-methoxyhomoproline hydrochloride to give the title compound as a pale brown powder (yield: 25%).
$^1$H-NMR Spectrum (400 MHz, $CDCl_3$) δ ppm:
7.28 (2H, dd, J = 8Hz, 7Hz), 6.95 (1H, t, J = 7Hz), 6.87 (2H, d, J = 8Hz), 4.90-4.82 (1H, m), 3.40 (1H, d, J = 11Hz), 3.39-3.32 (1H, m), 2.80-2.68 (1H, m), 2.67-2.27 (7H, m), 1.86-1.74 (1H, m).

(Reference Example 24)

(8aS)-2-Ethyhdene-1,2,3,5,6,7,8,8a-octahydroindolizin-7-one

A reaction similar to that of Reference Example 8-1) using ethyltriphenylphosphonium bromide instead of methyltriphenylphosphonium bromide, and reactions similar to those of Reference Examples 11-1), 11-2), 11-3) and 11-4) using the resulting (S)-1-benzyloxycarbonyl-4-ethylideneproline methyl ester were successively carried out to give the title compound as a brown oil (yield: 9%).
$^1$H-NMR Spectrum (400 MHz, $CDCl_3$) δ ppm:
5.45-5.30 (1H, m), 3.78 (0.5H, d, J = 3Hz), 3.64 (0.5H, d, J = 3Hz), 3.37-3.28 (1H, m), 2.94-2.85 (1H, m), 2.69-2.09 (8H, m), 1.68-1.61 (3H, m).

(Reference Example 25)

(8aS)-2-Propylidene-1,2,3,5,6,7,8,8a-octahydroindolizin-7-one

A reaction similar to that of Reference Example 8-1) using propyltriphenylphosphonium bromide instead of methyltriphenylphosphonium bromide, and reactions similar to those of Reference Examples 11-1), 11-2), 11-3) and 11-4) using the resulting (S)-1-benzyloxycarbonyl-4-propylideneproline methyl ester were successively carried out to give the title compound as a brown oil (yield: 10%).
$^1$H-NMR Spectrum (400 MHz, $CDCl_3$) δ ppm:
5.37-5.25 (1H, m), 3.78 (0.5H, d, J = 3Hz), 3.62 (0.5H, d, J = 3Hz), 3.36-3.28 (1H, m), 2.94-2.85 (1H, m), 2.69-2.10 (8H, m), 2.04-1.91 (2H, m), 1.01-0.92 (3H, m).

(Reference Example 26)

(8aS)-2-Benzylidene-1,2,3,5,6,7,8,8a-octahydroindolizin-7-one

A reaction similar to that of Reference Example 8-1) using benzyltriphenylphosphonium bromide instead of methyltriphenylphosphonium bromide, and reactions similar to those of Reference Examples 11-1), 11-2), 11-3) and 11-4) using the resulting (S)-1-benzyloxycarbonyl-4-benzylideneproline methyl ester were successively carried out to give the title compound as a brown oil (yield: 0.4%).
$^1$H-NMR Spectrum (400 MHz, $CDCl_3$) δ ppm:
7.41-7.26 (4H, m), 7.21-7.15 (1H, m), 6.39-6.32 (1H, m), 4.70-4.02 (1H, m), 3.89-3.72 (1H, m), 3.40-3.35 (1H, m), 3.26-3.10 (1H, m), 2.98-2.78 (1H, m), 2.75-2.38 (5H, m), 2.11-1.84 (1H, m).

(Reference Example 27)

(2S,8aS)-2-Ethyl-1,2,3,5,6,7,8,8a-octahydroindolizin-7-one

Reactions similar to those of Reference Examples 8-2), 8-3), 8-4), 8-5) and 8-6) were successively carried out using (S)-1-benzyloxycarbonyl-4-ethylideneproline methyl ester instead of (S)-1-benzyloxycarbonyl-4-methylideneproline methyl ester to give the title compound as a brown oil (yield: 10%).
$^1$H-NMR Spectrum (400 MHz, $CDCl_3$) δ ppm:
3.28-3.23 (1H, m), 2.87 (1H, d, J = 9Hz), 2.67-2.58 (1H, m), 2.56-2.40 (2H, m), 2.32-2.26 (4H, m), 2.13-2.05 (2H, m), 1.54-1.45 (2H, m), 1.18 (1H, d, J = 6Hz), 0.90 (3H, t, J = 7Hz).

(Reference Example 28)

(2S,8aS)-2-Propyl-1,2,3,5,6,7,8,8a-octahydroindolizin-7-one

Reactions similar to those of Reference Examples 8-2), 8-3), 8-4) 8-5) and 8-6) were successively carried out using (S)-1-benzyloxycarbonyl-4-propylideneproline methyl ester instead of (S)-1-benzyloxycarbonyl-4-methylideneproline methyl ester to give the title compound as a brown oil (yield: 24%).
$^1$H-NMR Spectrum (400 MHz, $CDCl_3$) δ ppm:
3.29-3.21 (1H, m), 2.87-2.73 (1H, m), 2.67-2.57 (1H, m), 2.53-2.41 (1H, m), 2.40-2.24 (3H, m), 2.22-2.09 (2H, m), 1.99-1.85 (1H, m), 1.83-1.56 (1H, m), 1.53-1.39 (2H, m), 1.37-1.22 (2H, m), 1.21-1.14 (1H, m), 0.93-0.86 (3H, m).

(Reference Example 29)

(2S,8aS)-2-Benzyl-1,2,3,5,6,7,8,8a-octahydroindolizin-7-one

Reactions similar to those of Reference Examples 8-2), 8-3), 8-4) 8-5) and 8-6) were successively carried out using (S)-1-benzyloxycarbonyl-4-benzylideneproline methyl ester instead of (S)-1-benzyloxycarbonyl-4-methylideneproline methyl ester to give the title compound as a brown oil (yield: 4%).
$^1$H-NMR Spectrum (400 MHz, $CDCl_3$) δ ppm:
7.38-7.28 (2H, m), 7.27-7.11 (3H, m), 3.36-3.23 (1H, m), 3.04-2.88 (1H, m), 2.86-2.21 (8H, m), 2.11-2.05 (1H, m), 2.02-1.72 (2H, m), 1.34-1.25 (2H, m).

(Reference Example 30)

(±)-1,2,3,5,6,7,8,8a-Octahydroindolizin-7-one

1) (±)-1-(t-Butoxycarbonyl)-N-methoxy-N-methylhomoprolinamide

In 100 ml of dichloromethane was dissolved 5.03 g (21.94 mmol) of (±)-1-(t-butoxycarbonyl)homoproline, and to this solution were added 2.57 g (26.33 mmol) of N-methoxy-N-methylhydroxyamine hydrochloride, 5.05 g (26.33 mmol) of 1-ethyl-3-(3'-dimethylaminopropyl)carbodiimide, and 7.65 ml (54.85 mmol) of triethylamine. The mixture was stirred at room temperature for 1 hour. Saturated aqueous ammonium chloride solution was added to the reaction mixture to separate the mixture. The organic layer was washed with water, dried over anhydrous sodium sulfate, and concentrated under reduced pressure. The residue was subjected to silica gel column chromatography (solvent; hexane:ethyl acetate = 1:2) to give 4.00 g of the title compound as a colorless oil (yield: 67%).
$^1$H-NMR Spectrum (400 MHz, $CDCl_3$) δ ppm:
4.28-4.12 (1H, br.s), 3.69 (3H, s), 3.45-3.25 (2H, br.s), 3.23-3.10 (3H, br.s), 3.09-2.83 (1H, m), 2.51-2.35 (1H, m), 2.14-1.98 (1H, m), 1.92-1.72 (3H, m), 1.46 (9H, s).

2) (±)-1-(t-Butoxycarbonyl)-2-(2-oxo-3-butenyl)pyrrolidine

In 40 ml of tetrahydrofuran was dissolved 4.00 g (14.69 mmol) of (±)-1-(t-butoxycarbonyl)-N-methoxy-N-methylhomoproline amide obtained in 1), and to this solution was added at -78°C under stirring 23.2 ml (22.04 mmol) of 0.95M vinyl magnesium bromide/tetrahydrofuran solution. The reaction mixture was stirred while being allowed to warm to room temperature. Saturated aqueous ammonium chloride solution was added to the reaction mixture, the mixture was extracted with ethyl acetate, and the organic layer was washed with water, dried over anhydrous sodium sulfate, and concentrated under reduced pressure. The resulting residue was subjected to silica gel column chromatography

(solvent; hexane:ethyl acetate = 3:1) to give 1.48 g of the title compound as a colorless oil (yield: 42%).
$^1$H-NMR Spectrum (500 MHz, $CDCl_3$) δ ppm:
6.42-6.20 (2H, m), 5.88 (1H, dd, J = 9Hz, 2Hz), 4.24-4.14 (1H, m), 3.47-3.23 (2.5H, m), 3.15-3.04 (0.5H, m), 2.60-2.44 (1H, m), 2.11-1.97 (1H, m), 1.90-1.77 (2H, m), 1.74-1.64 (1H, m), 1.46 (9H, s).

3) (±)-1,2,3,5,6,7,8,8a-Octahydroindolizin-7-one

In 2 ml of tetrahydrofuran was dissolved 140 mg (0.59 mmol) of (±)-1-(t-butoxycarbonyl)-2-(2-oxo-3-butenyl) pyrrolidine obtained in 2), and to this solution was added 1.76 ml (1.76 mmol) of 1N aqueous hydrochloric acid solution. The mixture was stirred at 70°C for 4 hours. Saturated aqueous sodium hydrogencarbonate solution was added to this reaction mixture and the mixture was extracted with dichloromethane. The organic layer was washed with water, dried over anhydrous magnesium sulfate and concentrated under reduced pressure to give 76 mg of the title compound as a slightly brown oil (yield: 93%).
$^1$H-NMR Spectrum (500 MHz, $CDCl_3$) δ ppm:
3.36-3.30 (1H, m), 3.19-3.13 (1H, m), 2.69-2.58 (1H, m), 2.55-2.50 (1H, m), 2.38-2.19 (5H, m), 2.02-1.93 (2H, m), 1.87-1.80 (1H, m), 1.59-1.50 (1H, m).

(Reference Example 31)

(±)-1,2,3,5,6,7,8,8a-Octahydroindolizin-7-one

1) (±)-1-(t-Butoxycarbonyl)-2-[4-(p-toluenesulfonyl)-2-oxobutyl]pyrrolidine

In 2 ml of tetrahydrofuran was dissolved 206 mg (10.86 mmol) of (±)-1-(t-butoxycarbonyl)-2-(2-oxo-3-butenyl) pyrrolidine obtained in Reference Example 30-2), and to this solution was added 107 mg (0.86 mmol) of 4-methylthiophenol. The mixture was stirred at room temperature for 2 hours. The reaction mixture was concentrated under reduced pressure. The resulting residue was dissolved in 2 ml of dichloromethane, and to this solution was added under ice cooling and stirring 468 mg (1.894 mmol) of 70 wt% m-chloroperbenzoic acid in small portions. The mixture was stirred at room temperature for 2 hours. To the reaction mixture were added successively 10% aqueous sodium thiosulfate solution and saturated aqueous sodium hydrogencarbonate solution, and the mixture was stirred and extracted with dichloromethane. The organic layer was washed with water, dried over anhydrous magnesium sulfate and concentrated under reduced pressure to give 322 mg of the title compound as a colorless oil (yield: 94%).
$^1$H-NMR, Spectrum (500 MHz, $CDCl_3$) δ ppm:
7.78 (2H, d, J = 8Hz), 7.37 (2H, br.d, J = 8Hz), 4.14-4.04 (1H, m), 3.48-3.22 (4H, m), 3.00-2.77 (3H, m), 2.49-2.35 (4H, m), 2.10-1.98 (1H, m), 1.86-1.74 (2H, m), 1.43 (9H, br.s), 1.33-1.22 (1H, m).

2) (±)-1,2,3,5,6,7,8,8a-Octahydroindolizin-7-one

In 3.2 ml of methanol was dissolved 322 mg (0.814 mmol) of (±)-1-(t-butoxycarbonyl)-2-[4-(p-toluenesulfonyl)-2-oxobutyl]pyrrolidine obtained in 1), and to this solution was added 0.61 ml (2.44 mmol) of 4N hydrochloric acid/dioxane solution. The mixture was stirred at 50°C for 30 minutes. The reaction mixture was concentrated under reduced pressure, the solvent and excess hydrochloric acid were distilled off, and saturated aqueous sodium hydrogencarbonate solution was added to the residue. The mixture was extracted with dichloromethane. The organic layer was washed with water, dried over anhydrous magnesium sulfate, and concentrated under reduced pressure. The residue was subjected to alumina column chromatography (solvent; ethyl acetate) to give 74 mg of the title compound as a slightly brown oil (yield: 65%). It was confirmed by $^1$H-NMR spectrum that the compound was (±)-1,2,3,5,6,7,8,8a-octahydroindolizin-7-one.

(Reference Example 32)

(8aS)-1,2,3,5,6,7,8,8a-Octahydroindolizin-7-one

1) (S)-1-Methylmalonylhomoproline

In 30 ml of dichloromethane was suspended 1.00 g (7.74 mmol) of (S)-homoproline, and to this suspension was added 1.08 ml (7.74 mmol) of triethylamine. To the mixture was added dropwise under ice cooling and stirring 0.83 ml (7.74 mmol) of methylmalonyl chloride, and the mixture was stirred at the same temperature for 15 minutes. The mixture was further stirred at room temperature for 30 minutes, and saturated aqueous sodium hydrogencarbonate

solution was added to this reaction mixture to separate the mixture. The aqueous layer was made pH 2 by the addition of concentrated hydrochloric acid and extracted with a mixture of dichloromethane:isopropanol = 4:1. The organic layer was washed with water, dried over anhydrous magnesium sulfate, and concentrated under reduced pressure. The resulting residue was washed with diisopropyl ether to give 1.42 g of the title compound as a pale yellow powder (yield: 80%).
Melting Point: 142-144°C
$^1$H-NMR Spectrum (500 MHz, $CDCl_3$) δ ppm:
4.40-4.38 (0.8H, m), 4.35-4.29 (0.2H, m), 3.76 (2.4H, s), 3.57-3.43 (3H, m), 3.42 (1.6H, s), 3.00 (0.8H, dd, J = 16Hz, 4Hz), 2.65 (0.2H, dd, J = 16Hz, 4Hz), 2.52 (0.2H, dd, J = 16Hz, 8Hz), 2.46 (0.8H, dd, J = 16Hz, 8Hz), 2.20-2.08 (1H, m), 2.07-1.92 (2H, m), 1.90-1.83 (1H, m).

2) (8aS)-6-Methoxycarbonyl-1,2,3,5,6,7,8,8a-octahydroindolizine-5,7-dione

In 6 ml of tetrahydrofuran was suspended 200 mg (0.87 mmol) of (S)-1-methylmalonylhomoproline obtained in 1), and to this suspension was added 156 mg (0.96 mmol) of carbonyldiimidazole. The mixture was stirred at room temperature for 30 minutes, 143 μl (0.96 mmol) of 1,8-diazabicyclo[5.4.0]-7-undecene was added thereto, and the mixture was further stirred for 1 hour. The reaction mixture was concentrated under reduced pressure, and to the residue were added dichloromethane and 1N hydrochloric acid solution to separate the mixture. The organic layer was washed with water, dried over anhydrous magnesium sulfate and concentrated under reduced pressure to give 183 mg of the title compound as a pale yellow powder (yield: quantitative).
$^1$H-NMR Spectrum (500 MHz, $CDCl_3$) δ ppm:
3.90 (3H, s), 3.76-3.69 (1H, m), 3.67-3.60 (1H, m), 3.53-3.44 (1H, m), 2.68 (1H, dd, J = 17Hz, 4Hz), 2.54 (1H, dd, J = 17Hz, 13Hz), 2.30-2.23 (1H, m), 2.08-2.00 (1H, m), 1.88-1.76 (1H, m), 1.70-1.56 (2H, m).

3) (8aS)-1,2,3,5,6,7,8,8a-Octahydroindolizine-5,7-dione

In 2 ml of 10% aqueous acetic acid solution was dissolved 183 mg (0.87 mmol) of (8aS)-6-methoxycarbonyl-1,2,3,5,6,7,8,8a-octahydroindolizine-5,7-dione obtained in 2), and the solution was stirred at 110°C for 30 minutes and cooled to room temperature. The reaction solution was made alkaline by the addition of saturated aqueous sodium hydrogencarbonate solution and extracted with dichloromethane:isopropanol = 4:1. The organic layer was washed with water, dried over anhydrous magnesium sulfate and concentrated under reduced pressure to give 117 mg of the title compound as a pale brown oil (yield: 87%).
$^1$H-NMR Spectrum (500 MHz, $CDCl_3$) δ ppm:
3.89-3.81 (1H, m), 3.72-3.64 (1H, m), 3.60-3.53 (1H, m), 3.27 (2H, dd, J = 24Hz, 20Hz), 2.85 (1H, dd, J = 17Hz, 3Hz), 2.36-2.30 (1H, m), 2.29 (1H, dd, J = 17Hz, 12Hz), 2.13-2.04 (1H,m), 1.97-1.86 (1H, m), 1.72-1.62 (1H, m).

4) (8aS)-7-(1-Pyrrolidinyl)-1,2,3,5,8,8a-hexahydroindolizin-5-one

In 1.2 ml of ethanol was dissolved 117 mg (0.76 mmol) of (8aS)-1,2,3,5,6,7,8,8a-octahydroindolizine-5,7-dione obtained in 3), and to this solution was added 128 μl (1.53 mmol) of pyrrolidine. The mixture was left to stand at room temperature for 5 minutes, and the solvent and excess pyrrolidine were distilled off under reduced pressure to give 160 mg of the title compound as a slightly yellow powder (yield: quantitative).
$^1$H-NMR Spectrum (500 MHz, $CDCl_3$) δ ppm:
4.60 (1H, s), 3.70-3.58 (2H, m), 3.50-3.41 (1H, m), 3.37-3.11 (4H, br.m), 2.62 (1H, dd, J = 16Hz, 5Hz), 2.30-2.14 (2H, m), 2.05-1.57 (7H, m).
HPLC determination of this compound using the conditions described below showed the absence of the R form, and it was confirmed that the compound had optical purity of 99%ee or above.

HPLC conditions

Column: CHIRALPAK OD (manufactured by Daicel Chemical Ind. Ltd., inner diameter 0.46 cm, length 25 cm)
Mobile phase: n-Hexane/isopropanol = 90/10
Flow rate: 1.0 ml/min.
Temperature: 40°C
Detection: 254 nm (UV)
Retention period: for S form: 27.23 minutes for R form: 32.30 minutes

5) (8aS)-1,2,3,5,6,7,8,8a-Octahydroindolizin-7-one

In 10 ml of tetrahydrofuran was dissolved 645 mg (3.13 mmol) of (8aS)-7-(1-pyrrolidinyl)-1,2,3,5,8,8a-hexahydroindolizin-5-one obtained in 4), and to this solution was added under ice cooling and stirring 356 mg (9.39 mmol) of lithium aluminum hydride. The mixture was stirred at room temperature overnight, and to the reaction mixture was added 1.44 ml of 1N aqueous sodium hydroxide solution. Ethanol was then added, and the insolubles were filtered off. The filtrate was concentrated under reduced pressure and the residue was subjected to alumina column chromatography (solvent; ethyl acetate) to give 236 mg of the title compound as a slightly brown oil (yield: 54%).
Boiling point: 75-78°C/6 mmHg
$^1$H-NMR Spectrum (500 MHz, $CDCl_3$) δ ppm:
3.36-3.30 (1H, m), 3.19-3.13 (1H, m), 2.67-2.58 (1H, m), 2.55-2.50 (1H, m), 2.38-2.19 (5H, m), 2.02-1.93 (2H, m), 1.87-1.80 (1H, m), 1.59-1.50 (1H, m).

$^1$H-NMR spectrum determination of this compound using a shift reagent [detailed description in W H. Pirkle and D. J. Hoover, Top. Stereochem., 13, 263(1982)] showed the absence of the R form, and it was confirmed that the compound had optical purity of 99%ee or above.

(Reference Example 33)

(2S,8aS)-2-Phenyl-1,2,3,5,6,7,8,8a-octahydroindolizin-7-one

1) (2S,4S)-1-Benzyloxycarbonyl-2-cyanomethyl-4-phenylpyrrolidine

Reduction, methanesulfonylation and cyanation were successively carried out in a manner similar to that in Reference Example 1-1) using (2S,4S)-1-benzyloxycarbonyl-4-phenylproline [J. Med. Chem., 31, 1148-1160 (1988)] instead of (2S,4R)-1-benzyloxycarbonyl-4-methoxyproline as the starting material, to give the title compound as a pale yellow oil (yield: 16%).
$^1$H-NMR Spectrum (400 MHz, $CDCl_3$) δ ppm:
7.58-7.29 (6H, m), 7.28-7.23 (2H, m), 7.22-7.19 (2H, m), 5.22-5.09 (2H, m), 4.31-4.20 (1H, m), 4.09-3.94 (1H, m), 3.75-3.41 (2H, m), 2.96-2.62 (2H, m), 2.36-2.27 (2H, m).

2) (2S,4S)-4-Phenylhomoproline

To 8.80 g (27.48 mmol) of (2S,4S)-1-benzyloxycarbonyl-2-cyanomethyl-4-phenylpyrrolidine obtained in 1) was added 44 ml of concentrated hydrochloric acid, and the mixture was stirred at 80°C for 15 hours. To the reaction mixture were added 100 ml of water and 50 ml of ethyl acetate, and the mixture was shaken thoroughly and then separated. The aqueous layer was concentrated to dryness under reduced pressure. The resulting solid (a mixture of the desired hydrochloride and ammonium chloride) was desalinated and purified with an ion exchange resin to give 3.66 g of the title compound as a white powder (yield: 65%).
$^1$H-NMR Spectrum (400 MHz, DMSO-$d_6$) δ ppm:
7.36-7.10 (5H, m), 3.56-3.40 (1H, m), 3.40-3.10 (2H, m), 2.72-2.58 (1H, m), 2.58-2.43 (2H, m), 2.20-2.00 (2H, m), 1.92-1.75 (2H, m).

3) (2S,8aS)-2-Phenyl-1,2,3,5,6,7,8,8a-octahydroindolizin-7-one

Reactions similar to those of Reference Examples 32-1), 32-2), 32-3), 32-4) and 32-5) were successively carried out using (2S,4S)-4-phenylhomoproline obtained in 2) instead of (S)-homoproline to give the title compound as a pale brown oil (yield: 35%).
$^1$H-NMR Spectrum (400 MHz, $CDCl_3$) δ ppm:
7.37-7.28 (2H, m), 7.27-7.21 (3H, m), 3.63-3.50 (2H, m), 3.49-3.32 (1H, m), 2.69-2.55 (3H, m), 2.47-2.40 (2H, m), 2.38-2.32 (2H, m), 2.17-2.10 (2H, m).

(Reference Example 34)

(2R,8aS)-2-(4-Fluorophenyl)-1,2,3,5,6,7,8,8a-octahydroindolizin-7-one

Reactions similar to those of Reference Examples 6-1), 6-2), 6-3), 6-4) and 6-5) were successively carried out using (S)-1-benzyloxycarbonyl-4-(4-fluorophenyl)-3-pyrroline-2-carboxylic acid methyl ester [J. Med. Chem., 31, 1148-1160 (1988)] instead of (S)-1-benzyloxycarbonyl-4-phenyl-3-pyrroline-2-carboxylic acid methyl ester as the starting material, to give the title compound as a white powder (yield: 11%).
$^1$H-NMR Spectrum (400 MHz, $CDCl_3$) δ ppm:
7.32 (2H, dd, J = 9Hz, 6Hz), 6.99 (2H, t, J = 6Hz), 3.39-3.28 (2H, m), 3.12 (1H, dd, J = 10Hz, 3Hz), 2.75-2.62 (2H,m), 2.57-2.32 (6H, m), 1.66-1.53 (1H, m).

(Reference Example 35)

(2R,8aS)-2-(4-Methylphenyl)-1,2,3,5,6,7,8,8a-octahydroindolizin-7-one

Reactions similar to those of Reference Examples 6-1), 6-2), 6-3), 6-4) and 6-5) were successively carried out using (S)-1-benzyloxycarbonyl-4-(4-methylphenyl)-3-pyrroline-2-carboxylic acid methyl ester [J. Med. Chem., 31, 1148-1160 (1988)] instead of (S)-1-benzyloxycarbonyl-4-phenyl-3-pyrroline-2-carboxylic acid methyl ester as the starting material, to give the title compound as colorless prism crystals (yield: 1%).
Melting Point: 91-92°C
$^1$H-NMR Spectrum (400 MHz, $CDCl_3$) δ ppm:
7.25 (2H, d, J = 8Hz), 7.12 (2H, d, J = 8Hz), 3.38-3.26 (2H, m), 3.15 (1H, dd, J = 10Hz, 3Hz), 2.73-2.62 (1H, m), 2.71 (1H, t, J = 10Hz), 2.56-2.32 (6H, m), 2.33 (3H, s), 1.68-1.57(1H, m).

(Reference Example 36)

(2S,8aS)-2-Butyl-1,2,3,5,6,7,8,8a-octahydroindolizin-7-one

Reactions similar to those of Reference Examples 8-2), 8-3), 8-4) 8-5) and 8-6) were successively carried out using (S)-1-benzyloxycarbonyl-4-butylideneproline methyl ester instead of (S)-1-benzyloxycarbonyl-4-methylideneproline methyl ester to give the title compound as a yellow oil (yield: 24%).
$^1$H-NMR Spectrum (400 MHz, $CDCl_3$) δ ppm:
3.31-3.23 (1H, m), 2.87-2.84 (1H, m), 2.67-2.58 (1H, m), 2.50-2.39 (2H, m), 2.36-2.26 (4H, m), 2.20-2.09 (2H, m), 1.53-1.39 (2H, m), 1.36-1.20 (4H, m), 1.19-1.15 (1H, m), 0.90 (3H, t, J = 7Hz).

(Reference Example 37)

(2S,8aS)-2-(4-Methylphenyl)-1,2,3,5,6,7,8,8a-octahydroindolizin-7-one

Reactions similar to those of Reference Examples 33-1), 33-2) and 33-3) were successively carried out using (2S,4S)-1-benzyloxycarbonyl-4-(4-methylphenyl)proline [J. Med. Chem., 31, 1148-1160 (1988)] instead of (2S,4R)-1-benzyloxycarbonyl-4-methoxyproline as the starting material, to give the title compound as pale yellow needle crystals (yield: 36%).
Melting Point: 45-47°C
$^1$H-NMR Spectrum (400 MHz, $CDCl_3$) δ ppm:
7.13 (4H, s), 3.60-3.47 (2H, m), 3.38-3.31 (1H, m), 2.70-2.55 (3H, m), 2.47-2.29 (4H, m), 2.33 (3H, s), 2.12-2.04(2H, m).

(Reference Example 38)

(2S,8aS)-2-(3-Methylphenyl)-1,2,3,5,6,7,8,8a-octahydroindolizin-7-one

Reactions similar to those of Reference Examples 33-1), 33-2) and 33-3) were successively carried out using (2S,4S)-1-benzyloxycarbonyl-4-(3-methylphenyl)proline [J. Med. Chem., 31, 1148-1160 (1988)] instead of (2S,4R)-1-benzyloxycarbonyl-4-methoxyproline as the starting material, to give the title compound as a yellow oil (yield: 16%).
$^1$H-NMR Spectrum (400 MHz, $CDCl_3$) δ ppm:
7.16 (1H, t, J = 8Hz), 7.00 (2H, s), 6.98(1H, s), 3.54-3.47 (2H, m), 3.38-3.21 (1H, m), 2.69-2.59 (3H, m), 2.45 (1H, dd, 12Hz J = 4Hz), 2.41 (1H, d, J = 2Hz), 2.37 (1H, d, J = 2Hz), 2.34 (3H, s), 2.10 (2H, t, J = 8Hz).

(Reference Example 39)

(2S,8aS)-2-(4-Methoxyphenyl)-1,2,3,5,6,7,8,8a-octahydroindolizin-7-one

Reactions similar to those of Reference Examples 33-1), 33-2) and 33-3) were successively carried out using (2S,4S)-1-benzyloxycarbonyl-4-(4-methoxyphenyl)proline [J. Med. Chem., 31, 1148-1160 (1988)] instead of (2S,4R)-1-benzyloxycarbonyl-4-methoxyproline as the starting material, to give the title compound as a pale yellow powder (yield: 31%).
$^1$H-NMR Spectrum (500 MHz, $CDCl_3$) δ ppm:
7.15 (2H, d, J = 8Hz), 6.85 (2H, d, J = 8Hz), 3.79 (3H, s), 3.58-3.46 (2H, m), 3.38-3.32 (1H, m), 2.70-2.53 (2H, m), 2.47-2.27 (4H, m), 2.13-2.01 (2H, m).

(Reference Example 40)

(2S,8aS)-2-(2-Methoxyphenyl)-1,2,3,5,6,7,8,8a-octahydroindolizin-7-one

Reactions similar to those of Reference Examples 33-1), 33-2) and 33-3) were successively carried out using (2S,4S)-1-benzyloxycarbonyl-4-(2-methoxyphenyl)proline [J. Med. Chem., 31, 1148-1160 (1988)] instead of (2S,4R)-1-benzyloxycarbonyl-4-methoxyproline as the starting material, to give the title compound as a slightly yellow powder (yield: 16%).
$^1$H-NMR Spectrum (500 MHz, $CDCl_3$) δ ppm:
7.23-7.17 (2H, m), 6.92 (1H, t, J = 7Hz), 6.86 (1H, d, J = 9Hz), 3.91-3.82 (1H, m), 3.82 (3H, s), 3.42 (1H, t, J = 8Hz), 3.38-3.32 (1H, m), 2.70-2.54 (3H, m), 2.47-2.33 (4H, m), 2.14-1.98 (2H, m).

(Reference Example 41)

(2S,8aS)-2-(3-Methoxyphenyl)-1,2,3,5,6,7,8,8a-octahydroindolizin-7-one

Reactions similar to those of Reference Examples 33-1), 33-2) and 33-3) were successively carried out using (2S,4S)-1-benzyloxycarbonyl-4-(3-methoxyphenyl)proline [J. Med. Chem., 31, 1148-1160 (1988)] instead of (2S,4R)-1-benzyloxycarbonyl-4-methoxyproline as the starting material, to give the title compound as a pale brown oil (yield: 22%).
$^1$H-NMR Spectrum (400 MHz, $CDCl_3$) δ ppm:
7.26-7.20 (1H, m), 6.83 (1H, d, J = 8Hz), 6.80-6.74 (2H, m), 3.81 (3H, s), 3.62-3.48 (2H, m), 3.41-3.31 (1H, m), 2.73-2.54 (3H,m), 2.51-2.31 (4H, m), 2.17-2.06 (2H, m).

(Reference Example 42)

(2S,8aS)-2-(4-t-Butylphenyl)-1,2,3,5,6,7,8,8a-octahydroindolizin-7-one

Reactions similar to those of Reference Examples 33-1), 33-2) and 33-3) were successively carried out using (2S,4S)-1-benzyloxycarbonyl-4-(4-t-butylphenyl)proline [J. Med. Chem., 31, 1148-1160 (1988)] instead of (2S,4R)-1-benzyloxycarbonyl-4-methoxyproline as the starting material, to give the title compound as an amorphous pale brown solid (yield: 21%).
$^1$H-NMR Spectrum (400 MHz, $CDCl_3$) δ ppm:
7.29 (2H, d, J = 8Hz), 7.13 (2H, d, J = 8Hz), 3.52 (1H, quintet, J = 8Hz), 3.47 (1H, t, J=8Hz), 3.33-3.28 (1H, m), 2.65-2.51 (3H, m), 2.42-2.28 (4H, m), 2.05 (2H, t, J = 8Hz), 1.31 (9H, s).

(Reference Example 43)

(2S,8aS)-2-(4-Ethoxyphenyl)-1,2,3,5,6,7,8,8a-octahydroindolizin-7-one

Reactions similar to those of Reference Examples 33-1), 33-2) and 33-3) were successively carried out using (2S,4S)-1-benzyloxycarbonyl-4-(4-ethoxyphenyl)proline [J. Med. Chem., 31, 1148-1160 (1988)] instead of (2S,4R)-1-benzyloxycarbonyl-4-methoxyproline as the starting material, to give the title compound as a white powder (yield: 28%).
$^1$H-NMR Spectrum (400 MHz, $CDCl_3$) δ ppm:
7.14 (2H, d, J = 9Hz), 6.84 (2H, d, J = 9Hz), 4.01 (2H, quartet, J = 7Hz), 3.59-3.45 (2H, m), 3.39-3.31 (1H, m), 2.72-2.54 (3H, m), 2.49-2.27 (4H,m), 2.16-2.01 (2H, m), 1.40 (3H, t, J = 7Hz).

(Reference Example 44)

(2S,8aS)-2-(3,4-Dimethylphenyl)-1,2,3,5,6,7,8,8a-octahydroindolizin-7-one

Reactions similar to those of Reference Examples 33-1), 33-2) and 33-3) were successively carried out using (2S,4S)-1-benzyloxycarbonyl-4-(3,4-dimethylphenyl)proline [J. Med. Chem., 31, 1148-1160 (1988)] instead of (2S,4R)-1-benzyloxycarbonyl-4-methoxyproline as the starting material, to give the title compound as an amorphous yellow solid (yield: 12%).
$^1$H-NMR Spectrum (400 MHz, $CDCl_3$) δ ppm:
7.07 (1H, d, J = 8Hz), 7.00 (1H, s), 6.97 (1H, d, J = 8Hz), 4.11-3.46 (2H, m), 3.37-3.33 (1H, m), 2.68-2.56 (3H, m), 2.46-2.29 (4H, m), 2.25 (3H, s), 2.24 (3H, s), 2.10-2.08 (2H, m).

(Reference Example 45)

(2S,8aS)-2-(4-Biphenyl)-1,2,3,5,6,7,8,8a-octahydroindolizin-7-one

Reactions similar to those of Reference Examples 33-1), 33-2) and 33-3) were successively carried out using (2S,4S)-1-benzyloxycarbonyl-4-(4-biphenyl)proline (obtained by the process described in J. Med. Chem., 1988, 31, 1148-1160) instead of (2S,4R)-1-benzyloxycarbonyl-4-methoxyproline as the starting material to give the title compound as colorless needle crystals (yield: 19%).
Melting Point: 112-114°C
$^1$H-NMR Spectrum (500 MHz, $CDCl_3$) δ ppm:
7.58 (2H, d, J = 8Hz), 7.54 (2H, d, J = 8Hz), 7.43 (2H, t, J = 8Hz), 7.36-7.29 (3H, m), 3.63 (1H, quintet, J = 8Hz), 3.55 (1H, t, J = 9Hz), 3.37 (1H, dd, J = 11Hz, 7Hz), 2.70-2.57 (3H, m), 2.49-2.35 (4H, m), 2.15 (2H, t, J = 9Hz).

(Reference Example 46)

3-Bromo-5-(3,4-difluorophenyl)-4-(pyridin-4-yl)pyrazole

A reaction similar to that of Example 5-1) was carried out using 5-(3,4-difluorophenyl)-4-(pyridin-4-yl)pyrazole (obtained by the process described in WO 00/31063) instead of 5-(4-fluorophenyl)-4-(pyridin-4-yl)pyrazole to give the title compound as a pale brown powder (yield: 61%).
Melting Point: 285-290°C
$^1$H-NMR Spectrum (400 MHz, DMSO-$d_6$) δ ppm:
14.15 (0.2H, br.s), 13.94 (0.8H, br.s), 8.60-8.58 (2H, d, J = 6Hz,), 7.53-7.42 (2H, m), 7.28-7.26 (2H, m), 7.13-7.11 (1H, m).

(Reference Example 47)

3-Bromo-5-phenyl-4-(pyridin-4-yl)pyrazole

A reaction similar to that of Example 5-1) was carried out using 5-phenyl-4-(pyridin-4-yl)pyrazole (obtained by the process described in WO 00/31063) instead of 5-(4-fluorophenyl)-4-(pyridin-4-yl)pyrazole to give the title compound as a yellow powder (yield: 93%).
$^1$H-NMR Spectrum (400 MHz, DMSO-$d_6$) δ ppm:
13.85 (1H, s), 8.58 (2H, d, J = 6Hz), 7.43-7.41 (3H, m), 7.35-7.30 (2H, m), 7.27 (2 H, d, J = 6Hz).

(Reference Example 48)

4-Bromo-2-(3-cyanophenyl)-3-(pyridin-4-yl)-1-triisopropylsilyl-1H-pyrrole

A reaction similar to that of Example 1-4) was carried out using 2-(3-cyanophenyl)-3-(pyridin-4-yl)-1-triisopropylsilyl-1H-pyrrole instead of 2-(4-fluorophenyl)-3-(pyridin-4-yl)-1-triisopropylsilyl-1H-pyrrole to give the title compound as an amorphous pale yellow solid (yield: 40%).
$^1$H-NMR Spectrum (400 MHz, $CDCl_3$) δ ppm:
8.38-8.36 (2H, dd, J = 5Hz, 2Hz), 7.45-7.25 (11H, m), 7.13-7.11 (1H, m), 6.97-6.95 (2H, dd, J = 5Hz, 2Hz), 6.78-6.69 (3H, m), 1.13 (9H, s).

(Reference Example 49)

4-Bromo-2-(4-fluoro-3-methoxyphenyl)-3-(pyridin-4-yl)-1-triisopropylsilyl-1H-pyrrol e

A reaction similar to that of Example 1-4) was carried out using 2-(4-fluoro-3-methoxyphenyl)-3-(pyridin-4-yl)-1-triisopropylsilyl-1H-pyrrole instead of 2-(4-fluorophenyl)-3-(pyridin-4-yl)-1-triisopropylsilyl-1H-pyrrole to give the title compound as a pale red powder (yield: 77%).
Melting Point: 190-195°C
$^1$H-NMR Spectrum (400 MHz, $CDCl_3$) δ ppm:
8.37-8.35 (2H, dd, J = 5Hz, 2Hz), 7.42-7.26 (11H, m), 7.05-7.03 (2H, dd, J = 5Hz, 2Hz), 6.34-6.30 (1H, dd, J = 11Hz, 8Hz), 6.17-6.15 (1H, dd, J = 8Hz, 2Hz), 5.99-5.96 (1H, m),3.01 (3H, s), 1.09 (9H, s).

(Reference Example 50)

4-Bromo-2-(3-methoxyphenyl)-3-(pyridin-4-yl)-1-triisopropylsilyl-1H-pyrrole

A reaction similar to that of Example 1-4) was carried out using 2-(3-methoxyphenyl)-3-(pyridin-4-yl)-1-triisopropylsilyl-1H-pyrrole instead of 2-(4-fluorophenyl)-3-(pyridin-4-yl)-1-triisopropylsilyl-1H-pyrrole to give the title compound as a white powder (yield: 77%).
$^1$H-NMR Spectrum (400 MHz, DMSO-$d_6$) δ ppm:
8.34 (2H, d, J = 5Hz), 7.46-7.41 (3H, m), 7.39-7.34 (7H, m), 7.25 (1H, s), 7.04 (2H, d, J = 4Hz), 6.64 (1H, t, J = 8Hz), 6.50-6.47 (1H, m), 6.17 (1H, s), 6.13 (1H, d, J = 8Hz), 3.14 (3H, s), 0.98 (9H, s).

(Reference Example 51)

(2R,8aS)-2-Azido-1,2,3,5,6,7,8,8a-octahydroindolizin-7-one

1) (2R,8aS)-2-Hydroxy-7,7-dimethoxy-1,2,3,5,6,7,8,8a-octahydroindolizine

In 5 ml of methanol was dissolved 0.748 g (2.78 mmol) of (2R,8aS)-2-(t-butyldimethylsilyloxy)-1,2,3,5,6,7,8,8a-octahydroindolizin-7-one obtained in Reference Example 2, and to this solution were added 0.91 ml (8.34 mmol) of trimethyl orthoformate and 0.479 g (2.78 mmol) of tosic acid. The mixture was refluxed with heating for 3 hours. A further 0.240 g (1.39 mmol) of tosic acid was added thereto, the mixture was heated at reflux for 1 hour, and the reaction mixture was cooled to room temperature. The reaction mixture was neutralized by the addition of 20% sodium methoxide solution in methanol, and the solvent was distilled off under reduced pressure. The residue was subjected to silica gel column chromatography (solvent; ethyl acetate:methanol:isopropyl amine = 20:1:1) to give 0.502 g of the title compound as a pale yellow oil (yield: 90%).
$^1$H-NMR Spectrum (400 MHz, $CDCl_3$) δ ppm:
4.50-4.44 (1H, m), 3.47 (1H, dd, J = 10Hz, 7Hz), 3.21 (3H, s), 3.17(3H, s), 2.90 (1H, ddd, J = 12Hz, 11Hz, 5Hz), 2.50-2.42 (1H, m), 2.28 (1H, ddd, J = 12Hz, 11Hz, 3Hz), 2.18-2.10 (2H, m), 2.02-1.96 (1H, m), 1.78-1.70 (2H, m), 1.60 (1H, dt, J = 13Hz, 5Hz), 1.22 (1H, t, J = 13Hz).

2) (2S,8aS)-2-Bromo-7,7-dimethoxy-1,2,3,5,6,7,8,8a-octahydroindolizine

To a solution of 3.27 g (12.5 mmol) of triphenylphosphine in 10 ml of tetrahydrofuran was added 1.96 ml (12.5 mmol) of diethyl azodicarboxylate and the mixture was stirred at room temperature for 20 minutes. To the reaction mixture were added a solution of 0.502 g (2.50 mmol) of (2R,8aS)-2-hydroxy-7,7-dimethoxy-1,2,3,5,6,7,8,8a-octahydroindolizine obtained in 1) in 5 ml of tetrahydrofuran and 2.16 g (25.0 mmol) of lithium bromide, and the mixture was stirred at the same temperature for 3 hours. The solvent was distilled off under reduced pressure, and the residue was dissolved in ethyl acetate and washed with water. The solution was dried over anhydrous magnesium sulfate and concentrated under reduced pressure. The resulting residue was subjected to silica gel column chromatography (solvent; ethyl acetate) to give 0.294 g of the title compound as a pale yellow oil (yield: 45%).
$^1$H-NMR Spectrum (400 MHz, $CDCl_3$) δ ppm:
4.38-4.32 (1H, m), 3.35 (1H, d, J = 12Hz), 3.21 (3H, s), 3.15 (3H, s), 2.96 (1H, ddd, J = 14Hz, 11Hz, 5Hz), 2.72 (1H, dd, J = 11Hz, 7Hz), 2.65-2.52 (1H, m), 2.18-2.09 (3H, m), 2.02-1.92 (2H, m), 1.76 (1H, dt, J = 13Hz, 4Hz), 1.42 (1H, dd, J = 13Hz, 12Hz).

3) (2R,8aS)-2-Azido-7,7-dimethoxy-1,2,3,5,6,7,8,8a-octahydroindolizine

To a solution of 0.294 g (1.11 mmol) of (2S,8aS)-2-bromo-7,7-dimethoxy-1,2,3,5,6,7,8,8a-octahydroindolizine obtained in 3) in 10 ml of dimethylformamide was added 0.289 g (4.45 mmol) of sodium azide and the mixture was stirred at 70°C for 1 hour. After cooling to room temperature, ethyl acetate was added to the reaction mixture, and the mixture was washed with water and dried over anhydrous magnesium sulfate. The residue obtained by concentrating the mixture under reduced pressure was subjected to silica gel column chromatography (solvent; ethyl acetate:methanol = 10:1) to give 0.164 g of the title compound as a pale yellow oil (yield: 65%).
$^1$H-NMR Spectrum (400 MHz, $CDCl_3$) δ ppm:
4.16-4.08 (1H, m), 3.41 (1H, dd, J = 9Hz, 7Hz), 3.21 (3H, s), 3.17 (3H, s), 2.90 (1H, ddd, J = 13Hz, 11Hz, 5Hz), 3.38-2.30 (1H, m), 2.27-2.19 (1H, m), 2.19-2.12 (2H, m), 2.00 (1H, ddd, J = 16Hz, 14Hz, 5Hz), 1.88 (1H, ddd, J = 13Hz, 6Hz, 2Hz), 1.80-1.74 (1H, m), 1.59 (1H, dt, J = 13Hz, 5Hz), 1.22 (1H, dd, J = 13Hz, 12Hz).

4) (2R,8aS)-2-Azido-1,2,3,5,6,7,8,8a-octahydroindolizin-7-one

In 5 ml of a solution of 0.5 N hydrochloric acid in dioxane was dissolved 0.164 g (0.725 mmol) of (2R,8aS)-2-azido-7,7-dimethoxy-1,2,3,5,6,7,8,8a-octahydroindolizine, the solution was stirred at 50°C for 21 hours, and a further 5 ml of a solution of 0.5 N hydrochloric acid in dioxane was added thereto. The solution was stirred at the same temperature for 9 hours. Subsequently 1 ml of a solution of 4N hydrochloric acid in dioxane was added thereto, the solution was stirred at 60°C for 6 hours, and a further 1 ml of a solution of 4N hydrochloric acid in dioxane was added thereto. The solution was stirred at the same temperature for 10 hours, and reaction solution was concentrated under reduced pressure to give 0.126 g of the title compound as a dark brown oil (yield: 97%).
$^1$H-NMR Spectrum (400 MHz, $CDCl_3$) δ ppm:
4.70-4.60 (1H, m), 4.29-4.14 (1H, m), 3.92-3.80 (1H, m), 3.64-3.50 (1H, m), 3.47-3.28 (2H, m), 3.20-3.08 (1H, m), 2.86-2.50 (4H, m), 3.37-3.29 (1H, m).

(Reference Example 52)

(2S,8aS)-2-(4-Ethylphenyl)-1,2,3,5,6,7,8,8a-octahydroindolizin-7-one

Reactions similar to those of Reference Examples 33-1), 33-2) and 33-3) were successively carried out using (2S,4S)-1-benzyloxycarbonyl-4-(4-ethylphenyl)proline (obtained in the process described in J. Med. Chem., 1988, 31, 1148-1160) instead of (2S,4R)-1-benzyloxycarbonyl-4-methoxyproline as the starting material, to give the title compound as a yellow oil (yield: 27%).
$^1$H-NMR Spectrum (400 MHz, $CDCl_3$) δ ppm:
7.18-7.12 (4H, m), 3.60-3.47 (2H, m), 3.38-3.31 (1H, m), 2.70-2.55 (5H, m), 2.47-2.31 (4H, m), 2.12-2.07 (2H, m), 1.23 (3H, t, J = 8Hz).

(Preparation Example 1)

Powders of the compound of Example 2

Powders are obtained by mixing 5 g of the compound of Production Example 1, 895 g of lactose, and 100 g of cornstarch using a blender.

(Preparation Example 2)

Granules of the compound of Example 12

After mixing 5 g of the compound of Production Example 2, 865 g of lactose, and 100 g of low substituted hydroxypropyl cellulose, 300 g of 10% aqueous hydroxypropyl cellulose solution is added thereto, and the mixture is kneaded. The kneaded mixture is granulated using an extruding granulator and dried to obtain granules.

(Preparation Example 3)

Capsules of the compound of Example 16

After mixing 5 g of the compound of Production Example 3, 115 g of lactose, 58 g of cornstarch, and 2 g of

magnesium stearate using a V-type mixer, 180 mg each of the mixture is filled into type 3 capsules to obtain capsules.

(Preparation Example 4)

Tablets of the compound of Example 46

After mixing 5 g of the compound of Production Example 4, 90 g of lactose, 34 g of cornstarch, 20 g of crystalline cellulose, and 1 g of magnesium stearate using a blender, the mixture is compressed by a tablet-making machine to obtain tablets.

(Preparation Example 5)

Formulations of the compound of Example 47

After mixing 5 g of the compound of Production Example 6, 25 g of dichlofenac sodium, 65 g of lactose, 34 g of cornstarch, 20 g of crystalline cellulose, and 1 g of magnesium stearate using a blender, the mixture is compressed by a tablet-making machine to obtain tablets.

(Test Example 1)

Inhibition of the Production of the Cytokines IL-1β and TNFα *in vitro in* Human

Whole Blood

This test was performed according to the method of Hartman, et al. [D.A. Hartman, S.J. Ochalski and R.P. Carlson; The effects of anti-inflammatory and antiallergic drugs on cytokine release after stimulation of human whole blood by lipopolysaccharide and zymosan A: Inflamm. Res., 44, 269 (1995)].

Peripheral blood samples were collected in the presence of heparin from healthy adult volunteers. 1000 μl of whole blood was added to an Eppendorf tube to which 2 μl of a dimethyl sulfoxide solution of the test compound had been previously added, after which 10 μl of lipopolysaccharide (E. coli O26: B6 origin, Difco) was added as a stimulant (final concentration of said lipopolysaccharide: 10 μg/ml). This was thoroughly mixed and then incubated for 6 hours at 37°C in the presence of 5% $CO_2$. At the end of the incubation, the mixture was cooled to 4°C to stop the reaction, followed immediately by centrifuging for 5 minutes at 14,000 rpm to separate and collect the supernatant plasma. The IL-1β and TNFα produced and released into the plasma were measured using a commercially available enzyme immunoassay (ELISA) kits [Cayman and Genzyme]. The inhibitory effect [$IC_{50}$ (μM)] on the production of cytokines was determined by the method of least squares from the amounts of the cytokines produced in the presence and absence of the test compound. The results for the inhibitory effect on TNFα production are as shown in Table 5 below.

Table 5

| Inhibitory Effect on TNFα Production *(in vitro)* | |
|---|---|
| Test Compound | $IC_{50}$ (nM) |
| Compound of Example 1 | 3.4 |
| Compound of Example 3 | 5.1 |
| Compound of Example 4 | 7.8 |
| Compound of Example 6 | 6.3 |
| Compound of Example 10 | 7.3 |
| Compound of Example 12 | 5.5 |
| Compound of Example 16 | 5.4 |
| Compound of Example 46 | 2.1 |

As shown in Table 5 above, compounds of the present invention demonstrated superior cytokine production inhibitory action in this test.

(Test Example 2)

Inhibition of the Production of TNFα *in vivo*

This test was performed according to the method of Ochalski, et al. [S.J. Ochalski, D.A. Hartman, M.T. Belfast, T.L. Walter, K.B. Glaser and R.P. Carlson; Inhibition of endotoxin-induced hypothermia and serum TNF-α levels in CD-1 mice by various pharmacological agents: Agents Actions 39, C52-C54 (1993)].

The production of TNFα was induced in mice by intravenous injection of lipopolysaccharide (E. coli 026: B6 origin, Difco) which was prepared to a concentration of 0.045 mg/ml using physiological saline. The saline preparation of lipopolysaccharide was administered at the rate of 10 ml/l kg of body weight into the caudal vein of Balb/c mice (males, age 5-7 weeks, body weight: approx. 22 g, Japan Charles River) which had been fasted overnight starting the day before the experiment. One hour after administration, the mice were laparotomized under ether anaesthesia and blood was collected from the abdominal vena cava. Blood collection was performed using a 1 ml volume disposable syringe equipped with a 23G needle which had been moistened with heparin on the inside wall. Following blood collection, the blood was immediately transferred to a 1.5 ml volume Eppendorf tube and centrifuged at 4°C and 14,000 rpm to separate the plasma. This plasma was then stored at -20°C until measurement of TNFα.

The measurement of the amount of TNFα was performed with a enzyme immunoassay (ELISA) kit (Mouse TNFα ELISA KIT, Genzyme).

To determine the inhibitory activity of the test compounds, each test compound was suspended in 0.5% tragacanth solution and then administered orally to the mice at a rate of 10 ml/l kg of body weight 30 minutes before injection of the lipopolysaccharide, and the average inhibition rates were calculated versus a control group. Those results are shown in Table 23 below.

Table 6

| Inhibitory Effect of TNFα Production (in vivo) | |
|---|---|
| Test Compound | Inhibition Rate (%) (dose: 5 mg/kg) |
| Compound of Example 8 | 91 |
| Compound of Example 10 | 91 |
| Compound of Example 16 | 90 |
| Compound of Example 23 | 90 |
| Compound of Example 25 | 92 |
| Compound of Example 30 | 90 |
| Compound of Example 31 | 92 |
| Compound of Example 41 | 91 |
| Compound of Example 46 | 93 |

As shown in Table 6 above, compounds of the present invention demonstrated superior TNFα production inhibitory action in this test.

(Test Example 3)

Inhibition Test of IL-1β Production (in vivo)

This test was performed according to the method of Griffiths, et al. [Richard J. Griffiths, Ethan J. Stam, James T. Downs and Ivan G. Otterness; ATP Induces the Release of IL-1 from LPS-Primed Cells In Vivo: J. Immunol., 154, 2821-2828 (1995)].

Production of IL-1β was induced by intraperitoneal injection of LPS and adenosine triphosphate (ATP) to mice. LPS (origin: E. coli 026:B6, Difco) prepared to 0.0045 mg/ml using physiological saline was administered at the rate of 10 ml/kg body weight into the abdominal cavities of Balb/c mice (males, age 5-7 weeks, body weights: roughly 22 g, Japan Charles River) that were fasted overnight starting the day before the experiment. Two hours later, 0.5 ml of ATP prepared to 6.03 mg/ml were additionally administered intraperitoneally using a physiological saline solution. Immediately after sacrificing the mice by asphyxiation using dry ice 0.5 hour after administration of ATP, 3 ml of washing PBS (containing heparin at 10 U/ml, PMSF at 0.25 mM, leupepsin at 1 μg/ml, pepstatin at 1 μg/ml and EDTA at 1 mM) were injected intraperitoneally to wash the abdominal cavity. A 1 ml volumetric disposable syringe equipped with a 21G needle was used to recover the washings. After recovering the washings, the washings from the abdominal cavity

were immediately transferred to 1.5 ml volumetric Eppendorf tubes followed by centrifuging at 4°C and 7,500 rpm to separate the supernatant. The supernatant was stored at -20°C until measurement of IL-1β.

The amounts of IL-1β were measured using an enzyme-linked immunosorbent assay (ELISA) kit (mouse ELISA Kit, Genzyme).

The test compound was suspended in 0.5% traganth solution and administered orally at the rate of 10 ml/kg body weight 30 minutes prior to injection of LPS. A minimum of three dose levels were administered to five mice each for each test compound. The average inhibition rates were calculated for each dose level versus a control group.

In this test, the compounds of the present invention demonstrated superior IL-1β production inhibitory action.

(Test Example 4)

Activity in Preventing the Development of Adjuvant-Induced Arthritis *in vivo*

The test was performed according to the method described by Winder et al. (Arthritis Rheum., 12, 472-482, 1969).

Heat-killed dried Mycobacterium butyricum (Difco Laboratories, Lot 679123) was ground on an agate mortar, and was then suspended in dry-sterilised liquid paraffin (first grade, Wako Pure Chemical Industries, Ltd.) to make a 2 mg/ml suspension. The resulting suspension was then sonicated and used as an adjuvant. Arthritis was induced by the intradermal injection of the adjuvant (100 μg of heat killed dried bacterium/0.05 ml of paraffin/paw) into the heel of the right hind paw of a Lewis rat (male, age 9 weeks, 190 g, Japan Charles River). The rats were assigned to groups of 5 animals each.

The test compounds, which had been suspended in a 0.5% aqueous sodium carboxymethyl cellulose solution, were administered orally at the rate of 5 ml/kg once a day from the day of injection of the adjuvant (day 0) to day 20.

The volumes of the right hind paw (adjuvant-injected paw) and left hind paw (non-injected paw) were measured on days 3, 5, 7, 10, 13, 15, 18 and 21 using a Plethysmometer™ (Ugo Basile), the hind paws being soaked from the toe to the hairline in the bath of the Plethysmometer™. The volumes of the swollen feet (adjuvant-injected right hind foot volume - non-injected left hind foot volume) were calculated. The percent inhibition of swelling of the injected foot of the treated animals as compared to that of the control animals on day 21 was calculated as follows.

Inhibition (%)

={1-(swollen foot volume of compound-treated animals)/

(swollen foot volume of control animals)} × 100

Those results are shown in Table 24 below.

Table 7

| Activity in Preventing the Development of Adjuvant-Induced Arthritis *in vivo* | |
|---|---|
| Test Compound | Inhibition rate (%) (dose: 10 mg/kg) |
| Compound of Example 2 | 84 |
| Compound of Example 7 | 81 |

As can be seen from Table 10 above, in this test, the compounds of the present invention showed excellent activity in preventing the development of adjuvant-induced arthritis.

(Test Example 5)

Activity in Preventing the Development of Arthritis Induced by Anti-Collagen Antibody *in vivo*

In this test, an anti-collagen antibody-induced mouse arthritis model was employed.

0.5 ml of an anti-collagen antibody solution (4 mg/ml, Arthritogenic mAb Cocktail: product of Immuno-Biological Laboratories Co., Ltd) was injected into the caudal vein of Balb/c mice (males, age 5-6 weeks old, Japan Charles River). Three days after injection, 0.1 ml [0.05 mg of lipopolysaccharide] of a lipopolysaccharide solution (0.5 mg/ml, Arthritogenic mAb Cocktail: product of Immuno-Biological Laboratories Co., Ltd) was administered to the mice by intraperitoneal injection.

The test compounds, which had been suspended in 0.5% tragacanth, were administered orally to the test animals at a rate of 10 ml/1 kg of body weight once per day for 7 days from the day when the anti-collagen antibody was administered. To the mice of the control group, 0.5% tragacanth solution was administered at the rate of 10 ml/kg of body weight once per day for 7 days from the day when the anti-collagen antibody was administered, instead of solutions of the test compounds.

After the administration of the test compounds (or 0.5% tragacanth solution), the degree of edema in the 4 paws of each test mouse was scored according to the following basis:

0: normal (edema is not observed);
1: edema is observed in one of the five toes;
2: edema is observed in two or more of the five toes;
3: the whole of the paw is swollen.

The degree of arthritis in the test mouse was evaluated by the total of the edema scores in the 4 paws. The rate of suppression was calculated from the degrees of arthritis of the control animals and of the animals treated with the test compounds. From the rates of suppression and the dosages, $ID_{50}$ values were calculated by the least squares method.

In this test, the compounds of the present invention showed excellent activity in preventing the development of arthritis induced by anti-collagen antibody.

(Test Example 6)

Activity in Treating Arthritis Induced by Anti-Collagen Antibody *in vivo*

In this test, an anti-collagen antibody-induced mouse arthritis model was employed.

0.5 ml of an anti-collagen antibody solution (4 mg/ml, Arthritogenic mAb Cocktail: product of Immune-Biological Laboratories Co., Ltd) was injected into the caudal vein of Balb/c mice (males, age 5-6 weeks old, Japan Charles River). Three days after injection, 0.1 ml of a lipopolysaccharide solution (0.5 mg/ml, Arthritogenic mAb Cocktail: product of Immuno-Biological Laboratories Co., Ltd) was administered to the mice by intraperitoneal injection.

7 days after the administration of the anti-collagen antibody solution, the degree of edema in the 4 paws of each test mouse was scored according to the basis shown in Test Example 5 above. Those mice in which edema in both the hind paws had been scored as "3" were selected and used in a treatment test.

Test compounds, which had been suspended in 0.5% tragacanth solution, were administered orally to the selected mice at a rate of 10 ml/kg of body weight once per day for 3 days. To the mice of the control group, 0.5% tragacanth solution was administered at a rate of 10 ml/kg of body weight once per day for 3 days instead of solutions of the test compounds.

After the administration of the test compounds (or tragacanth solution), the degree of arthritis in each test mouse was evaluated in the same manner as described in Test Example 5. The rates of treatment of arthritis induced by anti-collagen antibody were calculated from the degrees of arthritis of the control animals and of the compound-treated animals.

In this test, the compounds of the present invention showed excellent activity in treating arthritis induced by anti-collagen antibody

(Test Example 7)

Diabetes Inhibition Test (in vivo)

Cyclophosphamide (250 mg/kg) was administered intraperitoneally to female NOD mice at age 9 weeks and 11 weeks to quickly induce diabetes. The test compound was mixed into the powdered feed at 0.03% (equivalent to a dose of the test compound of 20-40 g/kg/day) or 0.1% (equivalent to a dose of the test compound of 90-130 g/kg/day), and the test compound was administered by feeding the animals the feed for 4 weeks starting on the day of the first administration of cyclophosphamide. Blood samples were collected following completion of administration of the test compound, blood glucose levels were measured, and those mice having a blood glucose level of 250 mg/dl or more were considered to be diabetic.

Furthermore, simultaneous to this test, a similar test was conducted by feeding animals powdered feed not containing any test compound in the same manner, and this group of animals was designated as the control group.

In this test, compounds of the present invention inhibited the onset of diabetes.

(Test Example 8)

Inhibition Test of Concanavalin A-Induced Liver Disorder (Preventive Effect)

Using five Balb/c mice (males, age 6-8 weeks) per group, a physiological saline solution (1.5 mg/ml) of Concanavalin A (Con A) was injected at the rate of 10 ml/kg into the caudal vein to induce liver disorder in each mouse (F. Gantner et al., Hepatology, 21, 190-198 (1995)).

The test compound was suspended in distilled water containing 0.5% traganth powder to a concentration of 3 mg/ml, and this suspension was orally administered to the mice at the rate of 10 ml/kg 30 minutes before injection of Con A (Group A).

Separately from this procedure, a group orally administered with distilled water containing 0.5% traganth powder instead of the suspension of the test compound (Group B), and a group that was injected with physiological saline instead of Con A physiological saline solution, and administered distilled water containing 0.5% traganth powder instead of the suspension of the test compound (Group C) were also prepared.

Blood samples were collected from the abdominal vena cava of the mice 6 hours after injection of Con A (or physiological saline) followed by separation of the serum. This serum was measured for glutamate oxaloacetate transaminase (AST) and glutamate pyruvate transaminase (ALT) levels in accordance with the method of Nakagawa et al. (J. Nakagawa et al., J. Pharmacol. Exp. Ther., 264, 496-500 (1993)).

The inhibition rate for increases in AST and ALT levels induced by injection of Con A were calculated according to the formulae indicated below from the resulting measured values.

$$\text{Inhibition rate (\%)} = \{1 - [(A-C)/(B-C)]\} \times 100$$

A: Average value of AST or ALT level of Group A
B: Average value of AST or ALT level of Group B
C: Average value of AST or ALT level of Group C

In this test, compounds of the present invention inhibited increases in AST and ALT levels (namely, liver disease) induced by Con A.

(Test Example 9)

Inhibition Test on Concanavalin A-Induced Liver Disorder (Therapeutic Effect)

Liver disorder was induced in Balb/c mice (males, age 6-8 weeks) by injection of Con A in the same manner as Test Example 8.

The test compound was administered to the mice 30 minutes after injection of Con A (Group A). Moreover, Groups B and C were prepared in the same manner as Test Example 8. Blood samples were collected from each mouse, the serum was separated, and AST and ALT levels were measured. This test was carried out entirely in the same manner as Test Example 8 with the exception of the time at which the test compound (or distilled water containing 0.5% traganth powder) was administered.

The inhibition rate for increases in AST and ALT levels induced by injection of Con A were calculated according to the formulae shown in Test Example 8 from the resulting measured values.

In this test, compounds of the present invention inhibited increases in AST and ALT levels (namely, liver disorder) induced by Con A even when administered following injection of Con A (namely, following treatment to induce liver disorder).

(Test Example 10)

Inhibition Test of Galactosamine/Lipopolysaccharide-Induced Liver Disorder (Preventive Effect)

Using 10 C57BL/6 mice (females, age 7 weeks) per group, a physiological saline solution containing 60 mg/ml of D-galactosamine (GalN) and 1 μg/ml of lipopolysaccharide (LPS) was injected into the abdominal cavity at the rate of 10 ml/kg to induce liver disorder in each mouse (M. Niehorster et al., Biochem. Pharmacol., 40, 1601-1603 (1990)).

The test compound was suspended in physiological saline containing 0.5% carboxymethyl cellulose sodium salt (CMC) powder to a concentration of 1 mg/ml, and this suspension was then orally administered to the mice at the

rate of 10 ml/kg 30 minutes before injection of GalN/LPS (Group A).

Separate from this procedure, a group administered physiological saline containing 0.5% CMC powder instead of the suspension of the test compound (Group B), and a group that was injected with physiological saline instead of physiological saline containing GalN/LPS, and administered physiological saline containing 0.5% CMC powder instead of the suspension of the test compound (Group C) were also prepared.

Blood samples were collected from the abdominal vena cava of the mice 6 hours after injection of GalN/LPS (or physiological saline) followed by separation of the plasma. This plasma was then measured for glutamate oxaloacetate transaminase (AST) and glutamate pyruvate transaminase (ALT) levels in the same manner as Test Example 8.

The inhibition rate for increases in AST and ALT levels was calculated in accordance with the formulae indicated in Test Example 8 from the resulting measured values.

Compounds of the present invention inhibited increases in AST and ALT levels (namely, liver disorder) induced by galactosamine and lipopolysaccharide, and demonstrated superior liver disease inhibitory effects in this test.

(Test Example 11)

Recovery Test on Galactosamine (GalN)-Lipopolysaccharide (LPS)-Induced Moribund Mice (Septicemia Model)

Septicaemia was induced by injecting GalN and LPS into the caudal veins of mice.

The GalN solution (Sigma) was prepared to a concentration of 1 g/5 ml using physiological saline, and the LPS solution (Sigma) was prepared to a concentration of 0.05 mg/5 ml using physiological saline, and these two types of solutions were mixed in equal amounts. The mixed solution was injected into the caudal veins of C3H/He.N mice (males, age 7 weeks, Japan Charles River, 10 mice per group) at the rate of 10 ml/kg to induce a moribund state caused by septicaemia.

The test compound was dissolved in physiological saline and injected into the caudal vein at the rate of 10 ml/kg immediately before injection of the GalN-LPS solution.

The mice were observed and the morbidity recorded for 3 days from induction of the moribund state to evaluate recovery effects.

Compounds of the present invention demonstrated superior recovery effects against septicaemia in this test.

[Industrial Applicability]

Since the compounds of the present invention have a superior action that inhibits the production of inflammatory cytokines (and particularly the production of IL-1$\beta$ and TNF$\alpha$), have satisfactory pharmacokinetics and oral absorption, and have superior safety, they are useful as pharmaceuticals for warm-blooded animals (and particularly humans), examples of which include analgesics, anti-inflammatory drugs, antiviral agents, as well as preventive or therapeutic agents for rheumatoid arthritis, osteoarthritis, allergic diseases, asthma, septicaemia, psoriasis, osteoporosis, autoimmune diseases (such as systemic lupus erythematosus, ulcerative colitis and Crohn's disease), diabetes, nephritis, hepatitis, tumors, ischemic heart disease, Alzheimer's disease and arteriosclerosis; preferably analgesics/anti-inflammatory drugs as well as preventive or therapeutic agents for rheumatoid arthritis, osteoarthritis, allergic diseases, septicaemia, psoriasis, osteoporosis, ulcerative colitis, diabetes, hepatitis, arteriosclerosis and Crohn's disease; and particularly preferably analgesics/anti-inflammatory drugs as well as preventive or therapeutic agents for articular rheumatism, osteoarthritis, septicaemia, psoriasis, Crohn's disease, ulcerative colitis, diabetes and hepatitis.

## Claims

1. A compound of the following formula (1):

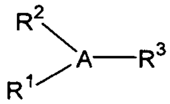

(I)

{wherein
A represents a trivalent group selected from pyrrole and pyrazole,
$R^1$ represents an aryl group; an aryl group substituted by group(s) selected from substituent group $\alpha$ and substituent group $\beta$; a heteroaryl group; or a heteroaryl group substituted by group(s) selected from substituent group $\alpha$ and

substituent group β,
$R^2$ represents a heteroaryl group having at least one nitrogen atom; or a heteroaryl group having at least one nitrogen atom substituted by group(s) selected from substituent group α and substituent group β and,
$R^3$ represents a group of the following formula (II):

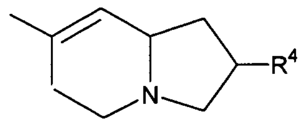

(II)

[wherein
$R^4$ represents a hydrogen atom, a hydroxyl group, a lower alkyl group, a lower alkoxy group, a group of the formula: $-NR^{\alpha}R^{\beta}$ (wherein $R^{\alpha}$ and $R^{\beta}$ are the same or different and each represents a hydrogen atom, a lower alkylsulfonyl group or a lower aliphatic acyl group), an aryl group, an aryl group substituted by group(s) selected from substituent group α and substituent group β, an aryl group substituted by an aryl group or an aryl group substituted by an aryloxy group,
substituent group α represents the group consisting of a hydroxyl group, a nitro group, a cyano group, a halogen atom, a lower alkoxy group, a halogeno lower alkoxy group, a lower acyloxy group, a lower alkylthio group, a halogeno lower alkylthio group and a group of formula: $-NR^aR^b$ (wherein $R^a$ and $R^b$ are the same or different and each represents a hydrogen atom, a lower alkyl group, a lower alkenyl group, a lower alkynyl group, an aralkyl group, a lower alkylsulfonyl group or a lower aliphatic acyl group, or $R^a$ and $R^b$, together with the nitrogen atom to which $R^a$ and $R^b$ are bonded, form a heterocyclyl group),
substituent group β represents the group consisting of a lower alkyl group, a lower alkenyl group, a lower alkynyl group, an aralkyl group, a cycloalkyl group, a lower alkyl group substituted by group(s) selected from substituent group α, a lower alkenyl group substituted by group(s) selected from substituent group α and an alkynyl group substituted by group(s) selected from substituent group α],
provided that the atoms on ring A to which $R^1$ and $R^3$ are bonded are each adjacent to the atom on ring A to which $R^2$ is bonded}
or a pharmacologically acceptable salt thereof, a pharmacologically acceptable ester thereof or other pharmacologically acceptable derivative thereof.

**2.** A compound according to Claim 1, wherein $R^1$ is an aryl group; or an aryl group substituted by group(s) selected from substituent group α and substituent group β, or a pharmacologically acceptable salt thereof, a pharmacologically acceptable ester thereof or other pharmacologically acceptable derivative thereof.

**3.** A compound according to Claim 1, wherein $R^1$ is phenyl, naphthyl, phenyl or naphthyl substituted by group(s) selected from substituent group α and substituent group β, or a pharmacologically acceptable salt thereof, a pharmacologically acceptable ester thereof or other pharmacologically acceptable derivative thereof.

**4.** A compound according to Claim 1, wherein $R^1$ is phenyl or phenyl substituted by group(s) selected from substituent group $\alpha^1$ and substituent group $\beta^1$,
substituent group $\alpha^1$ represents the group consisting of a cyano group, a halogen atom, a lower alkoxy group, a halogeno lower alkoxy group and a group of formula: $-NR^aR^b$ (wherein one of $R^a$ and $R^b$ represents a hydrogen atom or a lower alkyl group, and the other represents a hydrogen atom, a lower alkyl group or an aralkyl group),
substituent group $\beta^1$ represents the group consisting of a lower alkyl group, a halogeno lower alkyl group, a hydroxy - lower alkyl group, a nitro - lower alkyl group, an amino - lower alkyl group, a lower alkylamino - lower alkyl group, a di(lower alkyl)amino - lower alkyl group and an aralkylamino - lower alkyl group, or a pharmacologically acceptable salt thereof, a pharmacologically acceptable ester thereof or other pharmacologically acceptable derivative thereof.

**5.** A compound according to Claim 1, wherein $R^1$ is phenyl or phenyl substituted by group(s) selected from the substituent group consisting of a cyano group, a halogen atom, a lower alkoxy group, a halogeno lower alkyl group and a halogeno lower alkoxy group, or a pharmacologically acceptable salt thereof, a pharmacologically acceptable ester thereof or other pharmacologically acceptable derivative thereof.

**6.** A compound according to Claim 1, wherein $R^1$ is phenyl, 4-fluorophenyl, 3-fluorophenyl, 4-chlorophenyl, 3-chlorophenyl, 2,4-difluorophenyl, 3,4-difluorophenyl, 3,4-dichlorophenyl, 3,4,5-trifluorophenyl, 3-chloro-4-fluorophenyl, 3-difluoromethoxyphenyl, 3-trifluoromethoxyphenyl, 3-trifluoromethylphenyl, 3-methoxyphenyl, 3-cyanophenyl or 4-fluoro-3-methoxyphenyl, or a pharmacologically acceptable salt thereof, a pharmacologically acceptable ester thereof or other pharmacologically acceptable derivative thereof,

**7.** A compound according to Claim 1, wherein $R^1$ is phenyl, 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl, 3-trifluoromethylphenyl, 3-methoxyphenyl, 3-cyanophenyl or 4-fluoro-3-methoxyphenyl, or a pharmacologically acceptable salt thereof, a pharmacologically acceptable ester thereof or other pharmacologically acceptable derivative thereof.

**8.** A compound according to Claim 1, wherein $R^1$ is phenyl, 4-fluorophenyl, 3-fluorophenyl, 3-chlorophenyl, 3,4-difluorophenyl, 3-chloro-4-fluorophenyl or 3-trifluoromethylphenyl, or a pharmacologically acceptable salt thereof, a pharmacologically acceptable ester thereof or other pharmacologically acceptable derivative thereof.

**9.** A compound according to any one of Claims 1 to 8, wherein $R^2$ is a 5- or 6-membered heteroaryl group containing 1 or 2 nitrogen atoms; or a 5- or 6-membered heteroaryl group containing 1 or 2 nitrogen atoms substituted by group(s) selected from substituent group $\alpha$ and substituent group $\beta$, or a pharmacologically acceptable salt thereof, a pharmacologically acceptable ester thereof or other pharmacologically acceptable derivative thereof.

**10.** A compound according to any one of Claims 1 to 8, wherein $R^2$ is pyridyl, pyrimidinyl, or pyridyl or pyrimidinyl substituted by group(s) selected from substituent group $\alpha$ and substituent group $\beta$, or a pharmacologically acceptable salt thereof, a pharmacologically acceptable ester thereof or other pharmacologically acceptable derivative thereof.

**11.** A compound according to any one of Claims 1 to 8, wherein $R^2$ is 4-pyridyl, 4-pyrimidinyl, or 4-pyridyl or 4-pyrimidinyl substituted by group(s) selected from substituent group $\alpha$ and substituent group $\beta$, or a pharmacologically acceptable salt thereof, a pharmacologically acceptable ester thereof or other pharmacologically acceptable derivative thereof.

**12.** A compound according to any one of Claims 1 to 8, wherein $R^2$ is 4-pyridyl, 4-pyrimidinyl, or 4-pyridyl or 4-pyrimidinyl wherein the 2-position is substituted by one group selected from substituent group $\alpha$ and substituent group $\beta$, or a pharmacologically acceptable salt thereof, a pharmacologically acceptable ester thereof or other pharmacologically acceptable derivative thereof.

**13.** A compound according to any one of Claims 1 to 8, wherein $R^2$ is 4-pyridyl, 4-pyrimidinyl, or 4-pyridyl or 4-pyrimidinyl wherein the 2-position is substituted by one group selected from the substituent group consisting of methoxy, amino, methylamino, benzylamino and $\alpha$-methylbenzylamino, or a pharmacologically acceptable salt thereof, a pharmacologically acceptable ester thereof or other pharmacologically acceptable derivative thereof.

**14.** A compound according to any one of Claims 1 to 13, wherein $R^4$ is a group independently selected from a hydrogen atom, a lower alkyl group, a group of formula: $-NR^{\alpha}R^{\beta}$ (wherein $R^{\alpha}$ and $R^{\beta}$ are the same or different and each represents a hydrogen atom, a lower alkylsulfonyl group or a lower aliphatic acyl group), an aryl group and an aryl group substituted by group(s) selected from substituent group $\alpha$ and substituent group $\beta$, or a pharmacologically acceptable salt thereof, a pharmacologically acceptable ester thereof or other pharmacologically acceptable derivative thereof.

**15.** A compound according to any one of Claims 1 to 13, wherein $R^4$ is a group independently selected from a hydrogen atom, a methyl group, an ethyl group, a propyl group, a butyl group, an amino group, a phenyl group and a phenyl group substituted by group(s) selected from substituent group $\alpha$ and substituent group $\beta$, or a pharmacologically acceptable salt thereof, a pharmacologically acceptable ester thereof or other pharmacologically acceptable derivative thereof.

**16.** A compound according to any one of Claims 1 to 13, wherein $R^4$ is a methyl group, an ethyl group, a propyl group, a butyl group, a phenyl group; or a phenyl group substituted by 1 or 2 substituent(s) selected from the group consisting of a hydroxyl group, a halogen atom, a lower acyloxy group, a lower alkoxy group, a halogeno lower alkoxy group, a lower alkyl group and a halogeno lower alkyl group, or a pharmacologically acceptable salt thereof, a pharmacologically acceptable ester thereof or other pharmacologically acceptable derivative thereof.

**17.** A compound according to any one of Claims 1 to 13, wherein $R^4$ is a methyl group, an ethyl group, a propyl group, a butyl group, a phenyl group, or a phenyl group substituted by 1 or 2 substituent(s) selected from the group

consisting of a hydroxyl group, a fluorine atom, a chlorine atom, a bromine atom, a formyloxy group, an acetoxy group, a propionyloxy group, a butyryloxy group, an isobutyryloxy group, a valeryloxy group, an isovaleryloxy group, a pivaloyloxy group, a methyl group, an ethyl group, a propyl group, a butyl group, a tert-butyl group, a difluoromethyl group, a trifluoromethyl group, a methoxy group, an ethoxy group, a propoxy group, a butoxy group, a tert-butoxy group, a difluoromethoxy group and a trifluoromethoxy group, or a pharmacologically acceptable salt thereof, a pharmacologically acceptable ester thereof or other pharmacologically acceptable derivative thereof.

**18.** A compound according to any one of Claims 1 to 13, wherein $R^4$ is a methyl group, an ethyl group, a propyl group, a butyl group, a phenyl group; or a phenyl group substituted by 1 or 2 substituent(s) selected from the group consisting of a hydroxyl group, a fluorine atom, a chlorine atom, an acetyloxy group, a methyl group, an ethyl group, a methoxy group and an ethoxy group, or a pharmacologically acceptable salt thereof, a pharmacologically acceptable ester thereof or other pharmacologically acceptable derivative thereof.

**19.** A compound according to any one of Claims 1 to 18, wherein A is pyrrole, or a pharmacologically acceptable salt thereof, a pharmacologically acceptable ester thereof or other pharmacologically acceptable derivative thereof.

**20.** A compound according to any one of Claims 1 to 19, wherein the compound of formula (I) is a compound represented by any one of the following formulae:

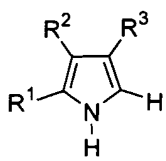

or

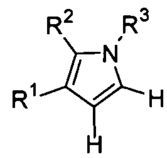

or a pharmacologically acceptable salt thereof, a pharmacologically acceptable ester thereof or other pharmacologically acceptable derivative thereof.

**21.** A compound according to any one of Claims 1 to 19, wherein the compound of formula (I) is a compound represented by the following formula:

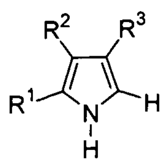

or a pharmacologically acceptable salt thereof, a pharmacologically acceptable ester thereof or other pharmacologically acceptable derivative thereof.

**22.** A compound according to any one of Claims 1 to 18, wherein A is pyrazole, or a pharmacologically acceptable salt thereof, a pharmacologically acceptable ester thereof or other pharmacologically acceptable derivative thereof.

**23.** A compound according to any one of Claims 1 to 18 and 22, wherein the compound of formula (I) is a compound represented by any one of the following formulae:

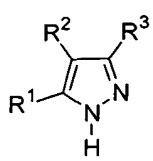

or

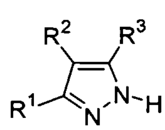

or a pharmacologically acceptable salt thereof, a pharmacologically acceptable ester thereof or other pharmacologically acceptable derivative thereof.

**24.** A compound according to any one of Claims 1 to 18 and 22, wherein the compound of formula (I) is a compound represented by the following formula:

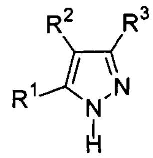

or a pharmacologically acceptable salt thereof, a pharmacologically acceptable ester thereof or other pharmacologically acceptable derivative thereof.

**25.** A compound according to Claim 1, wherein the compound of formula (I) is a compound represented by the following formula:

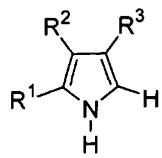

wherein $R^1$ is a phenyl group, a halogeno phenyl group or a halogeno lower alkylphenyl group,
$R^2$ is a pyridyl group,
$R^3$ is a group of the formula:

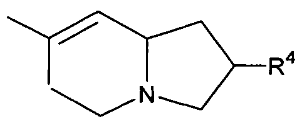

(wherein $R^4$ is a phenyl group or an alkyl group having from 1 to 4 carbon atoms), or a pharmacologically acceptable salt thereof, a pharmacologically acceptable ester thereof or other pharmacologically acceptable derivative thereof.

**26.** A compound according to Claim 25, wherein $R^1$ is a phenyl group, a fluorophenyl group, a chlorophenyl group, a difluorophenyl group, a chlorofluorophenyl group or a trifluoromethylphenyl group, or a pharmacologically acceptable salt thereof, a pharmacologically acceptable ester thereof or other pharmacologically acceptable derivative thereof.

**27.** A compound according to Claim 25, wherein $R^1$ is a fluorophenyl group and $R^3$ is a group of the following formula:

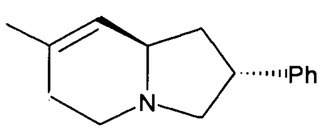

or a pharmacologically acceptable salt thereof, a pharmacologically acceptable ester thereof or other pharmacologically acceptable derivative thereof.

**28.** A compound according to Claim 25, wherein $R^1$ is a chlorofluorophenyl group and $R^3$ is a group of the following formula:

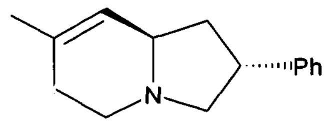

or a pharmacologically acceptable salt thereof, a pharmacologically acceptable ester thereof or other pharmacologically acceptable derivative thereof.

**29.** A compound according to Claim 1, wherein the compound of formula (I) is a compound represented by the following formula:

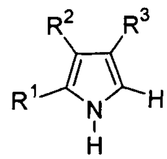

wherein $R^1$ is a 4-halogeno phenyl group or a halogeno lower alkylphenyl group,
$R^2$ is a pyridyl group,
$R^3$ is a group of the following formula:

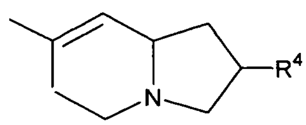

(wherein $R^4$ is a halogeno phenyl group, a 4-methylphenyl group or a 4-methoxyphenyl group), or a pharmacologically acceptable salt thereof, a pharmacologically acceptable ester thereof or other pharmacologically acceptable derivative thereof.

**30.** A compound according to Claim 29, wherein $R^1$ is a 4-fluorophenyl group or a trifluoromethylphenyl group and $R^4$ is a fluorophenyl group, a 4-methylphenyl group or a methoxyphenyl group, or a pharmacologically acceptable salt thereof, a pharmacologically acceptable ester thereof or other pharmacologically acceptable derivative thereof.

**31.** A compound according to Claim 1, wherein the compound is selected from the following compounds:

- 4-[(2S,8aS)-2-butyl-1,2,3,5,6,8a-hexahydroindolizin-7-yl]-2-(4-fluorophenyl)-3-(pyridin-4-yl)-1H-pyrrole,
- 2-(4-fluorophenyl)-4-[(2S,8aS)-2-phenyl-1,2,3,5,6,8a-hexahydroindolizin-7-yl]-3-(pyridin-4-yl)-1H-pyrrole,
- 2-(4-fluorophenyl)-4-[(2R,8aS)-2-(4-fluorophenyl)-1,2,3,5,6,8a-hexahydroindolizin-7-yl]-3-(pyridin-4-yl)-1H-pyrrole,
- 2-(3,4-difluorophenyl)-4-[(2S,8aS)-2-phenyl-1,2,3,5,6,8a-hexahydroindolizin-7-yl]-3-(pyridin-4-yl)-1H-pyrrole,
- 2-(4-fluorophenyl)-4-[(2S,8aS)-2-(4-methylphenyl)-1,2,3,5,6,8a-hexahydroindolizin-7-yl]-3-(pyridin-4-yl)-1H-pyrrole,
- 2-(3-chlorophenyl)-4-[(2S,8aS)-2-phenyl-1,2,3,5,6,8a-hexahydroindolizin-7-yl]-3-(pyridin-4-yl)-1H-pyrrole,
- 4-[(2S,8aS)-2-(4-methylphenyl)-1,2,3,5,6,8a-hexahydroindolizin-7-yl]-3-(pyridin-4-yl)-2-(3-trifluoromethylphenyl)-1H-pyrrole,

- 2-(4-fluorophenyl)-4-[(2S,8aS)-2-(4-methoxyphenyl)-1,2,3,5,6,8a-hexahydroindolizin-7-yl]-3-(pyridin-4-yl)-1H-pyrrole,

- 4-[(2S,8aS)-2-phenyl-1,2,3,5,6,8a-hexahydroindolizin-7-yl]-3-(pyridin-4-yl)-2-(3-trifluoromethylphenyl)-1H-pyrrole,

- 2-phenyl-4-[(2S,8aS)-2-phenyl-1,2,3,5,6,8a-hexahydroindolizin-7-yl]-3-(pyridin-4-yl)-1H-pyrrole,

- 4-[(2S,8aS)-2-(4-ethylphenyl)-1,2,3,5,6,8a-hexahydroindolizin-7-yl]-2-(4-fluorophenyl)-3-(pyridin-4-yl)-1H-pyrrole,

- 4-[(2S,8aS)-2-(4-ethylphenyl)-1,2,3,5,6,8a-hexahydroindolizin-7-yl]-2-(3,4-difluorophenyl)-3-(pyridin-4-yl)-1H-pyrrole and

- 2-(3-chloro-4-fluorophenyl)-4-[(2S,8aS)-2-phenyl-1,2,3,5,6,8a-hexahydroindolizin-7-yl]-3-(pyridin-4-yl)-1H-pyrrole,

or a pharmacologically acceptable salt thereof, a pharmacologically acceptable ester thereof or other pharmacologically acceptable derivative thereof.

**32.** The compound according to Claim 1, wherein the compound of formula (I) is a compound represented by any one of the following formulae:

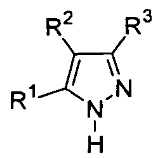

or

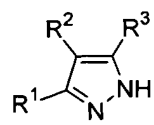

wherein $R^1$ is a phenyl group or a halogeno phenyl group,
$R^2$ is a pyridyl group and
$R^3$ is a group of the following formula:

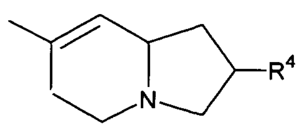

(wherein $R^4$ is a hydrogen atom, a lower alkyl group, a phenyl group, a lower alkylphenyl group or a lower alkoxyphenyl group), or a pharmacologically acceptable salt thereof, a pharmacologically acceptable ester thereof or other pharmacologically acceptable derivative thereof.

**33.** A compound according to Claim 32, wherein $R^1$ is a phenyl group, a fluorophenyl group or a chlorofluorophenyl group, and
$R^4$ is a hydrogen atom, an alkyl group having from 1 to 4 carbon atoms, a phenyl group, an alkylphenyl group having from 1 to 3 carbon atoms or an alkoxyphenyl group having from 1 to 3 carbon atoms,
or a pharmacologically acceptable salt thereof, a pharmacologically acceptable ester thereof or other pharmacologically acceptable derivative thereof.

**34.** A compound according to Claim 1, which is selected from the following:

- 3-[(2S,8aS)-2-butyl-1,2,3,5,6,8a-hexahydroindolizin-7-yl]-5-(4-fluorophenyl)-4-(pyridin-4-yl)pyrazole,

- 5-(4-fluorophenyl)-3-[(2S,8aS)-2-propyl-1,2,3,5,6,8a-hexahydroindolizin-7-yl]-4-(pyridin-4-yl)pyrazole,

- 5-(4-fluorophenyl)-3-[(2S,8aS)-2-phenyl-1,2,3,5,6,8a-hexahydroindolizin-7-yl]-4-(pyridin-4-yl)pyrazole,

- 5-(4-fluorophenyl)-3-[(2S,8aS)-2-(4-methylphenyl)-1,2,3,5,6,8a-hexahydroindolizin-7-yl]-4-(pyridin-4-yl)pyrazole and

- 5-(3-chloro-4-fluorophenyl)-3-[(2S,8aS)-2-phenyl-1,2,3,5,6,8a-hexahydroindolizin-7-yl]-4-(pyridin-4-yl)pyrazole,

or a pharmacologically acceptable salt thereof, a pharmacologically acceptable ester thereof or other pharmacologically acceptable derivative thereof.

**35.** A compound according to Claim 1, wherein the compound of formula (I) is a compound represented by the following formula:

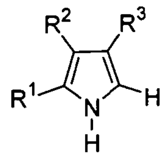

wherein $R^1$ is a lower alkoxyphenyl group or a lower alkoxy (halogeno) phenyl group,
$R^2$ is a pyridyl group, and
$R^3$ is a group of the following formula:

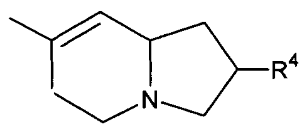

(wherein $R^4$ is a phenyl group), or a pharmacologically acceptable salt thereof, a pharmacologically acceptable ester thereof or other pharmacologically acceptable derivative thereof.

**36.** A compound according to Claim 35, wherein $R^1$ is a methoxyphenyl group or a fluoro(methoxy)phenyl group, or a pharmacologically acceptable salt thereof, a pharmacologically acceptable ester thereof or other pharmacologically acceptable derivative thereof.

**37.** A compound according to Claim 1, wherein the compound of formula (I) is a compound represented by the formula:

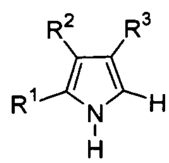

wherein $R^1$ is a 4-halogeno phenyl group,
$R^2$ is a pyridyl group, and
$R^3$ is a group of the following formula:

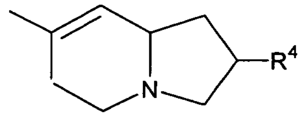

[wherein $R^4$ is a phenyl group substituted by a lower alkyl group other than a 4-methyl group, a phenyl group substituted by a lower alkoxy group other than a 4-methoxy group, a hydroxyphenyl group or a lower acyloxyphenyl group], or a pharmacologically acceptable salt thereof, a pharmacologically acceptable ester thereof or other pharmacologically acceptable derivative thereof.

**38.** A compound according to Claim 39, wherein $R^1$ is a 4-fluorophenyl group, and $R^4$ is a 3-methylphenyl group, an ethylphenyl group, a butylphenyl group, a 3-methoxyphenyl group, an ethoxyphenyl group, a hydroxyphenyl group or an acetoxy group, or a pharmacologically acceptable salt thereof, a pharmacologically acceptable ester thereof or other pharmacologically acceptable derivative thereof.

**39.** A compound according to Claim 1, wherein the compound of formula (I) is a compound represented by the formula:

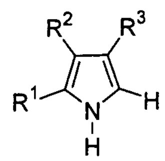

wherein $R^1$ is a phenyl group substituted by the halogen atom other than a 4-halogeno phenyl group,
$R^2$ is a pyridyl group, and
$R^3$ is a group of the following formula:

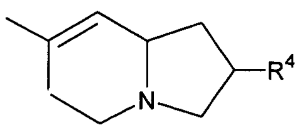

(wherein $R^4$ is a lower alkylphenyl group, a lower alkoxyphenyl group or a hydroxyphenyl group), or a pharmacologically acceptable salt thereof, a pharmacologically acceptable ester thereof or other pharmacologically acceptable derivative thereof,

**40.** A compound according to Claim 39, wherein $R^1$ is a chlorophenyl group or a difluorophenyl group and $R^4$ is a methylphenyl group, an ethylphenyl group, a propylphenyl group, an isopropylphenyl group, a butylphenyl group, a methoxyphenyl group, an ethoxyphenyl group, a propoxyphenyl group or a hydroxyphenyl group, or a pharmacologically acceptable salt thereof, a pharmacologically acceptable ester thereof or other pharmacologically acceptable derivatives thereof.

**41.** The compound according to Claim 1, which is selected from the following:

- 2-(4-fluorophenyl)-4-[(2S,8aS)-2-(3-methylphenyl)-1,2,3,5,6,8a-hexahydroindolizin-7-yl]-3-(pyridin-4-yl)-1H-pyrrole,
- 2-(3,4-difluorophenyl)-4-[(2S,8aS)-2-(4-methylphenyl)-1,2,3,5,6,8a-hexahydroindolizin-7-yl]-3-(pyridin-4-yl)-1H-pyrrole,
- 2-(3-chlorophenyl)-4-[(2S,8aS)-2-(4-methylphenyl)-1,2,3,5,6,8a-hexahydroindolizin-7-yl]-3-(pyridin-4-yl)-1H-pyrrole,
- 2-(3,4-difluorophenyl)-4-[(2S,8aS)-2-(4-methoxyphenyl)-1,2,3,5,6,8a-hexahydroindolizin-7-yl]-3-(pyridin-4-yl)-1H-pyrrole,
- 2-(4-fluorophenyl)-4-[(2S,8aS)-2-(3-methoxyphenyl)-1,2,3,5,6,8a-hexahydroindolizin-7-yl]-3-(pyridin-4-yl)-1H-pyrrole,
- 2-(3-chlorophenyl)-4-[(2S,8aS)-2-(4-methoxyphenyl)-1,2,3,5,6,8a-hexahydroindolizin-7-yl]-3-(pyridin-4-yl)-1H-pyrrole,
- 4-[(2S,8aS)-2-(4-ethoxyphenyl)-1,2,3,5,6,8a-hexahydroindolizin-7-yl]-2-(4-fluorophenyl)-3-(pyridin-4-yl)-1H-pyrrole,

- 2-(3,4-difluorophenyl)-4-[(2S,8aS)-2-(3-methoxyphenyl)-1,2,3,5,6,8a-hexahydroindolizin-7-yl]-3-(pyridin-4-yl)-1H-pyrrole,
- 2-(4-fluorophenyl)-4-[(2S,8aS)-2-(4-hydroxyphenyl)-1,2,3,5,6,8a-hexahydroindolizin-7-yl]-3-(pyridin-4-yl)-1H-pyrrole,
- 4-[(2S,8aS)-2-(4-acetoxyphenyl)-1,2,3,5,6,8a-hexahydroindolizin-7-yl]-2-(4-fluorophenyl)-3-(pyridin-4-yl)-1H-pyrrole,
- 2-(3,4-difluorophenyl)-4-[(2S,8aS)-2-(3-hydroxyphenyl)-1,2,3,5,6,8a-hexahydroindolizin-7-yl]-3-(pyridin-4-yl)-1H-pyrrole,
- 2-(4-fluoro-3-methoxyphenyl)-4-[(2S,8aS)-2-phenyl-1,2,3,5,6,8a-hexahydroindolizin-7-yl]-3-(pyridin-4-yl)-1H-pyrrole,
- 2-(3-methoxyphenyl)-4-[(2S,8aS)-2-phenyl-1,2,3,5,6,8a-hexahydroindolizin-7-yl]-3-(pyridin-4-yl)-1H-pyrrole),
- 4-[(2S,8aS)-2-(4-ethoxyphenyl)-1,2,3,5,6,8a-hexahydroindolizin-7-yl]-2-(3,4-difluorophenyl)-3-(pyridin-4-yl)-1H-pyrrole,
- 2-(4-fluorophenyl)-4-[(2S,8aS)-2-(3,4-dimethylphenyl)-1,2,3,5,6,8a-hexahydroindolizin-7-yl]-3-(pyridin-4-yl)-1H-pyrrole and
- 2-(3,4-difluorophenyl)-4-[(2S,8aS)-2-(3,4-dimethylphenyl)-1,2,3,5,6,8a-hexahydroindolizin-7-yl]-3-(pyridin-4-yl)-1H-pyrrole

or a pharmacologically acceptable salt thereof, a pharmacologically acceptable ester thereof or other pharmacologically acceptable derivative thereof.

**42.** A pharmaceutical composition comprising as an active ingredient a compound as defined in any one of Claims 1 to 41, or a pharmacologically acceptable salt thereof, a pharmacologically acceptable ester thereof or other pharmacologically acceptable derivative thereof.

**43.** A pharmaceutical composition according to Claim 42, used for inhibiting the production of inflammatory cytokines.

**44.** A pharmaceutical composition according to Claim 42, used as an antifebrile/analgesic, an anti-inflammatory agent or an anti-virus agent.

**45.** A pharmaceutical composition according to Claim 42, used for preventing or treating rheumatoid arthritis.

**46.** A pharmaceutical composition according to Claim 42, used for preventing or treating osteoarthritis.

**47.** A pharmaceutical composition according to Claim 42, used for preventing or treating septicaemia.

**48.** A pharmaceutical composition according to Claim 42, used for preventing or treating diabetes.

**49.** A pharmaceutical composition according to Claim 48, wherein the diabetes is I type diabetes.

**50.** A pharmaceutical composition according to Claim 42, used for preventing or treating psoriasis, Crohn's disease, ulcerative colitis or hepatitis.

**51.** A method for inhibiting the production of inflammatory cytokines, which comprises administering to a mammal an effective amount of a compound as defined in any one of Claims 1 to 41, or a pharmacologically acceptable salt thereof, a pharmacologically acceptable ester thereof or other pharmacologically acceptable derivative thereof.

**52.** The method according to Claim 51, wherein the mammal is a human being.

**53.** A method for treating or removing pain and/or inflammation which comprises administering to a mammal an effective amount of a compound as defined in any one of Claims 1 to 41, or a pharmacologically acceptable salt thereof, a pharmacologically acceptable ester thereof or other pharmacologically acceptable derivative thereof.

**54.** The method according to Claim 53, wherein the mammal is a human being.

**55.** A method for preventing or treating rheumatoid arthritis which comprises administering to a mammal an effective amount of a compound as defined in any one of Claims 1 to 41, or a pharmacologically acceptable salt thereof, a pharmacologically acceptable ester thereof or other pharmacologically acceptable derivative thereof.

**56.** The method according to Claim 55, wherein the mammal is a human being.

**57.** A method for preventing or treating osteoarthritis which comprises administering to a mammal an effective amount of a compound as defined in any one of Claims 1 to 41, or a pharmacologically acceptable salt thereof, a pharmacologically acceptable ester thereof or other pharmacologically acceptable derivative thereof.

**58.** The method according to Claim 57, wherein the mammal is a human being.

**59.** A method for preventing or treating septicaemia which comprises administering to a mammal an effective amount of a compound as defined in any one of Claims 1 to 41, or a pharmacologically acceptable salt thereof, a pharmacologically acceptable ester thereof or other pharmacologically acceptable derivative thereof.

**60.** The method according to Claim 59, wherein the mammal is a human being.

**61.** A method for preventing or treating diabetes which comprises administering to a mammal an effective amount of a compound as defined in any one of Claims 1 to 41, or a pharmacologically acceptable salt thereof, a pharmacologically acceptable ester thereof or other pharmacologically acceptable derivative thereof.

**62.** The method according to Claim 61, wherein the mammal is a human being.

**63.** The method according to Claim 61, wherein the diabetes is I type diabetes.

**64.** A method for preventing or treating psoriasis, Crohn's disease, ulcerative colitis or hepatitis which comprises administering to a mammal an effective amount of a compound as defined in any one of Claims 1 to 41, or a pharmacologically acceptable salt thereof, a pharmacologically acceptable ester thereof or other pharmacologically acceptable derivative thereof.

**65.** The method according to Claim 64, wherein the mammal is a human being.

## INTERNATIONAL SEARCH REPORT

International application No.
PCT/JP03/09110

**A. CLASSIFICATION OF SUBJECT MATTER**

Int.Cl[7] C07D471/04, A61K31/444, A61P1/00, 1/16, 3/10, 9/00, 9/10, 11/06, 13/12, 17/06, 19/02, 19/10, 25/04, 25/28, 29/00, 31/12, 35/00, 37/02, 37/08, 43/00

According to International Patent Classification (IPC) or to both national classification and IPC

**B. FIELDS SEARCHED**

Minimum documentation searched (classification system followed by classification symbols)

Int.Cl[7] C07D471/04, A61K31/444, A61P1/00, 1/16, 3/10, 9/00, 9/10, 11/06, 13/12, 17/06, 19/02, 19/10, 25/04, 25/28, 29/00, 31/12, 35/00, 37/02, 37/08, 43/00

Documentation searched other than minimum documentation to the extent that such documents are included in the fields searched

Electronic data base consulted during the international search (name of data base and, where practicable, search terms used)

CAPLUS(STN), CAOLD(STN), REGISTRY(STN), MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| X<br><br>Y | EP 1070711 A2 (SANKYO CO., LTD.), 24 January, 2001 (24.01.01), Pages 353 to 367<br>& EP 1070711 A3 & NO 2000003734 A<br>& EP 1243589 A1 & AU 2000048755 A<br>& BR 2000004534 A & CN 1295069 A<br>& JP 2001-247564 A | 1-21,25-31, 35-50<br>22-24,32-34 |
| Y | WO 00/31063 A1 (G.D. SEARLE & CO.), 02 June, 2000 (02.06.00), Pages 1209 to 1216<br>& US 6525059 B1 & US 6514977 B1<br>& EP 1144403 A1 & BR 9915420 A<br>& NO 2001002456 A & BG 105620 A<br>& US 6423713 B1 & JP 2002-530397 A | 22-24,32-34 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
13 August, 2003 (13.08.03)

Date of mailing of the international search report
26 August, 2003 (26.08.03)

Name and mailing address of the ISA/
Japanese Patent Office

Authorized officer

Facsimile No.

Telephone No.

Form PCT/ISA/210 (second sheet) (July 1998)

**INTERNATIONAL SEARCH REPORT**

International application No.
PCT/JP03/09110

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 03/15781 A1 (SANKYO CO., LTD.), 27 February, 2003 (27.02.03) & JP 2003-128551 A | 1-50 |
| P,X | WO 02/94267 A1 (SANKYO CO., LTD.), 28 November, 2002 (28.11.02) & JP 2003-040776 A | 1-50 |
| P,X | WO 02/80974 A1 (SANKYO CO., LTD.), 17 October, 2002 (17.10.02) & JP 2002-363104 A | 1-50 |
| P,X | JP 2002-284779 A (SANKYO CO., LTD.), 03 October, 2002 (03.10.02) (Family: none) | 1-50 |
| P,X | WO 02/57265 A1 (SANKYO CO., LTD.), 25 July, 2002 (25.07.02) & JP 2002-284782 A | 1-50 |
| P,X | WO 02/57264 A1 (SANKYO CO., LTD.), 25 July, 2002 (25.07.02) & JP 2002-284783 A | 1-50 |
| P,X | WO 02/57255 A1 (SANKYO CO., LTD.), 25 July, 2002 (25.07.02) & JP 2002-284780 A | 1-50 |
| P,X | WO 02/57254 A1 (SANKYO CO., LTD.), 25 July, 2002 (25.07.02) & JP 2002-284681 A | 1-50 |

Form PCT/ISA/210 (continuation of second sheet) (July 1998)

## INTERNATIONAL SEARCH REPORT

International application No.
PCT/JP03/09110

**Box I Observations where certain claims were found unsearchable (Continuation of item 2 of first sheet)**

This international search report has not been established in respect of certain claims under Article 17(2)(a) for the following reasons:

1. [X] Claims Nos.: 51-65
   because they relate to subject matter not required to be searched by this Authority, namely:
   Claims 51 to 65 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)